# EUROPEAN PATENT SPECIFICATION

(11) **EP 4 081 215 B1**
(45) Date of publication and mention of the grant of the patent: **20.05.2026**
(21) Application number: 20907145.5
(22) Date of filing: 26.12.2020
(51) Int. Cl.: A61K 31/436, A61K 31/47, C07D 215/12

(54) **METHODS AND COMPOSITIONS FOR INHIBITION OF DIHYDROOROTATE DEHYDROGENASE**
VERFAHREN UND ZUSAMMENSETZUNGEN ZUR HEMMUNG VON DIHYDROOROTATDEHYDROGENASE
MÉTHODES ET COMPOSITIONS POUR INHIBER LA DIHYDROOROTATE DÉSHYDROGÉNASE

(30) Priority: 26.12.2019 US 201962953708 P
(43) Date of publication of application: 02.11.2022
(73) Proprietor: Ohio State Innovation Foundation, Columbus, OH 43210 (US); Hendrix College, Conway AR 72032 (US)
(72) Inventor: BYRD, John C., Columbus, Ohio 43201 (US); BENNETT, Chad E., Columbus, Ohio 43201 (US); VIBHUTE, Sandip Madhukar, Columbus, Ohio 43202 (US); GOODWIN, Thomas E., Conway, Arkansas 72032 (US); HERTLEIN, Erin, Columbus, Ohio 43201 (US); ELGAMAL, Ola A., Columbus, Ohio 43201 (US); WILSON, Tyler Aron, Columbus, Ohio 43201 (US)
(74) Representative: Abel & Imray LLP
(86) International application number: PCT/US2020/067065
(87) International publication number: WO 2021/134042

(56) References cited:
- WO-A1-2019/246603
- US-A- 4 680 299
- MADAK JOSEPH T. ET AL: "Design, Synthesis, and Biological Evaluation of 4-Quinoline Carboxylic Acids as Inhibitors of Dihydroorotate Dehydrogenase", vol. 61, no. 12, 4 May 2018 (2018-05-04), US, pages 5162 - 5186, XP055790577, ISSN: 0022-2623, Retrieved from the Internet <URL:https://pubs.acs.org/doi/pdf/10.1021/acs.jmedchem.7b01862> DOI: 10.1021/acs.jmedchem.7b01862
- DATABASE PUBCHEM [online] 13 April 2018 (2018-04-13), Database accession no. 363456350
- DATABASE PUBCHEM [online] 23 June 2017 (2017-06-23), Database accession no. 340076842

## Description

### CROSS-REFERENCE TO RELATED APPLICATIONS

This Application claims the benefit of U.S. Provisional Application No. 62/953,708, filed on December 26, 2019.

### BACKGROUND

Proliferating cells require a supply of nucleotides for replication of DNA and transcription of genes to RNA, as well as for a variety of other metabolic processes. Cells can supply such nucleotides by de novo nucleotide synthesis pathways. An important step in the de novo synthesis pathway of pyrimidine nucleotides is the oxidation of dihydroorotate to form orotate. That reaction is catalyzed by dihydroorotate dehydrogenase (DHODH) and that step is one of the rate-limiting steps in the pyrimidine nucleotide synthesis pathway. DHODH has a sub-cellular location in the mitochondrial membrane and uses cytochrome C in the electron transport chain as an electron acceptor for the oxidation of dihydroorotate to orotate.

Under normal circumstances the intracellular pool of pyrimidine nucleotides can be replenished by a salvage pathway in which pyrimidine nucleotides are recycled. Although this DHODH-independent mechanism is sufficient for resting lymphocytes, 'activated' and proliferating lymphocytes need to substantially increase the available pyrimidine and so become dependent on de novo pyrimidine synthesis. Since orotate is a necessary intermediate in pyrimidine nucleotide synthesis, and since pyrimidine nucleotides are required for DNA replication, gene expression, and carbohydrate metabolism, inhibition of the DHODH enzyme can inhibit cell growth.

Moreover, rapidly proliferating cells require pyrimidines not only for cellular growth, but also for protein glycosylation, membrane lipid biosynthesis and strand break repair (e.g., see Fairbanks, et al., J. Biol. Chem. 270:29682-29689 (1995)). Under such conditions, in order to meet the increased demand, substantial quantities of pyrimidine nucleotides must be produced in rapidly proliferating cells. Accordingly, DHODH inhibitors are attractive candidates for treating proliferative disorders (e.g., see Liu, S., et al., Structure 8:25-31 (2000)), and various studies have shown that DHODH inhibitors can stop the proliferation of tumor cells in some circumstances (e.g., see Loffler, Eur. J. Biochem. 107:207-215 (1980)).

Other circumstances in which DHODH inhibitors have been identified as candidates for the clinical control of rapid cell division include activated immune cells, diseased skin cells, cancers, and infectious agents. Examples of DHODH inhibitors used or being developed for proliferative disorders include brequinar, leflunomide, and teriflunomide. Inhibitors of DHODH have further been disclosed for the treatment or prevention of autoimmune diseases, immune and inflammatory diseases, angioplastic-related disorders, viral, bacterial, and protozoic diseases.

Although DHODH is an attractive target for therapeutic intervention for a varieity of clinical conditions, including cancer, there remain significant issues with currently described compounds. For example, many of these compounds, including brequinar, suffer from being associated with poor bioavailability, due in part to the poor aqueous solubility and GI uptake. Accordingly, currently described DHODH inhibitors can have limited pharmaceutical efficacy due to such bioavailability issues.

Despite advances in research directed towards effective and therapeutically useful DHODH inhibitors, there remain a scarcity of compounds that are both efficacious and have the appropriate bioavailability properties. These needs and other needs are satisfied by the present disclosure.

### SUMMARY

In accordance with the purpose(s) of the disclosure, as embodied and broadly described herein, the disclosure, in one aspect, relates to compounds that are inhibitors of dihydroorotate dehydrogenase (DHODH), and the disclosed compounds have improved pharmacokinetic properties making them extremely useful for therapeutic intervention in a variety of disorders and diseases in which inhibition of DHODH can be clinically useful, e.g., cancer. In various aspects, the disclosed compounds are 6-substituted-2-([1,1'-biphenyl]-4-yl)quinoline-4-carboxylic acid analogs. In further aspects, the disclosed compounds can be used in methods of treating a cancer, such as a hematological cancer, including acute myeloid leukemia (AML), graft-versus-host-diseases, and disorders associated with T-cell proliferation. In some aspects, the disclosed compounds can demonstrate flip-flop kinetics when administered orally, i.e., pharmacokinetics in which the rate of absorption, rather than the rate of elimination, dominates the pharmacokinetics. Moreover, the disclosed compounds can demonstrate a sustained pharmacokinetic profile instead of an immediate release profile.

In accordance with a first aspect of the present invention, there is provided a compound, or a pharmaceutically acceptable salt thereof, according to claim1.

R¹ may be halogen or -SF₅. R¹ may be -F or -Cl. R¹ may be -F. R¹ may be -CI. R¹ may be -SF₅.

R^{6a} and R^{6b} may be independently selected from hydrogen, halogen, -SF₅, -CN, -N₃, -OH, -NH₂, -CHF₂, -CH₂F, and -CF₃.

R^{6a} and R^{6b} may be independently selected from halogen, -SF₅, -CN, -N₃, -OH, -NH₂, -CHF₂, -CH₂F, and -CF₃.

R^{6a} and R^{6b} may be independently selected from -F, -CI, -SF₅, -CN, -N₃, -OH, -NH₂, -CHF₂, -CH₂F, and -CF₃.

R^{6a} and R^{6c} may be independently selected from hydrogen, halogen, -SF₅, -CN, -N₃, -OH, -NH₂, -CHF₂, -CH₂F, and -CF₃.

R^{6a} and R^{6c} may be independently selected from halogen, -SF₅, -CN, -N₃, -OH, -NH₂, -CHF₂, -CH₂F, and -CF₃.

R^{6a} and R^{6c} may be independently selected from -F, -CI, -SF₅, -CN, -N₃, -OH, -NH₂, -CHF₂, -CH₂F, and -CF₃.

R^{6a} and R^{6d} may be independently selected from hydrogen, halogen, -SF₅, -CN, -N₃, -OH, -NH₂, -CHF₂, -CH₂F, and -CF₃.

R^{6a} and R^{6d} may be independently selected from halogen, -SF₅, -CN, -N₃, -OH, -NH₂, -CHF₂, -CH₂F, and -CF₃.

R^{6a} and R^{6d} may be independently selected from -F, -CI, -SF₅, -CN, -N₃, -OH, -NH₂, -CHF₂, -CH₂F, and -CF₃.

R^{6a} may be selected from -F, -CI, -SF₅, -CN, -N₃, -OH, and -NH₂.

R^{6a} may be selected from -F, -SF₅, -CN, -N₃, -OH, and -NH₂.

R^{6b} may be selected from -F, -CI, -SF₅, -CN, -N₃, -OH, and -NH₂.

R^{6a} may be selected from -F, -SF₅, -CN, -N₃, -OH, and -NH₂.

Each of R^{6c} and R^{6d} may be hydrogen.

R^{6a} may be selected from hydrogen, halogen, -SF₅, -CN, -N₃, -OH, -NH₂, C1-C3 alkyl, C1-C3 alkoxy, C1-C3 haloalkyl, C1-C3 aminoalkyl, and C1-C3 hydroxyalkyl; and wherein each of R^{6b}, R^{6c}, and R^{6d} is hydrogen, in which case R^{6a} may be selected from halogen, -SF₅, -CN, -N₃, -OH, -NH₂, -CHF₂, -CH₂F, and -CF₃, and R^{6a} may be selected from -F, -CI, -SF₅, -CN, -N₃, -OH, -NH₂, -CHF₂, -CH₂F, and -CF₃. And R^{6a} may be -F.

R^{6b} may be selected from hydrogen, halogen, -SF₅, -CN, -N₃, -OH, -NH₂, C1-C3 alkyl, C1-C3 alkoxy, C1-C3 haloalkyl, C1-C3 aminoalkyl, and C1-C3 hydroxyalkyl; and wherein each of R^{6a}, R^{6c}, and R^{6d} is hydrogen, in which case R^{6b} may be is selected from halogen, -SF₅, -CN, -N₃, -OH, -NH₂, -CHF₂, -CH₂F, and -CF₃, and R^{6b} may be selected from -F, -CI, -SF₅, -CN, -N₃, -OH, -NH₂, -CHF₂, -CH₂F, and -CF₃, and R^{6b} may be -F.

Each of R^{6a} and R^{6b} may be independently selected from halogen, -SF₅, -CN, -N₃, -OH, -NH₂, C1-C3 alkyl, C1-C3 alkoxy, C1-C3 haloalkyl, C1-C3 aminoalkyl, and C1-C3 hydroxyalkyl; and wherein each of R^{6c} and R^{6d} is hydrogen, in which case each of R^{6a} and R^{6b} may be independently selected from halogen, -SF₅, -CN, -N₃, -OH, -NH₂, -CHF₂, -CH₂F, and -CF₃, and each of R^{6a} and R^{6b} may be independently selected from -F, -CI, -SF₅, -CN, -N₃, -OH, -NH₂, -CHF₂, -CH₂F, and -CF₃, and each of R^{6a} and R^{6b} may be -F.

Each of R^{6a} and R^{6c} may be independently selected from halogen, -SF₅, -CN, -N₃, -OH, -NH₂, C1-C3 alkyl, C1-C3 alkoxy, C1-C3 haloalkyl, C1-C3 aminoalkyl, and C1-C3 hydroxyalkyl; and wherein each of R^{6b} and R^{6d} is hydrogen, in which case each of R^{6a} and R^{6c} may be independently selected from halogen, -SF₅, -CN, -N₃, -OH, -NH₂, -CHF₂, -CH₂F, and -CF₃, and each of R^{6a} and R^{6c} may be independently selected from -F, -CI, -SF₅, -CN, -N₃, -OH, -NH₂, -CHF₂, -CH₂F, and -CF₃, and each of R^{6a} and R^{6c} may be -F.

Each of R^{6a} and R^{6d} may be independently selected from halogen, -SF₅, -CN, -N₃, -OH, -NH₂, C1-C3 alkyl, C1-C3 alkoxy, C1-C3 haloalkyl, C1-C3 aminoalkyl, and C1-C3 hydroxyalkyl; and wherein each of R^{6b} and R^{6c} is hydrogen, in which case each of R^{6a} and R^{6d} may be independently selected from halogen, -SF₅, -CN, -N₃, -OH, -NH₂, -CHF₂, -CH₂F, and -CF₃, and each of R^{6a} and R^{6d} may be independently selected from -F, -CI, -SF₅, -CN, -N₃, -OH, -NH₂, -CHF₂, -CH₂F, and -CF₃, and each of R^{6a} and R^{6d} may be -F.

Each of R^{6b} and R^{6c} may be independently selected from halogen, -SF₅, -CN, -N₃, -OH, -NH₂, C1-C3 alkyl, C1-C3 alkoxy, C1-C3 haloalkyl, C1-C3 aminoalkyl, and C1-C3 hydroxyalkyl; and wherein each of R^{6a} and R^{6d} is hydrogen, in which case each of R^{6b} and R^{6c} may be independently selected from halogen, -SF₅, -CN, -N₃, -OH, -NH₂, -CHF₂, -CH₂F, and -CF₃, and each of R^{6b} and R^{6c} may be independently selected from -F, -CI, -SF₅, -CN, -N₃, -OH, -NH₂, -CHF₂, -CH₂F, and -CF₃, and each of R^{6b} and R^{6c} may be -F.

The compound may have a structure represented by a formula: 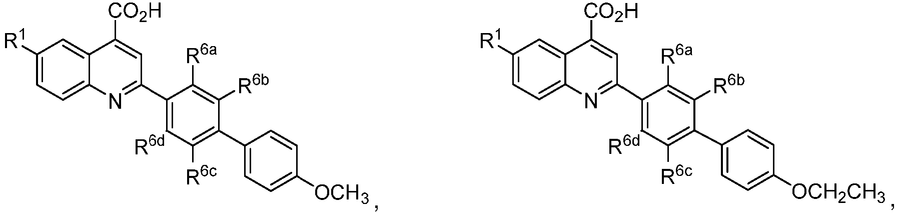 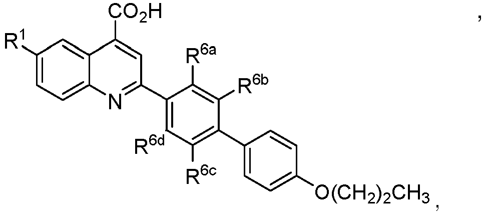 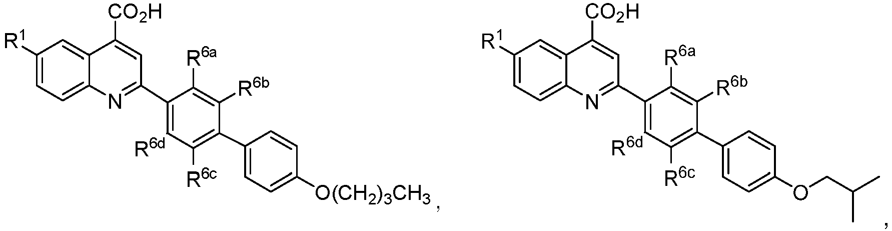 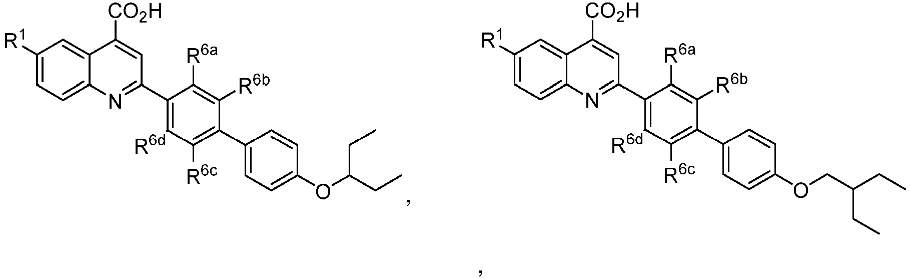 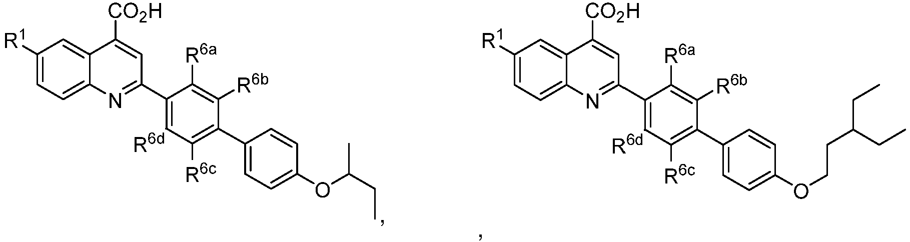 or combinations thereof.

The compound of may have a structure represented by a formula: 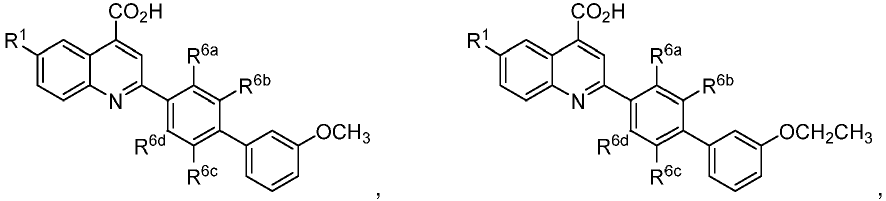 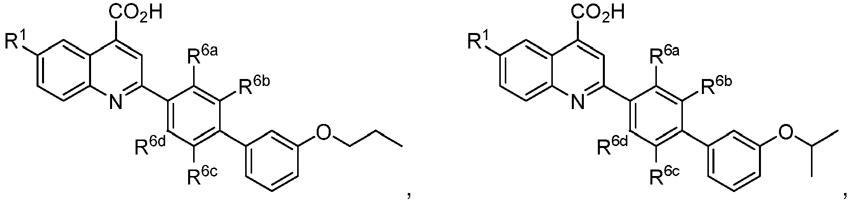 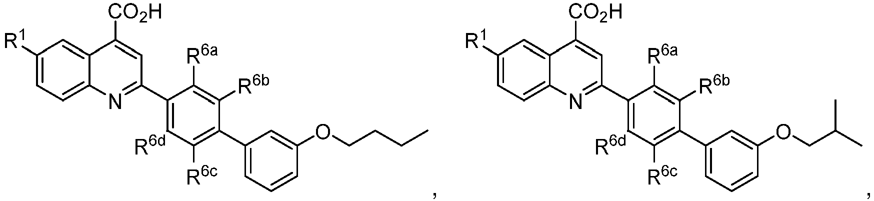 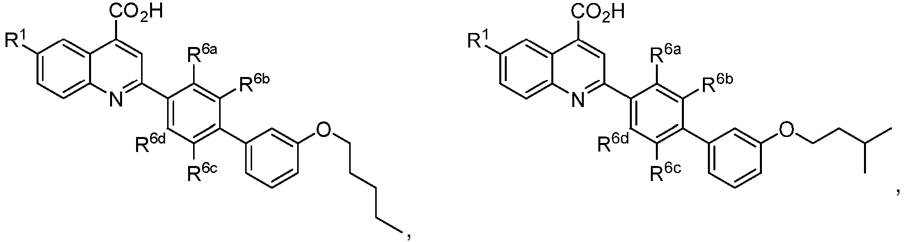 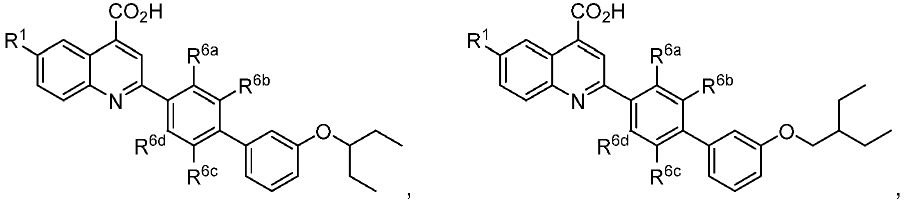 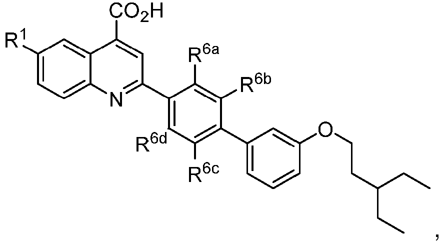 or combinations thereof.

The compound of may have a structure represented by a formula: 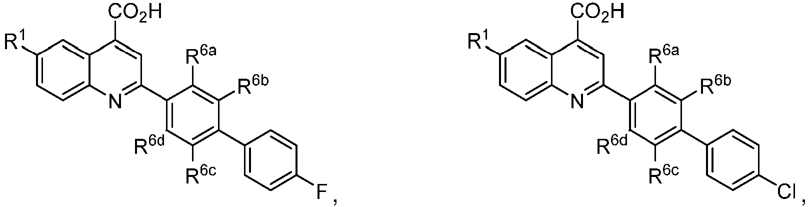 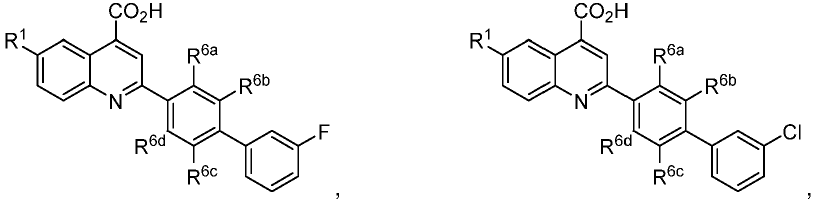 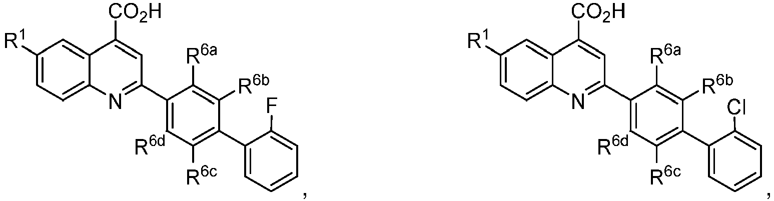 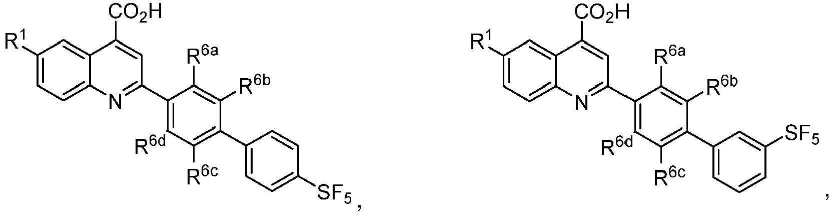 or combinations thereof.

The compound may have a structure represented by a formula: 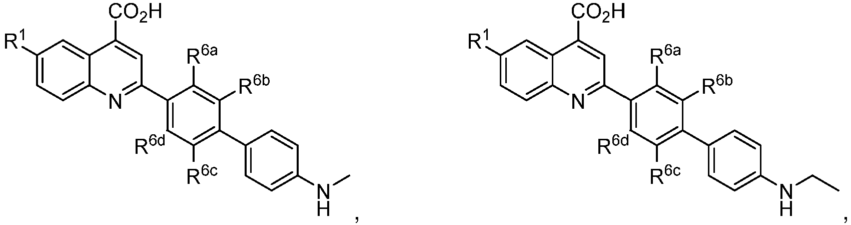 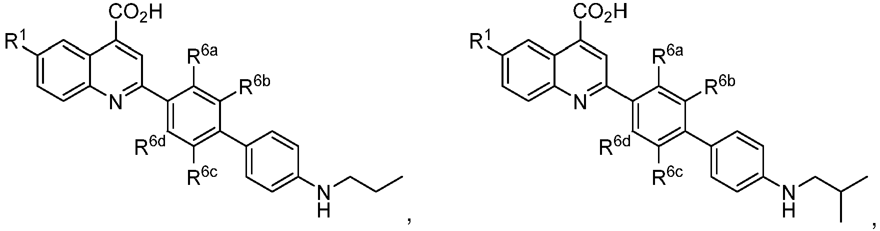 or combinations thereof.

. The compound may have a structure represented by a formula: 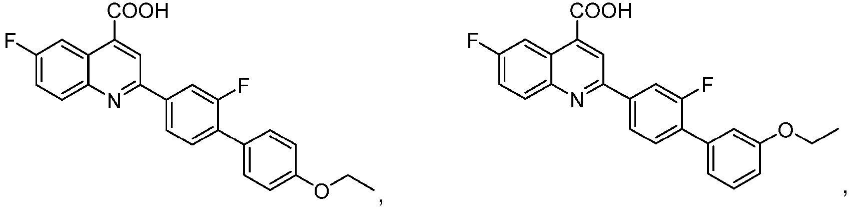 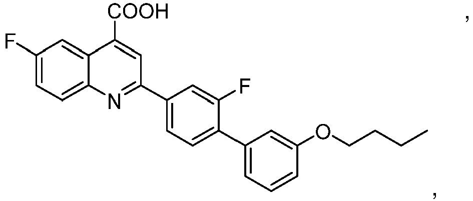 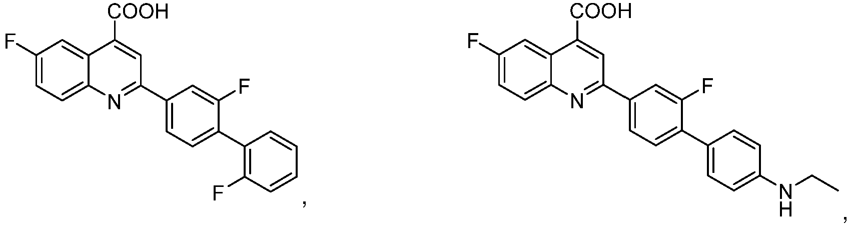 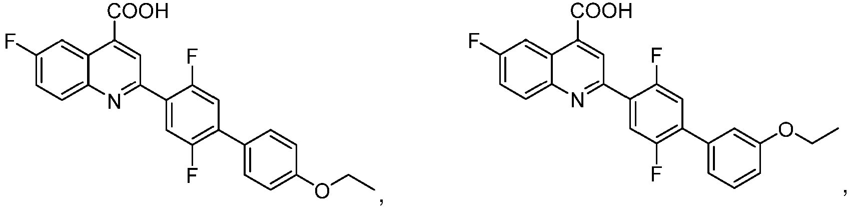 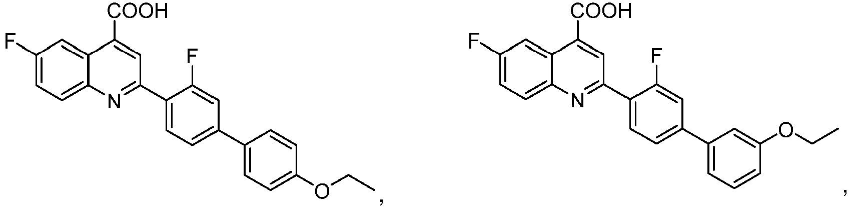 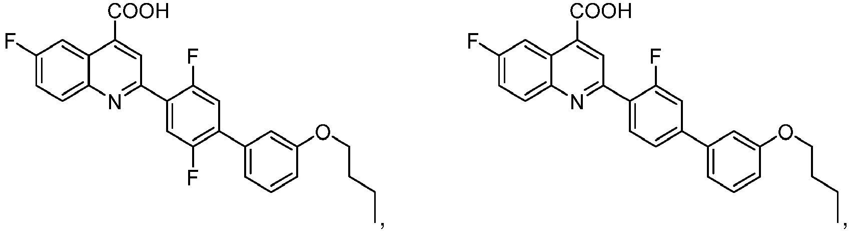 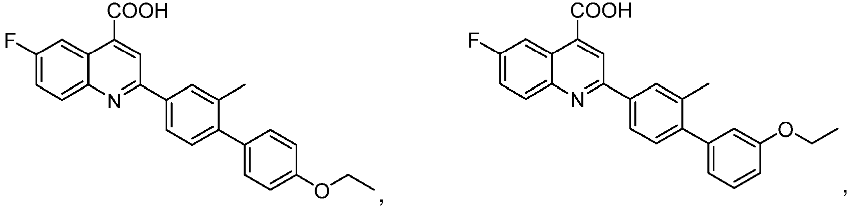 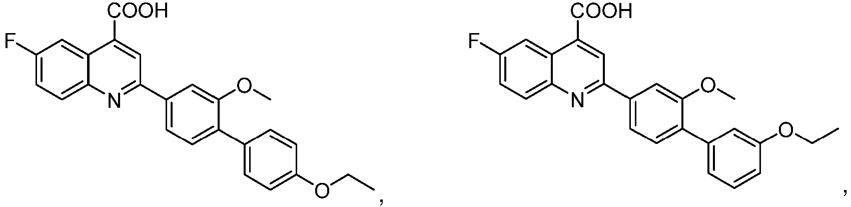 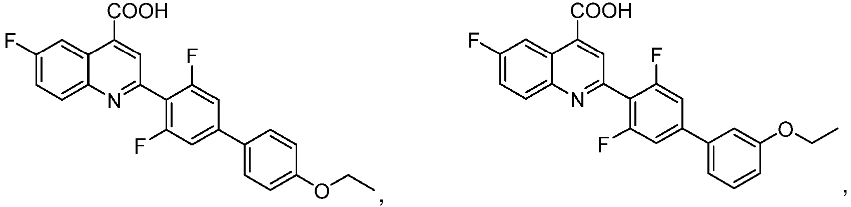 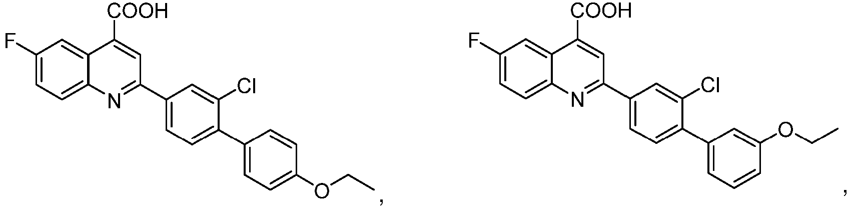 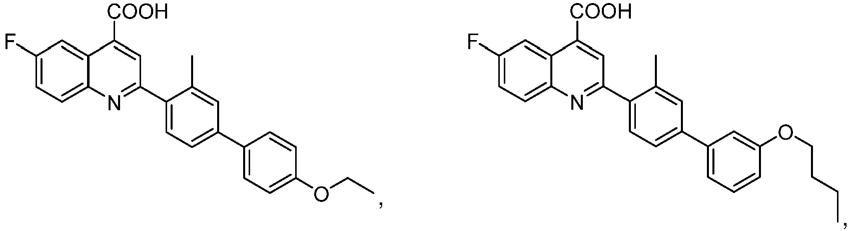 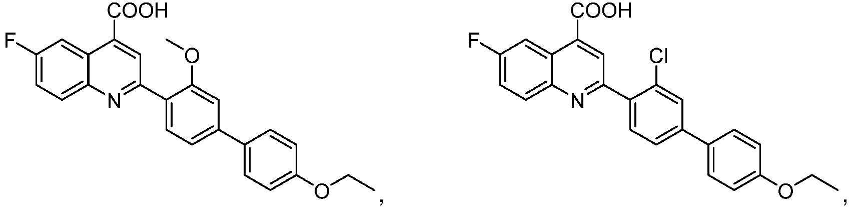 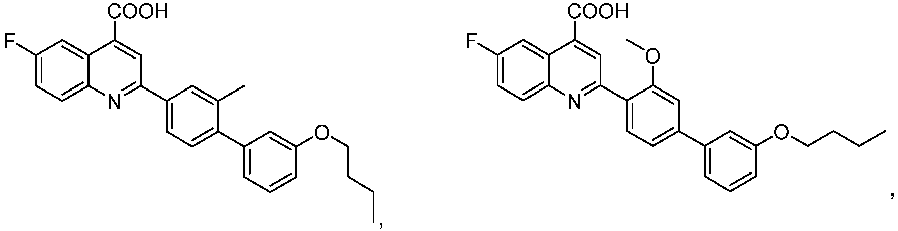 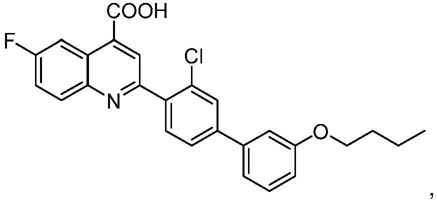 or combinations thereof.

The compound of may have a structure of:: 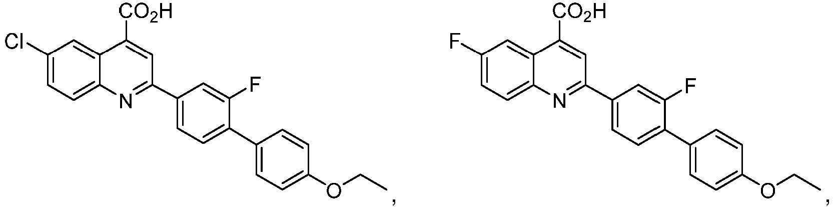 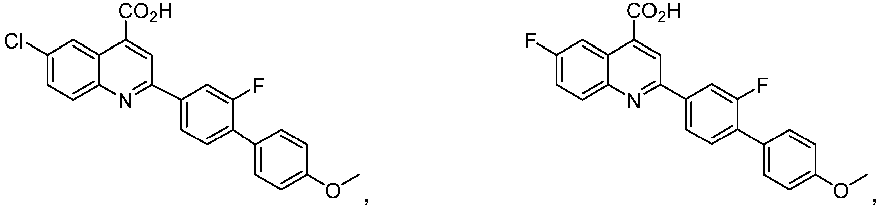 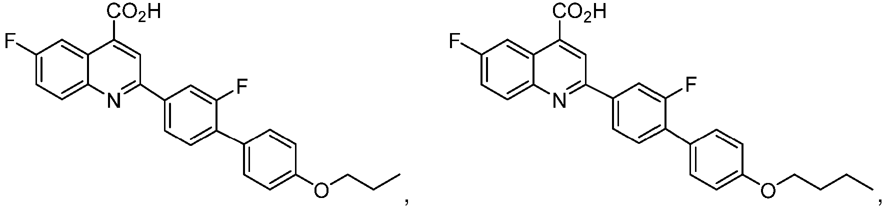 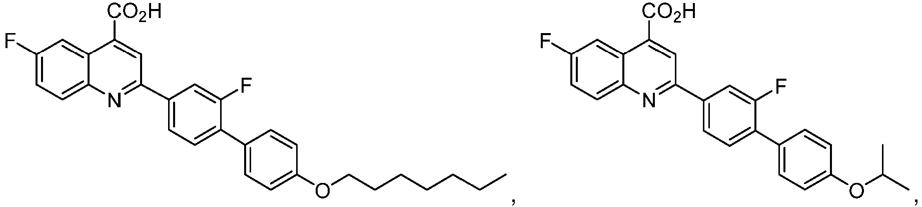 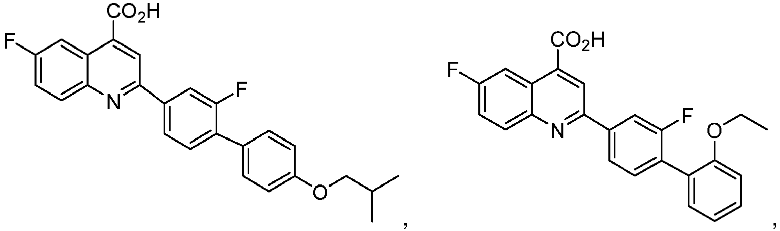 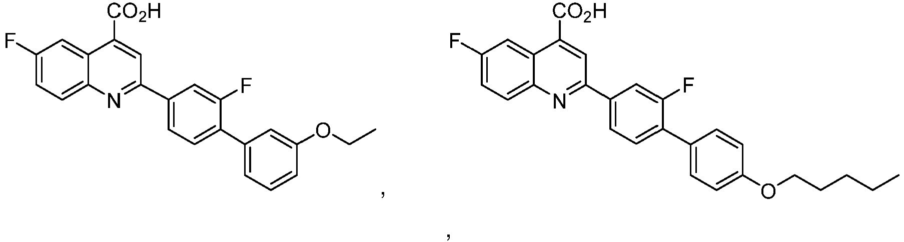 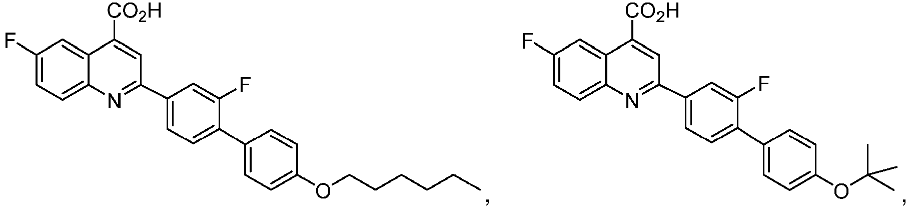 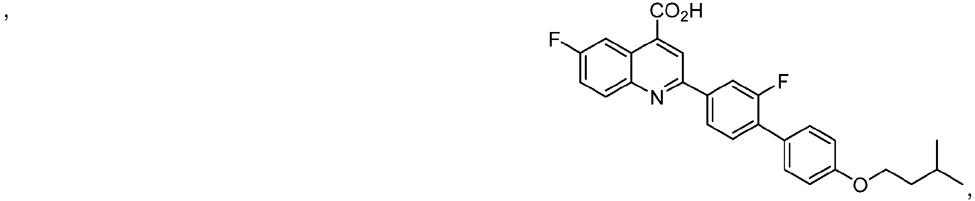 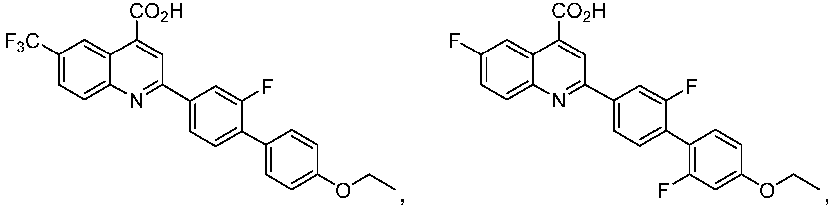 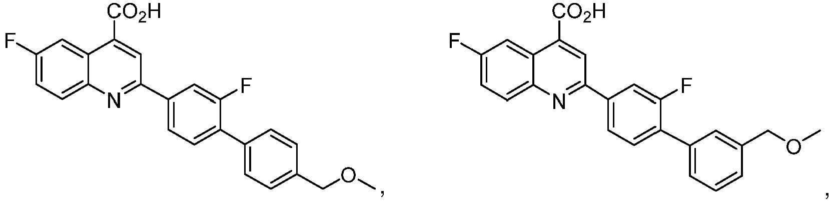 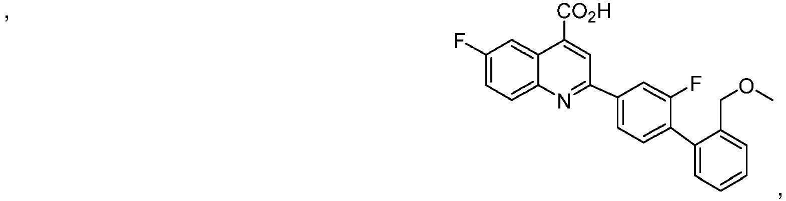 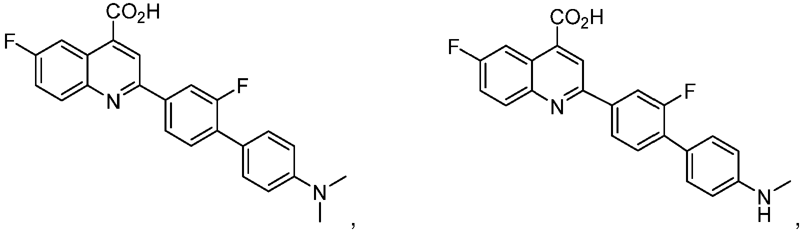 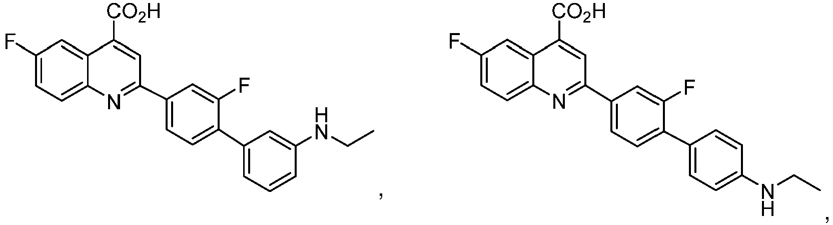 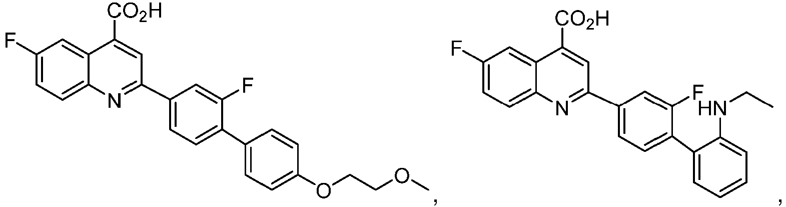 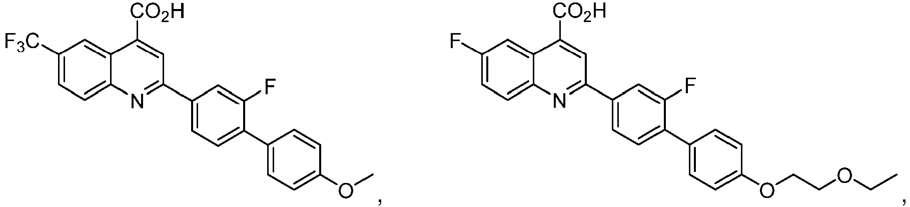 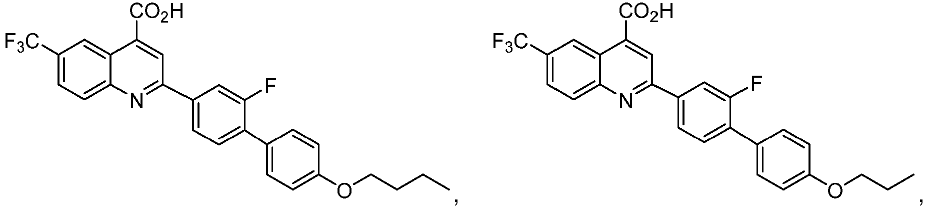 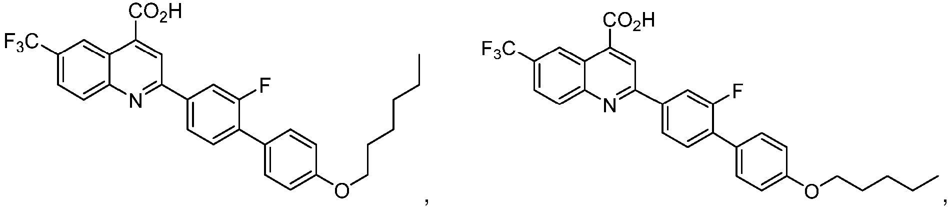 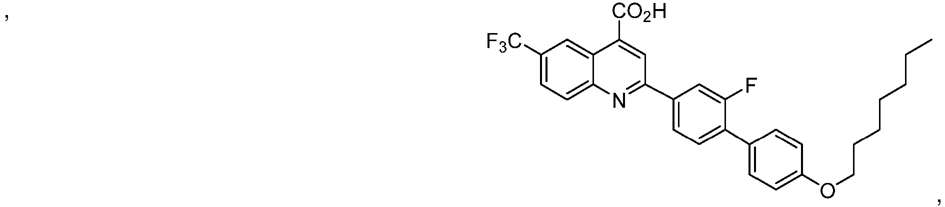 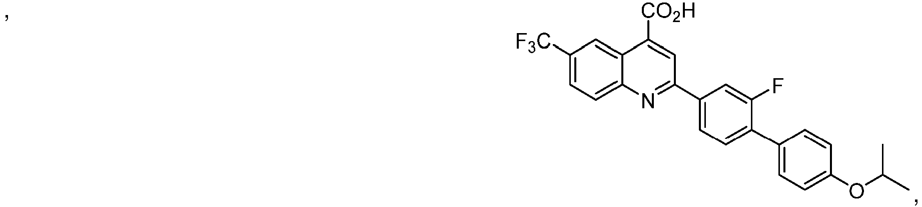 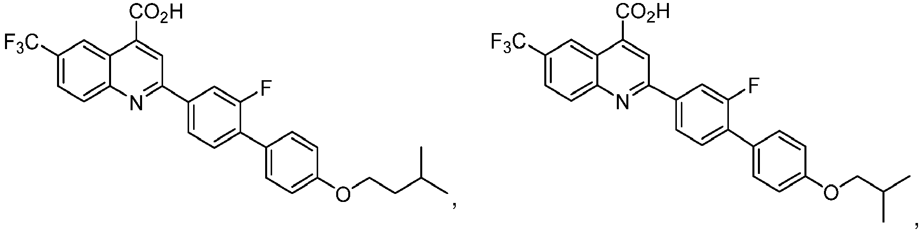 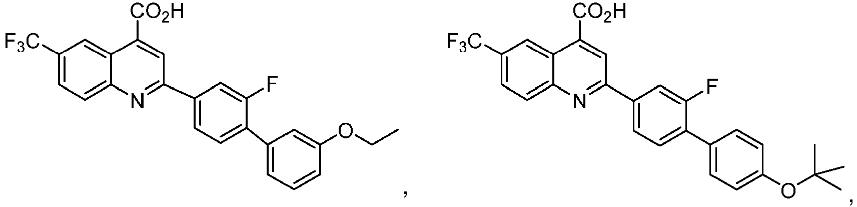 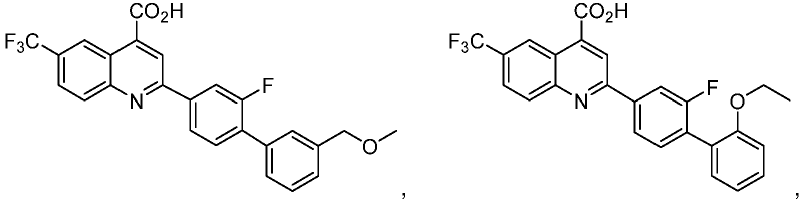 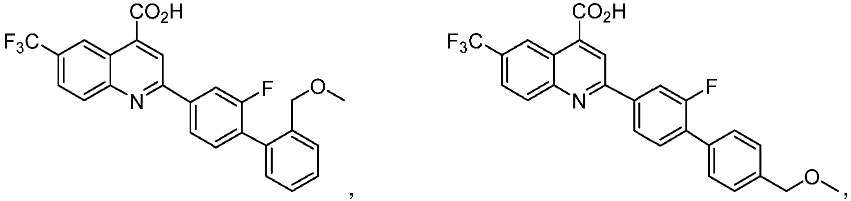 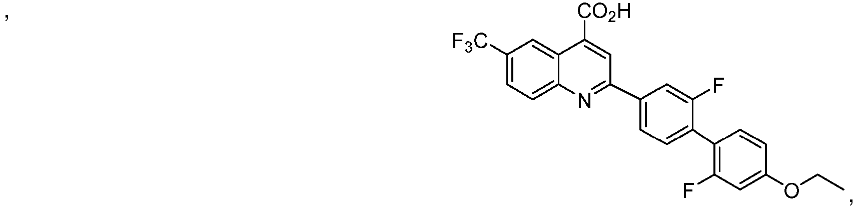 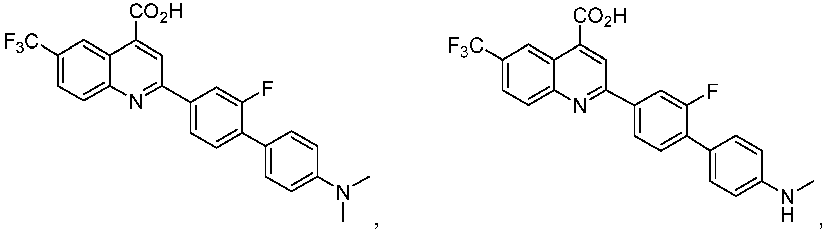 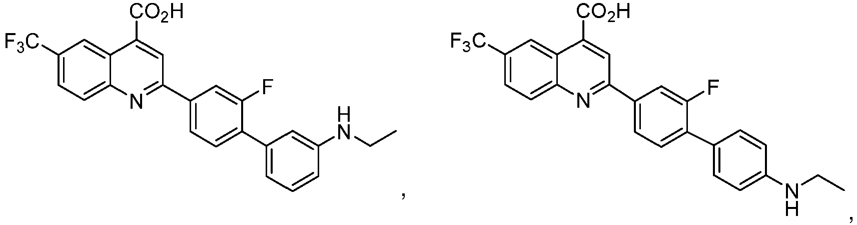 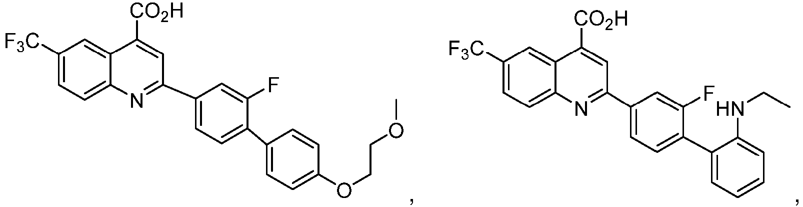 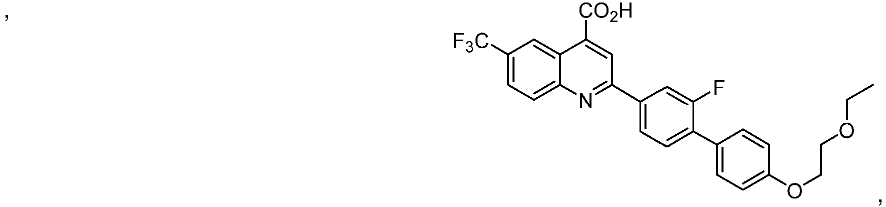 or a subgroup thereof.

The compound may have a structure of : 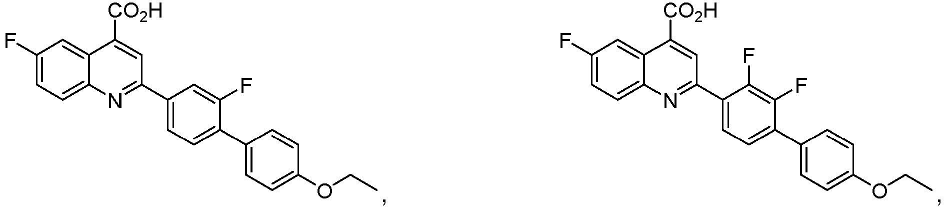 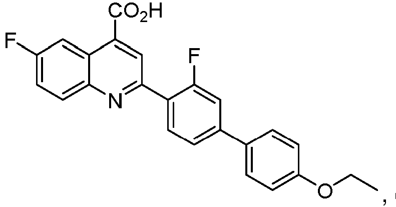 or a combination thereof.

The compound may be a pharmaceutically acceptable salt thereof comprising the conjugate base form of the compound, and a counter ion selected from Li+, K+, Na+, ammonium, tetramethylammonium, tetraethylammonium, Fe⁺², Cu⁺², Zn⁺², Mg⁺², Ca⁺², Al⁺³, Fe⁺³, and combinations thereof. The counterion may be Na⁺.

The compound may have a structure represented by a formula: 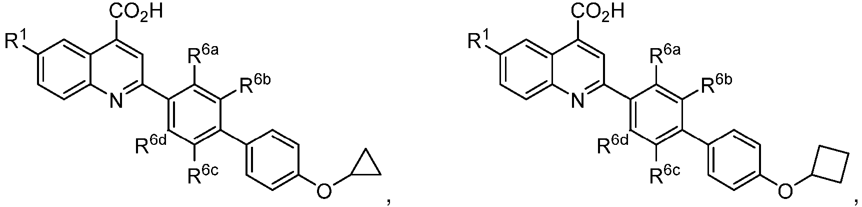 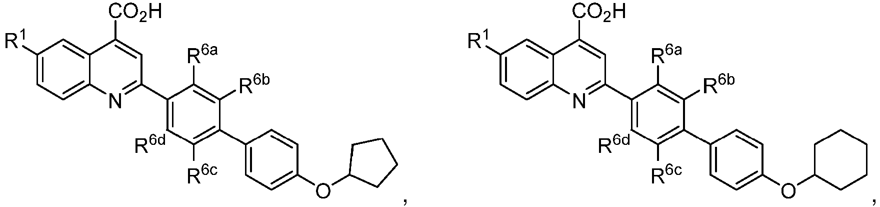 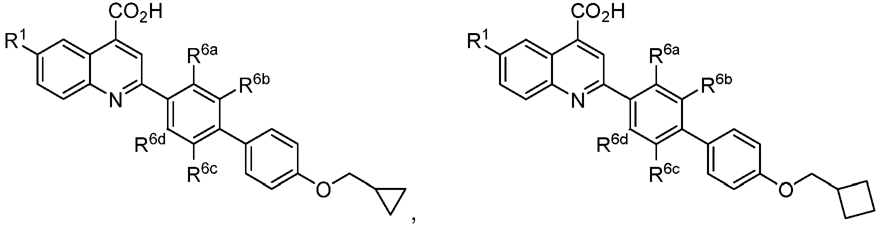 or combinations thereof.

. The compound may have a structure represented by a formula: 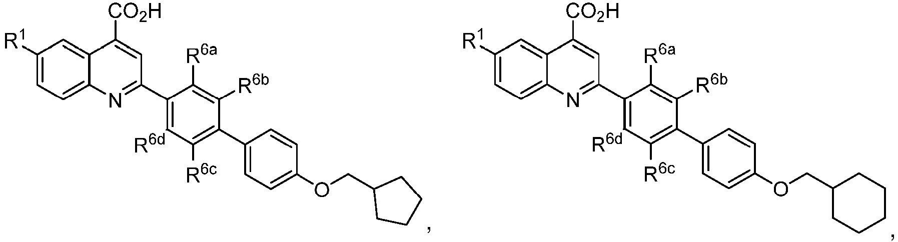 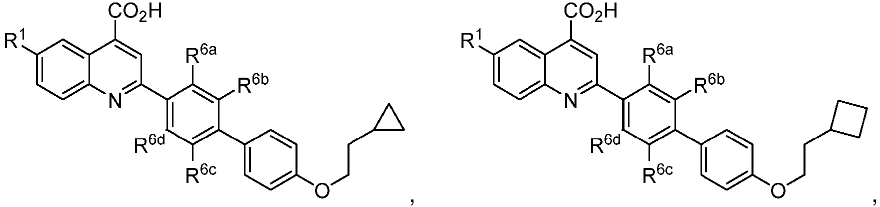 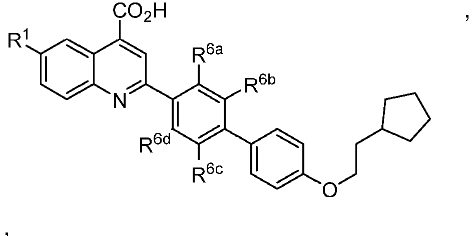 or combinations thereof.

The compound may have a structure represented by a formula: 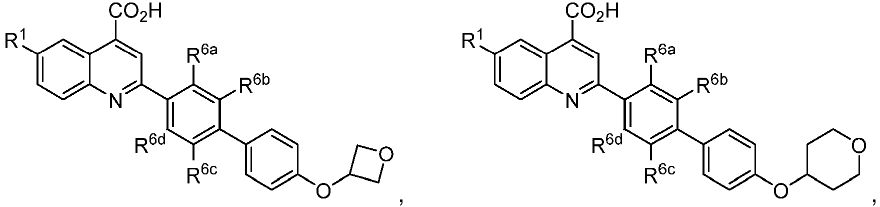 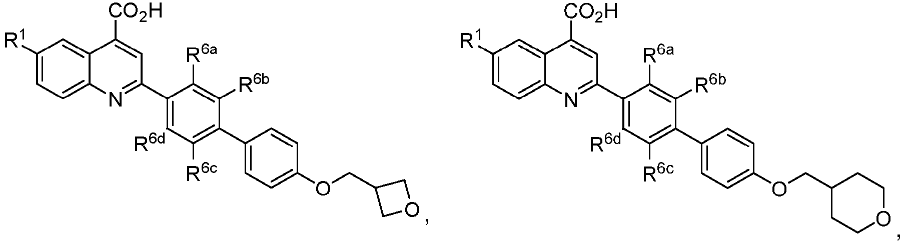 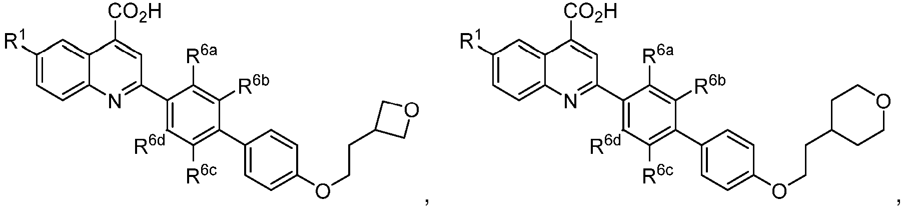 or combinations thereof.

The compound may have a structure represented by a formula: 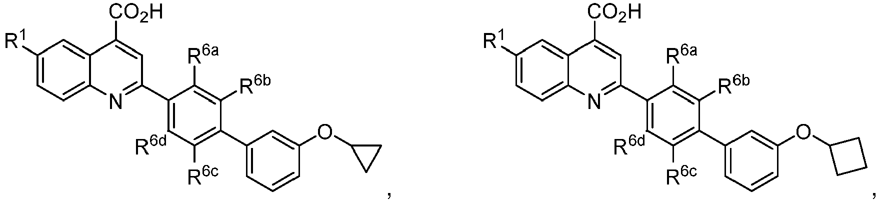 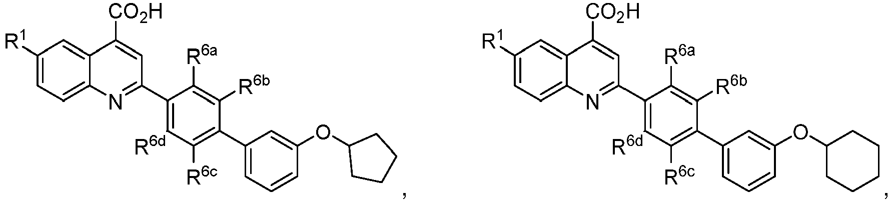 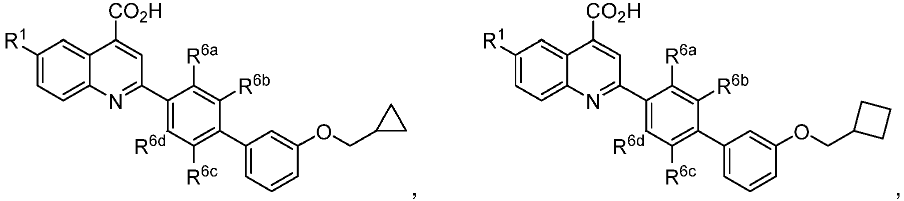 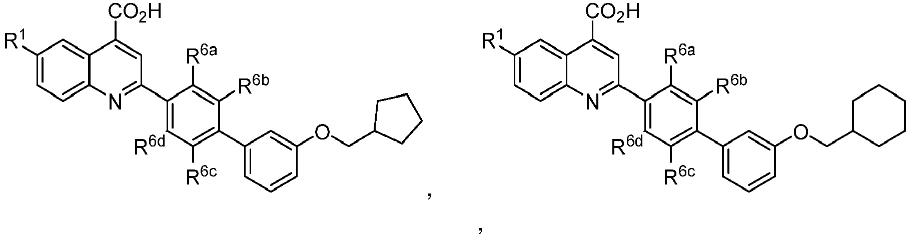 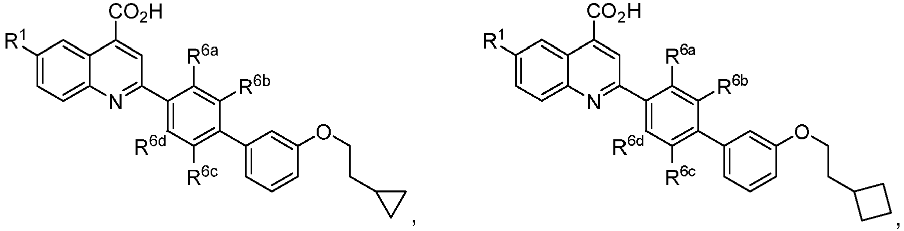 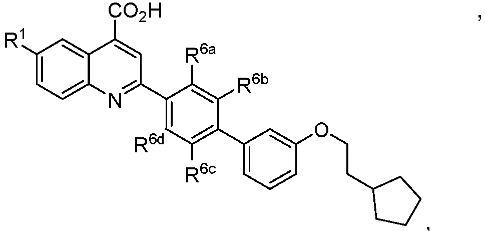 or combinations thereof.

The compound may have a structure represented by a formula: 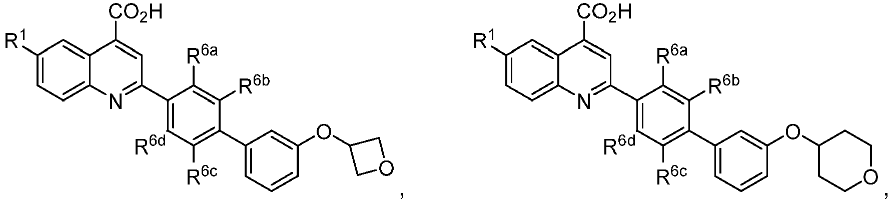 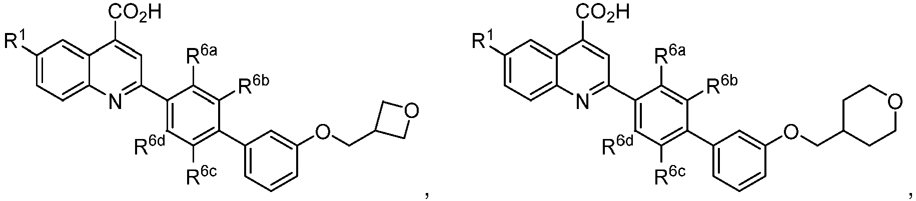 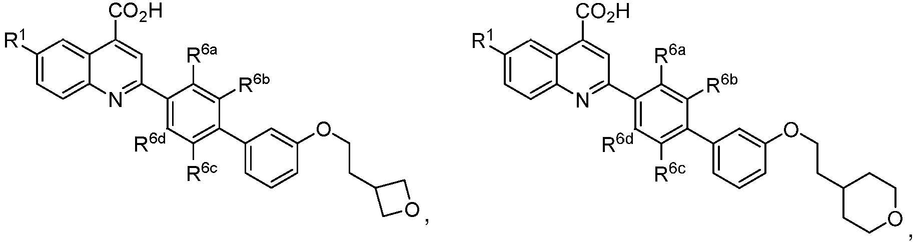 or combinations thereof.

The compound may have a structure represented by a formula: 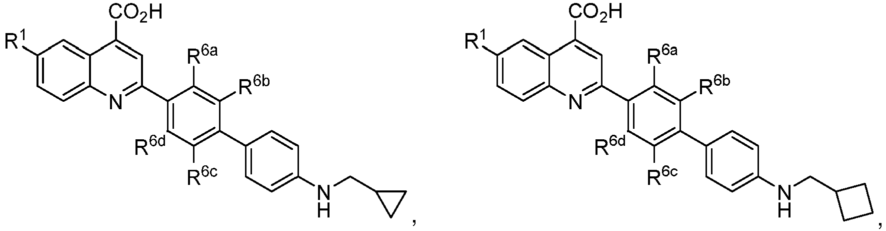 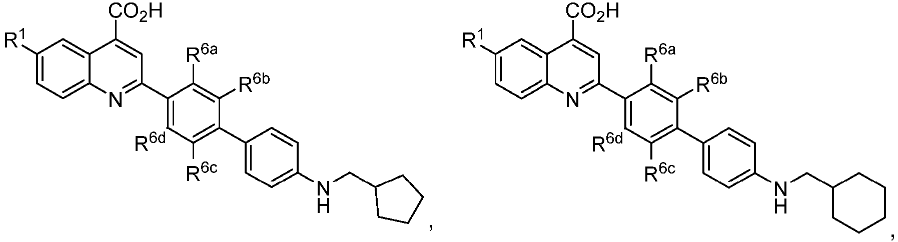 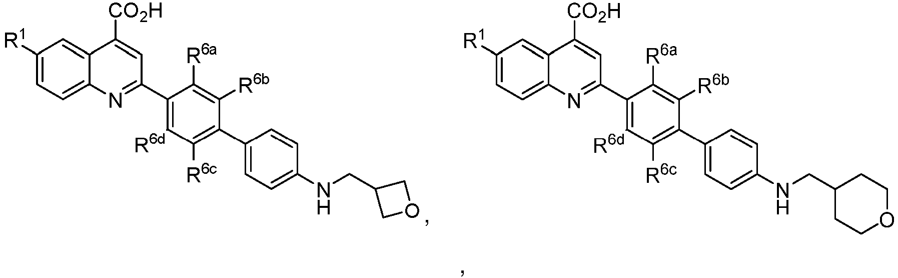 Or combinations thereof.

In accordance with a second aspect of the present invention, there is also provided a pharmaceutical composition in accordance with claim 10.

The pharmaceutical composition of may comprise at least one agent known to treat a cancer.

The at least one agent may be a DNA methyltransferase inhibitor, an HDAC-inhibitor, a glucocorticoid, an mTOR inhibitor, a cytotoxic agent, or combinations thereof.

The DNA methyltransferase inhibitor may be 5-aza-2'-deoxycytidine, 5-azacytidine, zebularin, epigallocatechin-3-gallate, procaine, or combinations thereof.

The HDAC-inhibitor may be vorinostat, entinostat, panbinostat, trichostatin A, mocetinostat, belinostat, dacinostat, givinostat, tubastatin A, pracinostat, droxinostat, quisinostat, romidepsin, valproic acid, AR-42 (OSU-HDAC42), tacedinaline, rocilinostat, apicidin, or combinations thereof.

The glucocorticoid may be dexamethasone, prednisolone, methylprednisolone, betamethasone, triamicinolone, fludrocortisone, beclomethasone, or combinations thereof.

The mTor inhibitor may be BEZ235, everolimus, temsirolimus, rapamycin, AZD8055, or combinations thereof.

The cytotoxic agent may be an alkylating agent, an antimetabolite agent, an antineoplastic antibiotic agent, a mitotic inhibitor agent, a mTor inhibitor agent or other chemotherapeutic agent.

The antineoplastic antibiotic agent may be selected from one or more of the group consisting of doxorubicin, mitoxantrone, bleomycin, daunorubicin, dactinomycin, epirubicin, idarubicin, plicamycin, mitomycin, pentostatin, and valrubicin, or a pharmaceutically acceptable salt, hydrate, solvate, or polymorph thereof.

The antimetabolite agent may be selected from one or more of the group consisting of gemcitabine, 5-fluorouracil, capecitabine, hydroxyurea, mercaptopurine, pemetrexed, fludarabine, nelarabine, cladribine, clofarabine, cytarabine, decitabine, pralatrexate, floxuridine, methotrexate, and thioguanine, or a pharmaceutically acceptable salt, hydrate, solvate, or polymorph thereof.

The alkylating agent may be selected from one or more of the group consisting of carboplatin, cisplatin, cyclophosphamide, chlorambucil, melphalan, carmustine, busulfan, lomustine, dacarbazine, oxaliplatin, ifosfamide, mechlorethamine, temozolomide, thiotepa, bendamustine, and streptozocin, or a pharmaceutically acceptable salt, hydrate, solvate, or polymorph thereof.

The mitotic inhibitor agent may be selected from one or more of the group consisting of irinotecan, topotecan, rubitecan, cabazitaxel, docetaxel, paclitaxel, etopside, vincristine, ixabepilone, vinorelbine, vinblastine, and teniposide, or a pharmaceutically acceptable salt, hydrate, solvate, or polymorph thereof.

The mTor inhibitor may be everolimus, sirolimus, temsirolimus, or combinations thereof.

The other chemotherapeutic agent may be an anthracycline, cytarabine, a purine analog, sorafenib, gemtuzumab ozogamicin, rituximab, or combinations thereof.

The anthracycline may be daunorubicin, idarubicin, or combinations thereof.

The purine analog may be cladribine, fludarabine, clofarabine, or combinations thereof.

The pharmaceutical composition may further comprise at least one agent known to treat GVHD.

The least one agent known to treat GVHD may be a steroid, an mTor inhibitor, a tyrosine kinase inhibitor, or other agent known to treat GVHD.

The steroid may be dexamethasone, prednisolone, methylprednisolone, betamethasone, triamicinolone, fludrocortisone, beclomethasone, or combinations thereof.

The tyrosine kinase inhibitor may be imatinib, ruxolitinib, or a combination thereof.

The mTor inhibitor may be everolimus, sirolimus, temsirolimus, or combinations thereof.

The other agent known to treat GVHD may be tacrolimus, clofazimine, psoralen, cyclosporine, alemtuzumab, infliximab, rituximab, etanercept, antithymocyte globulin, thalidomide, mycophenolate mofetil, pentostatin, methotrexate, halofuginone, hydroxychloroquine, or combinations thereof.

The pharmaceutical composition may further comprise the step of administering a therapeutically effective amount of at least one agent known to treat an autoimmune disorder or disease.

The at least one agent known to treat an autoimmune disorder or disease may be selected from the group consisting of: (a) disease modifying antirheumatic drugs; (b) nonsteroidal anti-inflammatory drugs; (c) COX-2 selective inhibitors; (d) COX-1 inhibitors; (e) immunosuppressive drugs, including p70S6 kinase inhibitors; and inosine monophosphate dehydrogenase inhibitors; (f) steroids; (g) biological response modifiers; and (h) other agents useful for the treatment of autoimmune disorders.

The disease modifying antirheumatic drug may be selected from methotrexate, gold salts, D-penicillamine, hydroxychloroquine, auranofin, sulfasalazine, and combinations thereof.

The nonsteroidal anitinflammatory drug may be selected from indomethacin, naproxen, diclofenac, ibuprofen, aspirin and aspirin analogs, acetaminophen, and combinations thereof.

Te COX-2 selective inhibitor may be selected from celecoxib, rofecoxib, etoricoxib, valdecoxib, lumiracoxib, and combinations thereof.

The immunosuppressive drug may be selected from a calcineurin inhibitor such as cyclosporin and FK506;a p70S6 kinase inhibitor such as sirolimus and rapamycin; an inosine monophosphate dehydrogenase inhibitor such as mycophenolate; leflunomide, cyclophosphamide, azathioprine, and combinations thereof.

The steroid may be selected from prednisone, betamethasone, budesonide and dexamethasone, and combinations thereof.

The biological response modifier may be selected from TNFα antagonists such as infliximab, adalimmab and etanercept; IL-1 receptor antagonists such as anakinra; humanized or chimeric antibodies or fusion proteins such as alefacept, efalizumab, daclizumab; anti-chemokine antibodies; anti-interleukin antibodies; and combinations thereof.

The other agent useful for the treatment of autoimmune disorder may be selected from hemokine receptor antagonists or modulators, cannabinoid receptor antagonists or modulators, inhibitors of matrix metalloproteinases, TNFα-converting enzymes, nitric oxide synthetases or phosphodiesterase IV, such as roflumilast or cilomilast; inhibitors of p38 MAP-kinase, the NF-kappaβ, pathway or IL-1 receptor associated kinase or inhibitors of interactions involving adhesion molecules such as LFA-1, VLA-4, ICAM-1, VCAM-1, α4β7, MAdCAM-1, and αvβ3; and combinations thereof.

In accordance with a third aspect of the present invention, there is also provided a compound in accordance with the first aspect of the present invention or a composition in accordance with the second aspect of the present invention, for use in the treatment of cancer, such as hematological cancer.

Also disclosed, but not according to the invention, are methods for the treatment of a disease or disorder in a mammal comprising the step of administering to the mammal a therapeutically effective amount of at least one compound, or a pharmaceutically acceptable salt thereof, in accordance with the invention or a pharmaceutical composition in accordance with the invention.

The mammal may be a human.

The mammal may have been diagnosed with a need for treatment of the disorder prior to the administering step.

The disorder or disease may be associated with abnormal, increased, or aberrant dihydroorotate dehydrogenase (DHODH) activity.

The disorder or disease can optionally be treated by inhibition of dihydroorotate dehydrogenase (DHODH) activity.

The method may further comprise the step of identifying a mammal in need of treatment of the disorder or disease.

The disorder may be a cancer.

The cancer may be selected from breast cancer, renal cancer, gastric cancer, colorectal cancer, ovarian cancer, prostate cancer, pancreatic cancer, brain cancer, genitourinary tract cancer, lymphatic system cancer, stomach cancer, larynx cancer, lung cancer, pancreatic cancer, breast cancer, and malignant melanoma.

The cancer may be a hematological cancer.

The hematological cancer may be leukemia, lymphoma, myeloma, myelodysplastic syndrome, or myeloproliferative neoplasm.

The hematological cancer may be chronic myeloid leukemia (CML), acute myeloid leukemia (AML), chronic lymphoid leukemia (CLL), acute lymphoid leukemia (ALL), hairy cell leukemia, chronic myelomonocytic leukemia (CMML), juvenile myelomonocyte leukemia (JMML), large granular lymphocytic leukemia (LGL), acute lymphocytic leukemia, acute lymphoblastic leukemia, B-cell lymphoma, T-cell-lymphoma, Hodgkin's lymphoma, non-Hodgkin's lymphoma, hairy cell lymphoma, Burkett's lymphoma, Hodgkin lymphoma, and non-Hodgkin lymphoma.

The hematological cancer may be chronic myeloid leukemia (CML) or acute myeloid leukemia (AML).

The method may further comprise the step of administering a therapeutically effective amount of at least one agent known to treat a cancer.

The at least one agent is optionally selected from uracil mustard, chlormethine, cyclophosphamide, ifosfamide, melphalan, chlorambucil, pipobroman, triethylenemelamine, triethylenethiophosphoramine, busulfan, carmustine, lomustine, streptozocin, dacarbazine, temozolomide, thiotepa, altretamine, methotrexate, 5-fluorouracil, floxuridine, cytarabine, 6-mercaptopurine, 6-thioguanine, fludarabine phosphate, pentostatin, bortezomib, vinblastine, vincristine, vinorelbine, vindesine, bleomycin, dactinomycin, daunorubicin, doxorubicin, epirubicin, dexamethasone, clofarabine, cladribine, pemextresed, idarubicin, paclitaxel, docetaxel, ixabepilone, mithramycin, topotecan, irinotecan, deoxycoformycin, mitomycin-C, L-asparaginase, interferons, etoposide, teniposide 17α-ethinylestradiol, diethylstilbestrol, testosterone, prednisone, fluoxymesterone, dromostanolone propionate, testolactone, megestrolacetate, tamoxifen, methylprednisolone, methyltestosterone, prednisolone, triamcinolone, chlorotrianisene, hydroxyprogesterone, aminoglutethimide, estramustine, medroxyprogesteroneacetate, leuprolide, flutamide, toremifene, goserelin, cisplatin, carboplatin, hydroxyurea, amsacrine, procarbazine, mitotane, mitoxantrone, levamisole, navelbene, anastrazole, letrazole, capecitabine, reloxafine, droloxafine, hexamethylmelamine, oxaliplatin, gefinitib, capecitabine, erlotinib, azacitidine, temozolomide, gemcitabine, and vasostatin.

The at least one agent is optionally a DNA methyltransferase inhibitor, an HDAC-inhibitor, a glucocorticoid, an mTOR inhibitor, a cytotoxic agent, or combinations thereof.

The DNA methyltransferase inhibitor is optionally 5-aza-2'-deoxycytidine, 5-azacytidine, zebularin, epigallocatechin-3-gallate, procaine, or combinations thereof.

The HDAC-inhibitor is optionally vorinostat, entinostat, panbinostat, trichostatin A, mocetinostat, belinostat, dacinostat, givinostat, tubastatin A, pracinostat, droxinostat, quisinostat, romidepsin, valproic acid, AR-42 (OSU-HDAC42), tacedinaline, rocilinostat, apicidin, or combinations thereof.

The glucocorticoid is optionally dexamethasone, prednisolone, methylprednisolone, betamethasone, triamicinolone, fludrocortisone, beclomethasone, or combinations thereof.

The mTor inhibitor is optionally BEZ235, everolimus, temsirolimus, rapamycin, AZD8055, or combinations thereof.

The cytotoxic agent is optionally an alkylating agent, an antimetabolite agent, an antineoplastic antibiotic agent, a mitotic inhibitor agent, a mTor inhibitor agent or other chemotherapeutic agent.

The antineoplastic antibiotic agent is optionally selected from one or more of the group consisting of doxorubicin, mitoxantrone, bleomycin, daunorubicin, dactinomycin, epirubicin, idarubicin, plicamycin, mitomycin, pentostatin, and valrubicin, or a pharmaceutically acceptable salt, hydrate, solvate, or polymorph thereof.

The antimetabolite agent is optionally selected from one or more of the group consisting of gemcitabine, 5-fluorouracil, capecitabine, hydroxyurea, mercaptopurine, pemetrexed, fludarabine, nelarabine, cladribine, clofarabine, cytarabine, decitabine, pralatrexate, floxuridine, methotrexate, and thioguanine, or a pharmaceutically acceptable salt, hydrate, solvate, or polymorph thereof.

The alkylating agent is optinally selected from one or more of the group consisting of carboplatin, cisplatin, cyclophosphamide, chlorambucil, melphalan, carmustine, busulfan, lomustine, dacarbazine, oxaliplatin, ifosfamide, mechlorethamine, temozolomide, thiotepa, bendamustine, and streptozocin, or a pharmaceutically acceptable salt, hydrate, solvate, or polymorph thereof.

The mitotic inhibitor agent is optionally selected from one or more of the group consisting of irinotecan, topotecan, rubitecan, cabazitaxel, docetaxel, paclitaxel, etopside, vincristine, ixabepilone, vinorelbine, vinblastine, and teniposide, or a pharmaceutically acceptable salt, hydrate, solvate, or polymorph thereof.

The mTor inhibitor is optionally everolimus, sirolimus, temsirolimus, or combinations thereof.

The other chemotherapeutic agent is optionally an anthracycline, cytarabine, a purine analog, sorafenib, gemtuzumab ozogamicin, rituximab, or combinations thereof.

The anthracycline is optionally daunorubicin, idarubicin, or combinations thereof.

The purine analog is optionally cladribine, fludarabine, clofarabine, or combinations thereof.

The at least one compound and the at least one agent are optionally administered sequentially.

The at least one compound and the at least one agent are optionally administered simultaneously.

The at least one compound and the at least one agent are optionally co-formulated.

The at least one compound and the at least one agent are optionally co-packaged.

The disorder is optionally mediated by T-cell proliferation.

The disorder is optionally psoriasis.

The disorder is optionally graft-versus-host disease (GVHD).

The GVHD is optionally associated with an organ transplant, an allograft, a xenograft, or a hematopoietic stem cell transplantation.

The GVHD may be acute GVHD.

The GVHD is optionally chronic GVHD.

The method may further comprise the step of administering a therapeutically effective amount of at least one agent known to treat GVHD.

The at least one agent known to treat GVHD may be a steroid, an mTor inhibitor, a tyrosine kinase inhibitor, or other agent known to treat GVHD.

The steroid may be dexamethasone, prednisolone, methylprednisolone, betamethasone, triamicinolone, fludrocortisone, beclomethasone, or combinations thereof.

The tyrosine kinase inhibitor may be imatinib, ruxolitinib, or a combination thereof.

The mTor inhibitor is optionally everolimus, sirolimus, temsirolimus, or combinations thereof.

The other agent known to treat GVHD is optionally tacrolimus, clofazimine, psoralen, cyclosporine, alemtuzumab, infliximab, rituximab, etanercept, antithymocyte globulin, thalidomide, mycophenolate mofetil, pentostatin, methotrexate, halofuginone, hydroxychloroquine, or combinations thereof.

The disorder may be associated with T-cell proliferation.

The disorder may be an autoimmune disorder or disease.

The autoimmune disorder or disease may be selected from lupus, rheumatoid arthritis, ankylosing spondylitis, glomerulonephritis, minimal change disease, ulcerative colitis, Crohn's disease, Addison's disease, adult Still's disease, alopecia areata, autoimmune hepatitis, autoimmune angioedema, Bechet's disease, pemphigoid and variants, celiac disease, chronic inflammatory demyelinating polyneuropathy, Churg-Straus syndrome, Crest syndrome, dermatomyositis, neuromyelitis optica, discoid lupus, fibromyalgia, giant cell arteritis, giant cell myocarditis, Goodpasteur's disease, evan's syndrome, autoimmune hemolytic anemia, immune thrombocytopenia, Henoch-Schonlein purpura, IgA nephropathy, IgG4 related sclerosing disease, juvenile arthritis, juvenile diabetes, Kawasaki disease, Leukocytoclastic vasculitis, mixed connective disease, multiple sclerosis, multifocal motor neuropathy, myasthenia gravis, autoimmune neutropenia, optic neuritis, peripheral neuropathy, POEMS syndrome, polymyositis, primary biliary cirrhosis, non-alcoholic hepatosteotosis and associated cirrhosis, psoriasis, scleroderma, sarcoidosis, temporal arteritis, vasculitis, and uveitis.

The method may further comprise the step of administering a therapeutically effective amount of at least one agent known to treat an autoimmune disorder or disease.

The at least one agent known to treat an autoimmune disorder or disease may be selected from the group consisting of: (a) disease modifying antirheumatic drugs; (b) nonsteroidal anitinflammatory drugs; (c) COX-2 selective inhibitors; (d) COX-1 inhibitors; (e) immunosuppressive drugs, including p70S6 kinase inhibitors; and inosine monophosphate dehydrogenase inhibitors; (f) steroids; (g) biological response modifiers; and (h) other agents useful for the treatment of autoimmune disorders.

The disease modifying antirheumatic drug may be selected from methotrexate, gold salts, D-penicillamine, hydroxychloroquine, auranofin, sulfasalazine, and combinations thereof.

The nonsteroidal anitinflammatory drug may be selected from indomethacin, naproxen, diclofenac, ibuprofen, aspirin and aspirin analogs, acetaminophen, and combinations thereof.

The COX-2 selective inhibitor may be selected from celecoxib, rofecoxib, etoricoxib, valdecoxib, lumiracoxib, and combinations thereof.

The immunosuppressive drug may be selected from a calcineurin inhibitor such as cyclosporin and FK506;a p70S6 kinase inhibitor such as sirolimus and rapamycin; an inosine monophosphate dehydrogenase inhibitor such as mycophenolate; leflunomide, cyclophosphamide, azathioprine, and combinations thereof.

The steroid may be selected from prednisone, betamethasone, budesonide and dexamethasone, and combinations thereof.

The biological response modifier may be selected from TNFα antagonists such as infliximab, adalimmab and etanercept; IL-1 receptor antagonists such as anakinra; humanized or chimeric antibodies or fusion proteins such as alefacept, efalizumab, daclizumab; anti-chemokine antibodies; anti-interleukin antibodies; and combinations thereof.

The other agent useful for the treatment of autoimmune disorder may be selected from hemokine receptor antagonists or modulators, cannabinoid receptor antagonists or modulators, inhibitors of matrix metalloproteinases, TNFα-converting enzymes, nitric oxide synthetases or phosphodiesterase IV, such as roflumilast or cilomilast; inhibitors of p38 MAP-kinase, the NF-κβ, pathway or IL-1 receptor associated kinase or inhibitors of interactions involving adhesion molecules such as LFA-1, VLA-4, ICAM-1, VCAM-1, α4β7, MAdCAM-1, and αvβ3; and combinations thereof.

Also disclosed, but not according to the invention, are methods for the treatment of a cancer in a mammal comprising the step of administering to the mammal a therapeutically effective amount of at least one compound, or a pharmaceutically acceptable salt thereof, in accordance with the invention or a pharmaceutical composition in accordance with the invention.

Also disclosed, but not according to the invention, are methods for the treatment of a graft-versus-host disease in a mammal comprising the step of administering to the mammal a therapeutically effective amount of at least one compound, or a pharmaceutically acceptable salt thereof, in accordance with the invention or a pharmaceutical composition in accordance with the invention.

Also disclosed, but not according to the invention, are methods for the treatment of a disease or disorder associated with T-cell proliferation in a mammal comprising the step of administering to the mammal a therapeutically effective amount of at least one compound, or a pharmaceutically acceptable salt thereof, in accordance with the invention or a pharmaceutical composition in accordance with the invention.

Also disclosed, but not claimed, are kits comprising a therapeutically effective amount of at least one compound, or a pharmaceutically acceptable salt thereof, in accordance with the invention or a pharmaceutical composition in accordance with the invention; and: (a) at least one agent known to treat a cancer, a host-versus-graft-disease, and/or a disorder associated with T-cell proliferation; and (b) instructions for treating a cancer, a host-versus-graft-disease, and/or a disorder associated with T-cell proliferation.

The at least one compound or the pharmaceutical composition and the at least one agent may be co-formulated.

The at least one compound or the pharmaceutical composition and the at least one agent may be co-packaged.

The kit may further comprise instructions to provide the compound in connection with surgery.

The instructions optionally provide that surgery is performed prior to the administering of at least one compound.

The instructions optionally provide that surgery is performed after the administering of at least one compound.

The instructions optionally provide that the administering of at least one compound is to effect presurgical debulking of a tumor.

The instructions may provide that surgery is performed at about the same time as the administering of at least one compound.

The kit may further comprise instructions to provide the at least one compound or the pharmaceutical composition in connection with radiotherapy.

Said instructions may provide that radiotherapy is performed prior to the administering of at least one compound.

The instructions may provide that radiotherapy is performed after the step of the administering of at least one compound.

The instructions may provide that radiotherapy is performed at about the same time as the step of the administering of at least one compound.

The kit may further comprise a plurality of dosage forms, the plurality comprising one or more doses; wherein each dose comprises a therapeutically effective amount of the at least one compound or the pharmaceutical composition and the at least one agent.

Each dose of the at least one compound or the pharmaceutical composition and the at least one agent may be co-formulated.

Each dose of the at least one compound or the pharmaceutical composition and the at least one agent may be co-packaged.

The dosage forms may be formulated for oral administration and/or intravenous administration.

The dosage forms may be formulated for oral administration.

The dosage forms may be formulated for intravenous administration.

The dosage form for the at least one compound or the pharmaceutical composition may be formulated for oral administration and the dosage form for the at least one agent is formulated for intravenous administration.

The dosage form for the at least one compound or the pharmaceutical composition may be formulated for intravenous administration and the dosage form for the at least one agent may be formulated for oral administration.

Also disclosed, but not claimed, are methods for manufacturing a medicament comprising combining at least one compound in accordance with the invention with a pharmaceutically acceptable carrier or diluent.

Also disclosed, but not claimed, are uses of a compound in accordance with the invention in the manufacture of a medicament for the treatment of a disease or disorder in a mammal such as a cancer, a disorder associated with T-cell proliferation, or a graft-versus-host-disease.

### BRIEF DESCRIPTION OF THE DRAWINGS

Many aspects of the present disclosure can be better understood with reference to the following drawings. The components in the drawings are not necessarily to scale, emphasis instead being placed upon clearly illustrating the principles of the present disclosure. Moreover, in the drawings, like reference numerals designate corresponding parts throughout the several views.

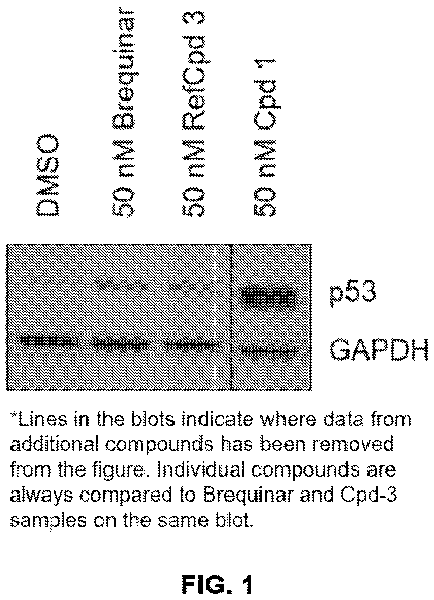

**FIG.** 1 shows representative data for the effect of a representative disclosed compound, Cpd1, on the expression of p53 in an AML cell-line as determined by immunoblot analysis.

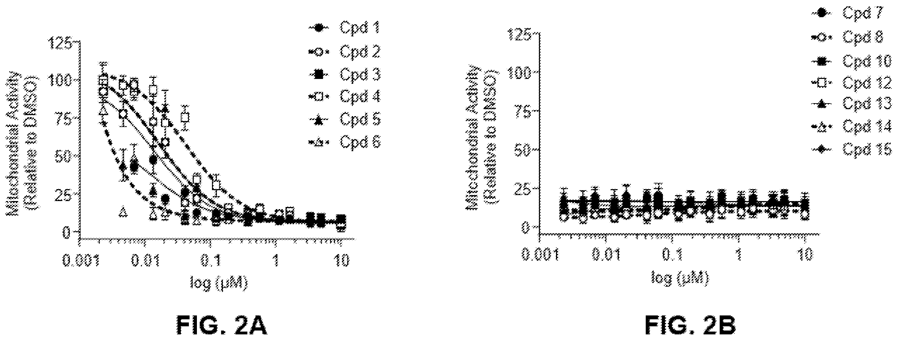

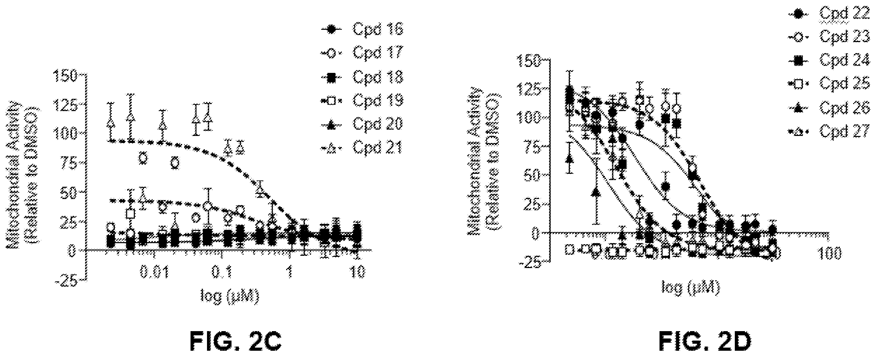

**FIGs. 2A-2D** show representative data for the activity of representative disclosed compounds in cell proliferation assays carried out using the MTS assay described herein below and inhibition curves used to determine IC₅₀ values in same. **FIG. 2A** shows representative data for the activity of representative disclosed compounds in cell proliferation assays carried out using the MTS assay described herein below and inhibition curves used to determine IC₅₀ values in same for compounds Cpd1-Cpd6 as indicated. **FIG. 2B** shows representative data for the activity of representative disclosed compounds in cell proliferation assays carried out using the MTS assay described herein below and inhibition curves used to determine IC₅₀ values in same for compounds Cpd7, Cpd8, Cpd10, and Cpd12-Cpd15 as indicated. **FIG. 2C** shows representative data for the activity of representative disclosed compounds in cell proliferation assays carried out using the MTS assay described herein below and inhibition curves used to determine IC₅₀ values in same for compounds Cpd16-Cpd21 as indicated. **FIG. 2D** shows representative data for the activity of representative disclosed compounds in cell proliferation assays carried out using the MTS assay described herein below and inhibition curves used to determine IC₅₀ values in same for compounds Cpd22-Cpd27 as indicated.

Additional advantages of the invention will be set forth in part in the description which follows, and in part will be obvious from the description, or can be learned by practice of the invention. The advantages of the invention will be realized and attained by means of the elements and combinations particularly pointed out in the appended claims. It is to be understood that both the foregoing general description and the following detailed description are exemplary and explanatory only and are not restrictive of the invention, as claimed.

### DETAILED DESCRIPTION

Many modifications and other embodiments disclosed herein will come to mind to one skilled in the art to which the disclosed compositions and methods pertain having the benefit of the teachings presented in the foregoing descriptions and the associated drawings. Therefore, it is to be understood that the disclosures are not to be limited to the specific embodiments disclosed and that modifications and other embodiments are intended to be included within the scope of the appended claims. The skilled artisan will recognize many variants and adaptations of the aspects described herein. These variants and adaptations are intended to be included in the teachings of this disclosure and to be encompassed by the claims herein.

Although specific terms are employed herein, they are used in a generic and descriptive sense only and not for purposes of limitation.

As will be apparent to those of skill in the art upon reading this disclosure, each of the individual embodiments described and illustrated herein has discrete components and features which may be readily separated from or combined with the features of any of the other several embodiments without departing from the scope or spirit of the present disclosure.

Any recited method can be carried out in the order of events recited or in any other order that is logically possible. That is, unless otherwise expressly stated, it is in no way intended that any method or aspect set forth herein be construed as requiring that its steps be performed in a specific order. Accordingly, where a method claim does not specifically state in the claims or descriptions that the steps are to be limited to a specific order, it is no way intended that an order be inferred, in any respect. This holds for any possible non-express basis for interpretation, including matters of logic with respect to arrangement of steps or operational flow, plain meaning derived from grammatical organization or punctuation, or the number or type of aspects described in the specification.

The publications discussed herein are provided solely for their disclosure prior to the filing date of the present application. Nothing herein is to be construed as an admission that the present invention is not entitled to antedate such publication by virtue of prior invention. Further, the dates of publication provided herein can be different from the actual publication dates, which can require independent confirmation.

While aspects of the present disclosure can be described and claimed in a particular statutory class, such as the system statutory class, this is for convenience only and one of skill in the art will understand that each aspect of the present disclosure can be described and claimed in any statutory class.

It is also to be understood that the terminology used herein is for the purpose of describing particular aspects only and is not intended to be limiting. Unless defined otherwise, all technical and scientific terms used herein have the same meaning as commonly understood by one of ordinary skill in the art to which the disclosed compositions and methods belong. It will be further understood that terms, such as those defined in commonly used dictionaries, should be interpreted as having a meaning that is consistent with their meaning in the context of the specification and relevant art and should not be interpreted in an idealized or overly formal sense unless expressly defined herein.

Prior to describing the various aspects of the present disclosure, the following definitions are provided and should be used unless otherwise indicated. Additional terms may be defined elsewhere in the present disclosure.

### Definitions

As used herein, "comprising" is to be interpreted as specifying the presence of the stated features, integers, steps, or components as referred to, but does not preclude the presence or addition of one or more features, integers, steps, or components, or groups thereof. Moreover, each of the terms "by", "comprising," "comprises", "comprised of," "including," "includes," "included," "involving," "involves," "involved," and "such as" are used in their open, non-limiting sense and may be used interchangeably. Further, the term "comprising" is intended to include examples and aspects encompassed by the terms "consisting essentially of" and "consisting of." Similarly, the term "consisting essentially of" is intended to include examples encompassed by the term "consisting of.

As used in the specification and the appended claims, the singular forms "a," "an" and "the" include plural referents unless the context clearly dictates otherwise. Thus, for example, reference to "a DHODH inhibitor," "a cancer," or "a pyrimidine nucleotide," includes, but is not limited to, combinations of two or more such DHODH inhibitors, cancers, or pyrimidine nucleotides, and the like.

It should be noted that ratios, concentrations, amounts, and other numerical data can be expressed herein in a range format. It will be further understood that the endpoints of each of the ranges are significant both in relation to the other endpoint, and independently of the other endpoint. It is also understood that there are a number of values disclosed herein, and that each value is also herein disclosed as "about" that particular value in addition to the value itself. For example, if the value "10" is disclosed, then "about 10" is also disclosed. Ranges can be expressed herein as from "about" one particular value, and/or to "about" another particular value. Similarly, when values are expressed as approximations, by use of the antecedent "about," it will be understood that the particular value forms a further aspect. For example, if the value "about 10" is disclosed, then "10" is also disclosed.

When a range is expressed, a further aspect includes from the one particular value and/or to the other particular value. For example, where the stated range includes one or both of the limits, ranges excluding either or both of those included limits are also included in the disclosure, e.g. the phrase "x to y" includes the range from 'x' to 'y' as well as the range greater than 'x' and less than 'y'. The range can also be expressed as an upper limit, e.g. 'about x, y, z, or less' and should be interpreted to include the specific ranges of 'about x', 'about y', and 'about z' as well as the ranges of 'less than x', less than y', and 'less than z'. Likewise, the phrase 'about x, y, z, or greater' should be interpreted to include the specific ranges of 'about x', 'about y', and 'about z' as well as the ranges of 'greater than x', greater than y', and 'greater than z'. In addition, the phrase "about 'x' to 'y'", where 'x' and 'y' are numerical values, includes "about 'x' to about 'y'".

It is to be understood that such a range format is used for convenience and brevity, and thus, should be interpreted in a flexible manner to include not only the numerical values explicitly recited as the limits of the range, but also to include all the individual numerical values or sub-ranges encompassed within that range as if each numerical value and sub-range is explicitly recited. To illustrate, a numerical range of "about 0.1% to 5%" should be interpreted to include not only the explicitly recited values of about 0.1% to about 5%, but also include individual values (e.g., about 1%, about 2%, about 3%, and about 4%) and the sub-ranges (e.g., about 0.5% to about 1.1%; about 5% to about 2.4%; about 0.5% to about 3.2%, and about 0.5% to about 4.4%, and other possible sub-ranges) within the indicated range.

As used herein, the terms "about," "approximate," "at or about," and "substantially" mean that the amount or value in question can be the exact value or a value that provides equivalent results or effects as recited in the claims or taught herein. That is, it is understood that amounts, sizes, formulations, parameters, and other quantities and characteristics are not and need not be exact, but may be approximate and/or larger or smaller, as desired, reflecting tolerances, conversion factors, rounding off, measurement error and the like, and other factors known to those of skill in the art such that equivalent results or effects are obtained. In some circumstances, the value that provides equivalent results or effects cannot be reasonably determined. In such cases, it is generally understood, as used herein, that "about" and "at or about" mean the nominal value indicated ±10% variation unless otherwise indicated or inferred. In general, an amount, size, formulation, parameter or other quantity or characteristic is "about," "approximate," or "at or about" whether or not expressly stated to be such. It is understood that where "about," "approximate," or "at or about" is used before a quantitative value, the parameter also includes the specific quantitative value itself, unless specifically stated otherwise.

As used herein, the terms "optional" or "optionally" means that the subsequently described event or circumstance can or cannot occur, and that the description includes instances where said event or circumstance occurs and instances where it does not.

As used herein, "dihydroorotate dehydrogenase" and "DHODH" can be used interchangeably, and refer to an enzyme encoded by a gene in humans with a cytogenetic location of 16q22.2 and a molecular location of base pairs 72,008,744 to 72,025,417 on chromosome 16 (Homo sapiens Annotation Release 109, GRCh38.p12). The gene structure in humans comprises 9 exons. DHODH has an EC classification of 1.3.1.1; an intracellular location within the mitochondria; and catalyzes the fourth enzymatic step in de novo pyrimidine biosynthesis. DHODH has also been referred to as DHOdehase; dihydroorotate dehydrogenase, mitochondrial; dihydroorotate dehydrogenase, mitochondrial precursor; dihydroorotate oxidase; human complement of yeast URA1; POADS; PYRD_HUMAN; and URA1.

The terms "inhibits", "inhibiting", or "inhibitor" of DHODH, as used herein, refer to inhibition of the enzyme DHODH, unless otherwise specified.

As used herein, "IC₅₀," is intended to refer to the concentration of a substance (e.g., a compound or a drug) that is required for 50% inhibition of a biological process, enzymatic reaction, or component of a biological or enzymatic process. For example, IC₅₀ refers to the half maximal (50%) inhibitory concentration (IC) of a substance as determined in a suitable assay. For example, an IC₅₀ for DHODH activity can be determined in an *in vitro* enzymatic assay using the methods described herein. Alternatively, an activity can be determined in a cell-based assay, including measurement of an activity or function associated with inhibition of the target process or enzyme. That is, DHODH activity can be indirectly determined in a cell-based assay of cell proliferation. It is believed that DHODH inhibition can lead to growth arrest or inhibition in suitable cell types. DHODH activity can be determined in a suitable cell, such as a primary AML cell or a AML cell-line, using an assay for cell-proliferation, such as an MTS assay as described herein, or a cell-colony forming assay as described herein. Suitable cell lines are described herein below.

As used herein, the term "immune" include cells of the immune system and cells that perform a function or activity in an immune response, such as, but not limited to, T-cells, B-cells, lymphocytes, macrophages, dendritic cells, neutrophils, eosinophils, basophils, mast cells, plasma cells, white blood cells, antigen presenting cells and natural killer cells.

As used herein, "brequinar" and "BQR," which can be used interchangeably, refer to the compound having a structure represented by the following formula: 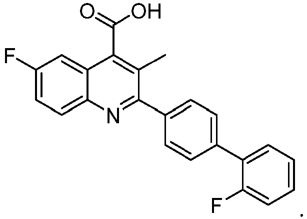 Brequinar can also be referred to by the IUPAC chemical name, or 6-fluoro-2-(2'-fluoro-1,1'-biphenyl-4-yl)-3-methyl-4-quinolinecarboxylic acid. Common salt forms are brequinar potassium and brequinar sodium (also referred to herein as BQR Na), which are the alkali metal salts of the conjugate base of the carboxylic acid. Brequinar is sometimes referred as DuP-785 or NSC-368390.

As used herein, "graft-versus-host-disease," "graft versus host disease," and GVHD can be used interchangeably, and refer to clinical complications following an allogeneic tissue transplant. It is commonly associated with stem cell or bone marrow transplant but the term also applies to other forms of tissue graft. Immune cells (white blood cells) in the tissue (the graft) recognize the recipient (the host) as "foreign". The transplanted immune cells then attack the host's body cells. GVHD can also occur after a blood transfusion if the blood products used have not been irradiated or treated with an approved pathogen reduction system.

As used herein, "administering" can refer to an administration that is oral, topical, intravenous, subcutaneous, transcutaneous, transdermal, intramuscular, intra-joint, parenteral, intra-arteriole, intradermal, intraventricular, intraosseous, intraocular, intracranial, intraperitoneal, intralesional, intranasal, intracardiac, intraarticular, intracavernous, intrathecal, intravireal, intracerebral, intracerebroventricular, intratympanic, intracochlear, rectal, vaginal, by inhalation, by catheters, stents or via an implanted reservoir or other device that administers, either actively or passively (e.g. by diffusion) a composition the perivascular space and adventitia. For example a medical device such as a stent can contain a composition or formulation disposed on its surface, which can then dissolve or be otherwise distributed to the surrounding tissue and cells. The term "parenteral" can include subcutaneous, intravenous, intramuscular, intra-articular, intra-synovial, intrasternal, intrathecal, intrahepatic, intralesional, and intracranial injections or infusion techniques. Administration can be continuous or intermittent. In various aspects, a preparation can be administered therapeutically; that is, administered to treat an existing disease or condition. In further various aspects, a preparation can be administered prophylactically; that is, administered for prevention of a disease or condition.

As used herein, "therapeutic agent" can refer to any substance, compound, molecule, and the like, which can be biologically active or otherwise can induce a pharmacologic, immunogenic, biologic and/or physiologic effect on a subject to which it is administered to by local and/or systemic action. A therapeutic agent can be a primary active agent, or in other words, the component(s) of a composition to which the whole or part of the effect of the composition is attributed. A therapeutic agent can be a secondary therapeutic agent, or in other words, the component(s) of a composition to which an additional part and/or other effect of the composition is attributed. The term therefore encompasses those compounds or chemicals traditionally regarded as drugs, vaccines, and biopharmaceuticals including molecules such as proteins, peptides, hormones, nucleic acids, gene constructs and the like. Examples of therapeutic agents are described in well-known literature references such as the Merck Index (14th edition), the Physicians' Desk Reference (64th edition), and The Pharmacological Basis of Therapeutics (12th edition), and they include, without limitation, medicaments; vitamins; mineral supplements; substances used for the treatment, prevention, diagnosis, cure or mitigation of a disease or illness; substances that affect the structure or function of the body, or pro-drugs, which become biologically active or more active after they have been placed in a physiological environment. For example, the term "therapeutic agent" includes compounds or compositions for use in all of the major therapeutic areas including, but not limited to, adjuvants; anti-infectives such as antibiotics and antiviral agents; analgesics and analgesic combinations, anorexics, anti-inflammatory agents, anti-epileptics, local and general anesthetics, hypnotics, sedatives, antipsychotic agents, neuroleptic agents, antidepressants, anxiolytics, antagonists, neuron blocking agents, anticholinergic and cholinomimetic agents, antimuscarinic and muscarinic agents, antiadrenergics, antiarrhythmics, antihypertensive agents, hormones, and nutrients, antiarthritics, antiasthmatic agents, anticonvulsants, antihistamines, antinauseants, antineoplastics, antipruritics, antipyretics; antispasmodics, cardiovascular preparations (including calcium channel blockers, beta-blockers, beta-agonists and antiarrythmics), antihypertensives, diuretics, vasodilators; central nervous system stimulants; cough and cold preparations; decongestants; diagnostics; hormones; bone growth stimulants and bone resorption inhibitors; immunosuppressives; muscle relaxants; psychostimulants; sedatives; tranquilizers; proteins, peptides, and fragments thereof (whether naturally occurring, chemically synthesized or recombinantly produced); and nucleic acid molecules (polymeric forms of two or more nucleotides, either ribonucleotides (RNA) or deoxyribonucleotides (DNA) including both double- and single-stranded molecules, gene constructs, expression vectors, antisense molecules and the like), small molecules (e.g., doxorubicin) and other biologically active macromolecules such as, for example, proteins and enzymes. The agent may be a biologically active agent used in medical, including veterinary, applications and in agriculture, such as with plants, as well as other areas. The term therapeutic agent also includes without limitation, medicaments; vitamins; mineral supplements; substances used for the treatment, prevention, diagnosis, cure or mitigation of disease or illness; or substances which affect the structure or function of the body; or pro- drugs, which become biologically active or more active after they have been placed in a predetermined physiological environment.

As used herein, "kit" means a collection of at least two components constituting the kit. Together, the components constitute a functional unit for a given purpose. Individual member components may be physically packaged together or separately. For example, a kit comprising an instruction for using the kit may or may not physically include the instruction with other individual member components. Instead, the instruction can be supplied as a separate member component, either in a paper form or an electronic form which may be supplied on computer readable memory device or downloaded from an internet website, or as recorded presentation.

As used herein, "instruction(s)" means documents describing relevant materials or methodologies pertaining to a kit. These materials may include any combination of the following: background information, list of components and their availability information (purchase information, etc.), brief or detailed protocols for using the kit, trouble-shooting, references, technical support, and any other related documents. Instructions can be supplied with the kit or as a separate member component, either as a paper form or an electronic form which may be supplied on computer readable memory device or downloaded from an internet website, or as recorded presentation. Instructions can comprise one or multiple documents and are meant to include future updates.

As used herein, "attached" can refer to covalent or non-covalent interaction between two or more molecules. Non-covalent interactions can include ionic bonds, electrostatic interactions, van der Waals forces, dipole-dipole interactions, dipole-induced-dipole interactions, London dispersion forces, hydrogen bonding, halogen bonding, electromagnetic interactions, π-π interactions, cation-π interactions, anion-π interactions, polar π-interactions, and hydrophobic effects.

As used interchangeably herein, "subject," "individual," or "patient" can refer to a vertebrate organism, such as a mammal (e.g. human). "Subject" can also refer to a cell, a population of cells, a tissue, an organ, or an organism, preferably to human and constituents thereof. It is understood that a vertebrate can be mammal, a fish, a bird, a reptile, or an amphibian. Thus, the subject of the herein disclosed methods can be a human, non-human primate, horse, pig, rabbit, dog, sheep, goat, cow, cat, guinea pig or rodent. The term does not denote a particular age or sex. Moreover, adult and newborn subjects, as well as fetuses, whether male or female, are intended to be covered. A patient refers to a subject afflicted with a clinical condition, disease or disorder. The term "patient" includes human and veterinary subjects.

As used herein, the terms "treating" and "treatment" can refer generally to obtaining a desired pharmacological and/or physiological effect. The effect can be, but does not necessarily have to be, prophylactic in terms of preventing or partially preventing a disease, symptom or condition thereof, such as a cancer, a disorder or disease associated with T-cell proliferation, or a graft-versus-host-disease. The effect can be therapeutic in terms of a partial or complete cure of a disease, condition, symptom or adverse effect attributed to the disease, disorder, or condition. The term "treatment" as used herein can include any treatment of a cancer, a disorder or disease associated with T-cell proliferation, or a graft-versus-host-disease in a subject, particularly a human and can include any one or more of the following: (a) preventing the disease from occurring in a subject which may be predisposed to the disease but has not yet been diagnosed as having it; (b) inhibiting the disease, i.e., arresting its development; and (c) relieving the disease, i.e., mitigating or ameliorating the disease and/or its symptoms or conditions. The term "treatment" as used herein can refer to both therapeutic treatment alone, prophylactic treatment alone, or both therapeutic and prophylactic treatment. Those in need of treatment (subjects in need thereof) can include those already with the disorder and/or those in which the disorder is to be prevented. As used herein, the term "treating", can include inhibiting the disease, disorder or condition, e.g., impeding its progress; and relieving the disease, disorder, or condition, e.g., causing regression of the disease, disorder and/or condition. Treating the disease, disorder, or condition can include ameliorating at least one symptom of the particular disease, disorder, or condition, even if the underlying pathophysiology is not affected, e.g., such as treating the pain of a subject by administration of an analgesic agent even though such agent does not treat the cause of the pain.

As used herein, "dose," "unit dose," or "dosage" can refer to physically discrete units suitable for use in a subject, each unit containing a predetermined quantity of a disclosed compound and/or a pharmaceutical composition thereof calculated to produce the desired response or responses in association with its administration.

As used herein, "therapeutic" can refer to treating, healing, and/or ameliorating a disease, disorder, condition, or side effect, or to decreasing in the rate of advancement of a disease, disorder, condition, or side effect.

As used herein, "effective amount" can refer to the amount of a disclosed compound or pharmaceutical composition provided herein that is sufficient to effect beneficial or desired biological, emotional, medical, or clinical response of a cell, tissue, system, animal, or human. An effective amount can be administered in one or more administrations, applications, or dosages. The term can also include within its scope amounts effective to enhance or restore to substantially normal physiological function.

As used herein, the term "therapeutically effective amount" refers to an amount that is sufficient to achieve the desired therapeutic result or to have an effect on undesired symptoms, but is generally insufficient to cause adverse side effects. The specific therapeutically effective dose level for any particular patient will depend upon a variety of factors including the disorder being treated and the severity of the disorder; the specific composition employed; the age, body weight, general health, sex and diet of the patient; the time of administration; the route of administration; the rate of excretion of the specific compound employed; the duration of the treatment; drugs used in combination or coincidental with the specific compound employed and like factors within the knowledge and expertise of the health practitioner and which may be well known in the medical arts. In the case of treating a particular disease or condition, in some instances, the desired response can be inhibiting the progression of the disease or condition. This may involve only slowing the progression of the disease temporarily. However, in other instances, it may be desirable to halt the progression of the disease permanently. This can be monitored by routine diagnostic methods known to one of ordinary skill in the art for any particular disease. The desired response to treatment of the disease or condition also can be delaying the onset or even preventing the onset of the disease or condition.

For example, it is well within the skill of the art to start doses of a compound at levels lower than those required to achieve the desired therapeutic effect and to gradually increase the dosage until the desired effect is achieved. If desired, the effective daily dose can be divided into multiple doses for purposes of administration. Consequently, single dose compositions can contain such amounts or submultiples thereof to make up the daily dose. The dosage can be adjusted by the individual physician in the event of any contraindications. It is generally preferred that a maximum dose of the pharmacological agents of the disclosure (alone or in combination with other therapeutic agents) be used, that is, the highest safe dose according to sound medical judgment. It will be understood by those of ordinary skill in the art however, that a patient may insist upon a lower dose or tolerable dose for medical reasons, psychological reasons or for virtually any other reasons.

A response to a therapeutically effective dose of a disclosed compound and/or pharmaceutical composition, for example, can be measured by determining the physiological effects of the treatment or medication, such as the decrease or lack of disease symptoms following administration of the treatment or pharmacological agent. Other assays will be known to one of ordinary skill in the art and can be employed for measuring the level of the response. The amount of a treatment may be varied for example by increasing or decreasing the amount of a disclosed compound and/or pharmaceutical composition, by changing the disclosed compound and/or pharmaceutical composition administered, by changing the route of administration, by changing the dosage timing and so on. Dosage can vary, and can be administered in one or more dose administrations daily, for one or several days. Guidance can be found in the literature for appropriate dosages for given classes of pharmaceutical products.

In the present disclosure, it is understood that in some cases, an effective amount or dose of a disclosed compound is the amount of the composition that is capable of inhibiting DHODH to provide a clinically meaningful decrease in the signs, symptoms, or causes of a disease, or any other desired alteration of a biological system, as a result of inhibiting DHODH. For example, an "effective amount" for therapeutic uses. In some aspects, an appropriate "effective" amount in any individual case is determined using techniques, such as a dose escalation study.

As used herein, the term "prophylactically effective amount" refers to an amount effective for preventing onset or initiation of a disease or condition.

As used herein, the term "prevent" or "preventing" refers to precluding, averting, obviating, forestalling, stopping, or hindering something from happening, especially by advance action. It is understood that where reduce, inhibit or prevent are used herein, unless specifically indicated otherwise, the use of the other two words is also expressly disclosed.

The term "pharmaceutically acceptable" describes a material that is not biologically or otherwise undesirable, *i.e.,* without causing an unacceptable level of undesirable biological effects or interacting in a deleterious manner.

The term "pharmaceutically acceptable salts", as used herein, means salts of the active principal agents which are prepared with acids or bases that are tolerated by a biological system or tolerated by a subject or tolerated by a biological system and tolerated by a subject when administered in a therapeutically effective amount. When compounds of the present disclosure contain relatively acidic functionalities, base addition salts can be obtained by contacting the neutral form of such compounds with a sufficient amount of the desired base, either neat or in a suitable inert solvent. Examples of pharmaceutically acceptable base addition salts include, but are not limited to; sodium, potassium, calcium, ammonium, organic amino, magnesium salt, lithium salt, strontium salt or a similar salt. When compounds of the present disclosure contain relatively basic functionalities, acid addition salts can be obtained by contacting the neutral form of such compounds with a sufficient amount of the desired acid, either neat or in a suitable inert solvent. Examples of pharmaceutically acceptable acid addition salts include, but are not limited to; those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydroiodic, or phosphorous acids and the like, as well as the salts derived from relatively nontoxic organic acids like acetic, propionic, isobutyric, maleic, malonic, benzoic, succinic, suberic, fumaric, lactic, mandelic, phthalic, benzenesulfonic, p-tolylsulfonic, citric, tartaric, methanesulfonic, and the like. Also included are salts of amino acids such as arginate and the like, and salts of organic acids like glucuronic or galactunoric acids and the like.

The term "pharmaceutically acceptable ester" refers to esters of compounds of the present disclosure which hydrolyze in vivo and include those that break down readily in the human body to leave the parent compound or a salt thereof. Examples of pharmaceutically acceptable, non-toxic esters of the present disclosure include C 1 -to-C 6 alkyl esters and C 5 -to-C 7 cycloalkyl esters, although C 1 -to-C 4 alkyl esters are preferred. Esters of disclosed compounds can be prepared according to conventional methods. Pharmaceutically acceptable esters can be appended onto hydroxy groups by reaction of the compound that contains the hydroxy group with acid and an alkylcarboxylic acid such as acetic acid, or with acid and an arylcarboxylic acid such as benzoic acid. In the case of compounds containing carboxylic acid groups, the pharmaceutically acceptable esters are prepared from compounds containing the carboxylic acid groups by reaction of the compound with base such as triethylamine and an alkyl halide, for example with methyl iodide, benzyl iodide, cyclopentyl iodide or alkyl triflate. They also can be prepared by reaction of the compound with an acid such as hydrochloric acid and an alcohol such as ethanol or methanol.

The term "pharmaceutically acceptable amide" refers to non-toxic amides of the present disclosure derived from ammonia, primary C 1 -to-C 6 alkyl amines and secondary C 1 -to-C 6 dialkyl amines. In the case of secondary amines, the amine can also be in the form of a 5- or 6-membered heterocycle containing one nitrogen atom. Amides derived from ammonia, C 1 -to-C 3 alkyl primary amides and C 1 -to-C 2 dialkyl secondary amides are preferred. Amides of disclosed compounds can be prepared according to conventional methods. Pharmaceutically acceptable amides can be prepared from compounds containing primary or secondary amine groups by reaction of the compound that contains the amino group with an alkyl anhydride, aryl anhydride, acyl halide, or aroyl halide. In the case of compounds containing carboxylic acid groups, the pharmaceutically acceptable amides are prepared from compounds containing the carboxylic acid groups by reaction of the compound with base such as triethylamine, a dehydrating agent such as dicyclohexyl carbodiimide or carbonyl diimidazole, and an alkyl amine, dialkylamine, for example with methylamine, diethylamine, and piperidine. They also can be prepared by reaction of the compound with an acid such as sulfuric acid and an alkylcarboxylic acid such as acetic acid, or with acid and an arylcarboxylic acid such as benzoic acid under dehydrating conditions such as with molecular sieves added. The composition can contain a compound of the present disclosure in the form of a pharmaceutically acceptable prodrug.

The term "pharmaceutically acceptable prodrug" or "prodrug" represents those prodrugs of the compounds of the present disclosure which are, within the scope of sound medical judgment, suitable for use in contact with the tissues of humans and lower animals without undue toxicity, irritation, allergic response, and the like, commensurate with a reasonable benefit/risk ratio, and effective for their intended use. Prodrugs of the present disclosure can be rapidly transformed in vivo to a parent compound having a structure of a disclosed compound, for example, by hydrolysis in blood. A thorough discussion is provided in T. Higuchi and V. Stella, Pro-drugs as Novel Delivery Systems, V. 14 of the A.C.S. Symposium Series, and in Edward B. Roche, ed., Bioreversible Carriers in Drug Design, American Pharmaceutical Association and Pergamon Press (1987).

The term "contacting" as used herein refers to bringing a disclosed compound or pharmaceutical composition in proximity to a cell, a target protein, or other biological entity together in such a manner that the disclosed compound or pharmaceutical composition can affect the activity of the a cell, target protein, or other biological entity, either directly; *i.e.,* by interacting with the cell, target protein, or other biological entity itself, or indirectly; *i.e.,* by interacting with another molecule, co-factor, factor, or protein on which the activity of the cell, target protein, or other biological entity itself is dependent.

It is understood, that unless otherwise specified, temperatures referred to herein are based on atmospheric pressure (i.e. one atmosphere).

As used herein, nomenclature for compounds, including organic compounds, can be given using common names, IUPAC, IUBMB, or CAS recommendations for nomenclature. When one or more stereochemical features are present, Cahn-Ingold-Prelog rules for stereochemistry can be employed to designate stereochemical priority, *E*/*Z*specification, and the like. One of skill in the art can readily ascertain the structure of a compound if given a name, either by systemic reduction of the compound structure using naming conventions, or by commercially available software, such as CHEMDRAW^{™} (Cambridgesoft Corporation, U.S.A.).

As used herein, the term "substituted" is contemplated to include all permissible substituents of organic compounds. In a broad aspect, the permissible substituents include acyclic and cyclic, branched and unbranched, carbocyclic and heterocyclic, and aromatic and nonaromatic substituents of organic compounds. Illustrative substituents include, for example, those described below. The permissible substituents can be one or more and the same or different for appropriate organic compounds. For purposes of this disclosure, the heteroatoms, such as nitrogen, can have hydrogen substituents and/or any permissible substituents of organic compounds described herein which satisfy the valences of the heteroatoms. This disclosure is not intended to be limited in any manner by the permissible substituents of organic compounds. Also, the terms "substitution" or "substituted with" include the implicit proviso that such substitution is in accordance with permitted valence of the substituted atom and the substituent, and that the substitution results in a stable compound, e.g., a compound that does not spontaneously undergo transformation such as by rearrangement, cyclization, elimination, etc. It is also contemplated that, in certain aspects, unless expressly indicated to the contrary, individual substituents can be further optionally substituted *(i.e.,* further substituted or unsubstituted).

In defining various terms, "A¹," "A²," "A³," and "A⁴" are used herein as generic symbols to represent various specific substituents. Similarly, "Ar¹," "Ar²," "Ar³," and "Ar⁴" are used herein as generic symbols to represent various specific aryl substituents. These symbols can be any substituent, not limited to those disclosed herein, and when they are defined to be certain substituents in one instance, they can, in another instance, be defined as some other substituents.

The term "aliphatic" or "aliphatic group," as used herein, denotes a hydrocarbon moiety that may be straight-chain *(i.e.,* unbranched), branched, or cyclic (including fused, bridging, and spirofused polycyclic) and may be completely saturated or may contain one or more units of unsaturation, but which is not aromatic. Unless otherwise specified, aliphatic groups contain 1-20 carbon atoms. Aliphatic groups include, but are not limited to, linear or branched, alkyl, alkenyl, and alkynyl groups, and hybrids thereof such as (cycloalkyl)alkyl, (cycloalkenyl)alkyl or (cycloalkyl)alkenyl.

The term "alkyl" as used herein is a branched or unbranched saturated hydrocarbon group of 1 to 24 carbon atoms, such as methyl, ethyl, *n*-propyl, isopropyl, *n*-butyl, isobutyl, *s-*butyl, *t*-butyl, *n*-pentyl, isopentyl, *s*-pentyl, neopentyl, hexyl, heptyl, octyl, nonyl, decyl, dodecyl, tetradecyl, hexadecyl, eicosyl, tetracosyl, and the like. The alkyl group can be cyclic or acyclic. The alkyl group can be branched or unbranched. The alkyl group can also be substituted or unsubstituted. For example, the alkyl group can be substituted with one or more groups including, but not limited to, alkyl, cycloalkyl, alkoxy, amino, ether, halide, hydroxy, nitro, silyl, sulfo-oxo, or thiol, as described herein. A "lower alkyl" group is an alkyl group containing from one to six (*e.g*., from one to four) carbon atoms. The term alkyl group can also be a C1 alkyl, C1-C2 alkyl, C1-C3 alkyl, C1-C4 alkyl, C1-C5 alkyl, C1-C6 alkyl, C1-C7 alkyl, C1-C8 alkyl, C1-C9 alkyl, C1-C10 alkyl, and the like up to and including a C1-C24 alkyl.

Throughout the specification "alkyl" is generally used to refer to both unsubstituted alkyl groups and substituted alkyl groups; however, substituted alkyl groups are also specifically referred to herein by identifying the specific substituent(s) on the alkyl group. For example, the term "halogenated alkyl" or "haloalkyl" specifically refers to an alkyl group that is substituted with one or more halide, *e.g.,* fluorine, chlorine, bromine, or iodine. Alternatively, the term "monohaloalkyl" specifically refers to an alkyl group that is substituted with a single halide, *e.g.* fluorine, chlorine, bromine, or iodine. The term "polyhaloalkyl" specifically refers to an alkyl group that is independently substituted with two or more halides, *i.e.* each halide substituent need not be the same halide as another halide substituent, nor do the multiple instances of a halide substituent need to be on the same carbon. The term "alkoxyalkyl" specifically refers to an alkyl group that is substituted with one or more alkoxy groups, as described below. The term "aminoalkyl" specifically refers to an alkyl group that is substituted with one or more amino groups. The term "hydroxyalkyl" specifically refers to an alkyl group that is substituted with one or more hydroxy groups. When "alkyl" is used in one instance and a specific term such as "hydroxyalkyl" is used in another, it is not meant to imply that the term "alkyl" does not also refer to specific terms such as "hydroxyalkyl" and the like.

As used herein "aminoalkyl" refers to a straight or branched chain alkyl group in which at least one hydrogen is replaced with an amino group, generally 1-3 amino groups. Non-limiting examples of aminoalkyl groups include -CH₂NH₂, -(CH₂)₂NH₂, -CHCH₃NH₂, -(CH₂)₂CHCH₃NH₂, -(CH₂)₂CHNH₂CH₂CH₃, -CHCH₃(CH₂)₂NH₂, and the like.

As used herein "alkylamino" refers to an amino group have at least one hydrogen replaced with an alkyl group. Thus, alkylamino refers to the group -NR^{a}R^{a}, wherein R^{a} and R^{b} are independently selected form H and alkyl, provided at least one of R^{a} or R^{b} is an alkyl. Non-limiting examples of alkylamino groups include -NHCH₃, -NHCH₂CH₃, -NH(CH₂)₂CH₃, -N(CH₃)₂, -N(CH₃)CH₂CH₃, -N(CH₃)(CH₂)₂CH₃, and the like.

As used herein "hydroxyalkyl" refers to a straight or branched chain alkyl group in which at least one hydrogen is replaced with an hydroxy group, generally 1-3 hydroxy groups. Non-limiting examples of hydroxyalkyl groups include -CH₂OH, -(CH₂)₂OH, -CHCH₃OH, -(CH₂)₂CHCH₃OH, -(CH₂)₂CHOHCH₂CH₃, -CHCH₃(CH₂)₂OH, and the like.

The term "alkanediyl", as used herein, unless otherwise indicated, means bivalent straight and branched chained saturated hydrocarbon radicals having carbon atoms. For example, "C1-C6 alkanediyl" would refer to bivalent straight and branched chained saturated hydrocarbon radicals having 1 to 6 carbon atoms, such as, for example, methylene, 1,2-ethanediyl (-CH₂CH₂-), propanediyl or 1,3-propanediyl (-(CH₂)₃-), butanediyl or 1,4-butanediyl (-(CH₂)₄-), pentanediyl or 1,5-pentanediyl (-(CH₂)₅-), hexanediyl or 1,6-hexanediyl (-(CH₂)₆-) and the branched isomers thereof (e.g., isopropanediyl (-CHCH₃CH₂-)). Alkanediyl groups can be further substituted, e.g., aminoalkanediyl or hydroxyalkanediyl.

As used herein, "aminoalkanediyl" refers to a straight or branched chain alkanediyl group in which at least one hydrogen is replaced with an amino group, generally 1-3 amino groups. Non-limiting examples of aminoalkanediyl groups include -CH₂NH-, -(CH₂)₂NH-, -CHCH₃NH-, -(CH₂)₂CHCH₃NH-, -(CH₂)₂CHNH₂(CH₂)₂-, -CH₂CHNH₂(CH₂)₂-, -CH₂NH(CH₂)₂-, -(CH₂)₂NH(CH₂)₂-, -CHCH₃(CH₂)₂NH-, and the like.

As used herein, "hydroxyalkanediyl" refers to a straight or branched chain alkanediyl group in which at least one hydrogen is replaced with a hydroxy group, generally 1-3 hydroxy groups. Non-limiting examples of hydroxyalkanediyl groups include-CHOH-, -CH₂CHOH-, -CCH₃OH-, -(CH₂)₂CCH₃OH-, -(CH₂)₂CHOH(CH₂)₂-, -CH₂CHOH(CH₂)₂-, -CHOH(CH₂)₂-, -CH₂CHOH(CH₂)₂-, -CHCH₃CH₂CHOH-, and the like.

The terms "alkoxy" and "alkoxyl" as used herein to refer to an alkyl or cycloalkyl group bonded through an ether linkage; that is, an "alkoxy" group can be defined as -OA¹ where A¹ is alkyl or cycloalkyl as defined above. "Alkoxy" also includes polymers of alkoxy groups as just described; that is, an alkoxy can be a polyether such as -OA¹-OA² or -OA¹-(OA²)ₐ-OA³, where "a" is an integer of from 1 to 200 and A¹, A², and A³ are alkyl and/or cycloalkyl groups.

The term "aromatic group" as used herein refers to a ring structure having cyclic clouds of delocalized π electrons above and below the plane of the molecule, where the π clouds contain (4n+2) π electrons. A further discussion of aromaticity is found in Morrison and Boyd, Organic Chemistry, (5th Ed., 1987), Chapter 13, entitled "Aromaticity," pages 477-497, incorporated herein by reference. The term "aromatic group" is inclusive of both aryl and heteroaryl groups.

The term "aryl" as used herein is a group that contains any carbon-based aromatic group including, but not limited to, benzene, naphthalene, phenyl, biphenyl, anthracene, and the like. The aryl group can be substituted or unsubstituted. The aryl group can be substituted with one or more groups including, but not limited to, alkyl, cycloalkyl, alkoxy, alkenyl, cycloalkenyl, alkynyl, cycloalkynyl, aryl, heteroaryl, aldehyde, -NH₂, carboxylic acid, ester, ether, halide, hydroxy, ketone, azide, nitro, silyl, sulfo-oxo, or thiol as described herein. The term "biaryl" is a specific type of aryl group and is included in the definition of "aryl." In addition, the aryl group can be a single ring structure or comprise multiple ring structures that are either fused ring structures or attached via one or more bridging groups such as a carbon-carbon bond. For example, biaryl to two aryl groups that are bound together via a fused ring structure, as in naphthalene, or are attached via one or more carbon-carbon bonds, as in biphenyl.

The term "cycloalkyl" as used herein is a non-aromatic carbon-based ring composed of at least three carbon atoms. Examples of cycloalkyl groups include, but are not limited to, cyclopropyl, cyclobutyl, cyclopentyl, cyclohexyl, norbornyl, and the like. The term "heterocycloalkyl" is a type of cycloalkyl group as defined above, and is included within the meaning of the term "cycloalkyl," where at least one of the carbon atoms of the ring is replaced with a heteroatom such as, but not limited to, nitrogen, oxygen, sulfur, or phosphorus. The cycloalkyl group and heterocycloalkyl group can be substituted or unsubstituted. The cycloalkyl group and heterocycloalkyl group can be substituted with one or more groups including, but not limited to, alkyl, cycloalkyl, alkoxy, amino, ether, halide, hydroxy, nitro, silyl, sulfo-oxo, or thiol as described herein.

The term "heteroalkyl" as used herein refers to an alkyl group containing at least one heteroatom. Suitable heteroatoms include, but are not limited to, O, N, Si, P and S, wherein the nitrogen, phosphorous and sulfur atoms are optionally oxidized, and the nitrogen heteroatom is optionally quaternized. Heteroalkyls can be substituted as defined above for alkyl groups.

The term "heteroaryl" as used herein refers to an aromatic group that has at least one heteroatom incorporated within the ring of the aromatic group. Examples of heteroatoms include, but are not limited to, nitrogen, oxygen, sulfur, and phosphorus, where N-oxides, sulfur oxides, and dioxides are permissible heteroatom substitutions. The heteroaryl group can be substituted or unsubstituted. The heteroaryl group can be substituted with one or more groups including, but not limited to, alkyl, cycloalkyl, alkoxy, amino, ether, halide, hydroxy, nitro, silyl, sulfo-oxo, or thiol as described herein. Heteroaryl groups can be monocyclic, or alternatively fused ring systems. Heteroaryl groups include, but are not limited to, furyl, imidazolyl, pyrimidinyl, tetrazolyl, thienyl, pyridinyl, pyrrolyl, N-methylpyrrolyl, quinolinyl, isoquinolinyl, pyrazolyl, triazolyl, thiazolyl, oxazolyl, isoxazolyl, oxadiazolyl, thiadiazolyl, isothiazolyl, pyridazinyl, pyrazinyl, benzofuranyl, benzodioxolyl, benzothiophenyl, indolyl, indazolyl, benzimidazolyl, imidazopyridinyl, pyrazolopyridinyl, and pyrazolopyrimidinyl. Further not limiting examples of heteroaryl groups include, but are not limited to, pyridinyl, pyridazinyl, pyrimidinyl, pyrazinyl, thiophenyl, pyrazolyl, imidazolyl, benzo[*d*]oxazolyl, benzo[d]thiazolyl, quinolinyl, quinazolinyl, indazolyl, imidazo[1,2-b]pyridazinyl, imidazo[1,2-a]pyrazinyl, benzo[c][1,2,5]thiadiazolyl, benzo[c][1,2,5]oxadiazolyl, and pyrido[2,3-b]pyrazinyl.

The term "heterocycle" as used herein can be used interchangeably and refer to single and multi-cyclic aromatic or non-aromatic ring systems in which at least one of the ring members is other than carbon. Thus, the term is inclusive of, but not limited to, "heterocycloalkyl," "heteroaryl," "bicyclic heterocycle," and "polycyclic heterocycle." Heterocycle includes pyridine, pyrimidine, furan, thiophene, pyrrole, isoxazole, isothiazole, pyrazole, oxazole, thiazole, imidazole, oxazole, including, 1,2,3-oxadiazole, 1,2,5-oxadiazole and 1,3,4-oxadiazole, thiadiazole, including, 1,2,3-thiadiazole, 1,2,5-thiadiazole, and 1,3,4-thiadiazole, triazole, including, 1,2,3-triazole, 1,3,4-triazole, tetrazole, including 1,2,3,4-tetrazole and 1,2,4,5-tetrazole, pyridazine, pyrazine, triazine, including 1,2,4-triazine and 1,3,5-triazine, tetrazine, including 1,2,4,5-tetrazine, pyrrolidine, piperidine, piperazine, morpholine, azetidine, tetrahydropyran, tetrahydrofuran, dioxane, and the like. The term heterocyclyl group can also be a C2 heterocyclyl, C2-C3 heterocyclyl, C2-C4 heterocyclyl, C2-C5 heterocyclyl, C2-C6 heterocyclyl, C2-C7 heterocyclyl, C2-C8 heterocyclyl, C2-C9 heterocyclyl, C2-C10 heterocyclyl, C2-C11 heterocyclyl, and the like up to and including a C2-C18 heterocyclyl. For example, a C2 heterocyclyl comprises a group which has two carbon atoms and at least one heteroatom, including, but not limited to, aziridinyl, diazetidinyl, dihydrodiazetyl, oxiranyl, thiiranyl, and the like. Alternatively, for example, a C5 heterocyclyl comprises a group which has five carbon atoms and at least one heteroatom, including, but not limited to, piperidinyl, tetrahydropyranyl, tetrahydrothiopyranyl, diazepanyl, pyridinyl, and the like. It is understood that a heterocyclyl group may be bound either through a heteroatom in the ring, where chemically possible, or one of carbons comprising the heterocyclyl ring.

The term "bicyclic heterocycle" as used herein refers to a ring system in which at least one of the ring members is other than carbon. Bicyclic heterocyclyl encompasses ring systems wherein an aromatic ring is fused with another aromatic ring, or wherein an aromatic ring is fused with a non-aromatic ring. Bicyclic heterocyclyl encompasses ring systems wherein a benzene ring is fused to a 5- or a 6-membered ring containing 1, 2 or 3 ring heteroatoms or wherein a pyridine ring is fused to a 5- or a 6-membered ring containing 1, 2 or 3 ring heteroatoms. Bicyclic heterocyclic groups include, but are not limited to, indolyl, indazolyl, pyrazolo[1,5-a]pyridinyl, benzofuranyl, quinolinyl, quinoxalinyl, 1,3-benzodioxolyl, 2,3-dihydro-1,4-benzodioxinyl, 3,4-dihydro-2H-chromenyl, 1H-pyrazolo[4,3-c]pyridin-3-yl; 1H-pyrrolo[3,2-b]pyridin-3-yl; and 1H-pyrazolo[3,2-b]pyridin-3-yl.

The term "heterocycloalkyl" as used herein refers to an aliphatic, partially unsaturated or fully saturated, 3- to 14-membered ring system, including single rings of 3 to 8 atoms and bi- and tricyclic ring systems. The heterocycloalkyl ring-systems include one to four heteroatoms independently selected from oxygen, nitrogen, and sulfur, wherein a nitrogen and sulfur heteroatom optionally can be oxidized and a nitrogen heteroatom optionally can be substituted. Representative heterocycloalkyl groups include, but are not limited to, pyrrolidinyl, pyrazolinyl, pyrazolidinyl, imidazolinyl, imidazolidinyl, piperidinyl, piperazinyl, oxazolidinyl, isoxazolidinyl, morpholinyl, thiazolidinyl, isothiazolidinyl, and tetrahydrofuryl.

The terms "amine" or "amino" as used herein are represented by the formula -NA¹A², where A¹ and A² can be, independently, hydrogen or alkyl, cycloalkyl, alkenyl, cycloalkenyl, alkynyl, cycloalkynyl, aryl, or heteroaryl group as described herein. A specific example of amino is -NH₂.

The term "carboxylic acid" as used herein is represented by the formula -C(O)OH.

The terms "halo," "halogen" or "halide," as used herein can be used interchangeably and refer to F, CI, Br, or I.

The term "hydroxyl" or "hydroxy" as used herein is represented by the formula -OH.

The term "nitro" as used herein is represented by the formula -NO₂.

The term "nitrile" or "cyano" as used herein is represented by the formula -CN.

"R¹," "R²," "R³," . . . "Rⁿ," where n is an integer, as used herein can, independently, possess one or more of the groups listed above. For example, if R¹ is a straight chain alkyl group, one of the hydrogen atoms of the alkyl group can optionally be substituted with a hydroxyl group, an alkoxy group, an alkyl group, a halide, and the like. Depending upon the groups that are selected, a first group can be incorporated within second group or, alternatively, the first group can be pendant (*i.e.,* attached) to the second group. For example, with the phrase "an alkyl group comprising an amino group," the amino group can be incorporated within the backbone of the alkyl group. Alternatively, the amino group can be attached to the backbone of the alkyl group. The nature of the group(s) that is (are) selected will determine if the first group is embedded or attached to the second group.

As described herein, compounds of the disclosure may contain "optionally substituted" moieties. In general, the term "substituted," whether preceded by the term "optionally" or not, means that one or more hydrogens of the designated moiety are replaced with a suitable substituent. Unless otherwise indicated, an "optionally substituted" group may have a suitable substituent at each substitutable position of the group, and when more than one position in any given structure may be substituted with more than one substituent selected from a specified group, the substituent may be either the same or different at every position. Combinations of substituents envisioned by this disclosure are preferably those that result in the formation of stable or chemically feasible compounds. In is also contemplated that, in certain aspects, unless expressly indicated to the contrary, individual substituents can be further optionally substituted (*i.e.,* further substituted or unsubstituted).

The term "stable," as used herein, refers to compounds that are not substantially altered when subjected to conditions to allow for their production, detection, and, in certain aspects, their recovery, purification, and use for one or more of the purposes disclosed herein.

The term "organic residue" defines a carbon containing residue, *i.e.,* a residue comprising at least one carbon atom, and includes but is not limited to the carbon-containing groups, residues, or radicals defined hereinabove. Organic residues can contain various heteroatoms, or be bonded to another molecule through a heteroatom, including oxygen, nitrogen, sulfur, phosphorus, or the like. Examples of organic residues include but are not limited alkyl or substituted alkyls, alkoxy or substituted alkoxy, mono or di-substituted amino, amide groups, etc. Organic residues can preferably comprise 1 to 18 carbon atoms, 1 to 15, carbon atoms, 1 to 12 carbon atoms, 1 to 8 carbon atoms, 1 to 6 carbon atoms, or 1 to 4 carbon atoms. In a further aspect, an organic residue can comprise 2 to 18 carbon atoms, 2 to 15, carbon atoms, 2 to 12 carbon atoms, 2 to 8 carbon atoms, 2 to 4 carbon atoms, or 2 to 4 carbon atoms.

A very close synonym of the term "residue" is the term "radical," which as used in the specification and concluding claims, refers to a fragment, group, or substructure of a molecule described herein, regardless of how the molecule is prepared. For example, a 2,4-thiazolidinedione radical in a particular compound has the structure: 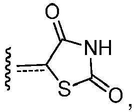 regardless of whether thiazolidinedione is used to prepare the compound. In some embodiments the radical (for example an alkyl) can be further modified *(i.e.,* substituted alkyl) by having bonded thereto one or more "substituent radicals." The number of atoms in a given radical is not critical to the present disclosure unless it is indicated to the contrary elsewhere herein.

"Organic radicals," as the term is defined and used herein, contain one or more carbon atoms. An organic radical can have, for example, 1-26 carbon atoms, 1-18 carbon atoms, 1-12 carbon atoms, 1-8 carbon atoms, 1-6 carbon atoms, or 1-4 carbon atoms. In a further aspect, an organic radical can have 2-26 carbon atoms, 2-18 carbon atoms, 2-12 carbon atoms, 2-8 carbon atoms, 2-6 carbon atoms, or 2-4 carbon atoms. Organic radicals often have hydrogen bound to at least some of the carbon atoms of the organic radical. One example, of an organic radical that comprises no inorganic atoms is a 5, 6, 7, 8-tetrahydro-2-naphthyl radical. In some embodiments, an organic radical can contain 1-10 inorganic heteroatoms bound thereto or therein, including halogens, oxygen, sulfur, nitrogen, phosphorus, and the like. Examples of organic radicals include but are not limited to an alkyl, substituted alkyl, cycloalkyl, substituted cycloalkyl, mono-substituted amino, di-substituted amino, acyloxy, cyano, carboxy, carboalkoxy, alkylcarboxamide, substituted alkylcarboxamide, dialkylcarboxamide, substituted dialkylcarboxamide, alkylsulfonyl, alkylsulfonyl, thioalkyl, thiohaloalkyl, alkoxy, substituted alkoxy, haloalkyl, haloalkoxy, aryl, substituted aryl, heteroaryl, heterocyclic, or substituted heterocyclic radicals, wherein the terms are defined elsewhere herein. A few non-limiting examples of organic radicals that include heteroatoms include alkoxy radicals, trifluoromethoxy radicals, acetoxy radicals, dimethylamino radicals and the like.

"Inorganic radicals," as the term is defined and used herein, contain no carbon atoms and therefore comprise only atoms other than carbon. Inorganic radicals comprise bonded combinations of atoms selected from hydrogen, nitrogen, oxygen, silicon, phosphorus, sulfur, selenium, and halogens such as fluorine, chlorine, bromine, and iodine, which can be present individually or bonded together in their chemically stable combinations. Inorganic radicals have 10 or fewer, or preferably one to six or one to four inorganic atoms as listed above bonded together. Examples of inorganic radicals include, but not limited to, amino, hydroxy, halogens, nitro, thiol, sulfate, phosphate, and like commonly known inorganic radicals. The inorganic radicals do not have bonded therein the metallic elements of the periodic table (such as the alkali metals, alkaline earth metals, transition metals, lanthanide metals, or actinide metals), although such metal ions can sometimes serve as a pharmaceutically acceptable cation for anionic inorganic radicals such as a sulfate, phosphate, or like anionic inorganic radical. Inorganic radicals do not comprise metalloids elements such as boron, aluminum, gallium, germanium, arsenic, tin, lead, or tellurium, or the noble gas elements, unless otherwise specifically indicated elsewhere herein.

As used herein, the term "derivative" refers to a compound having a structure derived from the structure of a parent compound (*e.g.,* a compound disclosed herein) and whose structure is sufficiently similar to those disclosed herein and based upon that similarity, would be expected by one skilled in the art to exhibit the same or similar activities and utilities as the claimed compounds, or to induce, as a precursor, the same or similar activities and utilities as the claimed compounds. Exemplary derivatives include salts, esters, amides, salts of esters or amides, and N-oxides of a parent compound.

Compounds described herein can contain one or more double bonds and, thus, potentially give rise to cis/trans (E/Z) isomers, as well as other conformational isomers. Unless stated to the contrary, the disclosure includes all such possible isomers, as well as mixtures of such isomers.

Unless stated to the contrary, a formula with chemical bonds shown only as solid lines and not as wedges or dashed lines contemplates each possible isomer, *e.g.,* each enantiomer and diastereomer, and a mixture of isomers, such as a racemic or scalemic mixture. Compounds described herein can contain one or more asymmetric centers and, thus, potentially give rise to diastereomers and optical isomers. Unless stated to the contrary, the present disclosure includes all such possible diastereomers as well as their racemic mixtures, their substantially pure resolved enantiomers, all possible geometric isomers, and pharmaceutically acceptable salts thereof. Mixtures of stereoisomers, as well as isolated specific stereoisomers, are also included. During the course of the synthetic procedures used to prepare such compounds, or in using racemization or epimerization procedures known to those skilled in the art, the products of such procedures can be a mixture of stereoisomers.

Many organic compounds exist in optically active forms having the ability to rotate the plane of plane-polarized light. In describing an optically active compound, the prefixes D and L or R and S are used to denote the absolute configuration of the molecule about its chiral center(s). The prefixes d and I or (+) and (-) are employed to designate the sign of rotation of plane-polarized light by the compound, with (-) or meaning that the compound is levorotatory. A compound prefixed with (+) or d is dextrorotatory. For a given chemical structure, these compounds, called stereoisomers, are identical except that they are non-superimposable mirror images of one another. A specific stereoisomer can also be referred to as an enantiomer, and a mixture of such isomers is often called an enantiomeric mixture. A 50:50 mixture of enantiomers is referred to as a racemic mixture. Many of the compounds described herein can have one or more chiral centers and therefore can exist in different enantiomeric forms. If desired, a chiral carbon can be designated with an asterisk (*). When bonds to the chiral carbon are depicted as straight lines in the disclosed formulas, it is understood that both the (R) and (S) configurations of the chiral carbon, and hence both enantiomers and mixtures thereof, are embraced within the formula. As is used in the art, when it is desired to specify the absolute configuration about a chiral carbon, one of the bonds to the chiral carbon can be depicted as a wedge (bonds to atoms above the plane) and the other can be depicted as a series or wedge of short parallel lines is (bonds to atoms below the plane). The Cahn-Ingold-Prelog system can be used to assign the (R) or (S) configuration to a chiral carbon.

Compounds described herein comprise atoms in both their natural isotopic abundance and in non-natural abundance. The disclosed compounds can be isotopically-labeled or isotopically-substituted compounds identical to those described, but for the fact that one or more atoms are replaced by an atom having an atomic mass or mass number different from the atomic mass or mass number typically found in nature. Examples of isotopes that can be incorporated into compounds of the disclosure include isotopes of hydrogen, carbon, nitrogen, oxygen, sulfur, fluorine and chlorine, such as ²H, ³H, ¹³C, ¹⁴C, ¹⁵N, ¹⁸O, ¹⁷O, ³⁵S, ¹⁸F, and ³⁶Cl, respectively. Compounds further comprise prodrugs thereof and pharmaceutically acceptable salts of said compounds or of said prodrugs which contain the aforementioned isotopes and/or other isotopes of other atoms are within the scope of this disclosure. Certain isotopically-labeled compounds of the present disclosure, for example those into which radioactive isotopes such as ³H and ¹⁴C are incorporated, are useful in drug and/or substrate tissue distribution assays. Tritiated, *i.e.,* ³H, and carbon-14, *i.e.,* ¹⁴C, isotopes are particularly preferred for their ease of preparation and detectability. Further, substitution with heavier isotopes such as deuterium, *i.e.,* ²H, can afford certain therapeutic advantages resulting from greater metabolic stability, for example increased in vivo half-life or reduced dosage requirements and, hence, may be preferred in some circumstances. Isotopically labeled compounds of the present disclosure and prodrugs thereof can generally be prepared by carrying out the procedures below, by substituting a readily available isotopically labeled reagent for a non- isotopically labeled reagent.

The compounds described in the disclosure can be present as a solvate. In some cases, the solvent used to prepare the solvate is an aqueous solution, and the solvate is then often referred to as a hydrate. The compounds can be present as a hydrate, which can be obtained, for example, by crystallization from a solvent or from aqueous solution. In this connection, one, two, three or any arbitrary number of solvent or water molecules can combine with the compounds according to the disclosure to form solvates and hydrates. Unless stated to the contrary, the disclosure includes all such possible solvates.

The term "co-crystal" means a physical association of two or more molecules which owe their stability through non-covalent interaction. One or more components of this molecular complex provide a stable framework in the crystalline lattice. In certain instances, the guest molecules are incorporated in the crystalline lattice as anhydrates or solvates, see e.g. "Crystal Engineering of the Composition of Pharmaceutical Phases. Do Pharmaceutical Co-crystals Represent a New Path to Improved Medicines?" Almarasson, O., et al., The Royal Society of Chemistry, 1889-1896, 2004. Examples of co-crystals include p-toluenesulfonic acid and benzenesulfonic acid.

It is known that chemical substances form solids which are present in different states of order which are termed polymorphic forms or modifications. The different modifications of a polymorphic substance can differ greatly in their physical properties. The compounds according to the disclosure can be present in different polymorphic forms, with it being possible for particular modifications to be metastable. Unless stated to the contrary, the disclosure includes all such possible polymorphic forms.

Certain materials, compounds, compositions, and components disclosed herein can be obtained commercially or readily synthesized using techniques generally known to those of skill in the art. For example, the starting materials and reagents used in preparing the disclosed compounds and compositions are either available from commercial suppliers such as Aldrich Chemical Co., (Milwaukee, Wis.), Acros Organics (Morris Plains, N.J.), Fisher Scientific (Pittsburgh, Pa.), or Sigma (St. Louis, Mo.) or are prepared by methods known to those skilled in the art following procedures set forth in references such as Fieser and Fieser's Reagents for Organic Synthesis, Volumes 1-17 (John Wiley and Sons, 1991); Rodd's Chemistry of Carbon Compounds, Volumes 1-5 and Supplementals (Elsevier Science Publishers, 1989); Organic Reactions, Volumes 1-40 (John Wiley and Sons, 1991); March's Advanced Organic Chemistry, (John Wiley and Sons, 4th Edition); and Larock's Comprehensive Organic Transformations (VCH Publishers Inc., 1989).

Unless otherwise expressly stated, it is in no way intended that any method set forth herein be construed as requiring that its steps be performed in a specific order. Accordingly, where a method claim does not actually recite an order to be followed by its steps or it is not otherwise specifically stated in the claims or descriptions that the steps are to be limited to a specific order, it is no way intended that an order be inferred, in any respect. This holds for any possible non-express basis for interpretation, including: matters of logic with respect to arrangement of steps or operational flow; plain meaning derived from grammatical organization or punctuation; and the number or type of embodiments described in the specification.

Disclosed are the components to be used to prepare the compositions of the disclosure as well as the compositions themselves to be used within the methods disclosed herein. These and other materials are disclosed herein, and it is understood that when combinations, subsets, interactions, groups, etc. of these materials are disclosed that while specific reference of each various individual and collective combinations and permutation of these compounds cannot be explicitly disclosed, each is specifically contemplated and described herein. For example, if a particular compound is disclosed and discussed and a number of modifications that can be made to a number of molecules including the compounds are discussed, specifically contemplated is each and every combination and permutation of the compound and the modifications that are possible unless specifically indicated to the contrary. Thus, if a class of molecules A, B, and C are disclosed as well as a class of molecules D, E, and F and an example of a combination molecule, A-D is disclosed, then even if each is not individually recited each is individually and collectively contemplated meaning combinations, A-E, A-F, B-D, B-E, B-F, C-D, C-E, and C-F are considered disclosed. Likewise, any subset or combination of these is also disclosed. Thus, for example, the sub-group of A-E, B-F, and C-E would be considered disclosed. This concept applies to all aspects of this application including, but not limited to, steps in methods of making and using the compositions of the disclosure. Thus, if there are a variety of additional steps that can be performed it is understood that each of these additional steps can be performed with any specific embodiment or combination of embodiments of the methods of the disclosure.

It is understood that the compositions disclosed herein have certain functions. Disclosed herein are certain structural requirements for performing the disclosed functions, and it is understood that there are a variety of structures that can perform the same function that are related to the disclosed structures, and that these structures will typically achieve the same result.

Described herein are compounds that can inhibit dihydroorotate dehydrogenase (DHODH) and have therapeutic or clinical utility for a disease or disorder that can be treated by inhibition of DHODH. Also described herein are methods of synthesizing the disclosed compounds. Also described herein are methods of administering the disclosed compounds to a subject in need thereof. In some aspects, the subject can have a disease or disorder associated with DHODH activity, such as a cancer, a disorder or disease associated with T-cell proliferation, or a graft-versus-host-disease. Other compositions, compounds, methods, features, and advantages of the present disclosure will be or become apparent to one having ordinary skill in the art upon examination of the following drawings, detailed description, and examples. It is intended that all such additional compositions, compounds, methods, features, and advantages be included within this description, and be within the scope of the present disclosure.

### Compounds.

In various aspects, the disclosed compounds are 6-substituted-2-([1,1'-biphenyl]-4-yl)quinoline-4-carboxylic acid analogs useful as inhibitors of dihydroxyorotate dehydrogenase, which have use as therapeutic agents in a variety of clinical conditions such as cancer, graft-versus-host disease, and disorders associated with T-cell proliferation.

In accordance with the present invention, there is provided compounds, or pharmaceutically acceptable salts thereof, in accordance with claim 1,

The compounds of the present invention may have a structure: 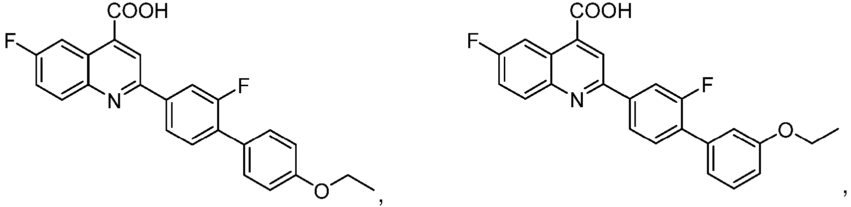 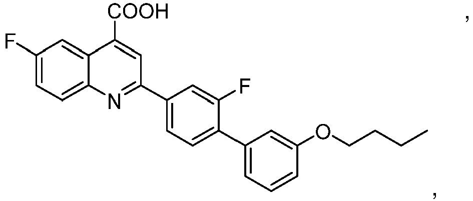 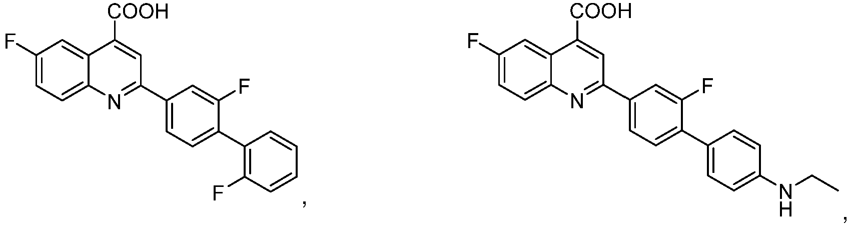 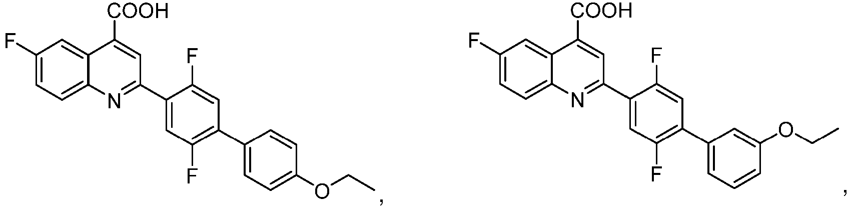 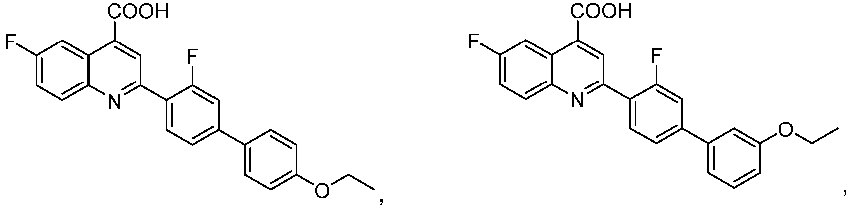 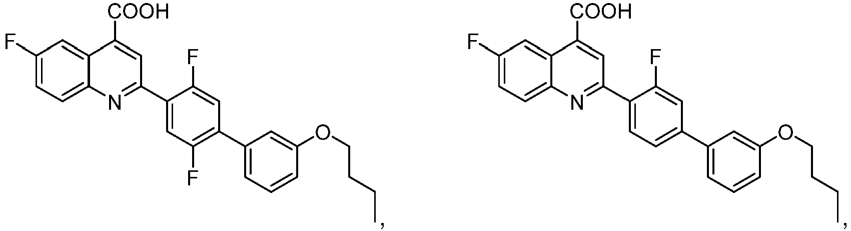 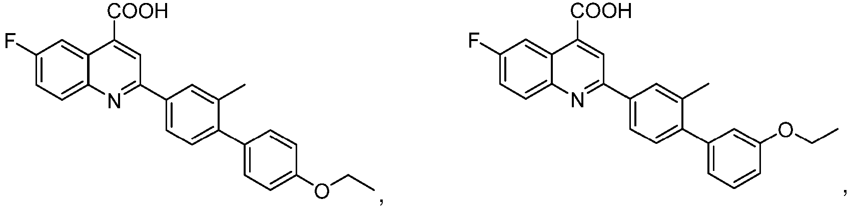 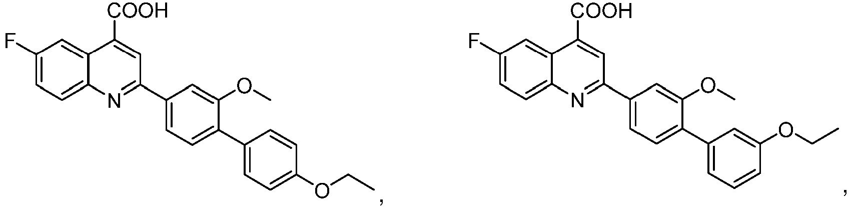 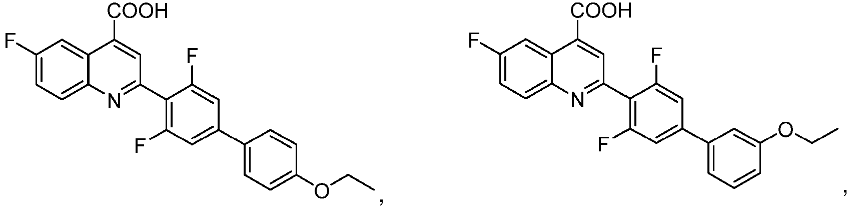 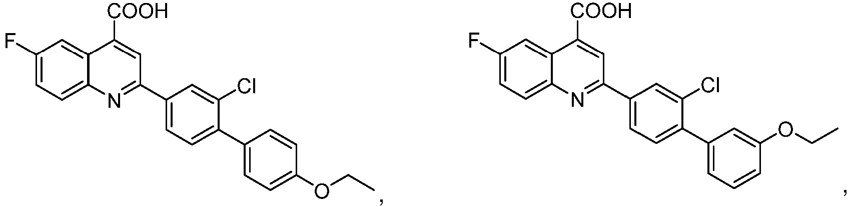 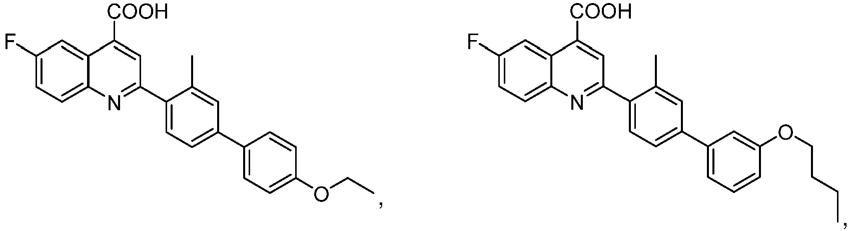 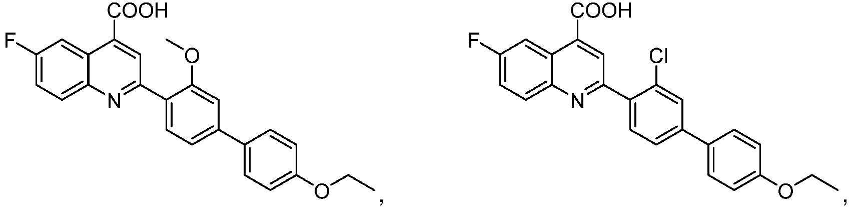 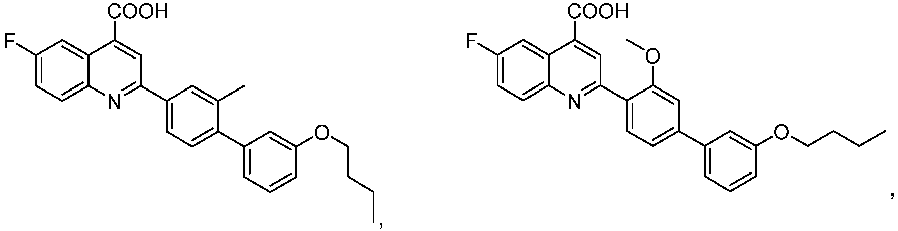 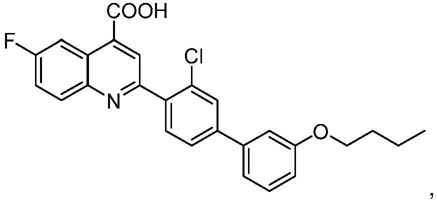 or combinations thereof.

The compounds of the present invention may have a structure: 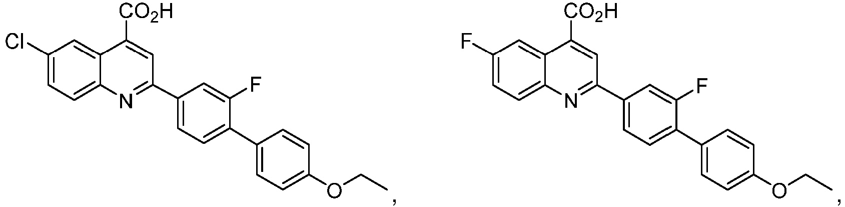 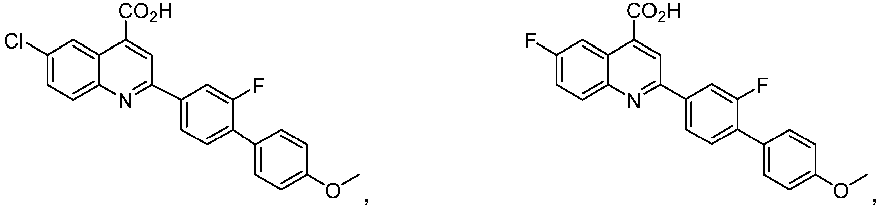 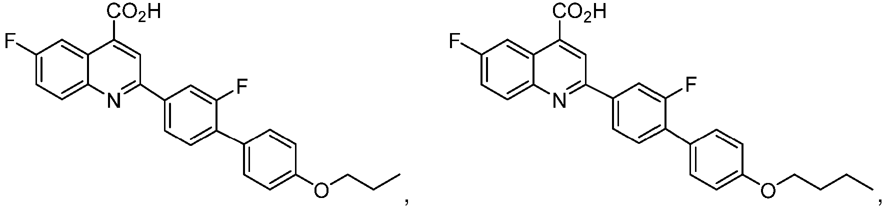 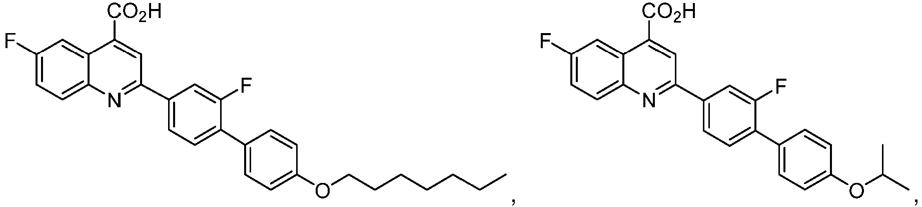 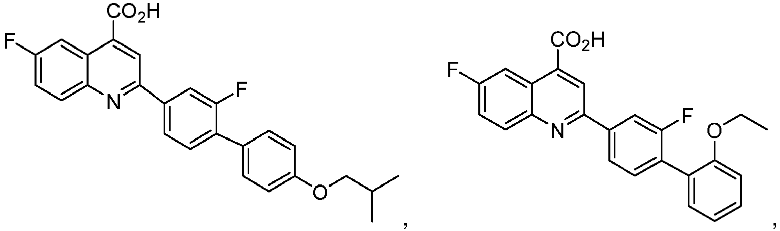 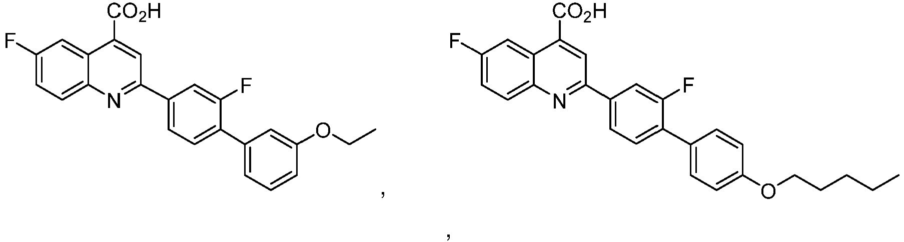 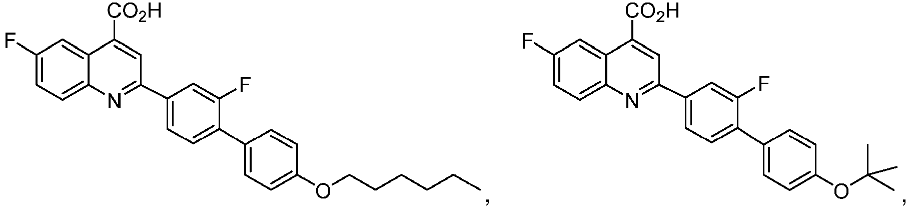 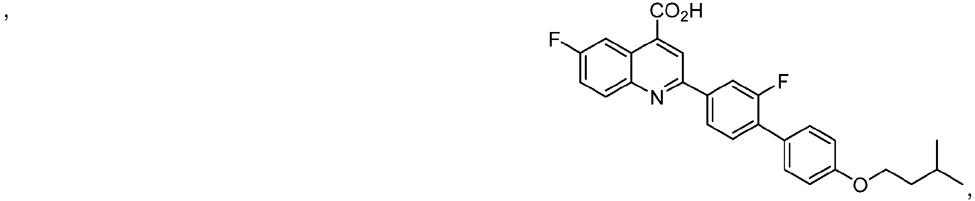 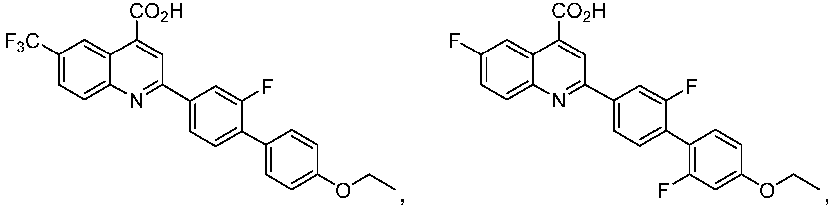 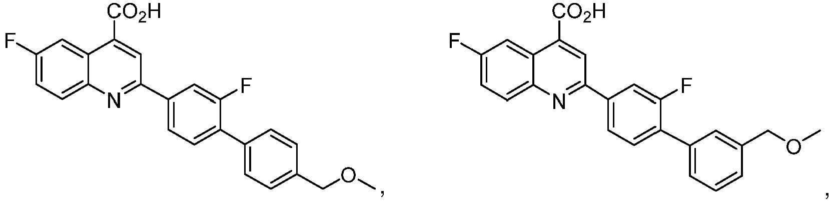 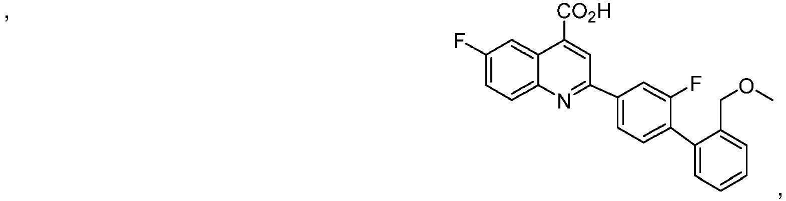 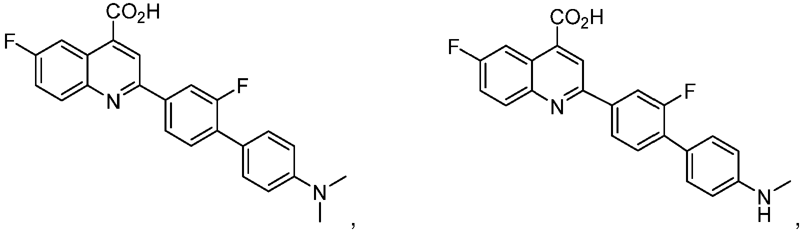 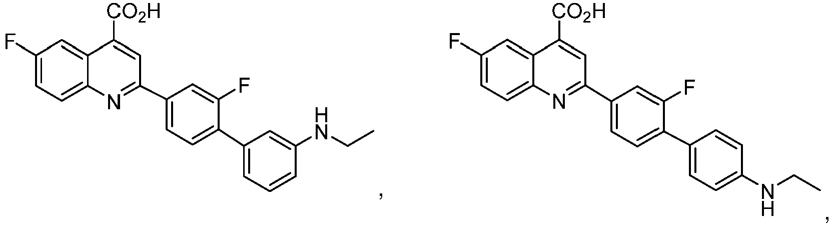 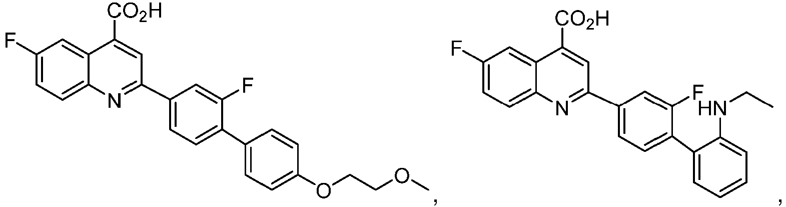 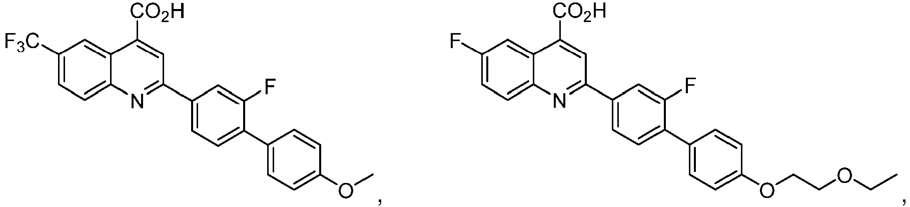 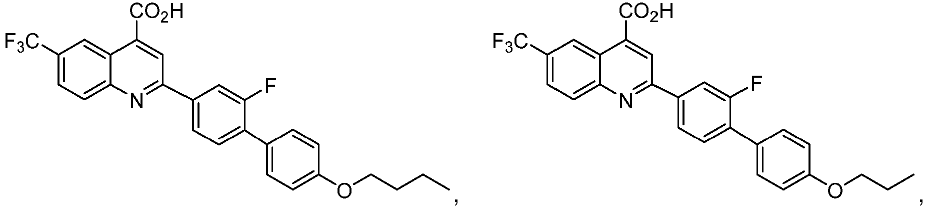 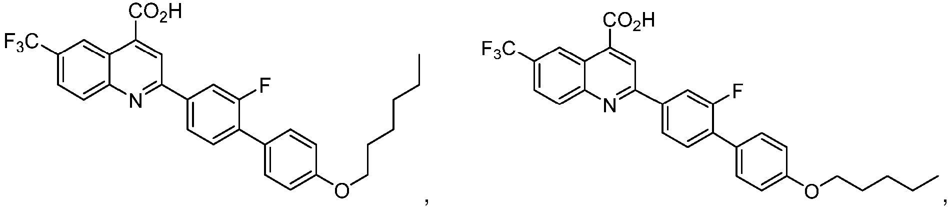 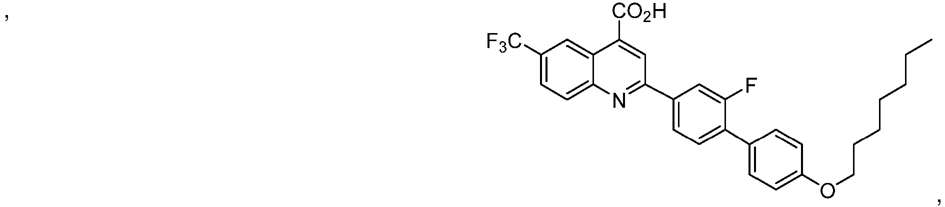 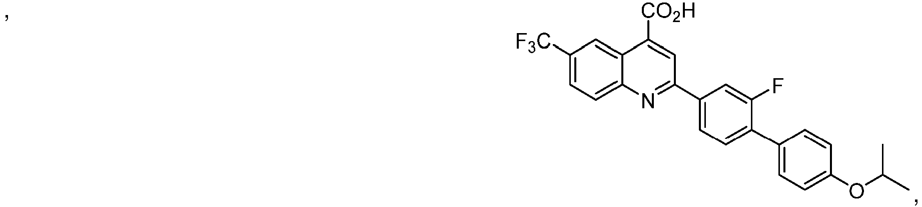 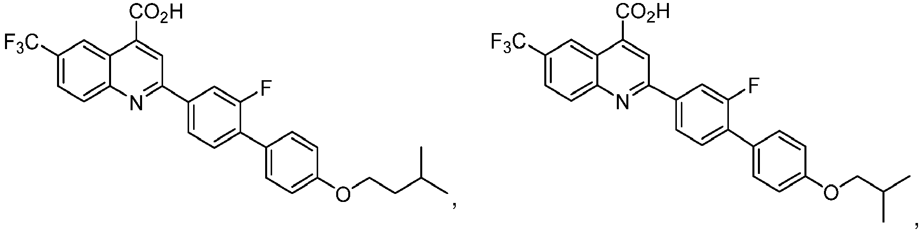 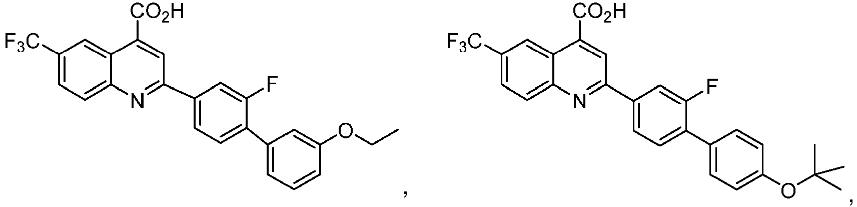 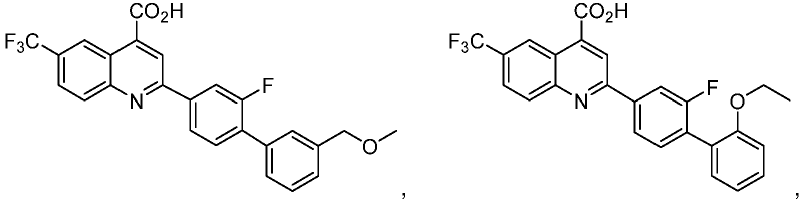 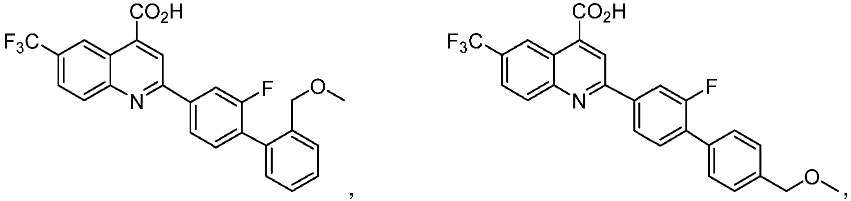 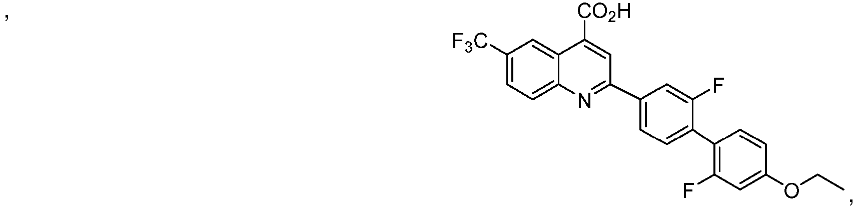 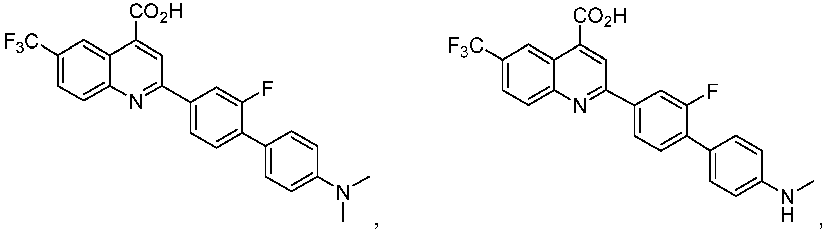 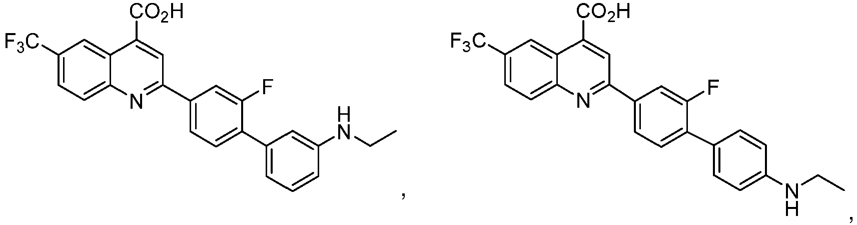 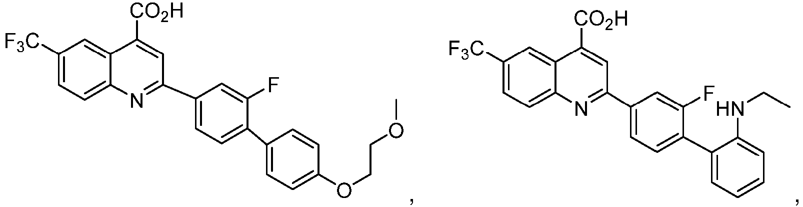 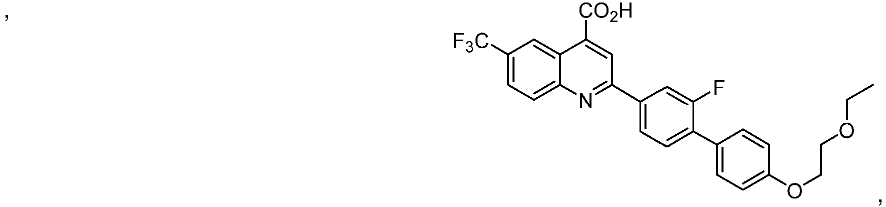 or a subgroup thereof.

The compounds of the present invention may have a structure: 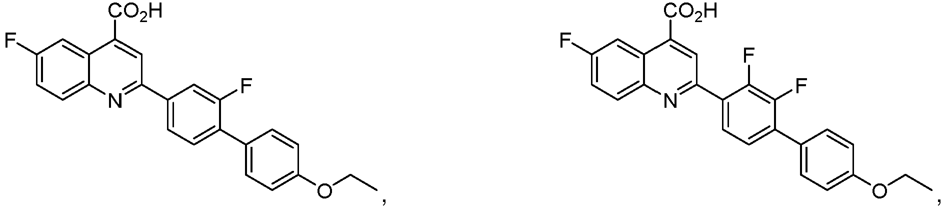 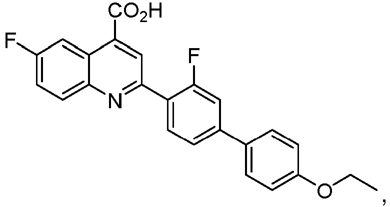 or a combination thereof.

The present invention also provides pharmaceutical compositions comprising a therapeutically effective amount of a compound, or a pharmaceutically acceptable salt thereof, in accordance with the present invention, and a pharmaceutically acceptable carrier.

It is contemplated herein that the compounds in accordance with the present invention further comprise their isotopically-labelled or isotopically-substituted variants, i.e., compounds identical to those described, but for the fact that one or more atoms are replaced by an atom having an atomic mass or mass number different from the atomic mass or mass number typically found in nature. Examples of isotopes that can be incorporated into compounds of the disclosure include isotopes of hydrogen, carbon, nitrogen, oxygen, phosphorous, fluorine and chlorine, such as ² H, ³ H, ¹³ C, ¹⁴ C, ¹⁵ N, ¹⁸ O, ¹⁷ O, ³⁵ S, ¹⁸ F and ³⁶ Cl, respectively. Compounds further comprise prodrugs thereof, and pharmaceutically acceptable salts of said compounds or of said prodrugs which contain the aforementioned isotopes and/or other isotopes of other atoms are within the scope of this disclosure. Certain isotopically-labelled compounds of the present disclosure, for example those into which radioactive isotopes such as ³H and ¹⁴C are incorporated, are useful in drug and/or substrate tissue distribution assays. Tritiated, i.e., ³H, and carbon-14, i.e., ¹⁴C, isotopes are particularly preferred for their ease of preparation and detectability. Further, substitution with heavier isotopes such as deuterium, i.e., ²H, can afford certain therapeutic advantages resulting from greater metabolic stability, for example increased in vivo half-life or reduced dosage requirements and, hence, may be preferred in some circumstances. Isotopically labelled compounds of the present disclosure and prodrugs thereof can generally be prepared by carrying out the procedures below, by substituting a readily available isotopically labelled reagent for a non- isotopically labelled reagent.

The compounds in accordance with the present invention can possess at least one center of asymmetry, they can be present in the form of their racemates, in the form of the pure enantiomers and/or diastereomers or in the form of mixtures of these enantiomers and/or diastereomers. The stereoisomers can be present in the mixtures in any arbitrary proportions. Provided this is possible, the compounds in accordance with the present invention can be present in the form of the tautomers.

Thus, methods which are known per se can be used, for example, to separate the compounds in accordance with the present invention which possess one or more chiral centers and occur as racemates into their optical isomers, i.e., enantiomers or diastereomers. The separation can be effected by means of column separation on chiral phases or by means of recrystallization from an optically active solvent or using an optically active acid or base or by means of derivatizing with an optically active reagent, such as an optically active alcohol, and subsequently cleaving off the residue.

The compounds in accordance with the present invention can be in the form of a co-crystal. The term "co-crystal" means a physical association of two or more molecules which owe their stability through non-covalent interaction. One or more components of this molecular complex provide a stable framework in the crystalline lattice. In certain instances, the guest molecules are incorporated in the crystalline lattice as anhydrates or solvates, see e.g. "Crystal Engineering of the Composition of Pharmaceutical Phases. Do Pharmaceutical Co-crystals Represent a New Path to Improved Medicines?" Almarasson, O., et. al., The Royal Society of Chemistry, 1889-1896, 2004. Preferred co-crystals include p-toluenesulfonic acid and benzenesulfonic acid.

The term "pharmaceutically acceptable co-crystal" means one that is compatible with the other ingredients of the formulation and not deleterious to the recipient thereof.

The compounds in accordance with the present invention can be isolated as solvates and, in particular, as hydrates of a disclosed compound, which can be obtained, for example, by crystallization from a solvent or from aqueous solution. In this connection, one, two, three or any arbitrary number of solvate or water molecules can combine with the compounds according to the disclosure to form solvates and hydrates.

The compounds in accordance with the present invention can be used in the form of salts derived from inorganic or organic acids. Pharmaceutically acceptable salts include salts of acidic or basic groups present in the disclosed compounds. Suitable pharmaceutically acceptable salts include base addition salts, including alkali metal salts, e.g., sodium or potassium salts; alkaline earth metal salts, e.g., calcium or magnesium salts; and salts formed with suitable organic ligands, e.g., quaternary ammonium salts, which may be similarly prepared by reacting the drug compound with a suitable pharmaceutically acceptable base. The salts can be prepared in situ during the final isolation and purification of the compounds of the present disclosure; or following final isolation by reacting a free base function, such as a secondary or tertiary amine, of a disclosed compound with a suitable inorganic or organic acid; or reacting a free acid function, such as a carboxylic acid, of a disclosed compound with a suitable inorganic or organic base.

Acidic addition salts can be prepared in situ during the final isolation and purification of a disclosed compound, or separately by reacting moieties comprising one or more nitrogen groups with a suitable acid. In various aspects, acids which may be employed to form pharmaceutically acceptable acid addition salts include such inorganic acids as hydrochloric acid, sulfuric acid and phosphoric acid and such organic acids as oxalic acid, maleic acid, succinic acid and citric acid. In a further aspect, salts further include, but are not limited, to the following: hydrochloride, hydrobromide, hydroiodide, nitrate, sulfate, bisulfate, phosphate, acid phosphate, isonicotinate, acetate, lactate, salicylate, citrate, tartrate, pantothenate, bitartrate, ascorbate, succinate, maleate, gentisinate, fumarate, gluconate, glucaronate, saccharate, formate, benzoate, glutamate, methanesulfonate, ethanesulfonate, benzensulfonate, p-toluenesulfonate, butyrate, camphorate, camphorsulfonate, digluconate, glycerophosphate, hemisulfate, heptanoate, hexanoate, fumarate, hydrochloride, 2-hydroxyethanesulfonate (isethionate), nicotinate, 2-naphthalenesulfonate, oxalate, pectinate, persulfate, 3-phenylpropionate, picrate, pivalate, propionate, succinate, tartrate, thiocyanate, phosphate, glutamate, bicarbonate, undecanoate, and pamoate (i.e., 1,1'-methylene-bis-(2-hydroxy-3-naphthoate)) salts. Also, basic nitrogen-containing groups can be quaternized with such agents as lower alkyl halides, such as methyl, ethyl, propyl, and butyl chloride, bromides, and iodides; dialkyl sulfates like dimethyl, diethyl, dibutyl, and diamyl sulfates, long chain halides such as decyl, lauryl, myristyl and stearyl chlorides, bromides and iodides, aralkyl halides like benzyl and phenethyl bromides, and others.

Basic addition salts can be prepared in situ during the final isolation and purification of a disclosed compound, or separately by reacting carboxylic acid moieties with a suitable base such as the hydroxide, carbonate or bicarbonate of a pharmaceutical acceptable metal cation or with ammonia, or an organic primary, secondary or tertiary amine. Pharmaceutical acceptable salts include, but are not limited to, cations based on the alkali and alkaline earth metals, such as sodium, lithium, potassium, calcium, magnesium, aluminum salts and the like, as well as nontoxic ammonium, quaternary ammonium, and amine cations, including, but not limited to ammonium, tetramethylammonium, tetraethylammonium, methylamine, dimethylamine, trimethylamine, triethylamine, ethylamine, and the like. Other representative organic amines useful for the formation of base addition salts include diethylamine, ethylenediamine, ethanolamine, diethanolamine, piperazine and the like. In further aspects, bases which may be used in the preparation of pharmaceutically acceptable salts include the following: ammonia, L-arginine, benethamine, benzathine, calcium hydroxide, choline, deanol, diethanolamine, diethylamine, 2-(diethylamino)-ethanol, ethanolamine, ethylenediamine, N-methyl-glucamine, hydrabamine, 1H-imidazole, L-lysine, magnesium hydroxide, 4-(2-hydroxyethyl)-morpholine, piperazine, potassium hydroxide, 1-(2-hydroxyethyl)-pyrrolidine, secondary amine, sodium hydroxide, triethanolamine, tromethamine and zinc hydroxide.

The compounds in accordance with the present invention can be conviently utilized as a component of a degrader molecule. Accordingly, in various aspects, a compound in accordance with the present invention can be used as a ligand, a linker, or an adjoining chemical structure within a proteolysis targeting complex or targeted protein degrader complex. For example, Proteolysis Targeting Chimera (PROTAC) technology is a rapidly emerging alternative therapeutic strategy with the potential to address many of the challenges currently faced in modern drug development programs. PROTAC technology employs small molecules that recruit target proteins for ubiquitination and removal by the proteasome (see, e.g., Bondeson and Crews, Annu Rev Pharmacol Toxicol. 2017 Jan 6; 57: 107-123; Lai et al. Angew Chem Int Ed Engl. 2016 Jan 11; 55(2): 807-810; and PCT Appl. No. PCT/US2018/061573).

The compounds in accordance with the present invention can further comprise linkage to a PROteolysis-TArgeting Chimera (PROTAC), thereby providing interaction with the intracellular ubiquitin-proteasome system to selectively degrade target protein. For example, in some instances, any one or more compounds can be utilized to form a composition, chimera, fusion, or complex having a protein degrading function. Some exemplary complexes can include a proteolysis-targeting chimaera (PROTAC) or a degronimid. As understood by a skilled artisan, such a complex is capable of uniting or combining cellular processes related to protein degradation to a specific target protein, wherein the cellular machinery and the target protein are complexed by a ligand, a linker, or an adjoining chemical structure.

### Methods of Making the Compounds.

Also described, but not claimed, are methods of making compounds useful as inhibitors of dihydroorotate dehydrogenase (DHODH), which can be useful in the treatment of clinical conditions, diseases, and disorders associated with DHODH dysfunction and other diseases in which DHODH is involved. In one aspect, the disclosure relates to the disclosed synthetic manipulations. The compounds in accordance with the present invention may comprise the products of the synthetic methods described herein. The compounds in accordance with the present invention may comprise a compound produced by a synthetic method described herein.

The compounds of the present invention can be prepared by employing reactions as shown in the disclosed schemes, in addition to other standard manipulations that are known in the literature, exemplified in the experimental sections or clear to one skilled in the art. The following examples are provided so that the disclosure might be more fully understood, are illustrative only, and should not be construed as limiting. For clarity, examples having a fewer substituent can be shown where multiple substituents are allowed under the definitions disclosed herein.

It is contemplated that each method can further comprise additional steps, manipulations, and/or components. It is also contemplated that any one or more step, manipulation, and/or component can be optionally omitted from the disclosure. It is understood that a disclosed method can be used to provide the disclosed compounds. It is also understood that the products of the methods can be employed in the compositions, kits, and uses described herein.

Substituted 6-substituted-2-([1,1'-biphenyl]-4-yl)quinoline-4-carboxylic acid analogs can be prepared generically by the synthetic scheme as shown below.

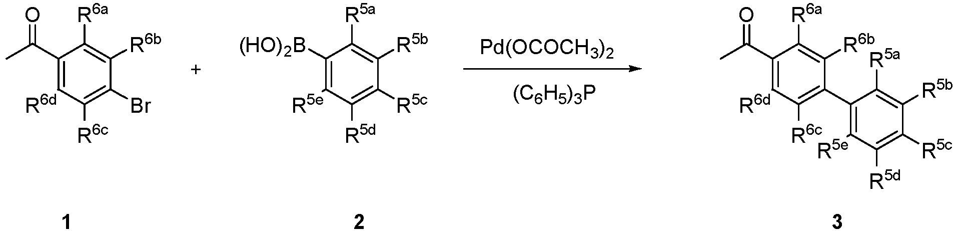

### Step 1 (Suzuki-Miyaura Reaction).

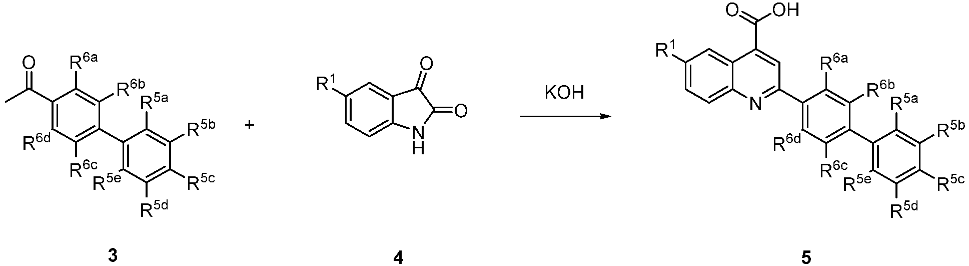

### Step 2 (Pfitzinger Reaction).

Compounds are represented in generic form, with substituents as noted in compound descriptions elsewhere herein. A more specific example is set forth below.

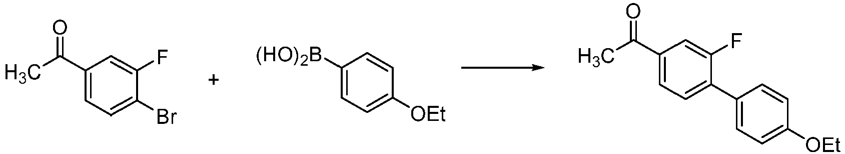

### Step 1 (Suzuki-Miyaura Reaction).

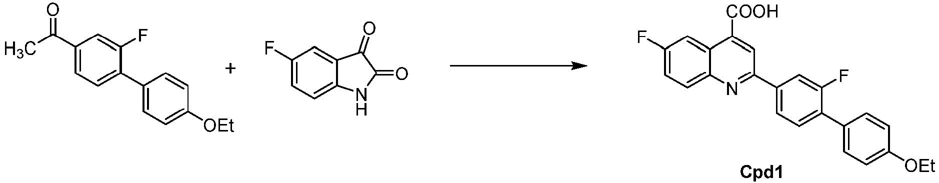

### Step 2 (Pfitzinger Reaction).

Compounds of the present invention, e.g. compounds of **Formula 5** can be prepared in a two-step reaction as shown above. Briefly, the synthesis of compound of **Formula 5** begin in **Step 1** with reaction of compounds of **Formulas 1** and **2** to yield compounds of **Formula 3.** Compounds of **Formula 1,** i.e., 4-halosubstituted phenone analogs, e.g., 3-fluoro-4-bromoacetophenone, and **Formula 2,** i.e., appropriately substituted phenylboronic acids, e.g., 4-ethoxyphenylboronic acid, can be obtained from commercial sources or can be readily prepared by skilled in the art according to methods described in the literature. For example, both 3-fluoro-4-bromophenone and 4-ethoxyphenylboronic acid are available commercially. The reaction of reaction of compounds of **Formulas 1** and **2** is typically carried at a molar ratio of **Formula 1** compound to **Formula 2** compound of about 25:1 to about 1:1 in a suitable solvent, e.g., 1-propanol, in the presence of palladium acetate and triphenylphosphine, at a suitable temperature, e.g. about 75 ºC to about 200 ºC, for a suitable period of time, e.g. about 10 minutes to about 2 hours, in order to ensure that the reaction is complete. The reaction is then cooled to a suitable temperature, e.g., room temperature, and then can be further cooled, e.g., to about 0 °C to obtain suitable crystals, which can be collected by filtration. Other suitable methods of isolating the product will be apparent to one skilled in the art.

In **Step 2,** the compound of **Formula 3,** isolated from **Step 1,** is reacted with compounds of **Formula 4** to yield the desired disclosed compound of **Formula 5** as shown above. Briefly, a mixture of the appropriate isatin, i.e., a compound of **Formula 4,** e.g., 5-fluoroisatin (5-fluoroindoline-2,3-dione), and a suitable base, e.g., aqueous potassium hydroxide solution (33%), are stirred and heated gently. To this solution, the slurry of a compound of **Formula 3,** e.g., 1-(4'-ethoxy-[1,1'-biphenyl]-4-yl)ethan-1-one, in an amount of about equimolar to the compound of **Formula 4,** and a suitable solvent is used to prepare the slurry, e.g., ethanol. The reaction mixture is then heated to a suitable temperature, e.g., reflux or about 70 °C to about 200 °C, for a suitable period of time, e.g., about 10 minutes to about 3 hours, in order to ensure that the reaction is complete. The reaction is then cooled to a suitable temperature, e.g., room temperature, and then can be further cooled, e.g., to about 0 °C to obtain suitable crystals, which can be collected by filtration. Other suitable methods of isolating the product will be apparent to one skilled in the art. The product may also be further purified if residual solvent is present, e.g., by methods known in the art.

### Pharmaceutical Compositions.

The present invention also relates to pharmaceutical compositions comprising a therapeutically effective amount of at least one compound or a pharmaceutically acceptable salt thereof, in accordance with the present invention. As used herein, "pharmaceutically-acceptable carriers" means one or more of a pharmaceutically acceptable diluents, preservatives, antioxidants, solubilizers, emulsifiers, coloring agents, releasing agents, coating agents, sweetening, flavoring and perfuming agents, and adjuvants. The disclosed pharmaceutical compositions can be conveniently presented in unit dosage form and prepared by any of the methods well known in the art of pharmacy and pharmaceutical sciences.

The pharmaceutical compositions of the present invention comprise a therapeutically effective amount of at least one compound, or a pharmaceutically acceptable salt thereof in accordance with the present invention as an active ingredient, a pharmaceutically acceptable carrier, optionally one or more other therapeutic agent, and optionally one or more adjuvant. The pharmaceutical compositions of the present invention include those suitable for oral, rectal, topical, pulmonary, nasal, and parenteral administration, although the most suitable route in any given case will depend on the particular host, and nature and severity of the conditions for which the active ingredient is being administered. The pharmaceutical composition of the present invention can be formulated to allow administration orally, nasally, via inhalation, parenterally, paracancerally, transmucosally, transdermally, intramuscularly, intravenously, intradermally, subcutaneously, intraperitonealy, intraventricularly, intracranially and intratumorally.

As used herein, "parenteral administration" includes administration by bolus injection or infusion, as well as administration by intravenous, intramuscular, intraarterial, intrathecal, intracapsular, intraorbital, intracardiac, intradermal, intraperitoneal, transtracheal, subcutaneous, subcuticular, intraarticular, subcapsular subarachnoid, intraspinal, epidural and intrasternal injection and infusion.

The pharmaceutical composition of the present invention may comprise a pharmaceutically acceptable carrier or diluent and, as active ingredient, a therapeutically effective amount of a compound or a pharmaceutically acceptable salt thereof, a hydrate thereof, a solvate thereof, a polymorph thereof, or a stereochemically isomeric form thereof, of the present invention. A compound a pharmaceutically acceptable salt, a hydrate thereof, a solvate thereof, a polymorph thereof, or a stereochemically isomeric form thereof, or any subgroup or combination thereof of the present invention may be formulated into various pharmaceutical forms for administration purposes.

Pharmaceutically acceptable salts can be prepared from pharmaceutically acceptable non-toxic bases or acids. For therapeutic use, salts of the disclosed compounds are those wherein the counter ion is pharmaceutically acceptable. However, salts of acids and bases which are non-pharmaceutically acceptable may also find use, for example, in the preparation or purification of a pharmaceutically acceptable compound. All salts, whether pharmaceutically acceptable or not, are contemplated by the present disclosure. Pharmaceutically acceptable acid and base addition salts are meant to comprise the therapeutically active non-toxic acid and base addition salt forms which the disclosed compounds are able to form.

A compound of the present invention comprising an acidic group or moiety, e.g., a carboxylic acid group, can be used to prepare a pharmaceutically acceptable salt. For example, such a disclosed compound may comprise an isolation step comprising treatment with a suitable inorganic or organic base. In some cases, it may be desirable in practice to initially isolate a compound from the reaction mixture as a pharmaceutically unacceptable salt and then simply convert the latter back to the free acid compound by treatment with an acidic reagent, and subsequently convert the free acid to a pharmaceutically acceptable base addition salt. These base addition salts can be readily prepared using conventional techniques, e.g., by treating the corresponding acidic compounds with an aqueous solution containing the desired pharmacologically acceptable cations and then evaporating the resulting solution to dryness, preferably under reduced pressure. Alternatively, they also can be prepared by mixing lower alkanolic solutions of the acidic compounds and the desired alkali metal alkoxide together, and then evaporating the resulting solution to dryness in the same manner as before.

Bases which can be used to prepare the pharmaceutically acceptable base-addition salts of the base compounds are those which can form non-toxic base-addition salts, i.e., salts containing pharmacologically acceptable cations such as, alkali metal cations (e.g., lithium, potassium and sodium), alkaline earth metal cations (e.g., calcium and magnesium), ammonium or other water-soluble amine addition salts such as N-methylglucamine-(meglumine), lower alkanolammonium and other such bases of organic amines. Derived from pharmaceutically acceptable organic non-toxic bases include primary, secondary, and tertiary amines, as well as cyclic amines and substituted amines such as naturally occurring and synthesized substituted amines. Such pharmaceutically acceptable organic non-toxic bases include, but are not limited to, ammonia, methylamine, ethylamine, propylamine, isopropylamine, any of the four butylamine isomers, betaine, caffeine, choline, dimethylamine, diethylamine, diethanolamine, dipropylamine, diisopropylamine, di-*n*-butylamine, N,N'-dibenzylethylenediamine, pyrrolidine, piperidine, morpholine, trimethylamine, triethylamine, tripropylamine, tromethamine, 2-diethylaminoethanol, 2-dimethylaminoethanol, ethanolamine, quinuclidine, pyridine, quinoline and isoquinoline; benzathine, *N*-methyl-D-glucamine, ethylenediamine, N-ethylmorpholine, N-ethylpiperidine, glucamine, glucosamine, methylglucamine, morpholine, piperazine, piperidine, polyamine resins, procaine, purines, theobromine, hydrabamine salts, and salts with amino acids such as, for example, histidine, arginine, lysine and the like. The foregoing salt forms can be converted by treatment with acid back into the free acid form.

A compound of the present invention comprising a protonatable group or moiety, e.g., an amino group, can be used to prepare a pharmaceutically acceptable salt. For example, such a compound may comprise an isolation step comprising treatment with a suitable inorganic or organic acid. In some cases, it may be desirable in practice to initially isolate a compound from the reaction mixture as a pharmaceutically unacceptable salt and then simply convert the latter back to the free base compound by treatment with a basic reagent, and subsequently convert the free base to a pharmaceutically acceptable acid addition salt. These acid addition salts can be readily prepared using conventional techniques, e.g., by treating the corresponding basic compounds with an aqueous solution containing the desired pharmacologically acceptable anions and then evaporating the resulting solution to dryness, preferably under reduced pressure. Alternatively, they also can be prepared by treating the free base form of the disclosed compound with a suitable pharmaceutically acceptable non-toxic inorganic or organic acid.

Acids which can be used to prepare the pharmaceutically acceptable acid-addition salts are those which can form non-toxic acid-addition salts, i.e., salts containing pharmacologically acceptable anions formed from their corresponding inorganic and organic acids. Exemplary, but non-limiting, inorganic acids include hydrochloric hydrobromic, sulfuric, nitric, phosphoric and the like. Exemplary, but non-limiting, organic acids include acetic, benzenesulfonic, benzoic, camphorsulfonic, citric, ethanesulfonic, fumaric, gluconic, glutamic, isethionic, lactic, maleic, malic, mandelicmethanesulfonic, mucic, pamoic, pantothenic, succinic, tartaric, p-toluenesulfonic acid and the like. In a further aspect, the acid-addition salt comprises an anion formed from hydrobromic, hydrochloric, maleic, phosphoric, sulfuric, and tartaric acids.

In practice, the compounds or pharmaceutically acceptable salts thereof, of the present invention can be combined as the active ingredient in intimate admixture with a pharmaceutical carrier according to conventional pharmaceutical compounding techniques. The carrier can take a wide variety of forms depending on the form of preparation desired for administration, e.g., oral or parenteral (including intravenous). Thus, the pharmaceutical compositions of the present disclosure can be presented as discrete units suitable for oral administration such as capsules, cachets or tablets each containing a predetermined amount of the active ingredient. Further, the compositions can be presented as a powder, as granules, as a solution, as a suspension in an aqueous liquid, as a non-aqueous liquid, as an oil-in-water emulsion or as a water-in-oil liquid emulsion. In addition to the common dosage forms set out above, the compounds of the present disclosure, and/or pharmaceutically acceptable salt(s) thereof, can also be administered by controlled release means and/or delivery devices. The compositions can be prepared by any of the methods of pharmacy. In general, such methods include a step of bringing into association the active ingredient with the carrier that constitutes one or more necessary ingredients. In general, the compositions are prepared by uniformly and intimately admixing the active ingredient with liquid carriers or finely divided solid carriers or both. The product can then be conveniently shaped into the desired presentation.

It is especially advantageous to formulate the aforementioned pharmaceutical compositions in unit dosage form for ease of administration and uniformity of dosage. The term "unit dosage form," as used herein, refers to physically discrete units suitable as unitary dosages, each unit containing a predetermined quantity of active ingredient calculated to produce the desired therapeutic effect in association with the required pharmaceutical carrier. That is, a "unit dosage form" is taken to mean a single dose wherein all active and inactive ingredients are combined in a suitable system, such that the patient or person administering the drug to the patient can open a single container or package with the entire dose contained therein, and does not have to mix any components together from two or more containers or packages. Typical examples of unit dosage forms are tablets (including scored or coated tablets), capsules or pills for oral administration; single dose vials for injectable solutions or suspension; suppositories for rectal administration; powder packets; wafers; and segregated multiples thereof. This list of unit dosage forms is not intended to be limiting in any way, but merely to represent typical examples of unit dosage forms.

The pharmaceutical compositions of the present invention comprise a compound or pharmaceutically acceptable salts thereof , of the present invention, as an active ingredient, a pharmaceutically acceptable carrier, and optionally one or more additional therapeutic agents. The pharmaceutical compositions of the present invention can include a pharmaceutically acceptable carrier and a compound, or a pharmaceutically acceptable salt thereof, of the present invention. A compound, or pharmaceutically acceptable salt thereof, of the present invention can also be included in a pharmaceutical composition in combination with one or more other therapeutically active compounds. The instant compositions include compositions suitable for oral, rectal, topical, and parenteral (including subcutaneous, intramuscular, and intravenous) administration, although the most suitable route in any given case will depend on the particular host, and nature and severity of the conditions for which the active ingredient is being administered. The pharmaceutical compositions can be conveniently presented in unit dosage form and prepared by any of the methods well known in the art of pharmacy.

Techniques and compositions for making dosage forms useful for materials and methods described herein are described, for example, in the following references: Modern Pharmaceutics, Chapters 9 and 10 (Banker & Rhodes, Editors, 1979); Pharmaceutical Dosage Forms: Tablets (Lieberman et al., 1981); Ansel, Introduction to Pharmaceutical Dosage Forms 2nd Edition (1976); Remington's Pharmaceutical Sciences, 17th ed. (Mack Publishing Company, Easton, Pa., 1985); Advances in Pharmaceutical Sciences (David Ganderton, Trevor Jones, Eds., 1992); Advances in Pharmaceutical Sciences Vol 7. (David Ganderton, Trevor Jones, James McGinity, Eds., 1995); Aqueous Polymeric Coatings for Pharmaceutical Dosage Forms (Drugs and the Pharmaceutical Sciences, Series 36 (James McGinity, Ed., 1989); Pharmaceutical Particulate Carriers: Therapeutic Applications: Drugs and the Pharmaceutical Sciences, Vol 61 (Alain Rolland, Ed., 1993); Drug Delivery to the Gastrointestinal Tract (Ellis Horwood Books in the Biological Sciences. Series in Pharmaceutical Technology; J. G. Hardy, S. S. Davis, Clive G. Wilson, Eds.); Modern Pharmaceutics Drugs and the Pharmaceutical Sciences, Vol 40 (Gilbert S. Banker, Christopher T. Rhodes, Eds.).

The compounds described herein are typically to be administered in admixture with suitable pharmaceutical diluents, excipients, extenders, or carriers (termed herein as a pharmaceutically acceptable carrier, or a carrier) suitably selected with respect to the intended form of administration and as consistent with conventional pharmaceutical practices. The deliverable compound will be in a form suitable for oral, rectal, topical, intravenous injection or parenteral administration. Carriers include solids or liquids, and the type of carrier is chosen based on the type of administration being used. The compounds may be administered as a dosage that has a known quantity of the compound.

Because of the ease in administration, oral administration can be a preferred dosage form, and tablets and capsules represent the most advantageous oral dosage unit forms in which case solid pharmaceutical carriers are obviously employed. However, other dosage forms may be suitable depending upon clinical population (e.g., age and severity of clinical condition), solubility properties of the specific disclosed compound used, and the like. Accordingly, the compounds of the present invention can be used in oral dosage forms such as pills, powders, granules, elixirs, tinctures, suspensions, syrups, and emulsions. In preparing the compositions for oral dosage form, any convenient pharmaceutical media can be employed. For example, water, glycols, oils, alcohols, flavoring agents, preservatives, coloring agents and the like can be used to form oral liquid preparations such as suspensions, elixirs and solutions; while carriers such as starches, sugars, microcrystalline cellulose, diluents, granulating agents, lubricants, binders, disintegrating agents, and the like can be used to form oral solid preparations such as powders, capsules and tablets. Because of their ease of administration, tablets and capsules are the preferred oral dosage units whereby solid pharmaceutical carriers are employed. Optionally, tablets can be coated by standard aqueous or nonaqueous techniques.

The disclosed pharmaceutical compositions in an oral dosage form can comprise one or more pharmaceutical excipient and/or additive. Non-limiting examples of suitable excipients and additives include gelatin, natural sugars such as raw sugar or lactose, lecithin, pectin, starches (for example corn starch or amylose), dextran, polyvinyl pyrrolidone, polyvinyl acetate, gum arabic, alginic acid, tylose, talcum, lycopodium, silica gel (for example colloidal), cellulose, cellulose derivatives (for example cellulose ethers in which the cellulose hydroxy groups are partially etherified with lower saturated aliphatic alcohols and/or lower saturated, aliphatic oxyalcohols, for example methyl oxypropyl cellulose, methyl cellulose, hydroxypropyl methyl cellulose, hydroxypropyl methyl cellulose phthalate), fatty acids as well as magnesium, calcium or aluminum salts of fatty acids with 12 to 22 carbon atoms, in particular saturated (for example stearates), emulsifiers, oils and fats, in particular vegetable (for example, peanut oil, castor oil, olive oil, sesame oil, cottonseed oil, corn oil, wheat germ oil, sunflower seed oil, cod liver oil, in each case also optionally hydrated); glycerol esters and polyglycerol esters of saturated fatty acids C₁₂H₂₄O₂ to C₁₈H₃₆O₂ and their mixtures, it being possible for the glycerol hydroxy groups to be totally or also only partly esterified (for example mono-, di- and triglycerides); pharmaceutically acceptable mono- or multivalent alcohols and polyglycols such as polyethylene glycol and derivatives thereof, esters of aliphatic saturated or unsaturated fatty acids (2 to 22 carbon atoms, in particular 10-18 carbon atoms) with monovalent aliphatic alcohols (1 to 20 carbon atoms) or multivalent alcohols such as glycols, glycerol, diethylene glycol, pentacrythritol, sorbitol, mannitol and the like, which may optionally also be etherified, esters of citric acid with primary alcohols, acetic acid, urea, benzyl benzoate, dioxolanes, glyceroformals, tetrahydrofurfuryl alcohol, polyglycol ethers with C1-C12-alcohols, dimethylacetamide, lactamides, lactates, ethylcarbonates, silicones (in particular medium-viscous polydimethyl siloxanes), calcium carbonate, sodium carbonate, calcium phosphate, sodium phosphate, magnesium carbonate and the like.

Other auxiliary substances useful in preparing an oral dosage form are those which cause disintegration (so-called disintegrants), such as: cross-linked polyvinyl pyrrolidone, sodium carboxymethyl starch, sodium carboxymethyl cellulose or microcrystalline cellulose. Conventional coating substances may also be used to produce the oral dosage form. Those that may for example be considered are: polymerizates as well as copolymerizates of acrylic acid and/or methacrylic acid and/or their esters; copolymerizates of acrylic and methacrylic acid esters with a lower ammonium group content (for example EudragitR RS), copolymerizates of acrylic and methacrylic acid esters and trimethyl ammonium methacrylate (for example EudragitR RL); polyvinyl acetate; fats, oils, waxes, fatty alcohols; hydroxypropyl methyl cellulose phthalate or acetate succinate; cellulose acetate phthalate, starch acetate phthalate as well as polyvinyl acetate phthalate, carboxy methyl cellulose; methyl cellulose phthalate, methyl cellulose succinate, -phthalate succinate as well as methyl cellulose phthalic acid half ester; zein; ethyl cellulose as well as ethyl cellulose succinate; shellac, gluten; ethylcarboxyethyl cellulose; ethacrylate-maleic acid anhydride copolymer; maleic acid anhydride-vinyl methyl ether copolymer; styrol-maleic acid copolymerizate; 2-ethyl-hexyl-acrylate maleic acid anhydride; crotonic acid-vinyl acetate copolymer; glutaminic acid/glutamic acid ester copolymer; carboxymethylethylcellulose glycerol monooctanoate; cellulose acetate succinate; polyarginine; and the like.

Plasticizing agents that may be considered as coating substances in the disclosed oral dosage forms are: citric and tartaric acid esters (acetyl-triethyl citrate, acetyl tributyl-, tributyl-, triethyl-citrate); glycerol and glycerol esters (glycerol diacetate, -triacetate, acetylated monoglycerides, castor oil); phthalic acid esters (dibutyl-, diamyl-, diethyl-, dimethyl-, dipropyl-phthalate), di-(2-methoxy- or 2-ethoxyethyl)-phthalate, ethylphthalyl glycolate, butylphthalylethyl glycolate and butylglycolate; alcohols (propylene glycol, polyethylene glycol of various chain lengths), adipates (diethyladipate, di-(2-methoxy- or 2-ethoxyethyl)-adipate; benzophenone; diethyl- and dibutylsebacate, dibutylsuccinate, dibutyltartrate; diethylene glycol dipropionate; ethyleneglycol diacetate, dibutyrate, dipropionate; tributyl phosphate, tributyrin; polyethylene glycol sorbitan monooleate (polysorbates such as Polysorbate 50); sorbitan monooleate; and the like.

Moreover, suitable binders, lubricants, disintegrating agents, coloring agents, flavoring agents, flow-inducing agents, and melting agents may be included as carriers. The pharmaceutical carrier employed can be, for example, a solid, liquid, or gas. Examples of solid carriers include, but are not limited to, lactose, terra alba, sucrose, glucose, methylcellulose, dicalcium phosphate, calcium sulfate, mannitol, sorbitol talc, starch, gelatin, agar, pectin, acacia, magnesium stearate, and stearic acid. Examples of liquid carriers are sugar syrup, peanut oil, olive oil, and water. Examples of gaseous carriers include carbon dioxide and nitrogen.

A binder can include, for example, starch, gelatin, natural sugars such as glucose or beta-lactose, corn sweeteners, natural and synthetic gums such as acacia, tragacanth, or sodium alginate, carboxymethylcellulose, polyethylene glycol, waxes, and the like. Lubricants used in these dosage forms include sodium oleate, sodium stearate, magnesium stearate, sodium benzoate, sodium acetate, sodium chloride, and the like. In a further aspect, a disintegrator can include, for example, starch, methyl cellulose, agar, bentonite, xanthan gum, and the like.

An oral dosage form, such as a solid dosage form, can comprise a disclosed compound that is attached to polymers as targetable drug carriers or as a prodrug. Suitable biodegradable polymers useful in achieving controlled release of a drug include, for example, polylactic acid, polyglycolic acid, copolymers of polylactic and polyglycolic acid, caprolactones, polyhydroxy butyric acid, polyorthoesters, polyacetals, polydihydropyrans, polycyanoacylates, and hydrogels, preferably covalently crosslinked hydrogels.

Tablets may contain the active ingredient in admixture with non-toxic pharmaceutically acceptable excipients which are suitable for the manufacture of tablets. These excipients may be, for example, inert diluents, such as calcium carbonate, sodium carbonate, lactose, calcium phosphate or sodium phosphate; granulating and disintegrating agents, for example, corn starch, or alginic acid; binding agents, for example starch, gelatin or acacia, and lubricating agents, for example magnesium stearate, stearic acid or talc. The tablets may be uncoated or they may be coated by known techniques to delay disintegration and absorption in the gastrointestinal tract and thereby provide a sustained action over a longer period.

A tablet containing a disclosed compound can be prepared by compression or molding, optionally with one or more accessory ingredients or adjuvants. Compressed tablets can be prepared by compressing, in a suitable machine, the active ingredient in a free-flowing form such as powder or granules, optionally mixed with a binder, lubricant, inert diluent, surface active or dispersing agent. Molded tablets can be made by molding in a suitable machine, a mixture of the powdered compound moistened with an inert liquid diluent.

A solid oral dosage form, such as a tablet, can be coated with an enteric coating to prevent ready decomposition in the stomach. In various aspects, enteric coating agents include, but are not limited to, hydroxypropylmethylcellulose phthalate, methacrylic acid-methacrylic acid ester copolymer, polyvinyl acetate-phthalate and cellulose acetate phthalate. Akihiko Hasegawa "Application of solid dispersions of Nifedipine with enteric coating agent to prepare a sustained-release dosage form" Chem. Pharm. Bull. 33:1615-1619 (1985). Various enteric coating materials may be selected on the basis of testing to achieve an enteric coated dosage form designed ab initio to have a preferable combination of dissolution time, coating thicknesses and diametral crushing strength (e.g., see S. C. Porter et al. "The Properties of Enteric Tablet Coatings Made From Polyvinyl Acetate-phthalate and Cellulose acetate Phthalate", J. Pharm. Pharmacol. 22:42p (1970)). In a further aspect, the enteric coating may comprise hydroxypropyl-methylcellulose phthalate, methacrylic acid-methacrylic acid ester copolymer, polyvinyl acetate-phthalate and cellulose acetate phthalate.

An oral dosage form can be a solid dispersion with a water soluble or a water insoluble carrier. Examples of water soluble or water insoluble carrier include, but are not limited to, polyethylene glycol, polyvinylpyrrolidone, hydroxypropylmethyl-cellulose, phosphatidylcholine, polyoxyethylene hydrogenated castor oil, hydroxypropylmethylcellulose phthalate, carboxymethylethylcellulose, or hydroxypropylmethylcellulose, ethyl cellulose, or stearic acid.

An oral dosage form can be in a liquid dosage form, including those that are ingested, or alternatively, administered as a mouth wash or gargle. For example, a liquid dosage form can include aqueous suspensions, which contain the active materials in admixture with excipients suitable for the manufacture of aqueous suspensions. In addition, oily suspensions may be formulated by suspending the active ingredient in a vegetable oil, for example arachis oil, olive oil, sesame oil or coconut oil, or in a mineral oil such as liquid paraffin. Oily suspensions may also contain various excipients. The pharmaceutical compositions of the present disclosure may also be in the form of oil-in-water emulsions, which may also contain excipients such as sweetening and flavoring agents.

For the preparation of solutions or suspensions it is, for example, possible to use water, particularly sterile water, or physiologically acceptable organic solvents, such as alcohols (ethanol, propanol, isopropanol, 1,2-propylene glycol, polyglycols and their derivatives, fatty alcohols, partial esters of glycerol), oils (for example peanut oil, olive oil, sesame oil, almond oil, sunflower oil, soya bean oil, castor oil, bovine hoof oil), paraffins, dimethyl sulphoxide, triglycerides and the like.

In the case of a liquid dosage form such as a drinkable solutions, the following substances may be used as stabilizers or solubilizers: lower aliphatic mono- and multivalent alcohols with 2-4 carbon atoms, such as ethanol, n-propanol, glycerol, polyethylene glycols with molecular weights between 200-600 (for example 1 to 40% aqueous solution), diethylene glycol monoethyl ether, 1,2-propylene glycol, organic amides, for example amides of aliphatic C1-C6-carboxylic acids with ammonia or primary, secondary or tertiary C1-C4-amines or C1-C4-hydroxy amines such as urea, urethane, acetamide, N-methyl acetamide, N,N-diethyl acetamide, N,N-dimethyl acetamide, lower aliphatic amines and diamines with 2-6 carbon atoms, such as ethylene diamine, hydroxyethyl theophylline, tromethamine (for example as 0.1 to 20% aqueous solution), aliphatic amino acids.

In preparing the disclosed liquid dosage form can comprise solubilizers and emulsifiers such as the following non-limiting examples can be used: polyvinyl pyrrolidone, sorbitan fatty acid esters such as sorbitan trioleate, phosphatides such as lecithin, acacia, tragacanth, polyoxyethylated sorbitan monooleate and other ethoxylated fatty acid esters of sorbitan, polyoxyethylated fats, polyoxyethylated oleotriglycerides, linolizated oleotriglycerides, polyethylene oxide condensation products of fatty alcohols, alkylphenols or fatty acids or also 1-methyl-3-(2-hydroxyethyl)imidazolidone-(2). In this context, polyoxyethylated means that the substances in question contain polyoxyethylene chains, the degree of polymerization of which generally lies between 2 and 40 and in particular between 10 and 20. Polyoxyethylated substances of this kind may for example be obtained by reaction of hydroxyl group-containing compounds (for example mono- or diglycerides or unsaturated compounds such as those containing oleic acid radicals) with ethylene oxide (for example 40 mole ethylene oxide per 1 mole glyceride). Examples of oleotriglycerides are olive oil, peanut oil, castor oil, sesame oil, cottonseed oil, corn oil. See also Dr. H. P. Fiedler "Lexikon der Hillsstoffe für Pharmazie, Kostnetik und angrenzende Gebiete" 1971, pages 191-195.

A liquid dosage form can further comprise preservatives, stabilizers, buffer substances, flavor correcting agents, sweeteners, colorants, antioxidants and complex formers and the like. Complex formers which may be for example be considered are: chelate formers such as ethylenediaminetetracetic acid, nitrilotriacetic acid, diethylenetriaminepentacetic acid and their salts.

It may optionally be necessary to stabilize a liquid dosage form with physiologically acceptable bases or buffers to a pH range of approximately 6 to 9. Preference may be given to as neutral or weakly basic a pH value as possible (up to pH 8).

In order to enhance the solubility and/or the stability of a disclosed compound in a disclosed liquid dosage form, a parenteral injection form, or an intravenous injectable form, it can be advantageous to employ α-, β- or γ-cyclodextrins or their derivatives, in particular hydroxyalkyl substituted cyclodextrins, e.g. 2-hydroxypropyl-β-cyclodextrin or sulfobutyl-β-cyclodextrin. Also co-solvents such as alcohols may improve the solubility and/or the stability of the compounds according to the present disclosure in pharmaceutical compositions.

A liquid dosage form, a parenteral injection form, or an intravenous injectable form can further comprise liposome delivery systems, such as small unilamellar vesicles, large unilamellar vesicles, and multilamellar vesicles. Liposomes can be formed from a variety of phospholipids, such as cholesterol, stearylamine, or phosphatidylcholines.

Pharmaceutical compositions of the present invention may be suitable for injection, such as parenteral administration, such as intravenous, intramuscular, or subcutaneous administration. Pharmaceutical compositions for injection can be prepared as solutions or suspensions of the active compounds in water. A suitable surfactant can be included such as, for example, hydroxypropylcellulose. Dispersions can also be prepared in glycerol, liquid polyethylene glycols, and mixtures thereof in oils. Further, a preservative can be included to prevent the detrimental growth of microorganisms.

Pharmaceutical compositions of the present invention suitable for parenteral administration can include sterile aqueous or oleaginous solutions, suspensions, or dispersions. Furthermore, the compositions can be in the form of sterile powders for the extemporaneous preparation of such sterile injectable solutions or dispersions. In some aspects, the final injectable form is sterile and must be effectively fluid for use in a syringe. The pharmaceutical compositions should be stable under the conditions of manufacture and storage; thus, preferably should be preserved against the contaminating action of microorganisms such as bacteria and fungi. The carrier can be a solvent or dispersion medium containing, for example, water, ethanol, polyol (*e.g.*, glycerol, propylene glycol and liquid polyethylene glycol), vegetable oils, and suitable mixtures thereof.

Injectable solutions, for example, can be prepared in which the carrier comprises saline solution, glucose solution or a mixture of saline and glucose solution. Injectable suspensions may also be prepared in which case appropriate liquid carriers, suspending agents and the like may be employed. In some aspects, a disclosed parenteral formulation can comprise about 0.01-0.1 M, e.g. about 0.05 M, phosphate buffer. In a further aspect, a disclosed parenteral formulation can comprise about 0.9% saline.

A parenteral pharmaceutical composition of the present invention can comprise pharmaceutically acceptable carriers such as aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include but not limited to water, alcoholic/aqueous solutions, emulsions or suspensions, including saline and buffered media. Parenteral vehicles can include mannitol, normal serum albumin, sodium chloride solution, Ringer's dextrose, dextrose and sodium chloride, lactated Ringer's and fixed oils. Intravenous vehicles include fluid and nutrient replenishers, electrolyte replenishers such as those based on Ringer's dextrose, and the like. Preservatives and other additives may also be present, such as, for example, antimicrobials, antioxidants, collating agents, inert gases and the like. A parenteral pharmaceutical composition of the present invention can comprise may contain minor amounts of additives such as substances that enhance isotonicity and chemical stability, e.g., buffers and preservatives. Also contemplated for injectable pharmaceutical compositions are solid form preparations that are intended to be converted, shortly before use, to liquid form preparations. Furthermore, other adjuvants can be included to render the formulation isotonic with the blood of the subject or patient.

In addition to the pharmaceutical compositions described herein above, the compounds of the present invention can also be formulated as a depot preparation. Such long acting formulations can be administered by implantation (e.g., subcutaneously or intramuscularly) or by intramuscular injection. Thus, for example, the compounds can be formulated with suitable polymeric or hydrophobic materials (e.g., as an emulsion in an acceptable oil) or ion exchange resins, or as sparingly soluble derivatives, e.g., as a sparingly soluble salt.

Pharmaceutical compositions of the present invention can be in a form suitable for topical administration. As used herein, the phrase "topical application" means administration onto a biological surface, whereby the biological surface includes, for example, a skin area (e.g., hands, forearms, elbows, legs, face, nails, anus and genital areas) or a mucosal membrane. By selecting the appropriate carrier and optionally other ingredients that can be included in the composition, as is detailed herein below, the compositions of the present disclosure may be formulated into any form typically employed for topical application. A topical pharmaceutical composition can be in a form of a cream, an ointment, a paste, a gel, a lotion, milk, a suspension, an aerosol, a spray, foam, a dusting powder, a pad, and a patch. Further, the compositions can be in a form suitable for use in transdermal devices. These formulations can be prepared, utilizing a compound of the present disclosure, or pharmaceutically acceptable salts thereof, via conventional processing methods. As an example, a cream or ointment is prepared by mixing hydrophilic material and water, together with about 5 wt% to about 10 wt% of the compound, to produce a cream or ointment having a desired consistency.

In the compositions suitable for percutaneous administration, the carrier optionally comprises a penetration enhancing agent and/or a suitable wetting agent, optionally combined with suitable additives of any nature in minor proportions, which additives do not introduce a significant deleterious effect on the skin. Said additives may facilitate the administration to the skin and/or may be helpful for preparing the desired compositions. These compositions may be administered in various ways, e.g., as a transdermal patch, as a spot-on, as an ointment.

Ointments are semisolid preparations, typically based on petrolatum or petroleum derivatives. The specific ointment base to be used is one that provides for optimum delivery for the active agent chosen for a given formulation, and, preferably, provides for other desired characteristics as well (e.g., emollience). As with other carriers or vehicles, an ointment base should be inert, stable, nonirritating and nonsensitizing. As explained in Remington: The Science and Practice of Pharmacy, 19th Ed., Easton, Pa.: Mack Publishing Co. (1995), pp. 1399-1404, ointment bases may be grouped in four classes: oleaginous bases; emulsifiable bases; emulsion bases; and water-soluble bases. Oleaginous ointment bases include, for example, vegetable oils, fats obtained from animals, and semisolid hydrocarbons obtained from petroleum. Emulsifiable ointment bases, also known as absorbent ointment bases, contain little or no water and include, for example, hydroxystearin sulfate, anhydrous lanolin and hydrophilic petrolatum. Emulsion ointment bases are either water-in-oil (W/O) emulsions or oil-in-water (O/W) emulsions, and include, for example, cetyl alcohol, glyceryl monostearate, lanolin and stearic acid. Preferred water-soluble ointment bases are prepared from polyethylene glycols of varying molecular weight.

Lotions are preparations that are to be applied to the skin surface without friction. Lotions are typically liquid or semiliquid preparations in which solid particles, including the active agent, are present in a water or alcohol base. Lotions are typically preferred for treating large body areas, due to the ease of applying a more fluid composition. Lotions are typically suspensions of solids, and oftentimes comprise a liquid oily emulsion of the oil-in-water type. It is generally necessary that the insoluble matter in a lotion be finely divided. Lotions typically contain suspending agents to produce better dispersions as well as compounds useful for localizing and holding the active agent in contact with the skin, such as methylcellulose, sodium carboxymethyl-cellulose, and the like.

Creams are viscous liquids or semisolid emulsions, either oil-in-water or water-in-oil. Cream bases are typically water-washable, and contain an oil phase, an emulsifier and an aqueous phase. The oil phase, also called the "internal" phase, is generally comprised of petrolatum and/or a fatty alcohol such as cetyl or stearyl alcohol. The aqueous phase typically, although not necessarily, exceeds the oil phase in volume, and generally contains a humectant. The emulsifier in a cream formulation is generally a nonionic, anionic, cationic or amphoteric surfactant. Reference may be made to Remington: The Science and Practice of Pharmacy, supra, for further information.

Pastes are semisolid dosage forms in which the bioactive agent is suspended in a suitable base. Depending on the nature of the base, pastes are divided between fatty pastes or those made from a single-phase aqueous gel. The base in a fatty paste is generally petrolatum, hydrophilic petrolatum and the like. The pastes made from single-phase aqueous gels generally incorporate carboxymethylcellulose or the like as a base. Additional reference may be made to Remington: The Science and Practice of Pharmacy, for further information.

Gel formulations are semisolid, suspension-type systems. Single-phase gels contain organic macromolecules distributed substantially uniformly throughout the carrier liquid, which is typically aqueous, but also, preferably, contain an alcohol and, optionally, an oil. Preferred organic macromolecules, i.e., gelling agents, are crosslinked acrylic acid polymers such as the family of carbomer polymers, e.g., carboxypolyalkylenes that may be obtained commercially under the trademark Carbopol^{™}. Other types of preferred polymers in this context are hydrophilic polymers such as polyethylene oxides, polyoxyethylene-polyoxypropylene copolymers and polyvinylalcohol; modified cellulose, such as hydroxypropyl cellulose, hydroxyethyl cellulose, hydroxypropyl methylcellulose, hydroxypropyl methylcellulose phthalate, and methyl cellulose; gums such as tragacanth and xanthan gum; sodium alginate; and gelatin. In order to prepare a uniform gel, dispersing agents such as alcohol or glycerin can be added, or the gelling agent can be dispersed by trituration, mechanical mixing or stirring, or combinations thereof.

Sprays generally provide the active agent in an aqueous and/or alcoholic solution which can be misted onto the skin for delivery. Such sprays include those formulated to provide for concentration of the active agent solution at the site of administration following delivery, e.g., the spray solution can be primarily composed of alcohol or other like volatile liquid in which the active agent can be dissolved. Upon delivery to the skin, the carrier evaporates, leaving concentrated active agent at the site of administration.

Foam compositions are typically formulated in a single or multiple phase liquid form and housed in a suitable container, optionally together with a propellant which facilitates the expulsion of the composition from the container, thus transforming it into a foam upon application. Other foam forming techniques include, for example the "Bag-in-a-can" formulation technique. Compositions thus formulated typically contain a low-boiling hydrocarbon, e.g., isopropane. Application and agitation of such a composition at the body temperature cause the isopropane to vaporize and generate the foam, in a manner similar to a pressurized aerosol foaming system. Foams can be water-based or aqueous alkanolic, but are typically formulated with high alcohol content which, upon application to the skin of a user, quickly evaporates, driving the active ingredient through the upper skin layers to the site of treatment.

Skin patches typically comprise a backing, to which a reservoir containing the active agent is attached. The reservoir can be, for example, a pad in which the active agent or composition is dispersed or soaked, or a liquid reservoir. Patches typically further include a frontal water permeable adhesive, which adheres and secures the device to the treated region. Silicone rubbers with self-adhesiveness can alternatively be used. In both cases, a protective permeable layer can be used to protect the adhesive side of the patch prior to its use. Skin patches may further comprise a removable cover, which serves for protecting it upon storage.

Examples of patch configuration which can be utilized with the present disclosure include a single-layer or multi-layer drug-in-adhesive systems which are characterized by the inclusion of the drug directly within the skin-contacting adhesive. In such a transdermal patch design, the adhesive not only serves to affix the patch to the skin, but also serves as the formulation foundation, containing the drug and all the excipients under a single backing film. In the multi-layer drug-in-adhesive patch a membrane is disposed between two distinct drug-in-adhesive layers or multiple drug-in-adhesive layers are incorporated under a single backing film.

Examples of pharmaceutically acceptable carriers that are suitable for pharmaceutical compositions for topical applications include carrier materials that are well-known for use in the cosmetic and medical arts as bases for e.g., emulsions, creams, aqueous solutions, oils, ointments, pastes, gels, lotions, milks, foams, suspensions, aerosols and the like, depending on the final form of the composition. Representative examples of suitable carriers according to the present disclosure therefore include, without limitation, water, liquid alcohols, liquid glycols, liquid polyalkylene glycols, liquid esters, liquid amides, liquid protein hydrolysates, liquid alkylated protein hydrolysates, liquid lanolin and lanolin derivatives, and like materials commonly employed in cosmetic and medicinal compositions. Other suitable carriers according to the present disclosure include, without limitation, alcohols, such as, for example, monohydric and polyhydric alcohols, e.g., ethanol, isopropanol, glycerol, sorbitol, 2-methoxyethanol, diethyleneglycol, ethylene glycol, hexyleneglycol, mannitol, and propylene glycol; ethers such as diethyl or dipropyl ether; polyethylene glycols and methoxypolyoxyethylenes (carbowaxes having molecular weight ranging from 200 to 20,000); polyoxyethylene glycerols, polyoxyethylene sorbitols, stearoyl diacetin, and the like.

Topical compositions of the present invention can, if desired, be presented in a pack or dispenser device, such as an FDA-approved kit, which may contain one or more unit dosage forms containing the active ingredient. The dispenser device may, for example, comprise a tube. The pack or dispenser device may be accompanied by instructions for administration. The pack or dispenser device may also be accompanied by a notice in a form prescribed by a governmental agency regulating the manufacture, use, or sale of pharmaceuticals, which notice is reflective of approval by the agency of the form of the compositions for human or veterinary administration. Such notice, for example, may include labeling approved by the U.S. Food and Drug Administration for prescription drugs or of an approved product insert. Compositions comprising the topical composition of the disclosure formulated in a pharmaceutically acceptable carrier may also be prepared, placed in an appropriate container, and labeled for treatment of an indicated condition.

Another patch system configuration which can be used by the present disclosure is a reservoir transdermal system design which is characterized by the inclusion of a liquid compartment containing a drug solution or suspension separated from the release liner by a semi-permeable membrane and adhesive. The adhesive component of this patch system can either be incorporated as a continuous layer between the membrane and the release liner or in a concentric configuration around the membrane. Yet another patch system configuration which can be utilized by the present invention is a matrix system design which is characterized by the inclusion of a semisolid matrix containing a drug solution or suspension which is in direct contact with the release liner. The component responsible for skin adhesion is incorporated in an overlay and forms a concentric configuration around the semisolid matrix.

Pharmaceutical compositions of the present invention can be in a form suitable for rectal administration wherein the carrier is a solid. It is preferable that the mixture forms unit dose suppositories. Suitable carriers include cocoa butter and other materials commonly used in the art. The suppositories can be conveniently formed by first admixing the composition with the softened or melted carrier(s) followed by chilling and shaping in molds.

Pharmaceutical compositions containing a compound, and/or pharmaceutically acceptable salts thereof, of the present invention can also be prepared in powder or liquid concentrate form.

The pharmaceutical composition (or formulation) may be packaged in a variety of ways. Generally, an article for distribution includes a container that contains the pharmaceutical composition in an appropriate form. Suitable containers are well known to those skilled in the art and include materials such as bottles (plastic and glass), sachets, foil blister packs, and the like. The container may also include a tamper proof assemblage to prevent indiscreet access to the contents of the package. In addition, the container typically has deposited thereon a label that describes the contents of the container and any appropriate warnings or instructions.

The pharmaceutical compositions of the present invention may, if desired, be presented in a pack or dispenser device which may contain one or more unit dosage forms containing the active ingredient. The pack may for example comprise metal or plastic foil, such as a blister pack. The pack or dispenser device may be accompanied by instructions for administration. The pack or dispenser may also be accompanied with a notice associated with the container in form prescribed by a governmental agency regulating the manufacture, use, or sale of pharmaceuticals, which notice is reflective of approval by the agency of the form of the drug for human or veterinary administration. Such notice, for example, may be the labeling approved by the U.S. Food and Drug Administration for prescription drugs, or the approved product insert. Pharmaceutical compositions comprising a disclosed compound formulated in a compatible pharmaceutical carrier may also be prepared, placed in an appropriate container, and labeled for treatment of an indicated condition.

The exact dosage and frequency of administration depends on the particular compound, a pharmaceutically acceptable salt, solvate, or polymorph thereof, a hydrate thereof, a solvate thereof, a polymorph thereof, or a stereochemically isomeric form thereof; the particular condition being treated and the severity of the condition being treated; various factors specific to the medical history of the subject to whom the dosage is administered such as the age; weight, sex, extent of disorder and general physical condition of the particular subject, as well as other medication the individual may be taking; as is well known to those skilled in the art. Furthermore, it is evident that said effective daily amount may be lowered or increased depending on the response of the treated subject and/or depending on the evaluation of the physician prescribing the compounds of the present disclosure.

Depending on the mode of administration, the pharmaceutical composition will comprise from 0.05 to 99 % by weight, preferably from 0.1 to 70 % by weight, more preferably from 0.1 to 50 % by weight of the active ingredient, and, from 1 to 99.95 % by weight, preferably from 30 to 99.9 % by weight, more preferably from 50 to 99.9 % by weight of a pharmaceutically acceptable carrier, all percentages being based on the total weight of the composition.

In the treatment conditions which require of inhibition dihydroorotate dehydrogenase activity an appropriate dosage level will generally be about 0.01 to 1000 mg per kg patient body weight per day and can be administered in single or multiple doses. In various aspects, the dosage level will be about 0.1 to about 500 mg/kg per day, about 0.1 to 250 mg/kg per day, or about 0.5 to 100 mg/kg per day. A suitable dosage level can be about 0.01 to 1000 mg/kg per day, about 0.01 to 500 mg/kg per day, about 0.01 to 250 mg/kg per day, about 0.05 to 100 mg/kg per day, or about 0.1 to 50 mg/kg per day. Within this range the dosage can be 0.05 to 0.5, 0.5 to 5.0 or 5.0 to 50 mg/kg per day. For oral administration, the compositions are preferably provided in the form of tablets containing 1.0 to 1000 mg of the active ingredient, particularly 1.0, 5.0, 10, 15, 20, 25, 50, 75, 100, 150, 200, 250, 300, 400, 500, 600, 750, 800, 900 and 1000 mg of the active ingredient for the symptomatic adjustment of the dosage of the patient to be treated. The compound can be administered on a regimen of 1 to 4 times per day, preferably once or twice per day. This dosing regimen can be adjusted to provide the optimal therapeutic response.

Such unit doses as described hereinabove and hereinafter can be administered more than once a day, for example, 2, 3, 4, 5 or 6 times a day. Such unit doses can be administered 1 or 2 times per day, so that the total dosage for a 70 kg adult is in the range of 0.001 to about 15 mg per kg weight of subject per administration. In a further aspect, dosage is 0.01 to about 1.5 mg per kg weight of subject per administration, and such therapy can extend for a number of weeks or months, and in some cases, years. It will be understood, however, that the specific dose level for any particular patient will depend on a variety of factors including the activity of the specific compound employed; the age, body weight, general health, sex and diet of the individual being treated; the time and route of administration; the rate of excretion; other drugs that have previously been administered; and the severity of the particular disease undergoing therapy, as is well understood by those of skill in the area.

A typical dosage can be one 1 mg to about 100 mg tablet or 1 mg to about 300 mg taken once a day, or, multiple times per day, or one time-release capsule or tablet taken once a day and containing a proportionally higher content of active ingredient. The time-release effect can be obtained by capsule materials that dissolve at different pH values, by capsules that release slowly by osmotic pressure, or by any other known means of controlled release.

It can be necessary to use dosages outside these ranges in some cases as will be apparent to those skilled in the art. Further, it is noted that the clinician or treating physician will know how and when to start, interrupt, adjust, or terminate therapy in conjunction with individual patient response.

Also described, but not claimed, is a method for the manufacture of a medicament for modulating dihydroorotate dehydrogenase activity (e.g., treatment of one or more disorders, such as a cancer or a graft-versus-host-disease, that can be treated via inhibition of dihydroorotate dehydrogenase dysfunction activity) in mammals (e.g., humans) comprising combining one or more disclosed compounds, products, or compositions with a pharmaceutically acceptable carrier or diluent Also described, but not claimed, is a method for manufacturing a medicament comprising combining at least one compound of the present invention with a pharmaceutically acceptable carrier or diluent.

The pharmaceutical compositions can further comprise other therapeutically active compounds, which are usually applied in the treatment of the above mentioned pathological or clinical conditions.

It is understood that the compositions of the present invention can be prepared from the compounds of the present invention.

As already mentioned, the present invention relates to a pharmaceutical composition comprising a therapeutically effective amount of a compound, a pharmaceutically acceptable salt, a hydrate thereof, a solvate thereof, a polymorph thereof, of the present invention, and a pharmaceutically acceptable carrier. Additionally, also described, but not claimed is a process for preparing such a pharmaceutical composition, characterized in that a pharmaceutically acceptable carrier is intimately mixed with a therapeutically effective amount of a compound according to the present invention.

As already mentioned, the present invention also relates to a pharmaceutical composition comprising a compound, a pharmaceutically acceptable salt, a hydrate thereof, a solvate thereof, a polymorph thereof, of the present invention, and one or more other drugs in the treatment, prevention, control, amelioration, or reduction of risk of diseases or conditions for a disclosed compound or the other drugs may have utility as well as to the use of such a composition for the manufacture of a medicament. Also described, but not claimed, is a combination of a compound, a pharmaceutically acceptable salt, a hydrate thereof, a solvate thereof, a polymorph thereof, of the present invention and a therapeutic agent that can be used to treat autoimmune diseases, immune and inflammatory diseases, destructive bone disorders, malignant neoplastic diseases, angiogenic-related disorders, viral diseases, and infectious diseases. Also described, but not claimed, is such a combination for use as a medicine. Also described, but not claimed, is a product comprising (a) compound, a product of a disclosed method of making, a pharmaceutically acceptable salt, a hydrate thereof, a solvate thereof, a polymorph thereof, of the present invention and (b) an additional therapeutic agent, as a combined preparation for simultaneous, separate or sequential use in the treatment or prevention of a condition in a mammal, including a human, the treatment or prevention of which is affected or facilitated by the modulatory effect of the disclosed compound and the additional therapeutic agent. The different drugs of such a combination or product may be combined in a single preparation together with pharmaceutically acceptable carriers or diluents, or they may each be present in a separate preparation together with pharmaceutically acceptable carriers or diluents.

### Methods of Using the Compounds.

The references to methods of treatment herein are to be interpreted as references to the compounds (and pharmaceutically acceptable salts thereof), pharmaceutical compositions and medicaments of the present invention for use in a method for treatment of the human (or animal) body by therapy (or for diagnosis).

Also described, but not in accordance with the present invention, are methods of treatment comprising administration of a therapeutically effective amount of a compound of the present invention or pharmaceutical composition of the present inventio to a subject in need thereof. In particular, the compounds and pharmaceutical compositions of the present invention can be used in methods of treating a disease or disorder that are associated with increased, aberrant, or dysfunctional levels of dihydroorotate dehydrogenase (DHODH) activity in a cell, tissue, or organism. That is, the compounds and pharmaceutical compositions of the present invention can be used to inhibit DHODH activity in a cell, tissue or organism to provide a clinical or therapeutic benefit to a subject which has been determined to or been diagnosed to have with increased, aberrant, or dysfunctional levels of dihydroorotate dehydrogenase (DHODH) activity.

The subject may have been diagnosed with a need for treatment prior to the administering step. In some aspects of the disclosed method, the subject has been diagnosed with a disorder treatable by inhibition of DHODH and/or a need for inhibition of DHODH prior to the administering step. The subject may have been diagnosed with a cancer, a disorder associated with T-cell proliferation, or a may be at risk for graft-versus-host disease or organ rejection following transplantation prior to the administering step. The subject may have been identified with a need for treatment prior to the administering step.

The compounds of the present invention can be used as single agents or in combination with one or more other drugs in the treatment, prevention, control, amelioration or reduction of risk of the aforementioned diseases, disorders and conditions for which compounds of formula I or the other drugs have utility, where the combination of drugs together are safer or more effective than either drug alone. The other drug(s) can be administered by a route and in an amount commonly used therefore, contemporaneously or sequentially with a disclosed compound. When a compound of the present invention is used contemporaneously with one or more other drugs, a pharmaceutical composition in unit dosage form containing such drugs and the compound of the present invention is preferred. However, the combination therapy can also be administered on overlapping schedules. It is also envisioned that the combination of one or more active ingredients and a compound of the present invention will be more efficacious than either as a single agent.

DHODH is an enzyme that catalyzes the fourth step in the de novo biosynthesis of pyrimidine. It converts dihydroorotate (DHO) to orotate (ORO). Human DHODH is a ubiquitous flavine mononucleotide (FMN) moiety flavoprotein. In a mammalian cell, DHODH is anchored at the inner mitochondrial leaflet and catalyzes the conversion of DHO to ORO, which represents the rate limiting step in the de novo pyrimidine biosynthesis. Kinetic studies indicate a sequential ping-pong mechanism for the conversion of DHO to ORO (e.g., see Knecht et al., Chem. Biol. Interact. 2000, 124, 61-76). The first half-reaction comprises the reduction of DHO to ORO. Electrons are transferred to the FMN which becomes oxidized to dihydroflavin mononucleotide (FMNH2). After dissociation of ORO from the enzyme, FMNH2 is regenerated by a ubiquinone molecule, which is recruited from the inner mitochondrial membrane. Kinetic and structural studies revealed two distinct binding sites for DHO/ORO and ubiquinone, respectively.

Human DHODH is composed of two domains, a large C-terminal domain (Met78-Arg396) and a smaller N-terminal domain (Met30-Leu68), connected by an extended loop. The large C-terminal domain can be best described as an α/β-barrel fold with a central barrel of eight parallel β strands surrounded by eight α helices. The redox site, formed by the substrate binding pocket and the site that binds the cofactor FMN, is located on this large C-terminal domain. The small N-terminal domain, on the other hand, consists of two α helices (labeled α1 and α2), both connected by a short loop. This small N-terminal domain harbors the binding site for the cofactor ubiquinone. The helices α1 and α2 span a slot of about 10×20 Å2 in the so-called hydrophobic patch, with the short α1-α2 loop at the narrow end of that slot. The slot forms the entrance to a tunnel that ends at the FMN cavity nearby the α1-α2 loop. This tunnel narrows toward the proximal redox site and ends with several charged or polar side chains (Gln47, Tyr356, Thr360, and Arg136). Structural clues, as discussed above, along with kinetic studies suggest that ubiquinone, which can easily diffuse into the mitochondrial inner membrane, uses this tunnel to approach the FMN cofactor for the redox reaction (e.g., see Baumgartner et al., J. Med. Chem. 2006, 49, 1239-1247).

In an organism, DHODH catalyzes the synthesis of pyrimidines, which are necessary for cell growth. An inhibition of DHODH inhibits the growth of (pathologically) fast proliferating cells, whereas cells which grow at normal speed may obtain their required pyrimidine bases from the normal metabolic cycle. The most important types of cells for the immune response, the lymphocytes, use exclusively the synthesis of pyrimidines for their growth and react particularly sensitively to DHODH inhibition.

DHODH inhibition results in decreased cellular levels of ribonucleotide uridine monophosphate (rUMP), thus arresting proliferating cells in the GI phase of the cell cycle. The inhibition of de novo pyrimidine nucleotide synthesis is of great interest in view of the observations that lymphocytes seem not to be able to undergo clonal expansion when this pathway is blocked. Substances that inhibit the growth of lymphocytes are important medicaments for the treatment of auto-immune diseases.

During homeostatic proliferation, the salvage pathway which is independent of DHODH seems sufficient for the cellular supply with pyrimidine bases. Only, cells with a high turnover and particularly T and B lymphocytes need the de novo pathway to proliferate. In these cells, DHODH inhibition stops the cell cycle progression suppressing DNA synthesis and consequently cell proliferation.

Therefore, inhibitors of DHODH show beneficial immunosuppressant and antiproliferative effects in human diseases characterized by abnormal and uncontrollable cell proliferation causing chronic inflammation and tissue destruction. The human enzyme dihydroorotate dehydrogenase (DHODH) represents a well-characterized target for small molecular weight Disease Modifying Antirheumatic Drugs (DMARDs).

Accordingly, also described, but not in accordance with the present invention, are methods of treating a variety of diseases or disorders, including, but not limited to, autoimmune diseases, immune and inflammatory diseases, destructive bone disorders, cancers and malignant neoplastic diseases, angiogenic-related disorders, viral diseases, and infectious diseases.

Also described, but not in accordance with the present invention, are methods for treating an immunological disorder, inflammatory disorder, cancer or other proliferative disease via inhibition of DHODH by administering to a subject in need of such treatment an effective amount of at least one compound or at least one pharmaceutical composition of the present invention.

Also described, but not in accordance with the present invention, is a method for treating an immunological disorder, inflammatory disorder, cancer or other proliferative disease via inhibition of DHODH by administering to a patient in need of such treatment an effective amount of at least one compound or at least one pharmaceutical composition of the present invention in combination (simultaneously or sequentially) with at least one other antiinflammatory, immunomodulator or anti-cancer agent.

An autoimmune disorder or disease that can be treated by the compounds or pharmaceutical compositions of the present invention include, but are not limited, one selected from lupus, rheumatoid arthritis, ankylosing spondylitis, glomerulonephritis, minimal change disease, ulcerative colitis, crohns disease, addison's disease, adult Still's disease, alopecia areata, autoimmune hepatitis, autoimmune angioedema, Bechet's disease, pemphigoid and variants, celiac disease, chronic inflammatory demyelinating polyneuropathy, churg-Straus syndrome, Crest syndrome, dermatomyositis, neuromyelitis optica, discoid lupus, fibromyalgia, giant cell arteritis, giant cell myocarditis, Goodpasteur's disease, evan's syndrome, autoimmune hemolytic anemia, immune thrombocytopenia, Henoch-Schonlein purpura, IgA nephropathy, IgG4 related sclerosing disease, juvenile arthritis, juvenile diabetes, Kawasaki disease, Leukocytoclastic vasculitis, mixed connective disease, multiple sclerosis, multifocal motor neuropathy, myasthenia gravis, autoimmune neutropenia, optic neuritis, peripheral neuropathy, POEMS syndrome, polymyositis, primary biliary cirrhosis, non-alcoholic hepatosteotosis and associated cirrhosis, psoriasis, scleroderma, sarcoidosis, temporal arteritis, vasculitis, and uveitis.

Autoimmune diseases that can be treated by the compounds or pharmaceutical compositions of the present invention include, but are not limited, to rheumatoid arthritis, psoriatic arthritis, systemic lupus erythematosus, multiple sclerosis, psoriasis, ankylosing spondilytis, Wegener's granulomatosis, polyarticular juvenile idiopathic arthritis, inflammatory bowel disease such as ulcerative colitis and Crohn's disease, Reiter's syndrome, fibromyalgia and type-1 diabetes.

Immune and inflammatory diseases that can be treated by the compounds or pharmaceutical compositions of the present invention include, but are not limited, to asthma, COPD, respiratory distress syndrome, acute or chronic pancreatitis, graft versus-host disease, chronic sarcoidosis, transplant rejection, contact dermatitis, atopic dermatitis allergic rhinitis, allergic conjunctivitis, Behget's syndrome, inflammatory eye conditions such as conjunctivitis and uveitis.

Also described, but not in accordance with the present invention, are methods for treating organ rejection diseases or ameliorating and/or preventing organ rejection diseases in patients pre-disposed to organ rejection by administering to a patient in need of such treatment an effective amount of at least one compound or pharmaceutical composition of the present invention. The patient may have received an organ transplant or is diagnosed as requiring an organ transplant. The organ transplant can include, but is not limited to, a transplanted organ of the kidney, liver, skin, heart, pancreas, lung, or combinations thereof.

Also described, but not in accordance with the present invention, are methods for treating EBV viral lymphoproliferation in the setting of tumor immunosuppression. The method of treating EBV viral lymphoproliferation can be to provide both continued organ transplantation preservation and also treatment of the underlying EBV lymphoproliferation.

Destructive bone disorders that can be treated by the compounds or pharmaceutical compositions of the present invention include, but are not limited, to osteoporosis, osteoarthritis and multiple myeloma-related bone disorder.

Cancers and malignant neoplastic that can be treated by the compounds or pharmaceutical compositions of the present invention include, but are not limited, to prostate, ovarian and brain cancer. Carcinoma, including that of the bladder, breast, colon, kidney, liver, lung, including small cell lung cancer, esophagus, gall bladder, ovary, pancreas, stomach, cervix, thyroid, prostate, and skin, including squamous cell carcinoma; hematopoietic tumors of lymphoid lineage, including leukemia, acute lymphocytic leukemia, acute lymphoblastic leukemia, B-cell lymphoma, T-cell lymphoma, Hodgkin's lymphoma, non-Hodgkins lymphoma, hairy cell lymphoma and Burkett's lymphoma; hematopoietic tumors of myeloid lineage, including acute and chronic myelogenous leukemias, myelodysplastic syndrome and promyelocytic leukemia; tumors of mesenchymal origin, including fibrosarcoma and rhabdomyosarcoma; tumors of the central and peripheral nervous system, including astrocytoma, neuroblastoma, glioma and schwannomas; and other tumors, including melanoma, seminoma, teratocarcinoma, osteosarcoma, xenoderoma pigmentosum, keratoctanthoma, thyroid follicular cancer and Kaposi's sarcoma.

Angiogenesis-related disorders that can be treated by the compounds or pharmaceutical compositions of the present invention include, but are not limited, to hemangiomas, ocular neovascularization, macular degeneration or diabetic retinopathy.

Viral diseases that can be treated by the compounds or pharmaceutical compositions of the present invention include, but are not limited, to HIV infection, hepatitis and cytomegalovirus infection.

Infectious diseases that can be treated by the compounds or pharmaceutical compositions of the present invention include, but are not limited, to sepsis, septic shock, endotoxic shock, Gram negative sepsis, toxic shock syndrome, Shigellosis and other protozoal infestations such as malaria.

The compounds or pharmaceutical compositions of the present invention can act as modulators of apoptosis, and accordingly, can be useful in the treatment of cancer (including but not limited to those types mentioned herein above), viral infections (including but not limited to herpes virus, poxvirus, Epstein-Barr virus, Sindbis virus and adenovirus), prevention of AIDS development in HIV-infected individuals, autoimmune diseases (including but not limited to systemic lupus, erythematosus, autoimmune mediated glomerulonephritis, rheumatoid arthritis, psoriasis, inflammatory bowel disease, and autoimmune diabetes mellitus), neurodegenerative disorders (including but not limited to Alzheimer's disease, AIDS-related dementia, Parkinson's disease, amyotrophic lateral sclerosis, retinitis pigmentosa, spinal muscular atrophy and cerebellar degeneration), myelodysplastic syndromes, aplastic anemia, ischemic injury associated with myocardial infarctions, stroke and reperfusion injury, arrhythmia, atherosclerosis, toxin-induced or alcohol related liver diseases, hematological diseases (including but not limited to chronic anemia and aplastic anemia), degenerative diseases of the musculoskeletal system (including but not limited to osteoporosis and arthritis) aspirin-sensitive rhinosinusitis, cystic fibrosis, multiple sclerosis, kidney diseases and cancer pain.

The compounds or pharmaceutical compositions of the present invention can act to modulate the level of cellular RNA and DNA synthesis. Accordingly, the compounds and pharmaceutical compositions of the present invention can be used in the treatment of viral infections (including but not limited to HIV, human papilloma virus, herpesvirus, poxvirus, Epstein-Barr virus, Sindbis virus and adenovirus).

The compounds or pharmaceutical compositions of the present invention can be used in the chemoprevention of cancer. Chemoprevention is understood to be a clinical intervention to inhibit the development of invasive cancer by either blocking the initiating mutagenic event or by blocking the progression of pre-malignant cells that have already suffered an insult or inhibiting tumor relapse. Accordingly, the compounds and pharmaceutical compositions of the present invention can be used in inhibiting tumor angiogenesis and metastasis.

The compounds and pharmaceutical compositions of the present invention can also be combined with other active compounds in the treatment of diseases wherein the inhibition of DHODH is known to show beneficial effect.

The diseases, conditions or disorders that can benefit from inhibition of DHODH include, but are not limited to, an immune system-related disease (e.g., an autoimmune disease), a disease or disorder involving inflammation (e.g., asthma, chronic obstructive pulmonary disease, rheumatoid arthritis, inflammatory bowel disease, glomerulonephritis, neuroinflammatory diseases, multiple sclerosis, uveitis and disorders of the immune system), cancer or other proliferative disease, hepatic diseases or disorders, renal diseases or disorders.

The compounds and pharmaceutical compositions of the present invention can be used as immunosuppressants to prevent transplant graft rejections, allogeneic or xenogeneic transplantation rejection (organ, bone marrow, stem cells, other cells and tissues), and graft-versus-host disease. In other embodiments, transplant graft rejections result from tissue or organ transplants. In further embodiments, graft-versus-host disease results from bone marrow or stem cell transplantation.

The compounds and pharmaceutical compositions of the present invention can be used in the treatment of a variety of inflammatory diseases including, but not limited to, inflammation, glomerulonephritis, uveitis, hepatic diseases or disorders, renal diseases or disorders, chronic obstructive pulmonary disease, rheumatoid arthritis, inflammatory bowel disease, vasculitis, dermatitis, osteoarthritis, inflammatory muscle disease, allergic rhinitis, vaginitis, interstitial cystitis, scleroderma, osteoporosis, eczema, allogeneic or xenogeneic transplantation, graft rejection, graft-versus-host disease, corneal transplant rejection, lupus erythematosus, systemic lupus erythematosus, proliferative lupus nephritis, type I diabetes, pulmonary fibrosis, dermatomyositis, thyroiditis, myasthenia gravis, autoimmune hemolytic anemia, cystic fibrosis, chronic relapsing hepatitis, primary biliary cirrhosis, allergic conjunctivitis, hepatitis and atopic dermatitis, asthma and Sjogren's syndrome.

The compounds and pharmaceutical compositions of the present invention can be used in the treatment of a variety of diseases including Felty's syndrome, Wegener's granulomatosis, Crohn's disease, sarcoidosis, Still's disease, pemphigoid, Takayasu arteritis, systemic sclerosis, relapsing polychondritis, refractory IgA nephropathy, SAPHO2 syndrome (SAS), cytomegalovirus infection including rhinitis or cyst, psoriasis, IGG4 disease, and multiple myeloma.

The compounds and pharmaceutical compositions of the present invention can be used in combination (administered together or sequentially) with known anti-cancer treatments such as radiation therapy or with cytostatic or cytotoxic or anticancer agents, such as for example, but not limited to, DNA interactive agents, such as cisplatin or doxorubicin; topoisomerase II inhibitors, such as etoposide; topoisomerase I inhibitors such as CPT-11 or topotecan; tubulin interacting agents, such as paclitaxel, docetaxel or the epothilones (for example ixabepilone), either naturally occurring or synthetic; hormonal agents, such as tamoxifen; thymidilate synthase inhibitors, such as 5-fluorouracil; and anti-metabolites, such as methotrexate, other tyrosine kinase inhibitors such as Iressa and OSI-774; angiogenesis inhibitors; BTK inhibitors, SYK inhibitors, ITK inhibitors, PI3-kinase inhibitors, FLT3 inhibitors, EGF inhibitors; PAK inhibitors, VEGF inhibitors; CDK inhibitors; SRC inhibitors; c-Kit inhibitors; Her1/2 inhibitors and monoclonal antibodies directed against growth factor receptors such as erbitux (EGF) and herceptin (Her2) and other protein kinase modulators as well. These agents can be used in combination with differentiation agents such as ATRA, EZH2 inhibitors, DNMT inhibitors, corticosteroids, IDH1 inhibitors, IDH2 inhibitors, and Vitamin C. These agents can be used in combination with small molecules that enhance DNA damage killing in cancer cells including PARP inhibitors, MDM2 inhibitors, NAMPT inhibitors, and HSP90 inhibitors. These agents can be used in combination with antibodies that target cell surface molecules on immune or cancer cells including but not limited to CD33, CD37, CD19, CD20, CD3, CD123, CD70, BAFFR, CD4, CD8, CD56, and CD38. These agents can be used in combination with antibodies or peptides which neutralize cytokines including, but not limited to IL1Beta, IL6, IL10, IL21, TNFA, TNFB, and IFN. These agents can be used in combination with cellular CAR-T cells to diminish cellular proliferation in the setting of significant cytokine release syndrome and neurotoxicity. These agents can be used to diminish T-cell proliferation, cytokine production, and neurotoxicity in combination with bi-specific antibodies or peptide molecules that target in a dual manner T-cells and immune/tumor cell antigens such as, but not limited to CD19, CD20 CD33, CD123, CD38, and CD37. These agents can be used to diminish T-cell proliferation and tissue damage caused by immune check point inhibitor antibodies to targets such as, but not limited to PD1, PDL1, CTLA4, and LAG3.

Diseases, disorders or conditions that can be treated or prevented using the compounds and pharmaceutical compositions of the present invention are capable of inhibiting DHODH, and accordingly, useful in the treatment of diseases, conditions or disorders involving inflammation and/or that are related to the immune system. These diseases include, but are not limited, to asthma, chronic obstructive pulmonary disease, rheumatoid arthritis, inflammatory bowel disease, glomerulonephritis, neuroinflammatory diseases such as multiple sclerosis, and disorders of the immune system.

The compounds and pharmaceutical compositions of the present invention can be used for treating immune and immune-related disorders, including, for example, chronic immune diseases/disorders, acute immune diseases/disorders, autoimmune and immunodeficiency diseases/disorders, diseases/disorders involving inflammation, organ transplant graft rejections and graft-versus-host disease and altered (e.g., hyperactive) immune responses. Other exemplary immune disorders that can be treated using the compounds and pharmaceutical compositions of the present invention include psoriasis, rheumatoid arthritis, vasculitis, inflammatory bowel disease, dermatitis, osteoarthritis, asthma, inflammatory muscle disease, allergic rhinitis, vaginitis, interstitial cystitis, scleroderma, osteoporosis, eczema, allogeneic or xenogeneic transplantation (organ, bone marrow, stem cells and other cells and tissues) graft rejection, graft-versus-host disease, lupus erythematosus, inflammatory disease, type I diabetes, pulmonary fibrosis, dermatomyositis, Sjogren's syndrome, thyroiditis (e.g., Hashimoto's and autoimmune thyroiditis), myasthenia gravis, autoimmune hemolytic anemia, multiple sclerosis, cystic fibrosis, chronic relapsing hepatitis, primary biliary cirrhosis, allergic conjunctivitis and atopic dermatitis.

Chronic graft-versus-host disease (cGVHD) is a primary cause of nonrelapse mortality after allogeneic hematopoietic stem cell transplantation (HSCT) (Baird K, Pavletic SZ. Curr Opin Hematol. 2006; 13(6):426-435; Lee SJ, Vogelsang G, Flowers ME. Biol Blood Marrow Transplant. 2003; 9(4):215-233; Pidala J, et al. Blood. 2011; 117(17):4651-4657; and Arai S, et al. Blood. 2011; 118(15):4242-4249). Drug therapy for cGVHD has been predominantly limited to steroids and calcineurin inhibitors, which are incompletely effective and associated with infections as well as long-term risks of toxicity (Holler, E. Best Pract Res Clin Haematol. 2007; 20(2):281-294). The disclosed compounds can be used for the treatment of cGVHD.

### Kits.

Also described, but not claimed, are kits comprising a therapeutically effective amount of at least one compound, or a pharmaceutically acceptable salt thereof, of the present invention or a disclosed pharmaceutical composition of the present invention; and: at least one agent known to treat a cancer, a host-versus-graft-disease, and/or a disorder associated with T-cell proliferation; and instructions for treating a cancer, a host-versus-graft-disease, and/or a disorder associated with T-cell proliferation.

The compounds and/or pharmaceutical compositions of the presenet invention can conveniently be presented as a kit, whereby two or more components, which may be active or inactive ingredients, carriers, diluents, and the like, are provided with instructions for preparation of the actual dosage form by the patient or person administering the drug to the patient. Such kits may be provided with all necessary materials and ingredients contained therein, or they may contain instructions for using or making materials or components that must be obtained independently by the patient or person administering the drug to the patient. In further aspects, a kit can include optional components that aid in the administration of the unit dose to patients, such as vials for reconstituting powder forms, syringes for injection, customized IV delivery systems, inhalers, etc. Additionally, a kit can contain instructions for preparation and administration of the compositions. The kit can be manufactured as a single use unit dose for one patient, multiple uses for a particular patient (at a constant dose or in which the individual compounds may vary in potency as therapy progresses); or the kit may contain multiple doses suitable for administration to multiple patients ("bulk packaging"). The kit components may be assembled in cartons, blister packs, bottles, tubes, and the like.

The disclosed kits can be packaged in a daily dosing regimen (e.g., packaged on cards, packaged with dosing cards, packaged on blisters or blow-molded plastics, etc.). Such packaging promotes products and increases patient compliance with drug regimens. Such packaging can also reduce patient confusion. Such kits may further contain instructions for use.

The kit may comprise one or more containers filled with one or more of the ingredients of the pharmaceutical compositions of the invention. Associated with such container(s) can be a notice in the form prescribed by a governmental agency regulating the manufacture, use or sale of pharmaceuticals or biological products, which notice reflects approval by the agency of manufacture, use or sale for human administration.

The kits can also comprise compounds and/or products co-packaged, co-formulated, and/or co-delivered with other components. For example, a drug manufacturer, a drug reseller, a physician, a compounding shop, or a pharmacist can provide a kit comprising a compound of the present invention and/or another component for delivery to a patient.

It is contemplated that the disclosed kits can be used in connection with the described methods of using or treating, and/or the compositions of the present invention.

### Research Tools.

The compounds and pharmaceutical compositions of the present invention have activity as inhibitors of DHODH activity or inhibitors of cell proliferation. As such, the compounds of the present invention are also useful as research tools. Accordingly, also described, but not claimed, is a method of using a compound of the invention as a research tool, the method comprising conducting a biological assay using a compound of the invention. Compounds of the invention can also be used to evaluate new chemical compounds. Thus, also described, but not claimed, is a method of evaluating a test compound in a biological assay, comprising: (a) conducting a biological assay with a test compound to provide a first assay value; (b) conducting the biological assay with a compound of the invention to provide a second assay value; wherein step (a) is conducted either before, after or concurrently with step (b); and (c) comparing the first assay value from step (a) with the second assay value from step (b). Exemplary biological assays include an *in vitro* DHODH enzymatic assay or in a cell culture-based assay measuring cell proliferation. Methods suitable for carrying out such assays are described herein. Also described, but not claimed, is a method of studying a biological system, e.g., a model animal for a clinical condition, or biological sample comprising a DHODH protein, the method comprising: (a) contacting the biological system or sample with a compound of the invention; and (b) determining the effects caused by the compound on the biological system or sample.

It is also to be understood that the terminology used herein is for the purpose of describing particular aspects only, and is not intended to be limiting. The skilled artisan will recognize many variants and adaptations of the aspects described herein. These variants and adaptations are intended to be included in the teachings of this disclosure and to be encompassed by the claims herein. Now having described the aspects of the present disclosure, in general, the following Examples describe some additional aspects of the present disclosure. While aspects of the present disclosure are described in connection with the following examples and the corresponding text and figures, there is no intent to limit aspects of the present disclosure to this description. On the contrary, the intent is to cover all alternatives, modifications, and equivalents included within the spirit and scope of the present disclosure.

### EXAMPLES

The following examples are put forth so as to provide those of ordinary skill in the art with a complete disclosure and description of how the compounds, compositions, articles, devices and/or methods claimed herein are made and evaluated, and are intended to be purely exemplary of the disclosure and are not intended to limit the scope of what the inventors regard as their disclosure. Efforts have been made to ensure accuracy with respect to numbers (*e.g*., amounts, temperature, etc.), but some errors and deviations should be accounted for. Unless indicated otherwise, parts are parts by weight, temperature is in °C or is at ambient temperature, and pressure is at or near atmospheric.

### 1. EXAMPLE 1: SYNTHESIS OF REPRESENTATIVE DISCLOSED COMPOUNDS

**Synthesis of 2-(4'-ethoxy-2-fluoro-[1,1'-biphenyl]-4-yl)-6-fluoroquinoline-4-carboxylic acid (Cpd1).** Following the synthetic procedure described herein below, the target compound, 2-(4'-ethoxy-2-fluoro-[1,1'-biphenyl]-4-yl)-6-fluoroquinoline-4-carboxylic acid **(Cpd1)** was prepared.

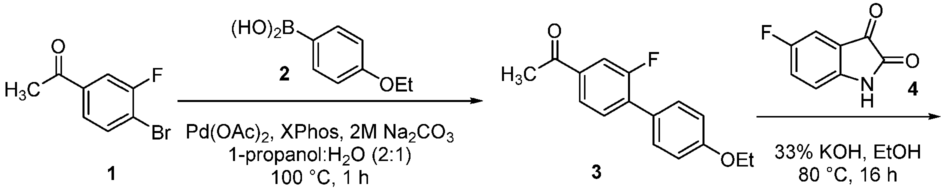

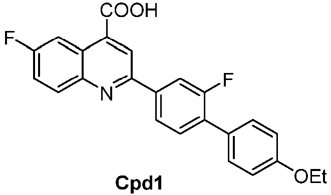

**Step 1** - **Suzuki coupling.** To a solution of 1-(4-bromo-3-fluorophenyl)ethan-1-one 1 (300 mg, 1.38 mmol), 4-ethoxyphenylboronic acid 2 (252 mg, 1.52 mmol) in 1-propanol (3.2 mL, 0.43 M), palladium acetate (1.5 mg, 0.007 mmol), XPhos (9 mg, 0.019 mmol), aqueous sodium carbonate ( 2M, 1.50 mL), and then water (0.10 mL) were added. The reaction mixture was stirred at 100 °C for 1 h (monitored by TLC) cooled to room temperature, diluted with EtOAc (40 mL) and water (30 mL), organic layer separated washed with brine (30 mL). Organic layer dried over anhydrous Na₂SO₄, filtered and concentrated under reduced pressure to obtain crude material. Obtained crude material was purified on flash column chromatography using methylene chloride/ hexanes to afford1-(4'-ethoxy-2-fluoro-[1,1'-biphenyl]-4-yl)ethan-1-one (3) as white solid (305 mg, 85% yield).

**Step 2** - **Pfitzinger Reaction.** The mixture of 5-fluoroisatin 4 (174 mg, 1.06 mmol) and aqueous potassium hydroxide solution (33%, 4.8 mL, 0.22 M) was stirred and heated gently until clear yellow solution formed. To this solution, the slurry of 1-(4'-ethoxy-2-fluoro-[1,1'-biphenyl]-4-yl)ethan-1-one 3 (300 mg, 1.16 mmol) in ethanol (7.1 mL, 0.15M) was added. The reaction mixture was heated up to reflux with stirring for 16 hours (reaction progress was monitored by TLC) at 80 °C, then cooled down to room temperature, ethanol was evaporated, and aqueous layer was acidified with aq. HCl (2M) to pH 2.

**Workup and Purification Procedure I:** The yellow solid was formed, the mixture was extracted with EtOAc (3 × 30 mL). Combined organic layer was washed with brine (30 mL). Organic layer dried over anhydrous Na₂SO₄ and concentrated to get crude material which was purified using flash column chromatography using methanol/dichloromethane as eluent to get yellow solid which was triturated with methanol, dichloromethane and/or ethyl acetate to obtain 2-(4'-ethoxy-2-fluoro-[1,1'-biphenyl]-4-yl)-6-fluoroquinoline-4-carboxylic acid **(Cpd1)** as an off-white solid.

**Workup and Purification Procedure II:** An alternative workup procedure for the Pfitzinger reaction is as follows. The acidified mixture with the resulting product that crashed out was stirred at room temperature for 2 h to get a free-flowing solid that was then filtered. The solid was washed with a small amount of methanol to remove trapped water and dried under vacuum. The resulting powder was triturated with dichloromethane, ethyl acetate, and/or methanol, filtered, and dried under vacuum to afford 2-(4'-ethoxy-2-fluoro-[1,1'-biphenyl]-4-yl)-6-fluoroquinoline-4-carboxylic acid **(Cpd1)** as an off-white solid (204 mg, 48% yield).

**Synthesis of 2-(3'-ethoxy-2-fluoro-[1,1'-biphenyl]-4-yl)-6-fluoroquinoline-4-carboxylic acid (Cpd2)**. Following synthetic procedures similar to those described for the preparation of **Cpd1,** the target compound 2-(3'-ethoxy-2-fluoro-[1,1'-biphenyl]-4-yl)-6-fluoroquinoline-4-carboxylic acid **(Cpd2)** was prepared as follows.

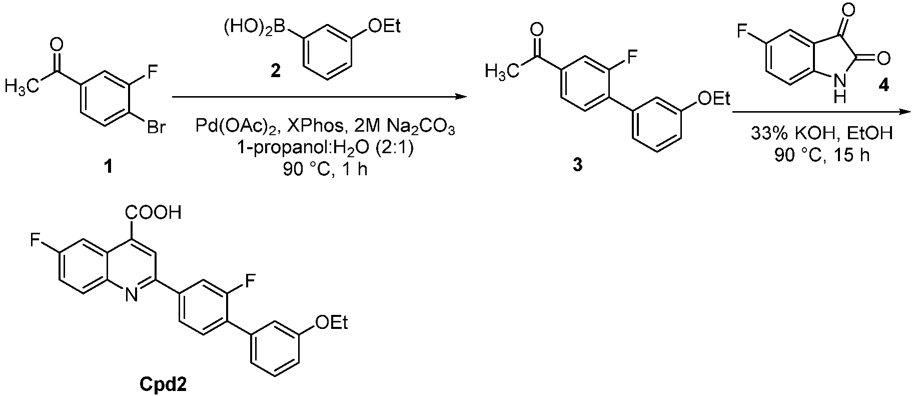

**Step 1** was conducted on 300 mg of 1-(4-bromo-3-fluorophenyl)ethan-1-one and 275 mg of (3-ethoxyphenyl)boronic acid to afford 1-(3'-ethoxy-2-fluoro-[1,1'-biphenyl]-4-yl)ethan-1-one as a light pink solid (327 mg, 90% yield). **Step 2** (workup/purification procedure I) was conducted on 125 mg of 5-fluoroisatin and 200 mg of 1-(3'-ethoxy-2-fluoro-[1,1'-biphenyl]-4-yl)ethan-1-one to afford 2-(3'-ethoxy-2-fluoro-[1,1'-biphenyl]-4-yl)-6-fluoroquinoline-4-carboxylic acid **(Cpd2)** as an off-white solid (60 mg, 20% yield).

**Synthesis of 2-(3'-butoxy-2-fluoro-[1,1'-biphenyl]-4-yl)-6-fluoroquinoline-4-carboxylic acid (Cpd3).** Following synthetic procedures similar to those described for the preparation of **Cpd1,** the target compound 2-(3'-butoxy-2-fluoro-[1,1'-biphenyl]-4-yl)-6-fluoroquinoline-4-carboxylic acid **(Cpd3)** was prepared as follows.

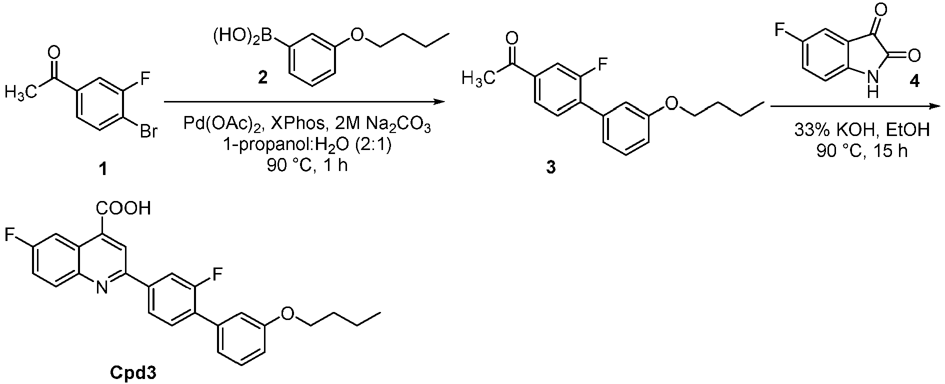

**Step 1** was conducted on 300 mg of 1-(4-bromo-3-fluorophenyl)ethan-1-one and 322 mg of (3-butoxyphenyl)boronic acid to afford 1-(3'-butoxy-2-fluoro-[1,1'-biphenyl]-4-yl)ethan-1-one as a colorless oil (369 mg, 93% yield). **Step 2** (workup/ purification procedure I) was conducted on 105 mg of 5-fluoroisatin and 200 mg of 1-(3'-butoxy-2-fluoro-[1,1'-biphenyl]-4-yl)ethan-1-one to afford 2-(3'-butoxy-2-fluoro-[1,1'-biphenyl]-4-yl)-6-fluoroquinoline-4-carboxylic acid **(Cpd3)** as an off-white solid (55 mg, 20% yield) after collecting only pure factions from column chromatography in ethyl acetate and hexane and triturating with hexanes.

**Synthesis of 2-(3'-butoxy-2-fluoro-[1,1'-biphenyl]-4-yl)-6-fluoroquinoline-4-carboxylic acid (Cpd4).** Following synthetic procedures similar to those described for the preparation of **Cpd1,** the target compound 2-(3'-butoxy-2-fluoro-[1,1'-biphenyl]-4-yl)-6-fluoroquinoline-4-carboxylic acid **(Cpd4),** was prepared as follows. Compound 4 is not in accordance with the present invention.

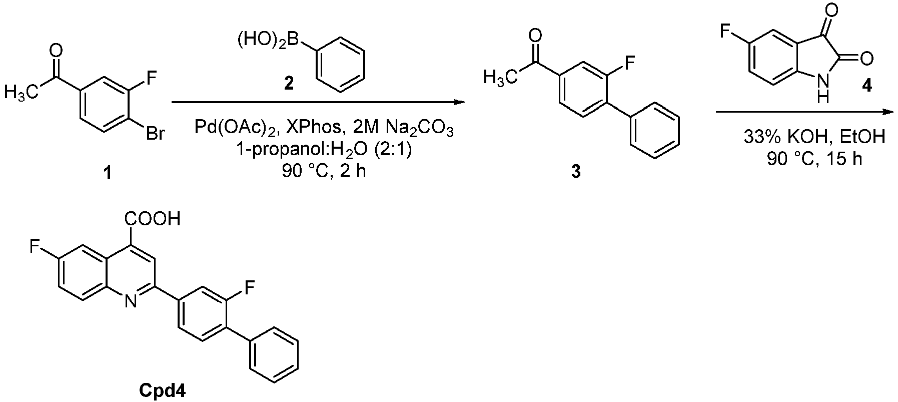

**Step 1** was conducted on 300 mg of 1-(4-bromo-3-fluorophenyl)ethan-1-one and 185 mg of phenylboronic acid to afford 1-(2-fluoro-[1,1'-biphenyl]-4-yl)ethan-1-one as white solid (265 mg, 89% yield). **Step 2** was conducted on 147 mg of 5-fluoroisatin and 200 mg of 1-(2-fluoro-[1,1'-biphenyl]-4-yl)ethan-1-one to afford 233 mg (~90% purity) of 2-(3'-butoxy-2-fluoro-[1,1'-biphenyl]-4-yl)-6-fluoroquinoline-4-carboxylic acid **(Cpd4).** Out of 233 mg, 110 mg was used to recrystallization using DMSO/water to afford as a white solid (54 mg, 19% yield).

**Synthesis of 2-(2,2'-difluoro-[1,1'-biphenyl]-4-yl)-6-fluoroquinoline-4-carboxylic acid (Cpd5).** Following synthetic procedures similar to those described for the preparation of **Cpd1,** the target compound 2-(2,2'-difluoro-[1,1'-biphenyl]-4-yl)-6-fluoroquinoline-4-carboxylic acid **(Cpd5)** was prepared as follows.

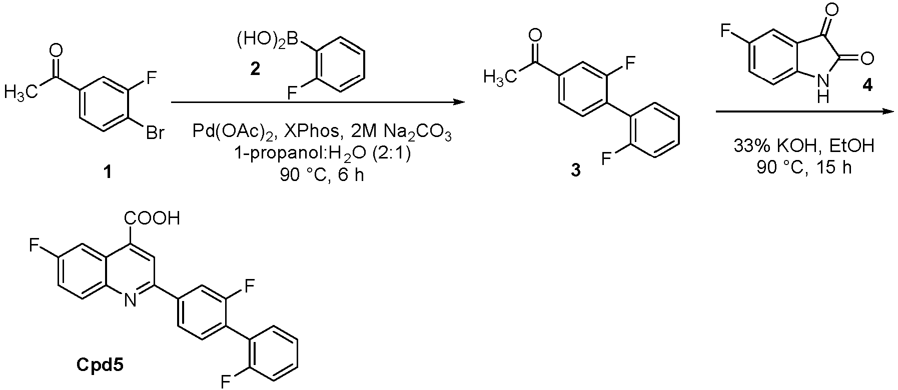

**Step 1** was conducted on 300 mg of 1-(4-bromo-3-fluorophenyl)ethan-1-one and 185 mg of (2-fluorophenyl)boronic acid to afford 1-(2,2'-difluoro-[1,1'-biphenyl]-4-yl)ethan-1-one as a white solid (164 mg, 46% yield). **Step 2** (workup/purification procedure II) was conducted on 108 mg of 5-fluoroisatin and 160 mg of 1-(2,2'-difluoro-[1,1'-biphenyl]-4-yl)ethan-1-one to afford 236 mg (~90% purity) of 2-(2,2'-difluoro-[1,1'-biphenyl]-4-yl)-6-fluoroquinoline-4-carboxylic acid **(Cpd5).** To improve the purity it was triturated with mixture of ethyl acetate once and 10%methanol/dichloromethane once to afford 2-(2,2'-difluoro-[1,1'-biphenyl]-4-yl)-6-fluoroquinoline-4-carboxylic acid **(Cpd5)** as an off-white solid (52 mg, 21% yield).

**Synthesis of 2-(4'-(ethylamino)-2-fluoro-[1,1'-biphenyl]-4-yl)-6-fluoroquinoline-4-carboxylic acid (Cpd6).** Following synthetic procedures similar to those described for the preparation of **Cpd1,** the target compound 2-(4'-(ethylamino)-2-fluoro-[1,1'-biphenyl]-4-yl)-6-fluoroquinoline-4-carboxylic acid **(Cpd6)** was prepared as follows.

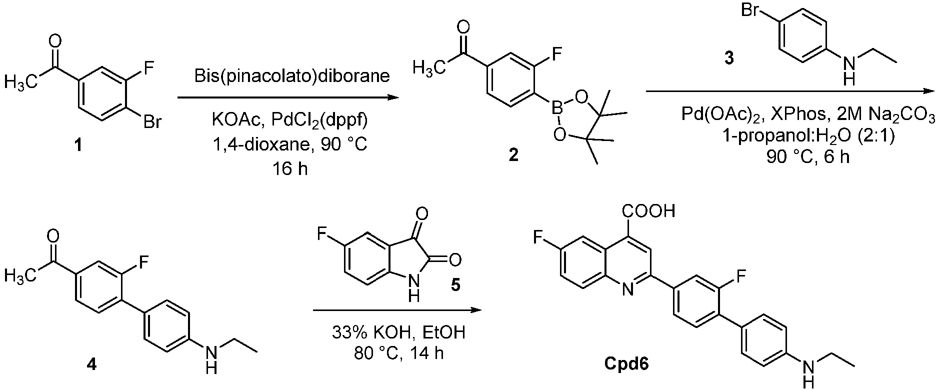

**Step 1.** To a solution of 1-(4-bromo-3-fluorophenyl)ethan-1-one 1 (50 mg, 2.30 mmol) in 1,4-dioxane (5 mL) was added bis(pinacolato)diborane (614 mg, 2.42 mmol) and potassium acetate (677 mg, 6.90 mmol). The reaction mixture was bubbled with argon for 5 min and PdCl₂(dppf) (84 mg, 0.12 mmol) was added and the reaction mixture was stirred at 90 °C for 16 h. The reaction mixture was diluted with ethyl acetate (50 mL) and water (50 mL). Organic layer separated, washed with brine (40 mL), dried over anhydrous sodium sulfate and concentrated to get crude material. Obtained crude material was purified using flash column chromatography using ethyl acetate/ hexanes to afford 1-(3-fluoro-4-(4,4,5,5-tetramethyl-1,3,2-dioxaborolan-2-yl)phenyl)ethan-1-one (460 mg, 76% yield) as an off-white solid. **Step 2** (workup/purification procedure II) was conducted on 450 mg of 1-(3-fluoro-4-(4,4,5,5-tetramethyl-1,3,2-dioxaborolan-2-yl)phenyl)ethan-1-one and 375 mg of 4-bromo-N-ethylaniline to afford 1-(4'-(ethylamino)-2-fluoro-[1,1'-biphenyl]-4-yl)ethan-1-one as a white solid (310 mg, 71% yield). Workup/purification was conducted on 88 mg of 5-fluoroisatin and 150 mg of 1-(4'-(ethylamino)-2-fluoro-[1,1'-biphenyl]-4-yl)ethan-1-one to afford 2-(4'-(ethylamino)-2-fluoro-[1,1'-biphenyl]-4-yl)-6-fluoroquinoline-4-carboxylic acid **(Cpd6)** as a white solid (93 mg, 43% yield).

**Synthesis of 2-(4'-ethoxy-2,5-difluoro-[1,1'-biphenyl]-4-yl)-6-fluoroquinoline-4-carboxylic acid (Ccd7).** Following synthetic procedures similar to those described for the preparation of **Cpd1,** the target compound 2-(4'-ethoxy-2,5-difluoro-[1,1'-biphenyl]-4-yl)-6-fluoroquinoline-4-carboxylic acid **(Cpd7)** was prepared as follows.

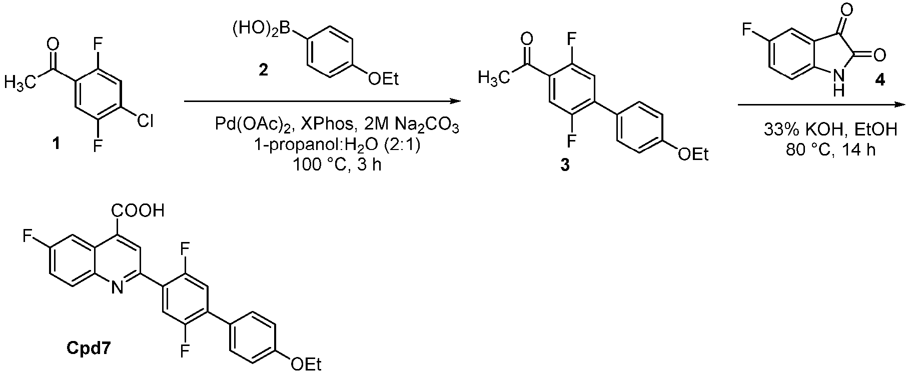

**Step 1** was conducted on 300 mg of 1-(4-chloro-2, 5-difluorophenyl)ethan-1-one and 274 mg of (4-ethoxyphenyl)boronic acid to afford 1-(4'-ethoxy-2,5-difluoro-[1,1'-biphenyl]-4-yl)ethan-1-one as an off-white solid (340 mg, 78% yield). **Step 2** (workup/purification procedure II) was conducted on 120 mg of 5-fluoroisatin and 200 mg of 1-(4'-ethoxy-2,5-difluoro-[1,1'-biphenyl]-4-yl)ethan-1-one to afford 2-(4'-ethoxy-2,5-difluoro-[1,1'-biphenyl]-4-yl)-6-fluoroquinoline-4-carboxylic acid **(Cpd7)** as an off-white solid (240 mg, 78% yield).

**Synthesis of 2-(3'-ethoxy-2,5-difluoro-[1,1'-biphenyl]-4-yl-6-fluoroquinoline-4-carboxylic acid (Cpd8).** Following synthetic procedures similar to those described for the preparation of **Cpd1,** the target compound 2-(3'-ethoxy-2,5-difluoro-[1,1'-biphenyl]-4-yl)-6-fluoroquinoline-4-carboxylic acid **(Cpd8)** was prepared as follows.

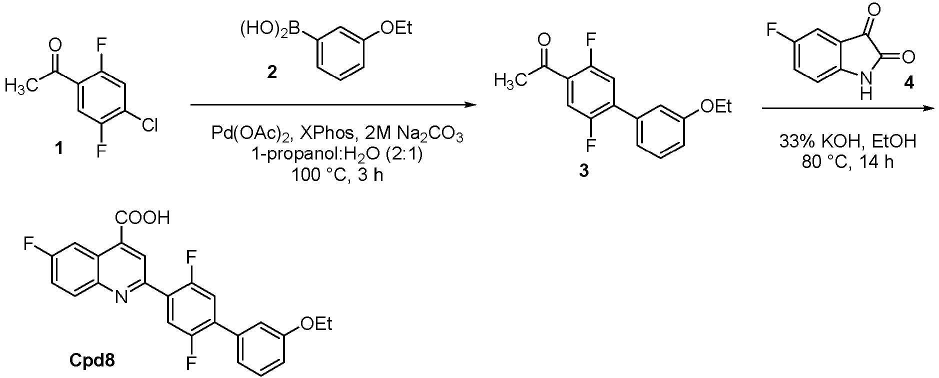

**Step 1** was conducted on 300 mg of 1-(4-chloro-2, 5-difluorophenyl)ethan-1-one and 274 mg of (3-ethoxyphenyl)boronic acid to afford 1-(3'-ethoxy-2,5-difluoro-[1,1'-biphenyl]-4-yl)ethan-1-one as an off-white solid (338 mg, 78% yield). **Step 2** (workup/purification procedure II) was conducted on 120 mg of 5-fluoroisatin and 200 mg of 1-(3'-ethoxy-2,5-difluoro-[1,1'-biphenyl]-4-yl)ethan-1-one to afford 260 mg (84%) of 2-(3'-ethoxy-2,5-difluoro-[1,1'-biphenyl]-4-yl)-6-fluoroquinoline-4-carboxylic acid **(Cpd8)** as an off-white solid (260 mg, 84% yield).

**Synthesis of 2-(4'-ethoxy-3-fluoro-[1,1'-biphenyl]-4-yl)-6-fluoroquinoline-4-carboxylic acid (Cpd9).** Following synthetic procedures similar to those described for the preparation of **Cpd1,** the target compound 2-(4'-ethoxy-3-fluoro-[1,1'-biphenyl]-4-yl)-6-fluoroquinoline-4-carboxylic acid **(Cpd9)** was prepared as follows.

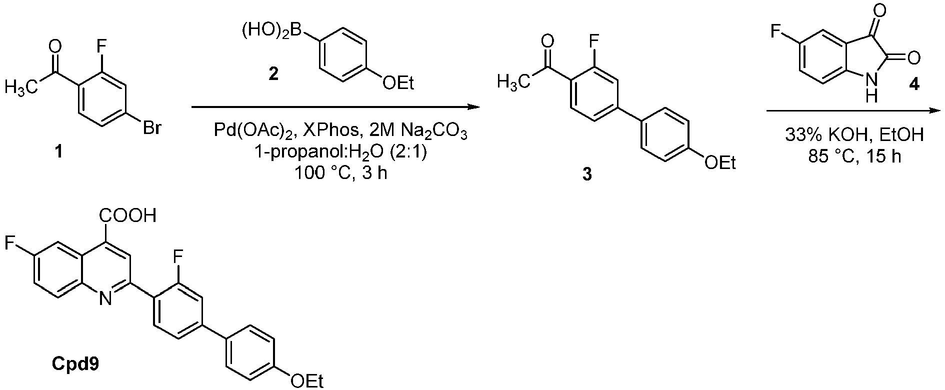

**Step 1** was conducted on 300 mg of 1-(4-bromo-2-fluorophenyl)ethan-1-one and 241 mg of (4-ethoxyphenyl)boronic acid to afford 1-(4'-ethoxy-3-fluoro-[1,1'-biphenyl]-4-yl)ethan-1-one as an off-white solid (300 mg, 84% yield). **Step 2** (workup/purification procedure II) was conducted on 127 mg of 5-fluoroisatin and 200 mg of 1-(4'-ethoxy-3-fluoro-[1,1'-biphenyl]-4-yl)ethan-1-one to afford 2-(4'-ethoxy-3-fluoro-[1,1'-biphenyl]-4-yl)-6-fluoroquinoline-4-carboxylic acid **(Cpd9)** as an off-white solid (150 mg, 48% yield).

**Synthesis of 2-(3'-ethoxy-3-fluoro-[1,1'-biphenyl]-4-yl)-6-fluoroquinoline-4-carboxylic acid (Cpd10).** Following synthetic procedures similar to those described for the preparation of **Cpd1,** the target compound 2-(3'-ethoxy-3-fluoro-[1,1'-biphenyl]-4-yl)-6-fluoroquinoline-4-carboxylic acid **(Cpd10)** was prepared as follows.

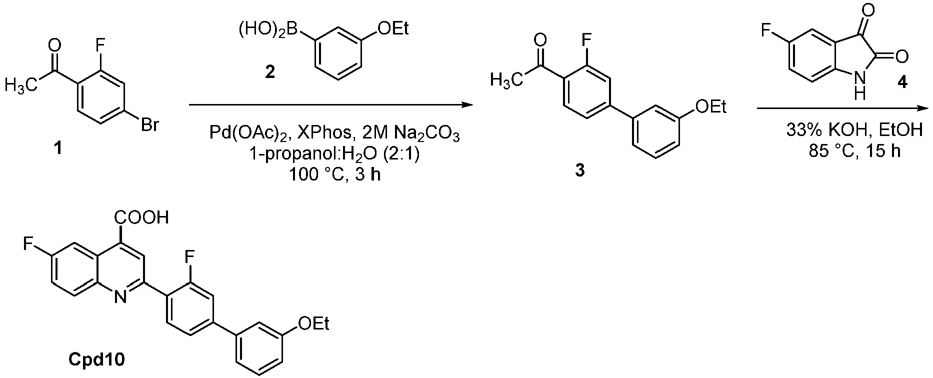

**Step 1** was conducted on 300 mg of 1-(4-bromo-2-fluorophenyl)ethan-1-one and 241 mg of (3-ethoxyphenyl)boronic acid to afford 313 mg (88%) of 1-(3'-ethoxy-3-fluoro-[1,1'-biphenyl]-4-yl)ethan-1-one as an off-white solid (313 mg, 88% yield). **Step 2** (workup/purification procedure II) was conducted on 127 mg of 5-fluoroisatin and 200 mg of 1-(4'-ethoxy-3-fluoro-[1,1'-biphenyl]-4-yl)ethan-1-one to afford 65 mg (21%) of 2-(3'-ethoxy-3-fluoro-[1,1'-biphenyl]-4-yl)-6-fluoroquinoline-4-carboxylic acid **(Cpd10)** as an off-white solid (65 mg, 21% yield).

**Synthesis of 2-(3'-butoxy-2,5-difluoro-[1,1'-biphenyl]-4-yl)-6-fluoroquinoline-4-carboxylic acid (Cpd11).** Following synthetic procedures similar to those described for the preparation of **Cpd1,** the target compound 2-(3'-butoxy-2,5-difluoro-[1,1'-biphenyl]-4-yl)-6-fluoroquinoline-4-carboxylic acid **(Cpd11)** was prepared as follows.

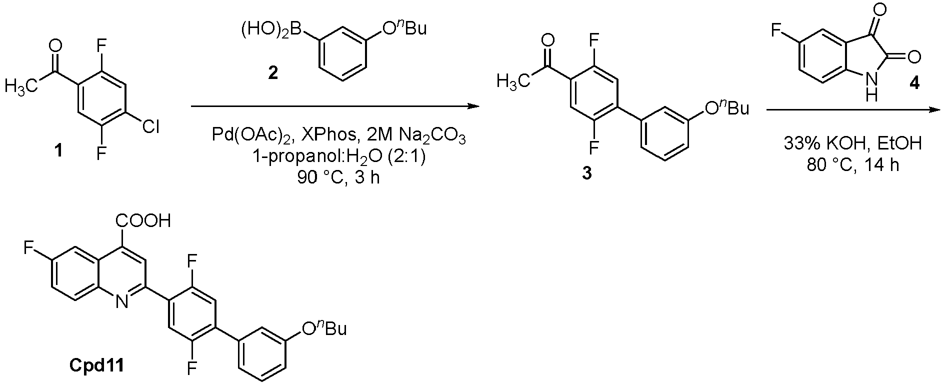

**Step 1** was conducted on 200 mg of 1-(4-chloro-2, 5-difluorophenyl)ethan-1-one and 214 mg of (3-butoxyphenyl)boronic acid to afford 1-(3'-butoxy-2,5-difluoro-[1,1'-biphenyl]-4-yl)ethan-1-one as an off-white solid (214 mg, 66% yield). **Step 2** (workup/purification procedure II) was conducted on 114 mg of 5-fluoroisatin and 200 mg of 1-(3'-butoxy-2,5-difluoro-[1,1'-biphenyl]-4-yl)ethan-1-one to afford 46 mg (15%) of 2-(3'-butoxy-2,5-difluoro-[1,1'-biphenyl]-4-yl)-6-fluoroquinoline-4-carboxylic acid **(Cpd11)** as ab off-white solid (46 mg, 15% yield).

**Synthesis of 2-(3'-butoxy-3-fluoro-[1,1'-biphenyl]-4-yl)-6-fluoroquinoline-4-carboxylic acid (Cpd12).** Following synthetic procedures similar to those described for the preparation of **Cpd1,** the target compound 2-(3'-butoxy-3-fluoro-[1,1'-biphenyl]-4-yl)-6-fluoroquinoline-4-carboxylic acid **(Cpd12)** was prepared as follows.

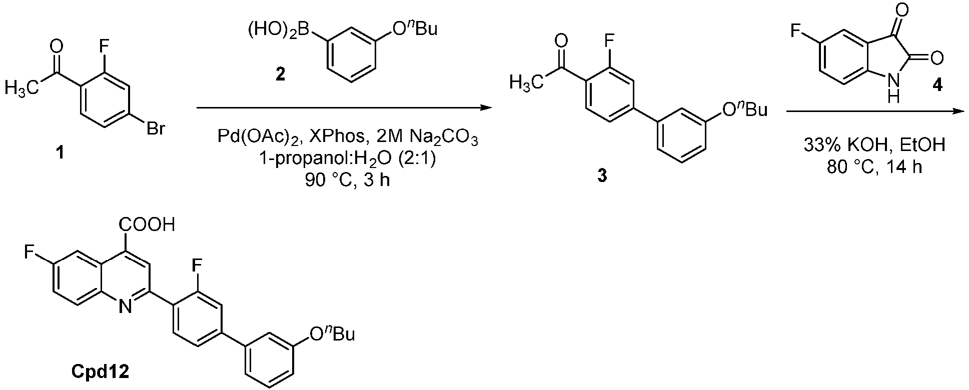

**Step** 1 was conducted on 200 mg of 1-(4-bromo-2-fluorophenyl)ethan-1-one and 188 mg of (3-butoxyphenyl)boronic acid to afford 1-(3'-butoxy-3-fluoro-[1,1'-biphenyl]-4-yl)ethan-1-one as an off-white solid (230 mg, 66% yield). **Step 2** (workup/purification procedure II) was conducted on 114 mg of 5-fluoroisatin and 200 mg of 1-(3'-butoxy-3-fluoro-[1,1'-biphenyl]-4-yl)ethan-1-one to afford 2-(3'-butoxy-3-fluoro-[1,1'-biphenyl]-4-yl)-6-fluoroquinoline-4-carboxylic acid **(Cpd12)** as an off-white solid (89 mg, 29%).

**Synthesis of 2-(4'-ethoxy-2-methyl-[1,1'-biphenyl]-4-yl)-6-fluoroquinoline-4-carboxylic acid (Cpd13).** Following synthetic procedures similar to those described for the preparation of **Cpd1,** the target compound 2-(4'-ethoxy-2-methyl-[1,1'-biphenyl]-4-yl)-6-fluoroquinoline-4-carboxylic acid **(Cpd13)** was prepared as follows.

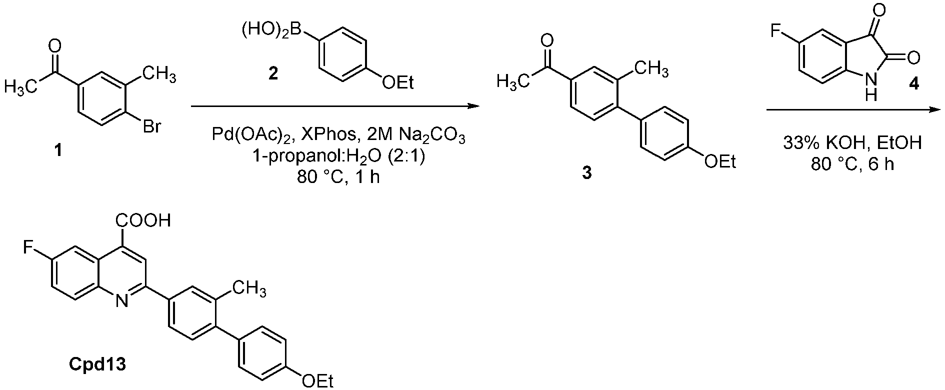

**Step** 1 was conducted on 300 mg of 1-(4-bromo-3-methylphenyl)ethan-1-one and 245 mg of (4-ethoxyphenyl)boronic acid to afford 1-(4'-ethoxy-2-methyl-[1,1'-biphenyl]-4-yl)ethan-1-one as an off-white solid (223 mg, 62% yield). **Step 2** (workup/purification procedure II) was conducted on 130 mg of 5-fluoroisatin and 200 mg of 1-(4'-ethoxy-2-methyl-[1,1'-biphenyl]-4-yl)ethan-1-one to afford 2-(4'-ethoxy-2-methyl-[1,1'-biphenyl]-4-yl)-6-fluoroquinoline-4-carboxylic acid **(Cpd13)** as a white solid (171 mg, 54%).

**Synthesis of 2-(4'-ethoxy-2-methyl-[1,1'-biphenyl]-4-yl)-6-fluoroquinoline-4-carboxylic acid (Cpd14).** Following synthetic procedures similar to those described for the preparation of **Cpd1,** the target compound 2-(4'-ethoxy-2-methyl-[1,1'-biphenyl]-4-yl)-6-fluoroquinoline-4-carboxylic acid **(Cpd14)** was prepared as follows.

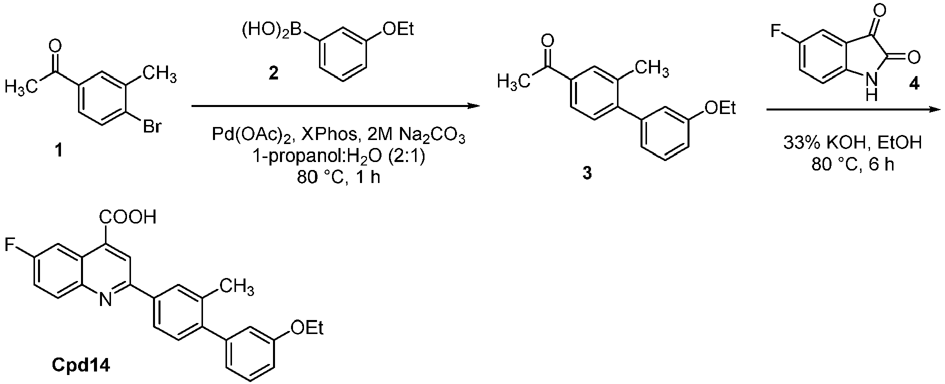

**Step 1** was conducted on 300 mg of 1-(4-bromo-3-methylphenyl)ethan-1-one and 245 mg of (3-ethoxyphenyl)boronic acid to afford 1-(3'-ethoxy-2-methyl-[1,1'-biphenyl]-4-yl)ethan-1-one as a white solid (212 mg, 59% yield). **Step 2** (workup/purification procedure II) was conducted on 130 mg of 5-fluoroisatin and 200 mg of 1-(3'-ethoxy-2-methyl-[1,1'-biphenyl]-4-yl)ethan-1-one to afford 70 mg (22%) of 2-(4'-ethoxy-2-methyl-[1,1'-biphenyl]-4-yl)-6-fluoroquinoline-4-carboxylic acid **(Cpd14)** as a white solid (70 mg, 22% yield).

**Synthesis of 2-(4'-ethoxy-2-methoxy-[1,1'-biphenyl]-4-yl)-6-fluoroquinoline-4-carboxylic acid (Cpd15).** Following synthetic procedures similar to those described for the preparation of **Cpd1,** the target compound 2-(4'-ethoxy-2-methoxy-[1,1'-biphenyl]-4-yl)-6-fluoroquinoline-4-carboxylic acid **(Cpd15)** was prepared as follows.

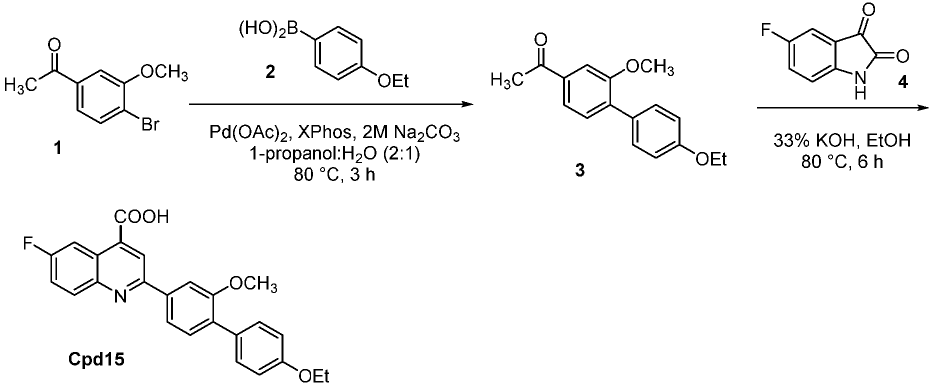

**Step 1** was conducted on 300 mg of 1-(4-bromo-3-methoxyphenyl)ethan-1-one and 228 mg of (4-ethoxyphenyl)boronic acid to afford 1-(4'-ethoxy-2-methoxy-[1,1'-biphenyl]-4-yl)ethan-1-one as a white solid (157 mg, 44% yield). **Step 2** (workup/purification procedure II) was conducted on 91 mg of 5-fluoroisatin and 150 mg of -(4'-ethoxy-2-methoxy-[1,1'-biphenyl]-4-yl)ethan-1-one to afford 2-(4'-ethoxy-2-methoxy-[1,1'-biphenyl]-4-yl)-6-fluoroquinoline-4-carboxylic acid **(Cpd15)** as a yellow solid (165 mg, 72% yield).

**Synthesis of 2-(3'-ethoxy-2-methoxy-[1,1'-biphenyl]-4-yl)-6-fluoroquinoline-4-carboxylic acid (Cpd16).** Following synthetic procedures similar to those described for the preparation of **Cpd1,** the target compound 2-(3'-ethoxy-2-methoxy-[1,1'-biphenyl]-4-yl)-6-fluoroquinoline-4-carboxylic acid **(Cpd16)** was prepared as follows.

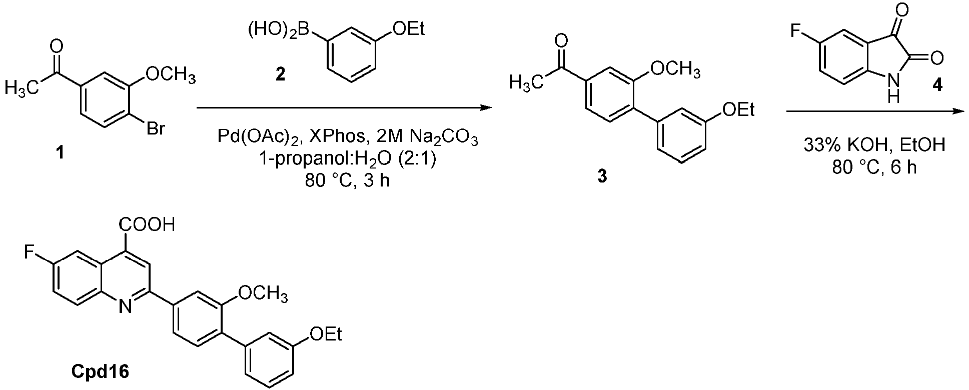

**Step 1** was conducted on 300 mg of 1-(4-bromo-3-methoxyphenyl)ethan-1-one and 228 mg of (3-ethoxyphenyl)boronic acid to afford 276 mg (78%) of 1-(3'-ethoxy-2-methoxy-[1,1'-biphenyl]-4-yl)ethan-1-one as a colorless oil (276 mg, 78% yield). **Step 2** (workup/purification procedure II) was conducted on 122 mg of 5-fluoroisatin and 200 mg of 1-(3'-ethoxy-2-methoxy-[1,1'-biphenyl]-4-yl)ethan-1-one to afford 2-(3'-ethoxy-2-methoxy-[1,1'-biphenyl]-4-yl)-6-fluoroquinoline-4-carboxylic acid **(Cpd16)** as a yellow solid (100 mg, 32% yield).

**Synthesis of 2-(4'-ethoxy-3,5-difluoro-[1,1'-biphenyl]-4-yl)-6-fluoroquinoline-4-carboxylic acid (Cpd17).** Following synthetic procedures similar to those described for the preparation of **Cpd1,** the target compound 2-(4'-ethoxy-3,5-difluoro-[1,1'-biphenyl]-4-yl)-6-fluoroquinoline-4-carboxylic acid **(Cpd17)** was prepared as follows.

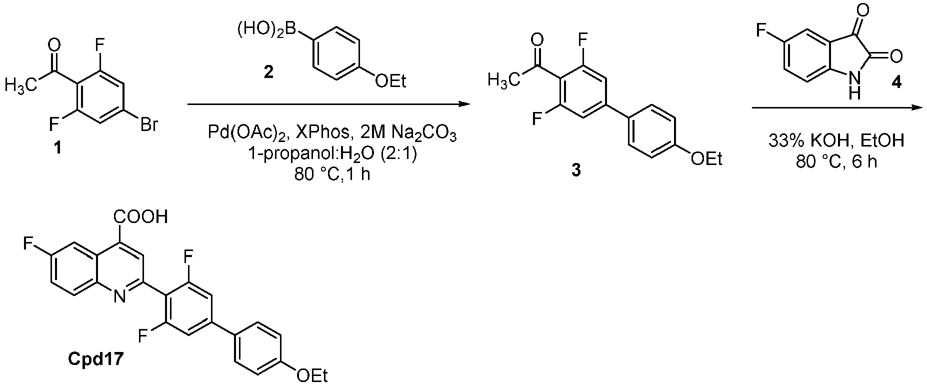

**Step 1** was conducted on 300 mg of 1-(4-bromo-2,6-difluorophenyl)ethan-1-one and 222 mg of (4-ethoxyphenyl)boronic acid to afford 1-(4'-ethoxy-3,5-difluoro-[1,1'-biphenyl]-4-yl)ethan-1-one as a white solid (291 mg, 83% yield). **Step 2** (workup/purification procedure II) was conducted on 120 mg of 5-fluoroisatin and 200 mg of 1-(4'-ethoxy-3,5-difluoro-[1,1'-biphenyl]-4-yl)ethan-1-one to get 293 mg of 2-(4'-ethoxy-3,5-difluoro-[1,1'-biphenyl]-4-yl)-6-fluoroquinoline-4-carboxylic acid however to attain required purity the solid was first triturated with dichloromethane, then with methanol to get 100 mg (~90% purity) solid. Dissolved into 2 mL of DMSO and added drop wisely into the water (10 mL), stirred for 2 h. Obtained solid material was filtered, washed with water and dried to afford of 2-(4'-ethoxy-3,5-difluoro-[1,1'-biphenyl]-4-yl)-6-fluoroquinoline-4-carboxylic acid **(Cpd17)** as a yellow solid (89 mg, 48% yield).

**Synthesis of 2-(4'-ethoxy-3,5-difluoro-[1,1'-biphenyl]-4-yl)-6-fluoroquinoline-4-carboxylic acid (Cpd18).** Following synthetic procedures similar to those described for the preparation of **Cpd1,** the target compound 2-(4'-ethoxy-3,5-difluoro-[1,1'-biphenyl]-4-yl)-6-fluoroquinoline-4-carboxylic acid **(Cpd18)** was prepared as follows.

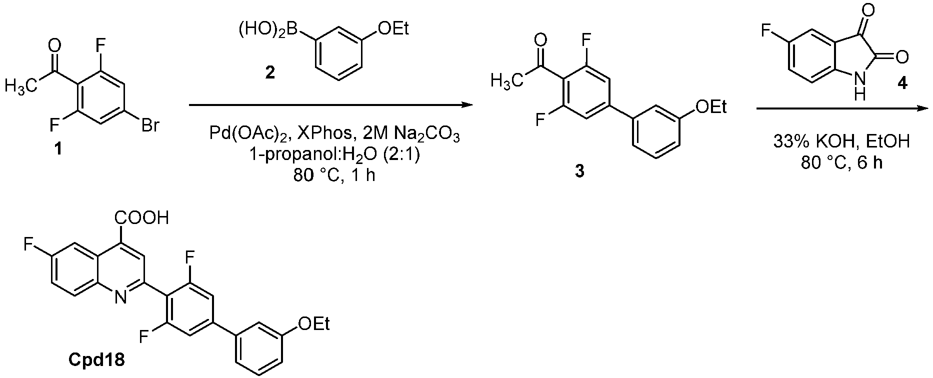

**Step 1** was conducted on 300 mg of 1-(4-bromo-2,6-difluorophenyl)ethan-1-one and 222 mg of (3-ethoxyphenyl)boronic acid to afford 1-(3'-ethoxy-3,5-difluoro-[1,1'-biphenyl]-4-yl)ethan-1-one as a white solid (315 mg, 89% yield). **Step 2** (workup/purification procedure II) was conducted on 120 mg of 5-fluoroisatin and 200 mg of 1-(3'-ethoxy-3,5-difluoro-[1,1'-biphenyl]-4-yl)ethan-1-one to get 100 mg of 2-(3'-ethoxy-2-methoxy-[1,1'-biphenyl]-4-yl)-6-fluoroquinoline-4-carboxylic acid however to attain required purity the solid was dissolved into minimum amount of ethyl acetate and solution was added with hexanes, obtained solid was filtered washed with hexanes to get 56 mg (~90% purity) of solid. Dissolved into1 mL of DMSO and added drop wisely into the water (10 mL), stirred for 4 h. Obtianed solid material was filtered, washed with water and dried to afford 2-(4'-ethoxy-3,5-difluoro-[1,1'-biphenyl]-4-yl)-6-fluoroquinoline-4-carboxylic acid **(Cpd18)** as a light pink solid (41 mg, 13%).

**Synthesis of 2-(2-chloro-4'-ethoxy-[1,1'-biphenyl]-4-yl)-6-fluoroquinoline-4-carboxylic acid (Cpd19).** Following synthetic procedures similar to those described for the preparation of **Cpd1,** the target compound 2-(2-chloro-4'-ethoxy-[1,1'-biphenyl]-4-yl)-6-fluoroquinoline-4-carboxylic acid **(Cpd19)** was prepared as follows.

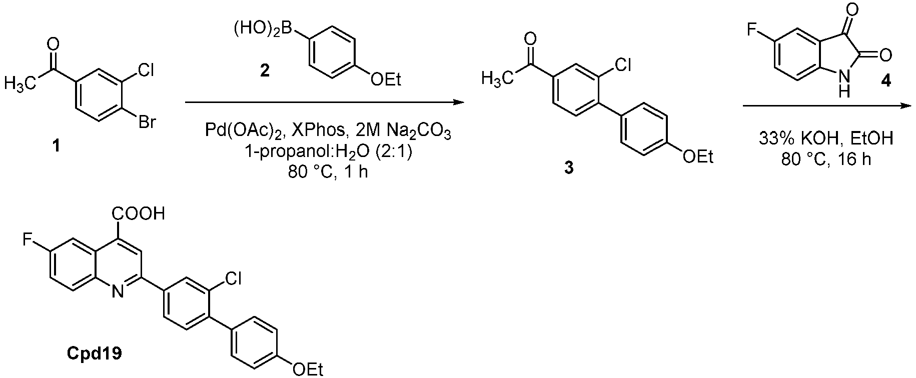

**Step 1** was conducted on 300 mg of 1-(4-bromo-3-chlorophenyl)ethan-1-one and 213 mg of (4-ethoxyphenyl)boronic acid to afford 1-(2-chloro-4'-ethoxy-[1,1'-biphenyl]-4-yl)ethan-1-one as a white solid (233 mg, 66%). **Step 2** (workup/purification procedure II) was conducted on 121 mg of 5-fluoroisatin and 200 mg of 1-(2-chloro-4'-ethoxy-[1,1'-biphenyl]-4-yl)ethan-1-one to afford 2-(2-chloro-4'-ethoxy-[1,1'-biphenyl]-4-yl)-6-fluoroquinoline-4-carboxylic acid **(Cpd19)** as an off-white solid (181 mg, 72%)

**Synthesis of 2-(2-chloro-3'-ethoxy-[1,1'-biphenyl]-4-yl)-6-fluoroquinoline-4-carboxylic acid (Cpd20).** Following synthetic procedures similar to those described for the preparation of **Cpd1,** the target compound 2-(2-chloro-3'-ethoxy-[1,1'-biphenyl]-4-yl)-6-fluoroquinoline-4-carboxylic acid **(Cpd20)** was prepared as follows.

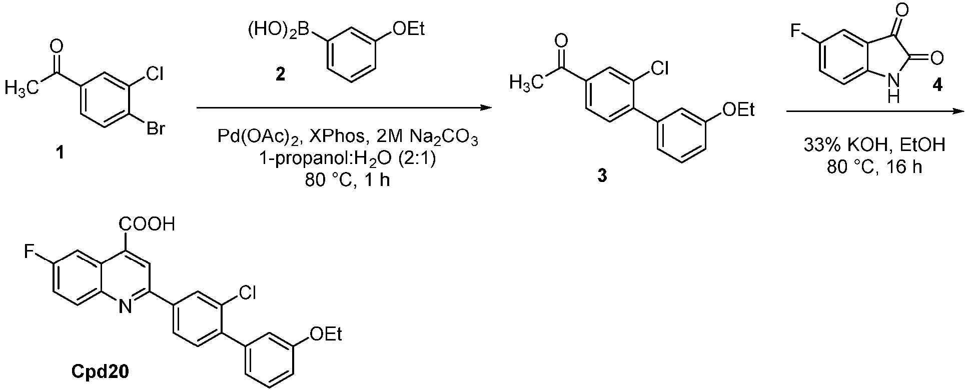

**Step 1** was conducted on 300 mg of 1-(4-bromo-3-chlorophenyl)ethan-1-one and 213 mg of (3-ethoxyphenyl)boronic acid to afford 1-(2-chloro-3'-ethoxy-[1,1'-biphenyl]-4-yl)ethan-1-one as a colorless oil (250 mg, 71%). **Step 2** (workup/purification procedure II) was conducted on 121 mg of 5-fluoroisatin and 200 mg of 1-(2-chloro-3'-ethoxy-[1,1'-biphenyl]-4-yl)ethan-1-one to afford 2-(2-chloro-3'-ethoxy-[1,1'-biphenyl]-4-yl)-6-fluoroquinoline-4-carboxylic acid **(Cpd20)** as a light pink solid (136 mg, 44%).

**Synthesis of 2-(4'-ethoxy-3-methyl-[1,1'-biphenyl]-4-yl)-6-fluoroquinoline-4-carboxylic acid (Cpd21).** Following synthetic procedures similar to those described for the preparation of **Cpd1,** the target compound 2-(4'-ethoxy-3-methyl-[1,1'-biphenyl]-4-yl)-6-fluoroquinoline-4-carboxylic acid **(Cpd21)** was prepared as follows.

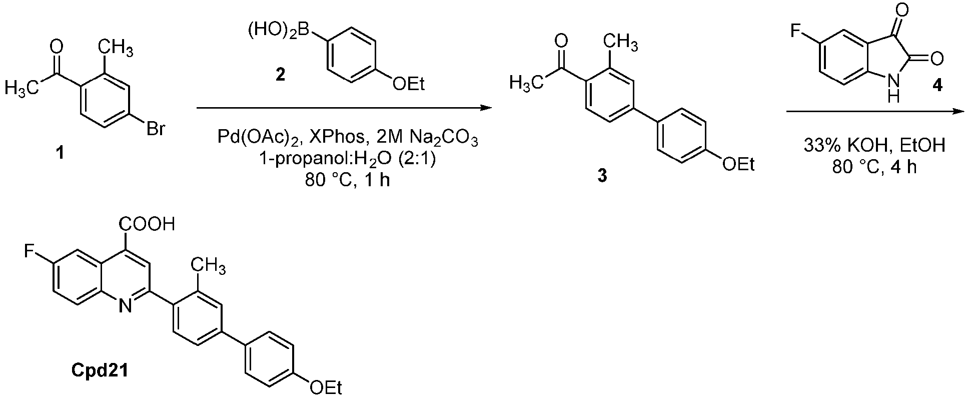

**Step** 1 was conducted on 300 mg of 1-(4-bromo-2-methylphenyl)ethan-1-one and 245 mg of (4-ethoxyphenyl)boronic acid to afford 1-(4'-ethoxy-3-methyl-[1,1'-biphenyl]-4-yl)ethan-1-one as a white solid (247 mg, 69% yield). **Step** 2 (workup/purification procedure II) was conducted on 143 mg of 5-fluoroisatin and 200 mg of 1-(4'-ethoxy-3-methyl-[1,1'-biphenyl]-4-yl)ethan-1-one to afford 2-(4'-ethoxy-3-methyl-[1,1'-biphenyl]-4-yl)-6-fluoroquinoline-4-carboxylic acid **(Cpd21)** as an off-white solid (184 mg, 58% yield).

**Synthesis of 2-(3'-butoxy-3-methyl-[1,1'-biphenyl]-4-yl)-6-fluoroquinoline-4-carboxylic acid (Cpd22).** Following synthetic procedures similar to those described for the preparation of **Cpd1,** the target compound 2-(3'-butoxy-3-methyl-[1,1'-biphenyl]-4-yl)-6-fluoroquinoline-4-carboxylic acid **(Cpd22)** was prepared as follows.

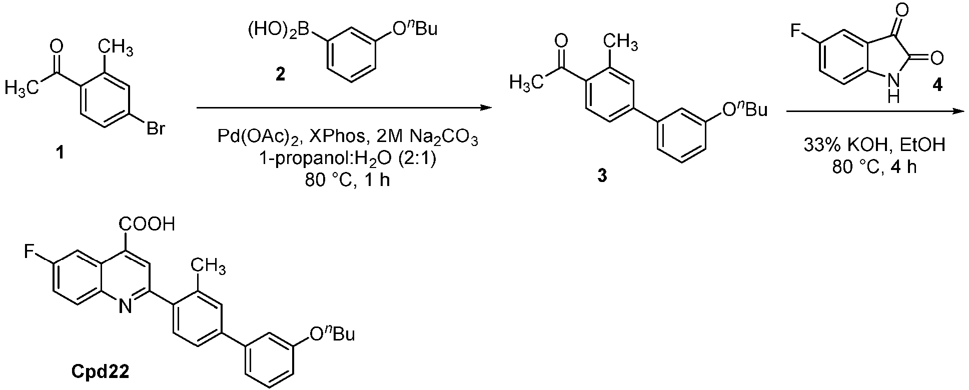

**Step 1** was conducted on 300 mg of 1-(4-bromo-2-methylphenyl)ethan-1-one and 273 mg of (3-butoxyphenyl)boronic acid to afford 1-(3'-butoxy-3-methyl-[1,1'-biphenyl]-4-yl)ethan-1-one as a pale yellow viscous oil (335 mg, 84% yield). **Step 2** (workup/purification procedure-I) was conducted on 129 mg of 5-fluoroisatin and 200 mg of 1-(3'-butoxy-3-methyl-[1,1'-biphenyl]-4-yl)ethan-1-one to afford 2-(3'-butoxy-3-methyl-[1,1'-biphenyl]-4-yl)-6-fluoroquinoline-4-carboxylic acid **(Cpd22)** as a white solid (45 mg, 15% yield).

**Synthesis of 2-(4'-ethoxy-3-methoxy-[1,1'-biphenyl]-4-yl)-6-fluoroquinoline-4-carboxylic acid (Cpd23).** Following synthetic procedures similar to those described for the preparation of **Cpd1,** the target compound 2-(4'-ethoxy-3-methoxy-[1,1'-biphenyl]-4-yl)-6-fluoroquinoline-4-carboxylic acid **(Cpd23)** was prepared as follows.

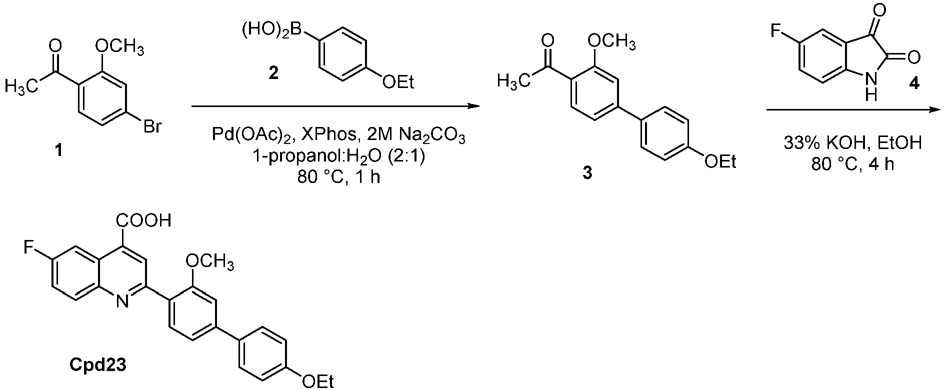

**Step 1** was conducted on 300 mg of 1-(4-bromo-2-methoxyphenyl)ethan-1-one and 239 mg of (4-ethoxyphenyl)boronic acid to afford 1-(4'-ethoxy-3-methoxy-[1,1'-biphenyl]-4-yl)ethan-1-one as a white solid (319 mg, 90% yield). **Step 2 (workup and purification procedure-II)** was conducted on 134 mg of 5-fluoroisatin and 200 mg of 1-(4'-ethoxy-3-methoxy-[1,1'-biphenyl]-4-yl)ethan-1-one to afford 2-(4'-ethoxy-3-methoxy-[1,1'-biphenyl]-4-yl)-6-fluoroquinoline-4-carboxylic acid **(Cpd23)** as a pale yellow solid (59 mg, 19% yield).

**Synthesis of 2-(3-chloro-4'-ethoxy-[1,1'-biphenyl]-4-yl)-6-fluoroquinoline-4-carboxylic acid (Cpd24).** Following synthetic procedures similar to those described for the preparation of **Cpd1,** the target compound 2-(3-chloro-4'-ethoxy-[1,1'-biphenyl]-4-yl)-6-fluoroquinoline-4-carboxylic acid **(Cpd24)** was prepared as follows.

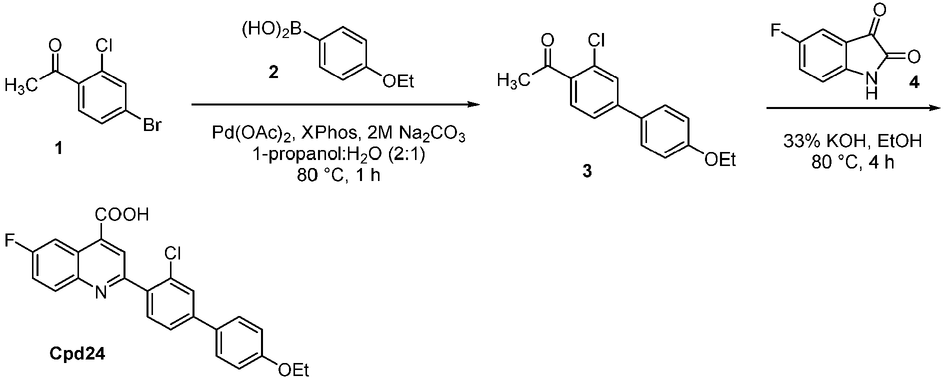

**Step** 1 was conducted on 300 mg of 1-(4-bromo-2-chlorophenyl)ethan-1-one and 224 mg of (4-ethoxyphenyl)boronic acid at 80 °C for 1 h. Another reaction on same scale was conducted at room temperature over 3 days. The workup and purification was carried out as described for Cpd79 to afford 1-(3-chloro-4'-ethoxy-[1,1'-biphenyl]-4-yl)ethan-1-one as a white solid (334 mg, 47% yield). **Step 2** (workup/purification procedure II) was conducted on 132 mg of 5-fluoroisatin and 200 mg of 1-(3-chloro-4'-ethoxy-[1,1'-biphenyl]-4-yl)ethan-1-one to afford 2-(3-chloro-4'-ethoxy-[1,1'-biphenyl]-4-yl)-6-fluoroquinoline-4-carboxylic acid (**Cpd24**) as an off-white solid (164 mg, 53%).

**Synthesis of 2-(3'-butoxy-2-methyl-[1,1'-biphenyl]-4-yl)-6-fluoroquinoline-4-carboxylic acid (Cpd25).** Following synthetic procedures similar to those described for the preparation of **Cpd1,** the target compound 2-(3'-butoxy-2-methyl-[1,1'-biphenyl]-4-yl)-6-fluoroquinoline-4-carboxylic acid (**Cpd25**) was prepared as follows.

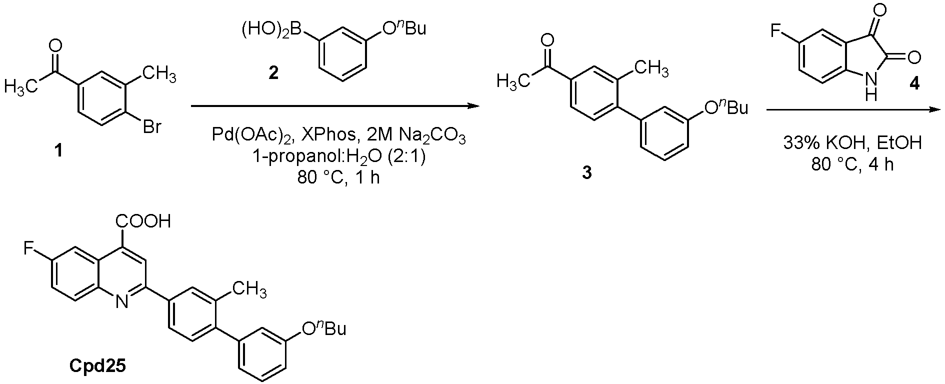

**Step 1** was conducted on 300 mg of 1-(4-bromo-3-methylphenyl)ethan-1-one and 257 mg of (3-butoxyphenyl)boronic acid to afford 1-(3'-butoxy-2-methyl-[1,1'-biphenyl]-4-yl)ethan-1-one as a white solid (226 mg, 57% yield). **Step 2** (workup/purification procedure II) was conducted on 129 mg of 5-fluoroisatin and 200 mg of 1-(3'-butoxy-2-methyl-[1,1'-biphenyl]-4-yl)ethan-1-one to afford 2-(3'-butoxy-2-methyl-[1,1'-biphenyl]-4-yl)-6-fluoroquinoline-4-carboxylic acid (**Cpd25**) as an off-white solid (255 mg, 84% yield).

**Synthesis of 2-(3'-butoxy-3-methoxy-[1,1'-biphenyl]-4-yl)-6-fluoroquinoline-4-carboxylic acid (Cpd26).** Following synthetic procedures similar to those described for the preparation of **Cpd1,** the target compound 2-(3'-butoxy-3-methoxy-[1,1'-biphenyl]-4-yl)-6-fluoroquinoline-4-carboxylic acid (**Cpd26**) was prepared as follows.

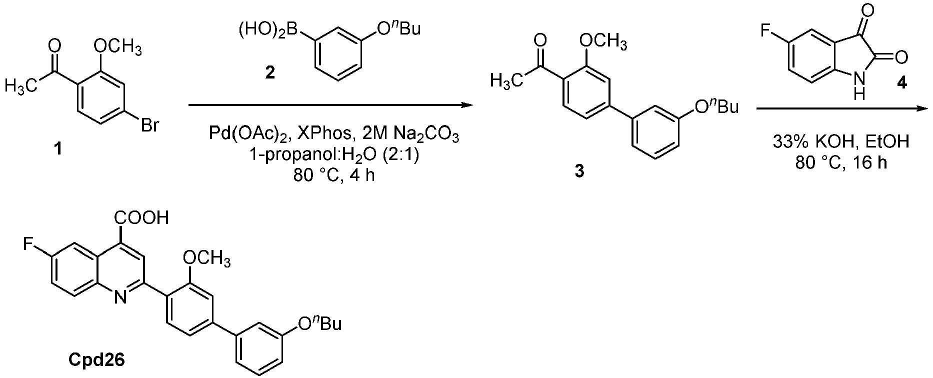

**Step 1** was conducted on 300 mg of 1-(4-bromo-2-methoxyphenyl)ethan-1-one and 280 mg of (3-butoxyphenyl)boronic acid to afford 1-(3'-butoxy-3-methoxy-[1,1'-biphenyl]-4-yl)ethan-1-one as a colorless oil (300 mg, 78%). **Step 2** (workup/purification procedure II) was conducted on 122 mg of 5-fluoroisatin and 200 mg of 1-(3'-butoxy-3-methoxy-[1,1'-biphenyl]-4-yl)ethan-1-one to afford 2-(3'-butoxy-3-methoxy-[1,1'-biphenyl]-4-yl)-6-fluoroquinoline-4-carboxylic acid (**Cpd26**) as an off-white solid (208 mg, 70% yield).

**Synthesis of 2-(3'-butoxy-3-methoxy-[1,1'-biphenyl]-4-yl)-6-fluoroquinoline-4-carboxylic acid (Cpd27).** Following synthetic procedures similar to those described for the preparation of **Cpd1,** the target compound 2-(3'-butoxy-3-methoxy-[1,1'-biphenyl]-4-yl)-6-fluoroquinoline-4-carboxylic acid (**Cpd27**) was prepared as follows.

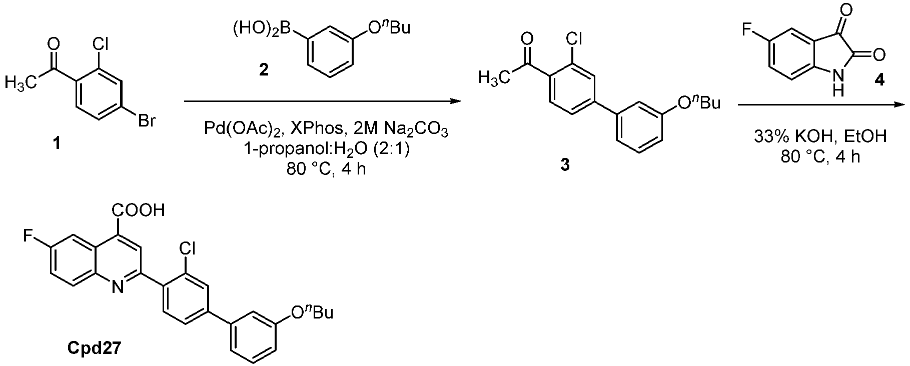

**Step 1** was conducted on 634 mg of 1-(4-bromo-2-chlorophenyl)ethan-1-one and 527 mg of (3-butoxyphenyl)boronic acid to afford 1-(3'-butoxy-3-chloro-[1,1'-biphenyl]-4-yl)ethan-1-one as a colorless oil (412 mg, 50% yield). **Step 2** (workup/purification procedure II) was conducted on 120 mg of 5-fluoroisatin and 200 mg of 1-(3'-butoxy-3-chloro-[1,1'-biphenyl]-4-yl)ethan-1-one to afford 2-(3'-butoxy-3-methoxy-[1,1'-biphenyl]-4-yl)-6-fluoroquinoline-4-carboxylic acid **(Cpd27)** as an off-white solid (189 mg, 64% yield).

The compounds disclosed above were assessed by mass spectrometry and the data are given below in **Table 1.**

**Table 1.**

| **Name** | **Mass Calculated for Formula** | **Calculated Mass** | **Observed Mass** |
|---|---|---|---|
| Cpd1 | C₂₄H₁₈F₂NO₃ | 406.1255 | 406.1 |
| Cpd2 | C₂₄H₁₈F₂NO₃ | 406.1255 | 406.1 |
| Cpd3 | C₂₆H₂₂F₂NO₃ | 434.1568 | 434.2 |
| Cpd4 | C₂₂H₁₂F₂NO₂ | 360.0836 | 360.0 |
| Cpd5 | C₂₂H₁₁F₃NO₂ | 378.0742 | 378.0 |
| Cpd6 | C₂₄H₁₇F₂N₂O₂ | 403.1258 | 403.1 |
| Cpd7 | C₂₄H₁₅F₃NO₃ | 422.1004 | 422.1 |
| Cpd8 | C₂₄H₁₅F₃NO₃ | 422.1004 | 422.1 |
| Cpd9 | C₂₄H₁₆F₂NO₃ | 404.1098 | 404.1 |
| Cpd10 | C₂₄H₁₆F₂NO₃ | 404.1098 | 404.1 |
| Cpd11 | C₂₆H₁₉F₃NO₃ | 450.1317 | 450.1 |
| Cpd12 | C₂₆H₂ₒF₂NO₃ | 432.1411 | 432.1 |
| Cpd13 | C₂₅H₁₉FNO₃ | 400.1349 | 400.1 |
| Cpd14 | C₂₅H₁₉FNO₃ | 400.1349 | 400.1 |
| Cpd15 | C₂₅H₁₉FNO₄ | 416.1298 | 416.1 |
| Cpd16 | C₂₅H₁₉FNO₄ | 416.1298 | 416.1 |
| Cpd17 | C₂₄H₁₅F₃NO₃ | 422.1004 | 422.1 |
| Cpd18 | C₂₄H₁₅F₃NO₃ | 422.1004 | 422.1 |
| Cpd19 | C₂₄H₁₆ClFNO₃ | 420.0803 | 420.1 |
| Cpd20 | C₂₄H₁₆ClFNO₃ | 420.0803 | 420.1 |
| Cpd21 | C₂₅H₁₉FNO₃ | 400.1349 | 400.1 |
| Cpd22 | C₂₇H₂₃FNO₃ | 428.1662 | 428.2 |
| Cpd23 | C₂₅H₁₉FNO₄ | 416.1298 | 416.2 |
| Cpd24 | C₂₄H₁₆ClFNO₃ | 420.0803 | 420.1 |
| Cpd25 | C₂₇H₂₃FNO₃ | 428.1662 | 428.2 |
| Cpd26 | C₂₇H₂₃FNO₄ | 444.1611 | 444.2 |
| Cpd27 | C₂₆H₂₀ClFNO₃ | 448.1116 | 448.2 |

Analogous procedures (i.e., using starting materials with different substituents) can be used to prepare compounds with different substituents following the general procedures disclosed herein.

### 2. EXAMPLE 2: BIOLOGICAL ACTIVITY OF REPRESENTATIVE DISCLOSED COMPOUNDS

DHODH Enzymatic Assay: DHODH activity was determined at 25 °C following the reduction of 2,6-dichloroindophenol sodium salt (DCIP) at 600 nm (ε = 18 800 M⁻¹ cm⁻¹) on a spectrophotometer. The reaction medium used contained 50 mM Tris-HCI, pH 8.0, 0.1% Triton X-100, 0.1 mM LDHO, 0.025 mM CoQ1, and 0.06 mM DCIP. The reaction was started by addition of the enzyme. The inhibitory potency of the compounds was evaluated by measuring the initial velocity of the reaction either in the absence or in the presence of the compounds at the indicated concentrations. The DHODH enzyme used was the recombinant human enzyme prepared as previously described (Hélène Munier-Lehmann, et al., J. Med. Chem. 2015, 58:860-877).

MTS assay for cell growth/viability: Mitochondrial activity was measured to determine cell proliferation using an MTS assay (tetrazolium dye 3'[4,5-dimethylthiazol-2-yl]-2,5-diphenyl-tetrazolium bromide). Metabolically active cells convert MTS tetrazolium salt into a purple formazan product that is soluble in tissue culture medium. The amount of formazan measured at 490 nm absorbance is proportional to the number of proliferating cells. MTS assays in AML cell lines were carried out with 20K cells plated per well in 96-well plates with Cpd3 or brequinar in a dose series ranging from 0.0001 to 10 µM. Triplicate wells were set up for each condition. At 96 hours, the MTS reagent was added and after approximately 4 hours the plates were read in a spectrophotometer.

Biological Activity: Cell proliferation assays were carried out using the MTS assay described herein using two AML cell lines (i.e., MOLM13 and OCI-AML3) with varied genetic backgrounds. The assays were carried out in a blinded fashion. Data for a representative compound is shown in **Tables 2** and **3.**

**Table 2.**

| **Name** | **OCI-AML3 cell line IC₅₀ (nM)** | **MOLM13 cell line IC₅₀ (nM)** |
|---|---|---|
| Cpd1 | 19.5 | 14.47 |

**Table 3.**

| **Name** | **h-DHODH IC₅₀ (nM)** | **MOLM-13 cell line IC₅₀ (nM)** |
|---|---|---|
| **Cpd1** | 2.43 | 13 |
| **Cpd2** | <0.5 | 16 |
| **Cpd3** | <0.5 | 16 |
| **Cpd4** | 0.72 | 41 |
| **Cpd5** | 1.66 | 14 |
| **Cpd6** | 1.34 | < 2 |
| **Cpd7** | 1.4 | < 2 |
| **Cpd8** | <0.51 | < 2 |
| **Cpd9** | 5.5 | ND* |
| **Cpd10** | 0.7 | < 2 |
| **Cpd11** | <0.51 | ND* |
| **Cpd12** | 0.7 | < 2 |
| **Cpd13** | 1.5 | < 2 |
| **Cpd14** | 1.4 | < 2 |
| **Cpd15** | 4.2 | < 2 |
| **Cpd16** | 1.3 | < 2 |
| **Cpd17** | 6.2 | 43 |
| **Cpd18** | 1.2 | < 2 |
| **Cpd19** | 1.1 | < 2 |
| **Cpd20** | 0.6 | < 2 |
| **Cpd21** | 11.6 | 538 |
| **Cpd22** | <0.51 | 34 |
| **Cpd23** | 16.0 | 532 |
| **Cpd24** | 22.2 | 464 |
| **Cpd25** | <0.51 | < 2 |
| **Cpd26** | 0.7 | 11 |
| **Cpd27** | <0.51 | 16 |

| | | |
|---|---|---|
| Studies used a dose range of 2 nM to 10000 nM. Compounds with cellular IC50 < 2 nM could not be accurately determined. | | |

The data show growth arrest in AML cell lines at submicromolar to micromolar concentrations (IC₅₀ ranges from 14.5 - 19.5 µM), which is more potent similar to treatment with a reference compound, brequinar (BQR) for Cpd1 in one study. In a second study, several disclosed compounds were tested in MOLM-13 cell line, and the data show a number of compounds effecting growth arrest in this cell-line in range of <2 nM to several hundred nanomolar, and a large number showing growth arrest at about <50 nM. Representative growth arrest data are shown in **FIGs. 2A-2D****.** The data show growth IC₅₀ for inhibition of DHODH activity at low nanomolar concentrations (IC₅₀ ranges from <0.5 nM - 22.2 nM).

**FIG. 1** shows representative data and analysis for the effect of representative disclosed compounds p53 expression. Data were obtained for the effect of a comparator compound, RefCpd3, along with controls for vehicle treatment, and treatment with a reference compound, brequinar (indicated as "BQR" in the figures) using methods as described herein above. Briefly, AML cell lines were treated with vehicle, 50 nM test compound (designated by HOSU and a number), 50 nM RefCpd3 or 50 nM BQR for 24 hours. Lysates were prepared and immunoblots were performed for p53, with GAPDH used as a loading control. The structure for RefCpd3 is as follows, and was prepared as described in Intl. Pat. Appl. No. PCT/US19/38622, which is incorporated herein by reference:

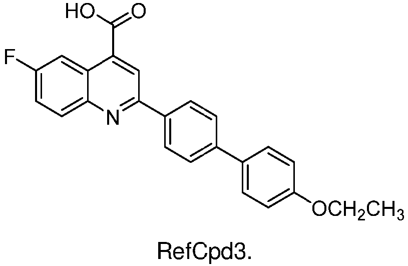

It should be emphasized that the above-described examples are merely possible examples of implementations set forth for a clear understanding of the principles of the disclosure.

## Claims

1. A compound having a formula represented by a structure: 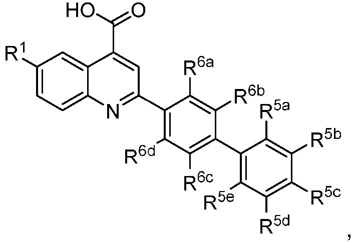
wherein R¹ is selected from halogen, -SF₅, -CF₃, and -CF₂CF₃;
wherein R^{5a} is -R²⁰, and -R²⁰ is selected from halogen, -C2-C7 alkylamino and -C2-C7 alkoxy; and wherein each of R^{5b}, R^{5c}, R^{5d}, and R^{5e} is independently selected from hydrogen, halogen, -SF₅, -CN, -N₃, -OH, -NH₂, -CF₃, and -CF₂CF₃; or
wherein R^{5b} is -R²⁰, and -R²⁰ is selected from halogen, -C2-C7 alkylamino and -C2-C7 alkoxy; and wherein each of R^{5a}, R^{5c}, R^{5d}, and R^{5e} is independently selected from hydrogen, halogen, -SF₅, -CN, -N₃, -OH, -NH₂, -CF₃, and -CF₂CF₃; or
wherein R^{5c} is -R²⁰, and -R²⁰ is selected from halogen, -C2-C7 alkylamino and -C2-C7 alkoxy; and wherein each of R^{5a}, R^{5b}, R^{5d}, and R^{5e} is independently selected from hydrogen, halogen, -SF₅, -CN, -N₃, -OH, -NH₂, -CF₃, and -CF₂CF₃; and
wherein each of R^{6a}, R^{6b}, R^{6c}, and R^{6d} is independently selected from hydrogen, halogen, -SF₅, -CN, -N₃, -OH, -NH₂, C1-C3 alkyl, C1-C3 alkoxy, C1-C3 haloalkyl, C1-C3 aminoalkyl, and C1-C3 hydroxyalkyl, provided that at least one of R^{6a}, R^{6b}, R^{6c}, and R^{6d} is not hydrogen;
or a pharmaceutically acceptable salt thereof.

2. The compound of claim 1, wherein R¹ is selected from halogen, -SF₅, -CF₃, and - CF₂CF₃.

3. The compound of claim 2, wherein R¹ is -F.

4. The compound of claim 1, wherein R¹ is selected from -SF₅, -CF₃, and -CF₂CF₃.

5. The compound of claim 1, wherein R^{5a} is -R²⁰; wherein R²⁰ is halogen; and wherein each of R^{5b}, R^{5c}, R^{5d}, and R^{5e} is independently selected from hydrogen, halogen, -SF₅, - CN, -N₃, -OH, -NH₂, -CF₃, and -CF₂CF₃; or
wherein R^{5a} is -R²⁰; wherein R²⁰ is selected from -C2-C7 alkylamino and -C2-C7 alkoxy; and wherein each of R^{5b}, R^{5c}, R^{5d}, and R^{5e} is independently selected from hydrogen, halogen, -SF₅, -CN, -N₃, -OH, -NH₂, -CF₃, and -CF₂CF₃

6. The compound of claim 1, wherein R^{5b} is -R²⁰; wherein R²⁰ is halogen; and wherein each of R^{5a}, R^{5c}, R^{5d}, and R^{5e} is independently selected from hydrogen, halogen, -SF₅, - CN, -N₃, -OH, -NH₂, -CF₃, and -CF₂CF₃; or
wherein R^{5b} is -R²⁰; wherein R²⁰ is selected from -C2-C7 alkylamino and -C2-C7 alkoxy; and wherein each of R^{5a}, R^{5c}, R^{5d}, and R^{5e} is independently selected from hydrogen, halogen, -SF₅, -CN, -N₃, -OH, -NH₂, -CF₃, and -CF₂CF₃

7. The compound of claim 1, wherein R^{5c} is -R²⁰; wherein R²⁰ is halogen; and wherein each of R^{5a}, R^{5b}, R^{5d}, and R^{5e} is independently selected from hydrogen, halogen, -SF₅, - CN, -N₃, -OH, -NH₂, -CF₃, and -CF₂CF₃; or
wherein R^{5c} is -R²⁰; wherein R²⁰ is selected from -C2-C7 alkylamino and -C2-C7 alkoxy; and wherein each of R^{5a}, R^{5b}, R^{5d}, and R^{5e} is independently selected from hydrogen, halogen, -SF₅, -CN, -N₃, -OH, -NH₂, -CF₃, and -CF₂CF₃.

8. The compound of claim 1, having a structure represented by a formula: 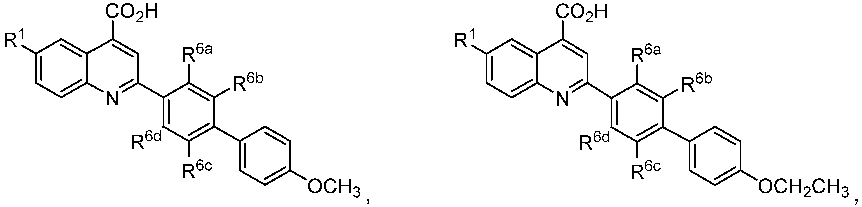 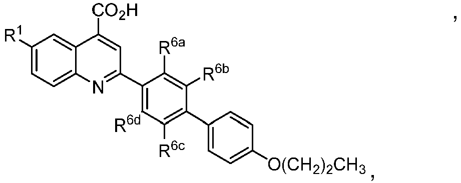 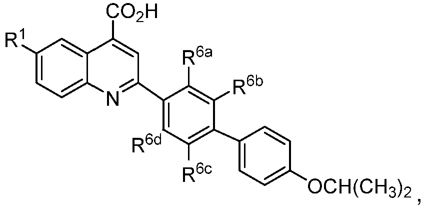 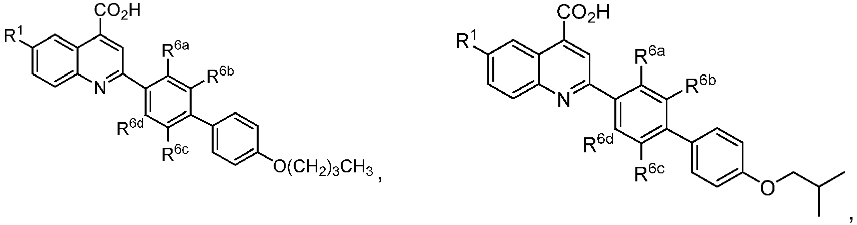 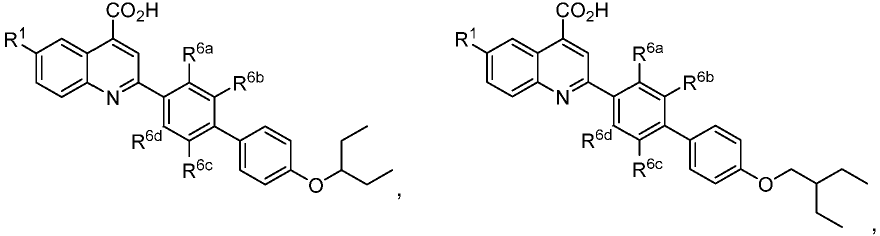 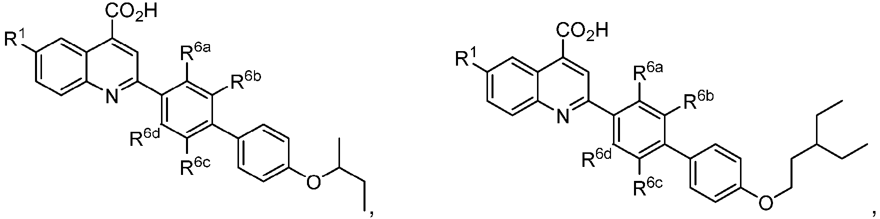 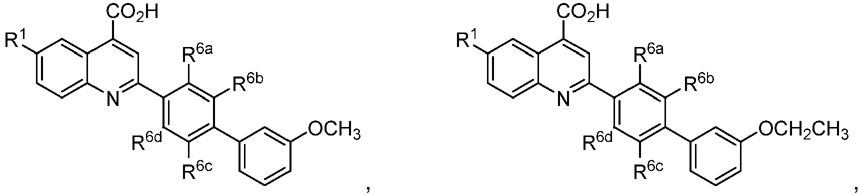 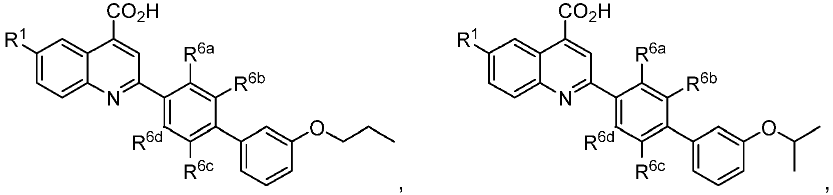 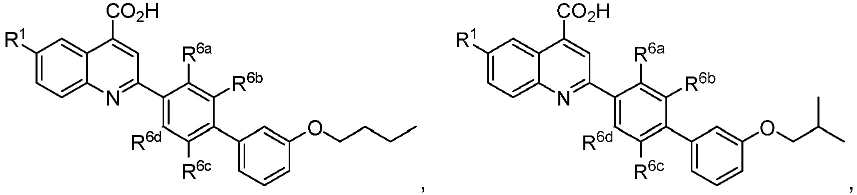 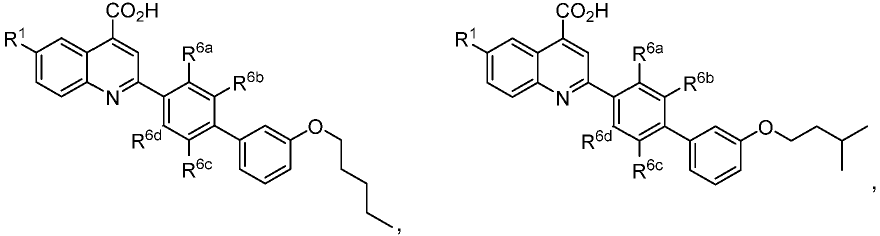 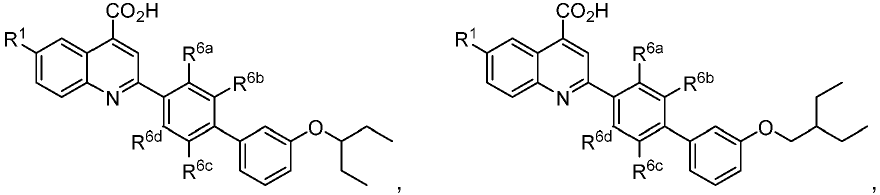 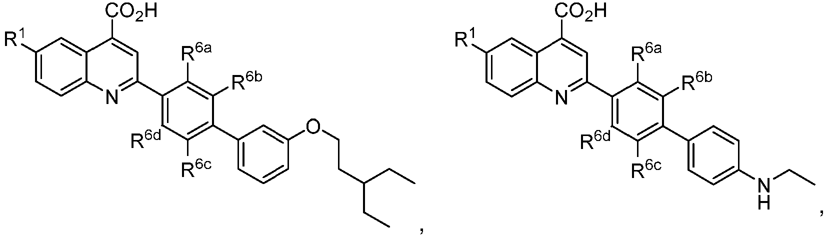 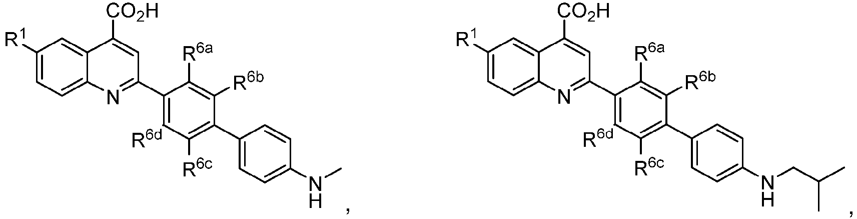 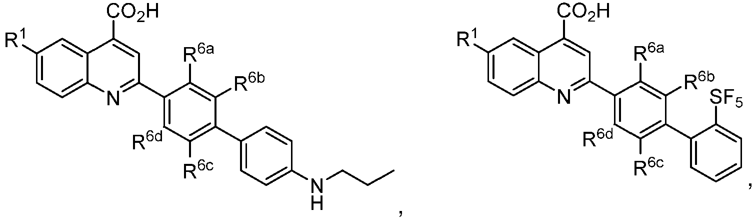 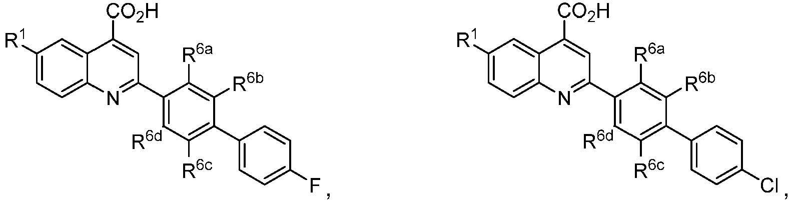 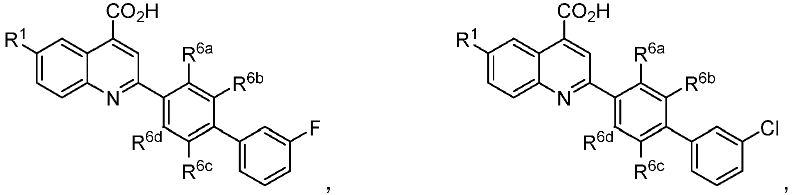 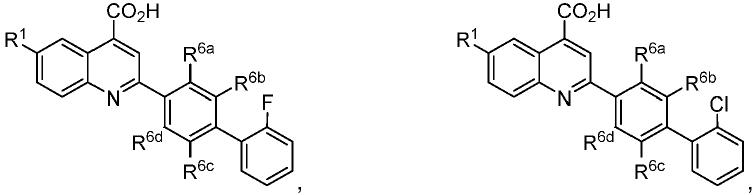 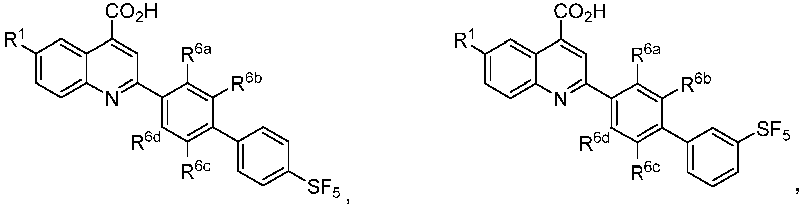 or combinations thereof.

9. The compound of claim 1, having a structure represented by a formula: 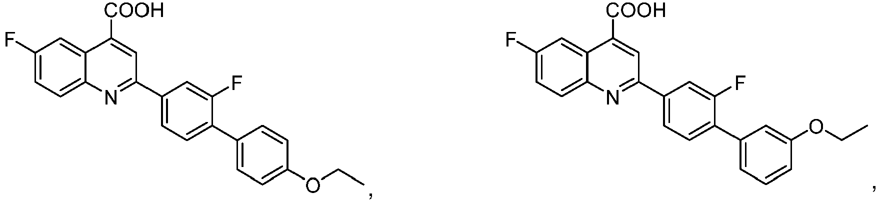 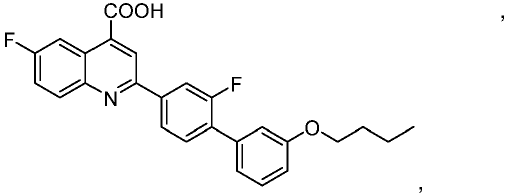 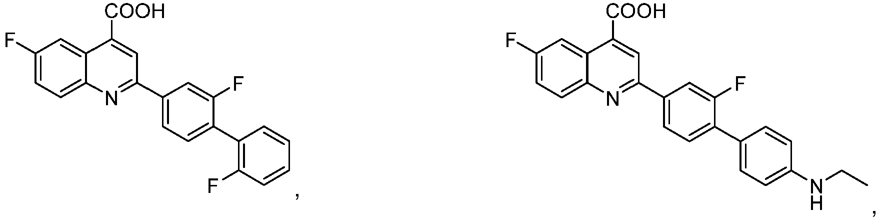 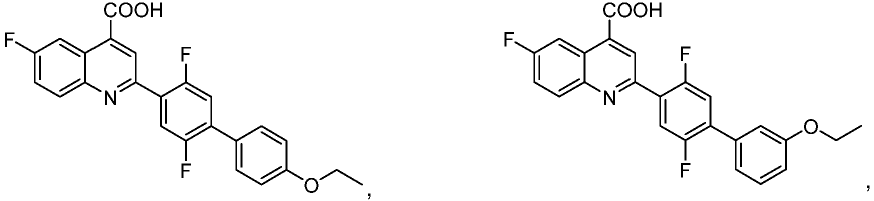 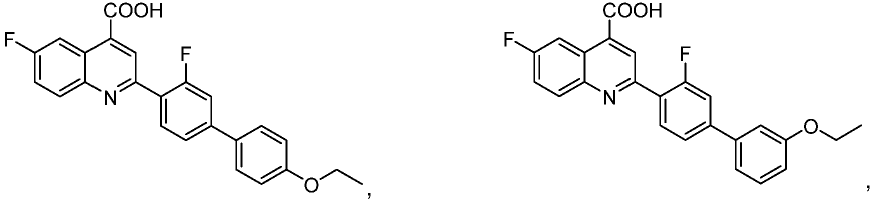 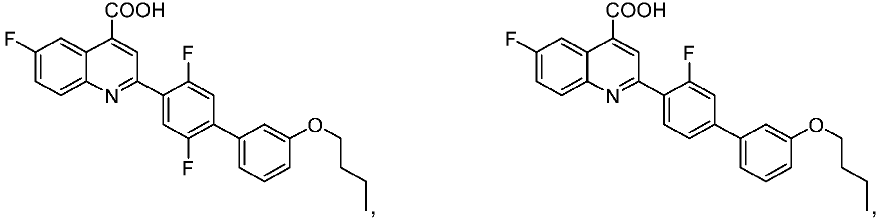 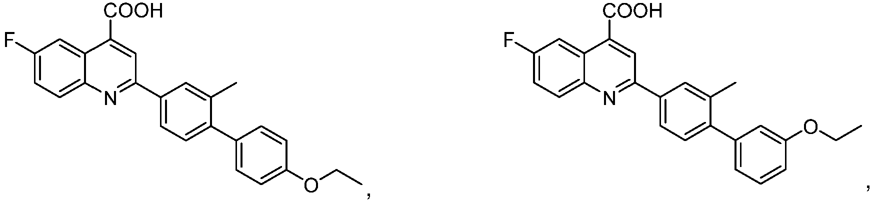 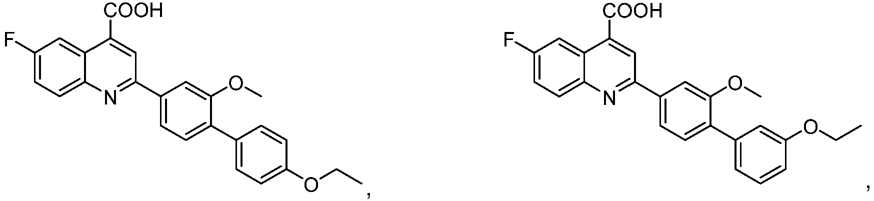 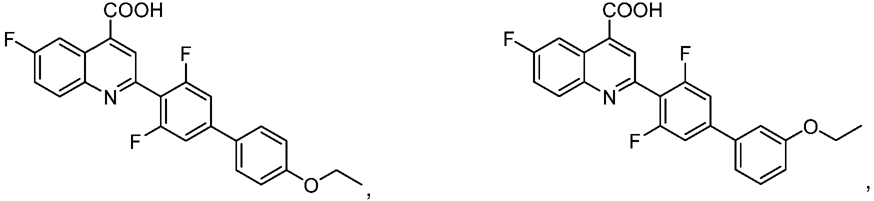 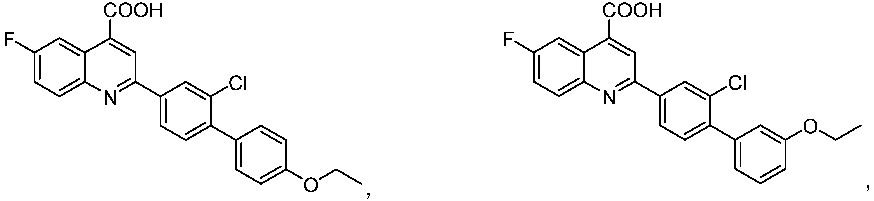 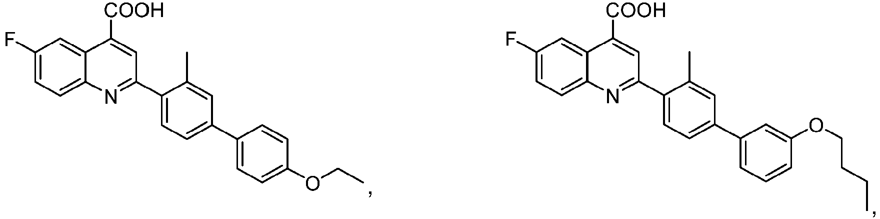 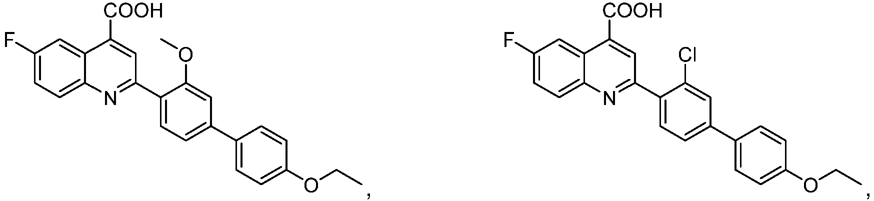 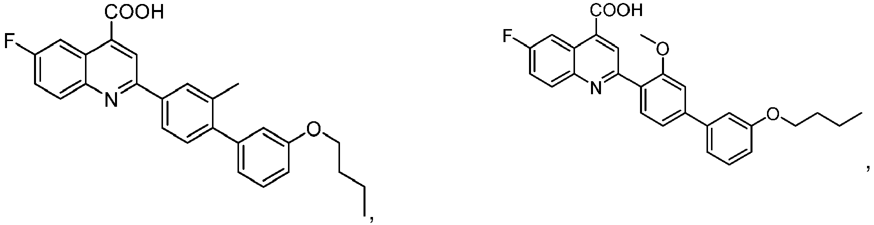 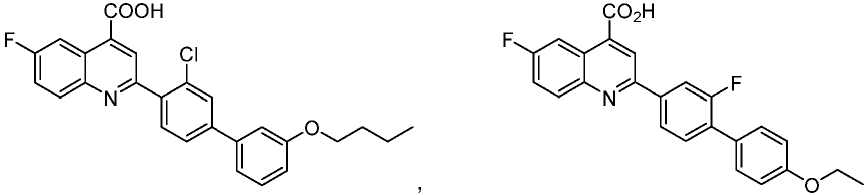 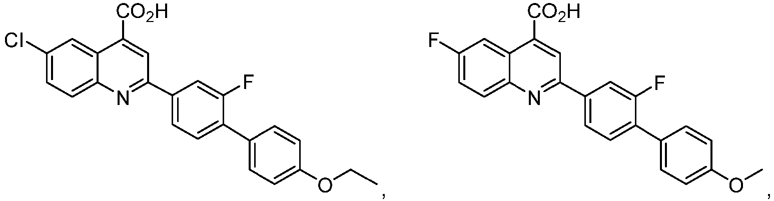 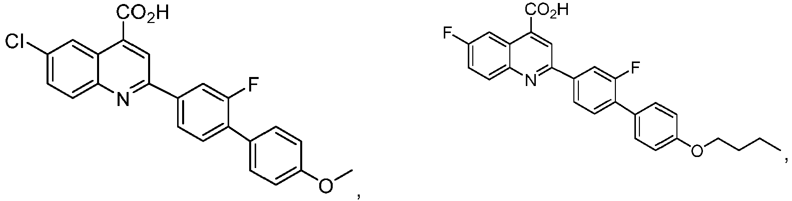 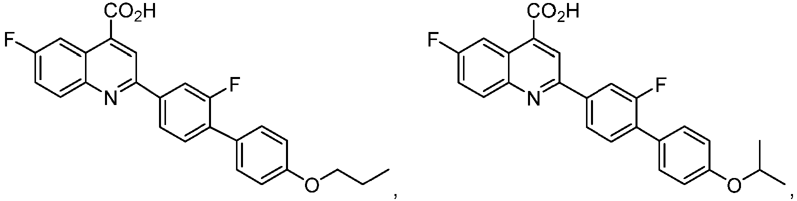 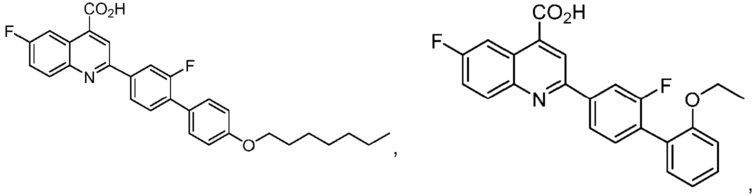 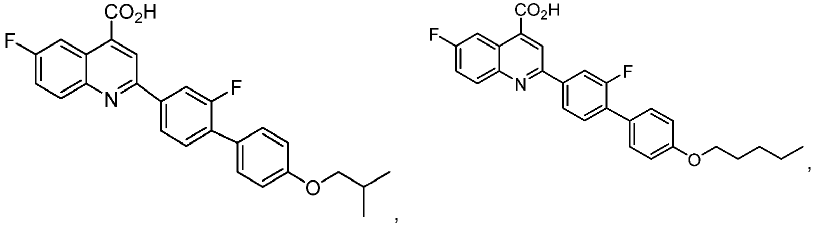 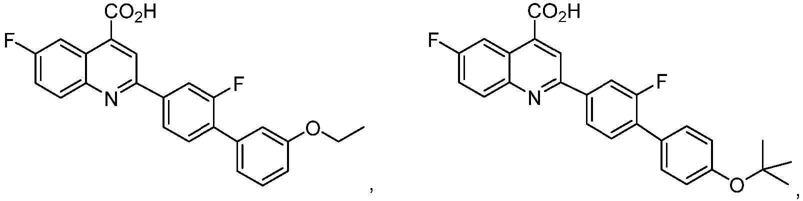 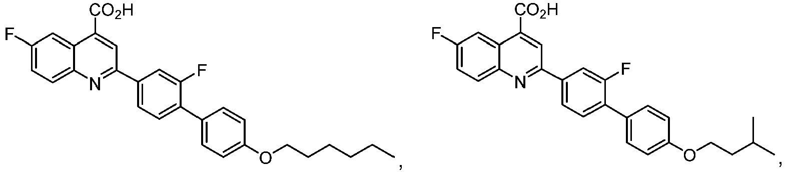 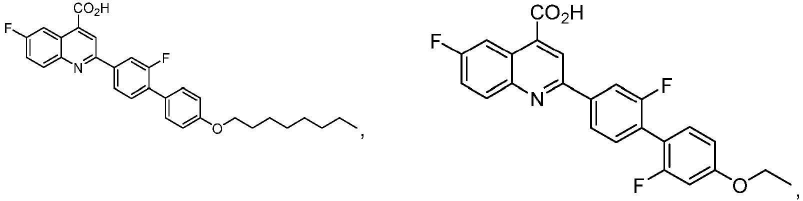 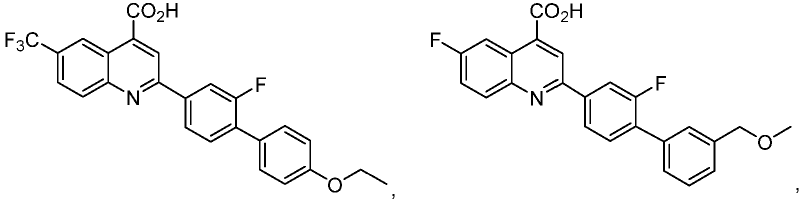 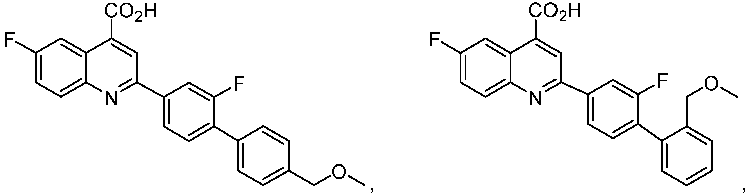 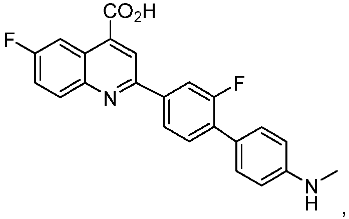 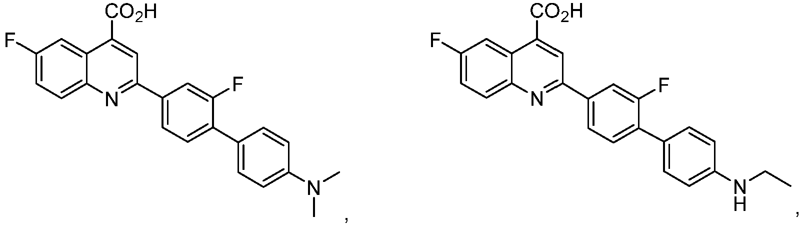 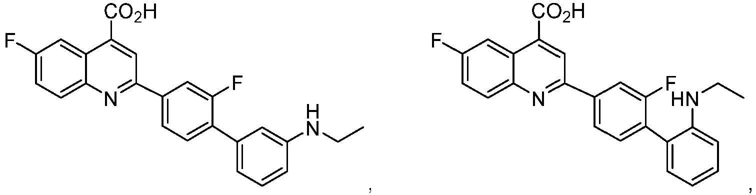 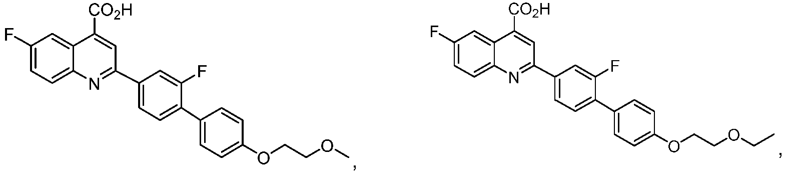 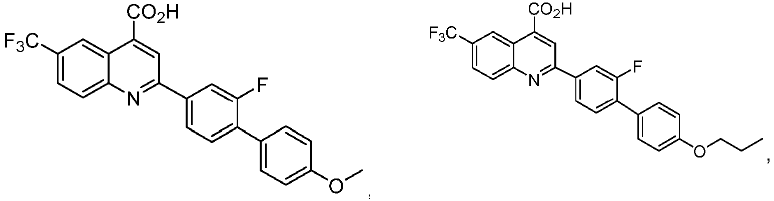 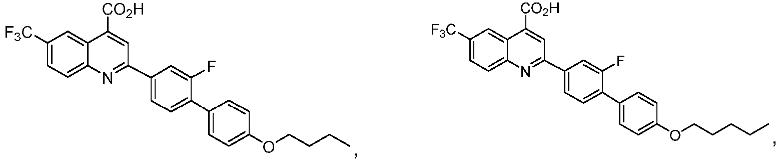 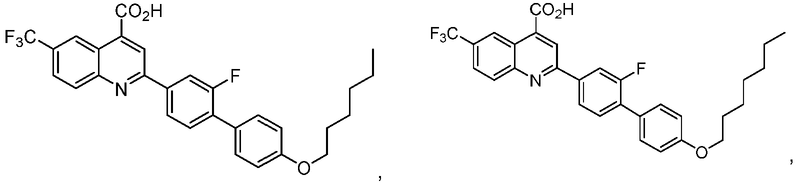 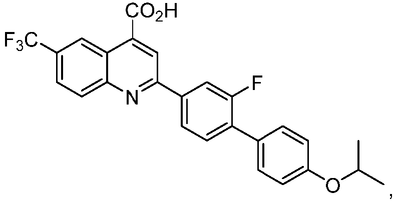 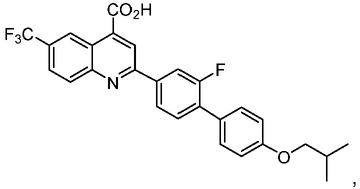 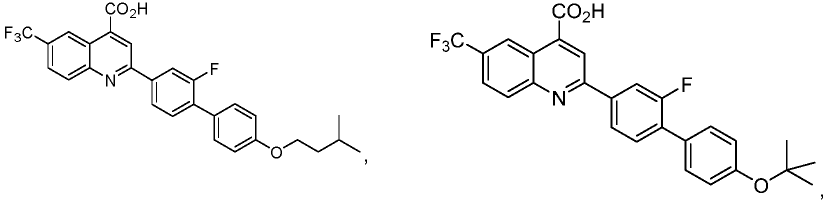 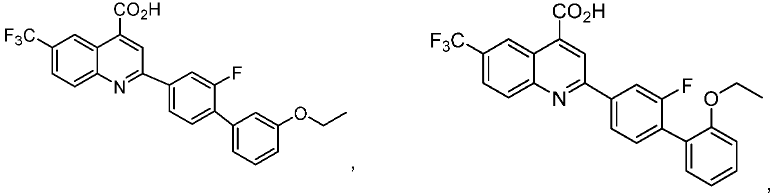 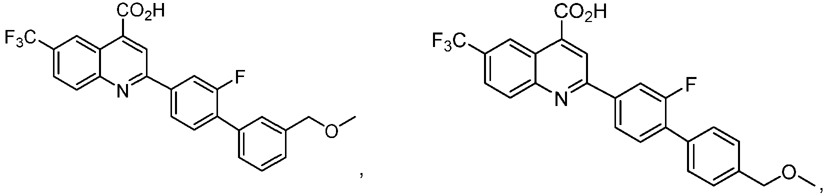 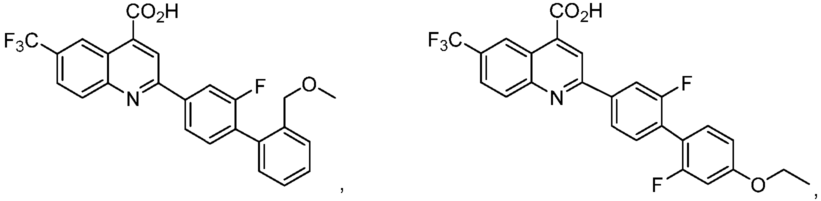 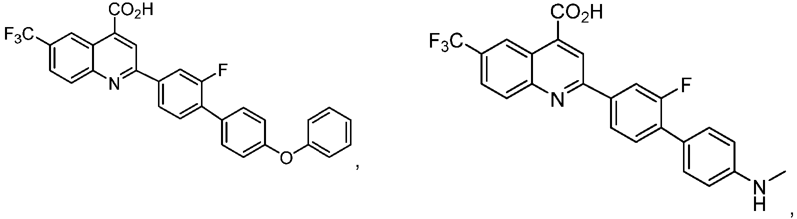 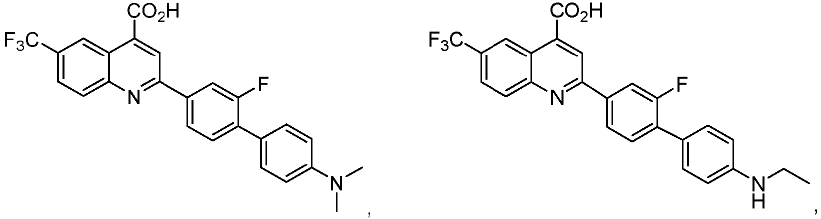 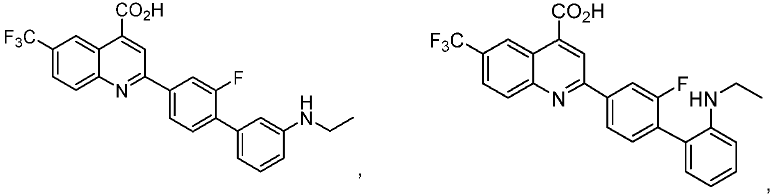 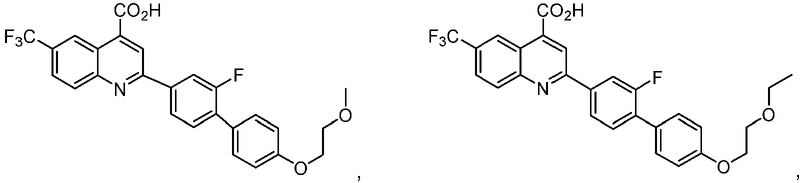 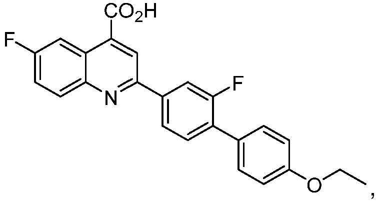 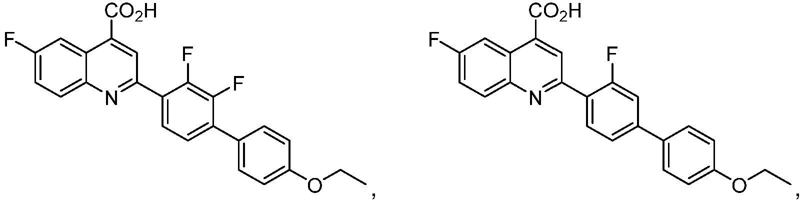 or a combination thereof.

10. A pharmaceutical composition comprising a therapeutically effective amount of a compound of any of claims 1 to 9, or a pharmaceutically acceptable salt thereof, and a pharmaceutically acceptable carrier.

11. A compound of any of claims 1 to 9, or a composition of claim 10, for use in treatment of cancer.

12. The compound or composition of claim 11 for use in treatment of hematological cancer.

13. The compound or composition of claim 12, wherein the hematological cancer is selected from chronic myeloid leukemia (CML), acute myeloid leukemia (AML), chronic lymphoid leukemia (CLL), acute lymphoid leukemia (ALL), hairy cell leukemia, chronic myelomonocytic leukemia (CMML), juvenile myelomonocyte leukemia (JMML), large granular lymphocytic leukemia (LGL), acute lymphocytic leukemia, acute lymphoblastic leukemia, B-cell lymphoma, T-cell-lymphoma, Hodgkin's lymphoma, non-Hodgkin's lymphoma, hairy cell lymphoma, Burkett's lymphoma, Hodgkin lymphoma, and non-Hodgkin lymphoma.

14. The compound or composition of claim 12, wherein the hematological cancer is chronic myeloid leukemia (CML) or acute myeloid leukemia (AML).

## Patentansprüche

1. Eine Verbindung mit einer Formel, dargestellt durch eine Struktur: 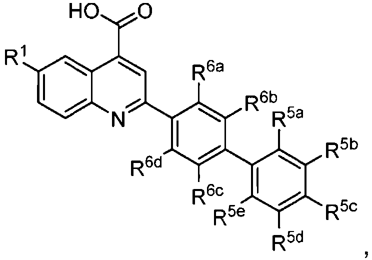
wobei R¹ ausgewählt ist aus Halogen, -SF₅, -CF₃ und -CF₂CF₃,
wobei R^{5a} -R²⁰ ist und -R²⁰ ausgewählt ist aus Halogen, -C2-C7-Alkylamino und -C2-C7-alkoxy, und wobei jeder der Reste R^{5b}, R^{5c}, R^{5d} und R^{5e} unabhängig ausgewählt ist aus Wasserstoff, Halogen, -SF₅, -CN, -N₃, -OH, -NH₂, -CF₃ und -CF₂CF₃, oder
wobei R^{5b} -R²⁰ ist und -R²⁰ ausgewählt ist aus Halogen, -C2-C7-Alkylamino und -C2-C7-Alkoxy, und wobei jeder der Reste R^{5a}, R^{5c}, R^{5d} und R^{5e} unabhängig ausgewählt ist aus Wasserstoff, Halogen, -SF₅, -CN, -N₃, -OH, -NH₂, -CF₃ und -CF₂CF₃, oder
wobei R^{5c} -R²⁰ ist und -R²⁰ ausgewählt ist aus Halogen, -C2-C7-Alkylamino und -C2-C7-alkoxy, und wobei jeder der Reste R^{5a}, R^{5b}, R^{5d} und R^{5e} unabhängig ausgewählt ist aus Wasserstoff, Halogen, -SF₅, -CN, -N₃, -OH, -NH₂, -CF₃ und -CF₂CF₃, und
wobei jeder der Reste R^{6a}, R^{6b}, R^{6c} und R^{6d} unabhängig ausgewählt ist aus Wasserstoff, Halogen, -SF₅, -CN, -N₃, -OH, -NH₂, C1-C3-Alkyl, C1-C3-Alkoxy, C1-C3-Halogenalkyl, C1-C3-Aminoalkyl und C1-C3-Hydroxyalkyl, mit der Maßgabe, dass wenigstens einer der Reste R^{6a}, R^{6b}, R^{6c} und R^{6d} nicht Wasserstoff ist,
oder ein pharmazeutisch annehmbares Salz davon.

2. Die Verbindung nach Anspruch 1, wobei R¹ ausgewählt ist aus Halogen, -SF₅, -CF₃ und -CF₂CF₃.

3. Die Verbindung nach Anspruch 2, wobei R¹ -F ist.

4. Die Verbindung nach Anspruch 1, wobei R¹ ausgewählt ist aus -SF₅, -CF₃ und -CF₂CF₃.

5. Die Verbindung nach Anspruch 1, wobei R^{5a} -R²⁰ ist, wobei R²⁰ Halogen ist, und wobei jeder der Reste R^{5b}, R^{5c}, R^{5d} und R^{5e} unabhängig ausgewählt ist aus Wasserstoff, Halogen, -SF₅, -CN, -N₃, -OH, -NH₂, -CF₃ und -CF₂CF₃, oder
wobei R^{5a} -R²⁰ ist, wobei R²⁰ ausgewählt ist aus -C2-C7-Alkylamino und -C2-C7-Alkoxy, und wobei jeder der Reste R^{5b}, R^{5c}, R^{5d} und R^{5e} unabhängig ausgewählt ist aus Wasserstoff, Halogen, -SF₅, -CN, -N₃, -OH, -NH₂, -CF₃ und -CF₂CF₃.

6. Die Verbindung nach Anspruch 1, wobei R^{5b} -R²⁰ ist, wobei R²⁰ Halogen ist, und wobei jeder der Reste R^{5a}, R^{5c}, R^{5d} und R^{5e} unabhängig ausgewählt ist aus Wasserstoff, Halogen, -SF₅, -CN, -N₃, -OH, -NH₂, -CF₃ und -CF₂CF₃, oder
wobei R^{5b} -R²⁰ ist, wobei R²⁰ ausgewählt ist aus -C2-C7-Alkylamino und -C2-C7-Alkoxy, und wobei jeder der Reste R^{5a} R^{5c}, R^{5d} und R^{5e} unabhängig ausgewählt ist aus Wasserstoff, Halogen, -SF₅, -CN, -N₃, -OH, -NH₂, -CF₃ und -CF₂CF₃.

7. Die Verbindung nach Anspruch 1, wobei R^{5c} -R²⁰ ist, wobei R²⁰ Halogen ist, und wobei jeder der Reste R^{5a} R^{5b}, R^{5d} und R^{5e} unabhängig ausgewählt ist aus Wasserstoff, Halogen, -SF₅, -CN, -N₃, -OH, -NH₂, -CF₃ und -CF₂CF₃, oder
wobei R^{5c} -R²⁰ ist, wobei R²⁰ ausgewählt ist aus -C2-C7-Alkylamino und -C2-C7-Alkoxy, und wobei jeder der Reste R^{5a}, R^{5b}, R^{5d} und R^{5e} unabhängig ausgewählt ist aus Wasserstoff, Halogen, -SF₅, -CN, -N₃, -OH, -NH₂, -CF₃ und -CF₂CF₃.

8. Die Verbindung nach Anspruch 1 mit einer Struktur, dargestellt durch eine Formel: 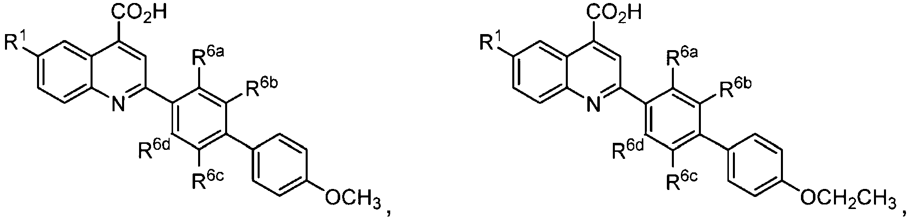 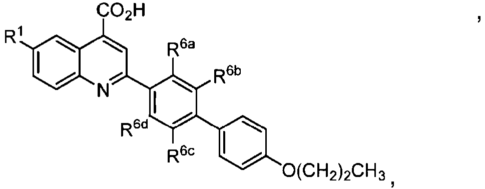 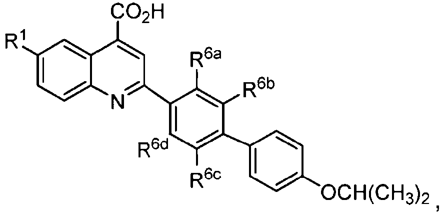 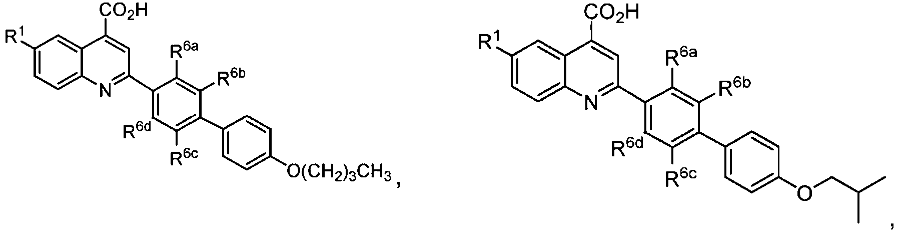 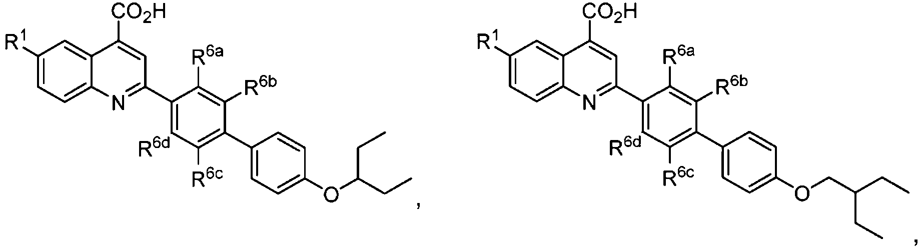 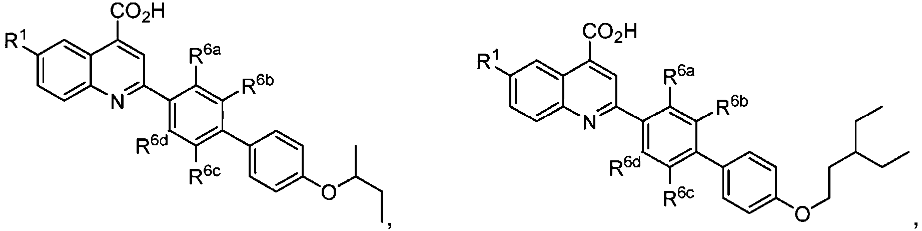 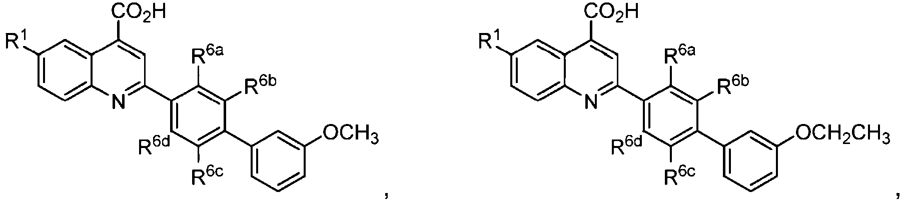 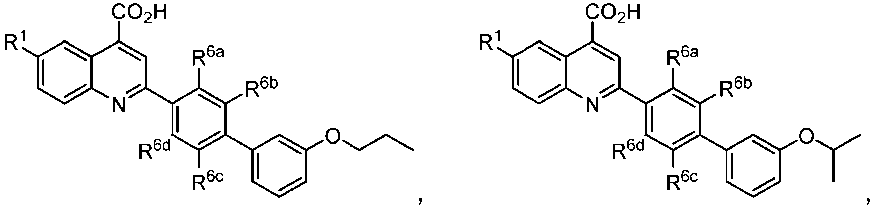 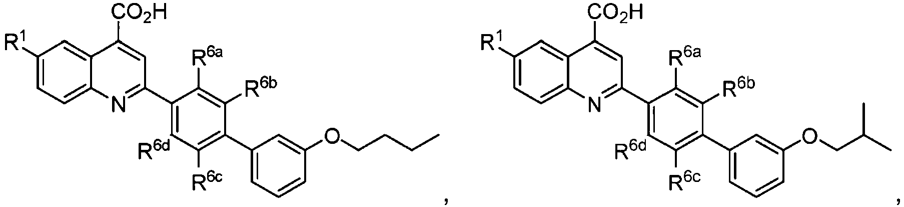 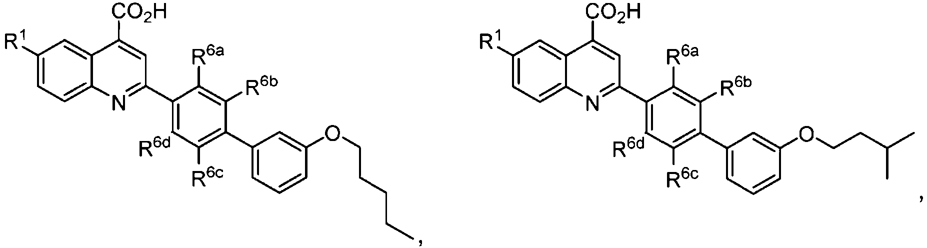 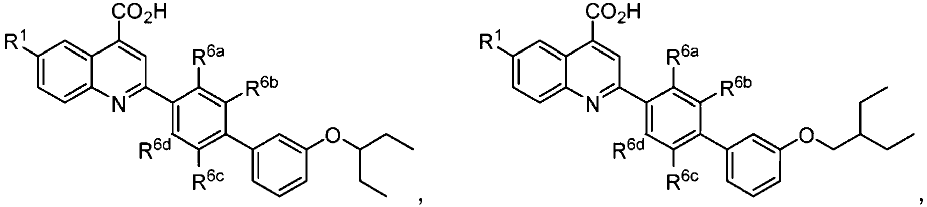 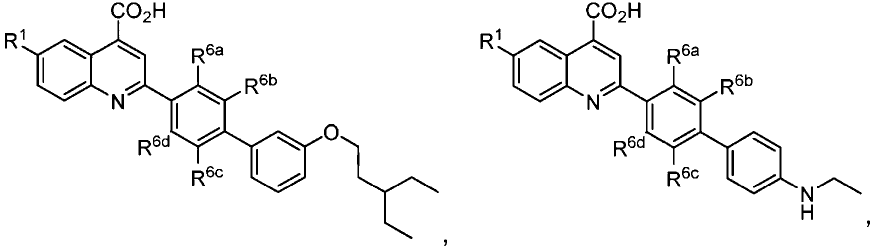 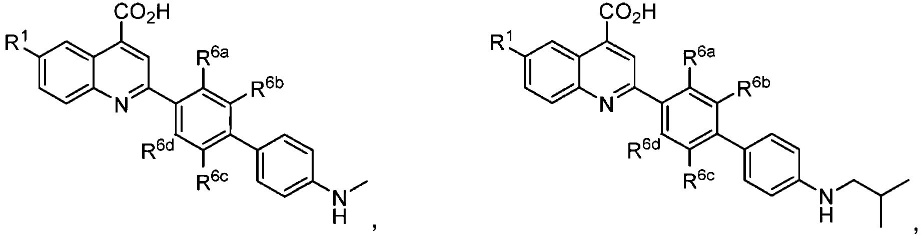 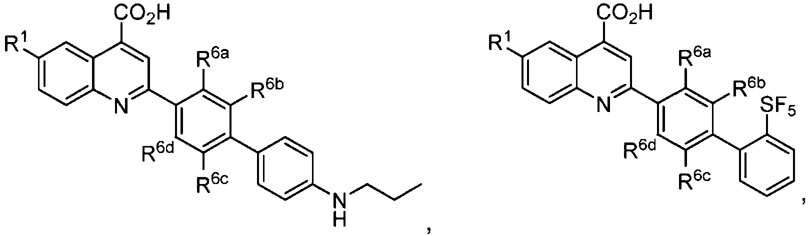 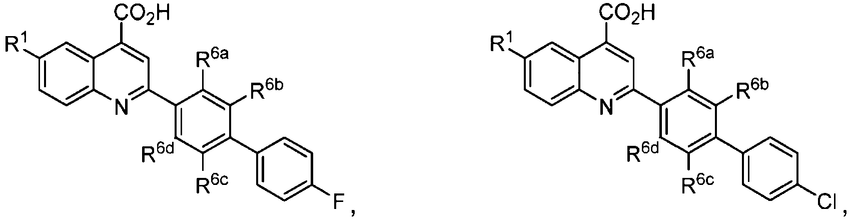 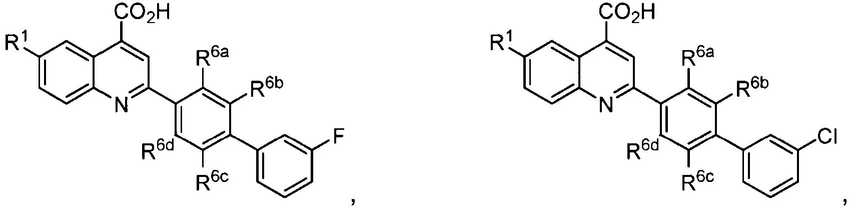 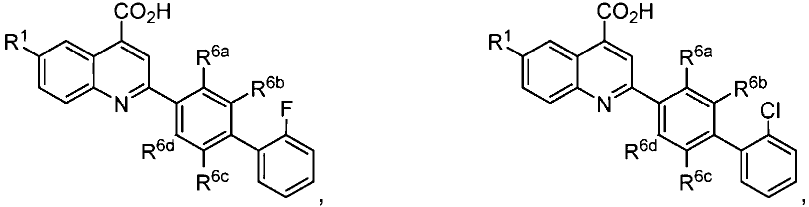 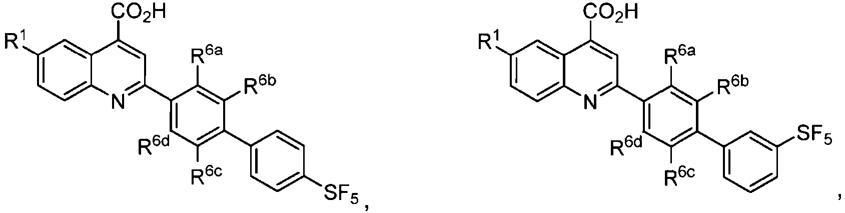 oder Kombinationen davon.

9. Die Verbindung nach Anspruch 1 mit einer Struktur, dargestellt durch eine Formel: 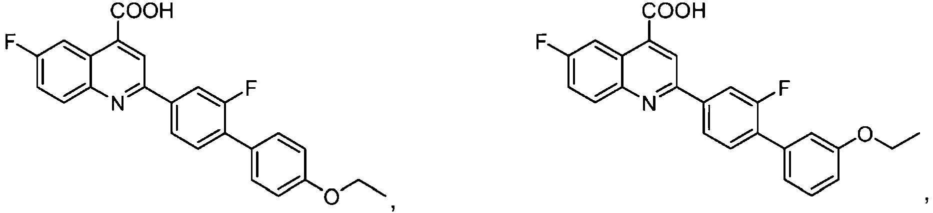 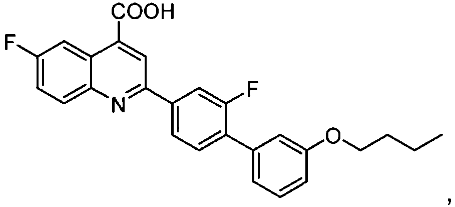 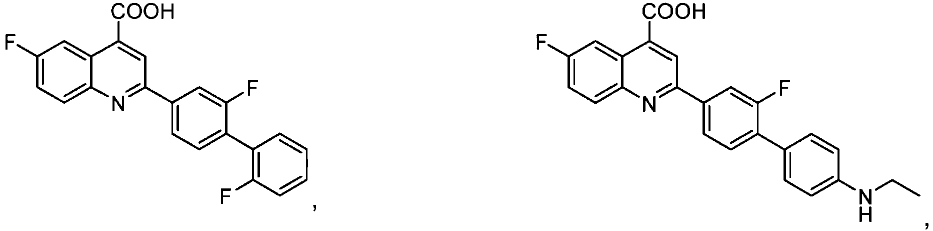 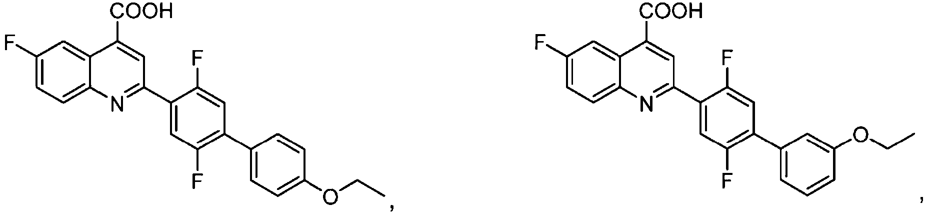 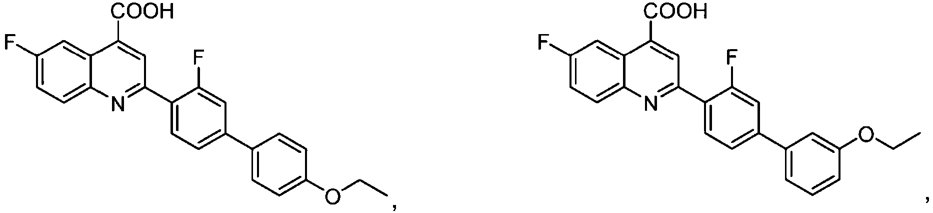 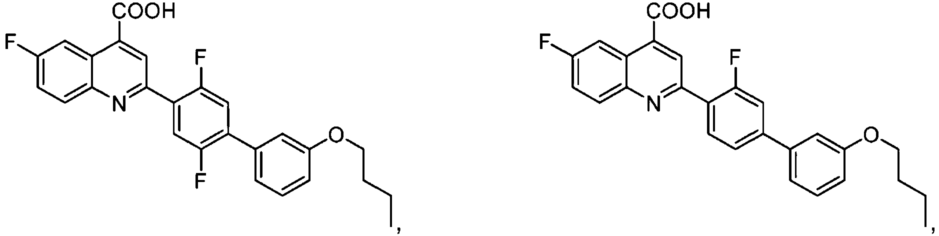 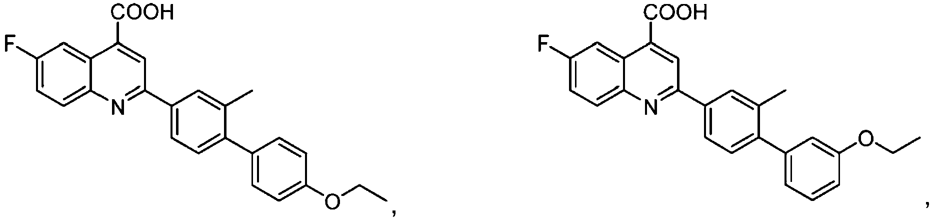 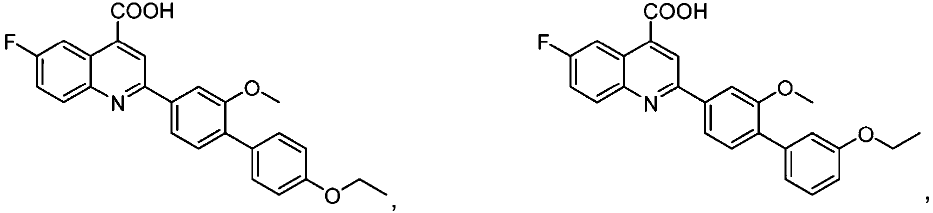 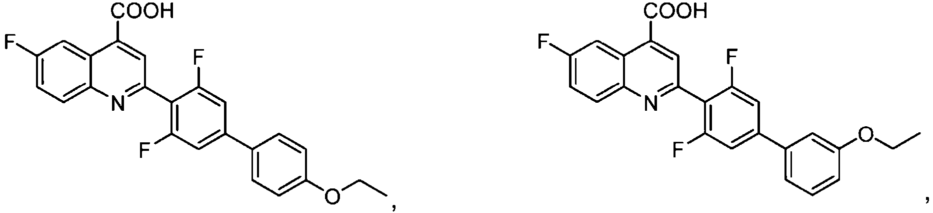 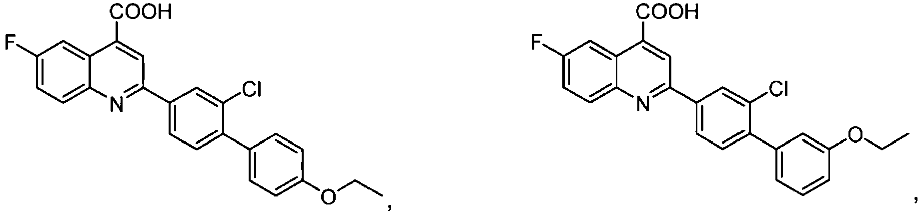 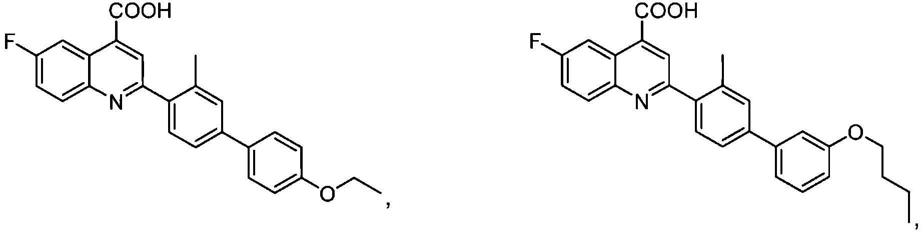 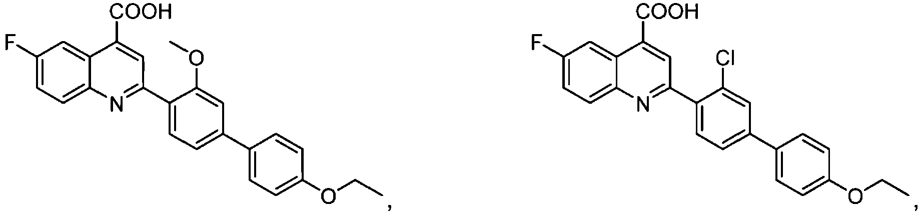 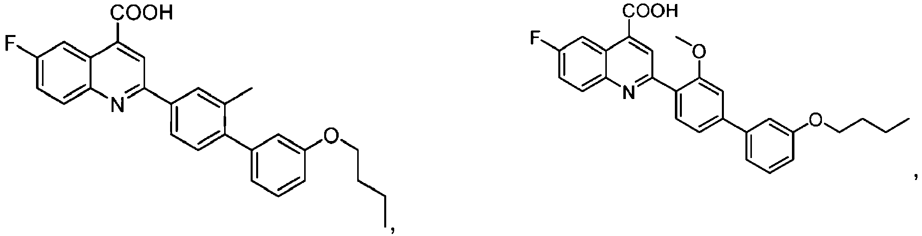 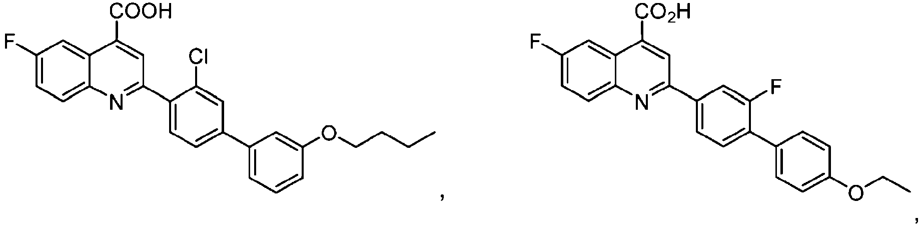 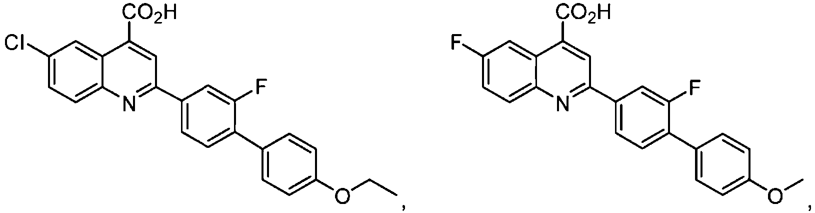 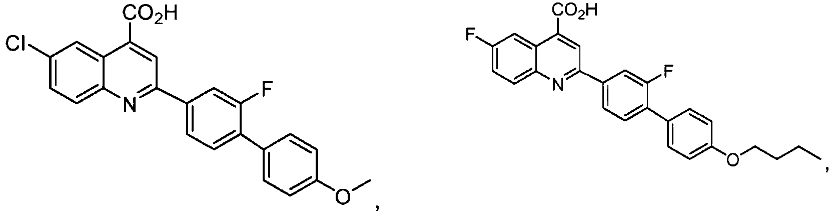 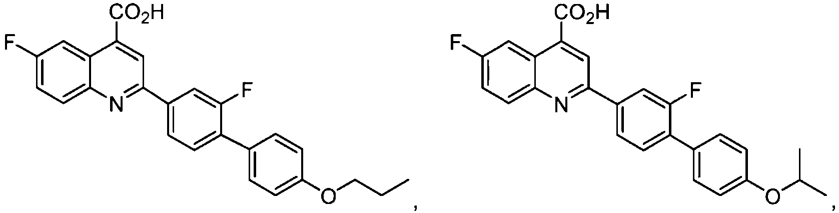 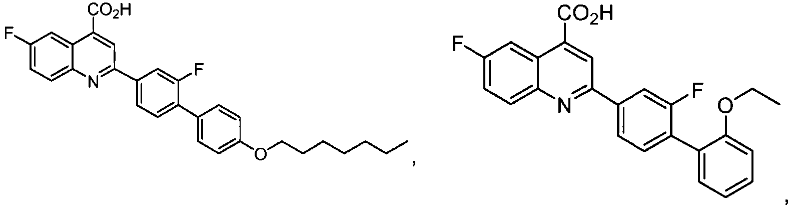 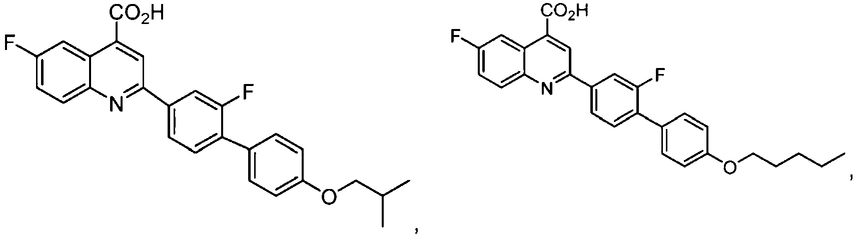 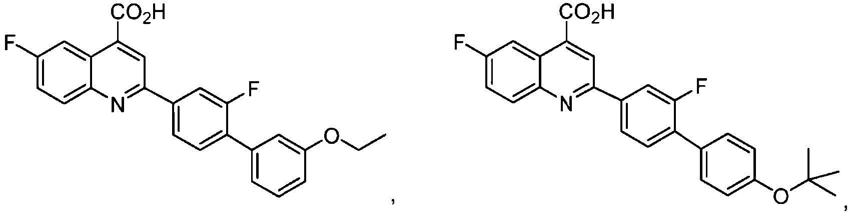 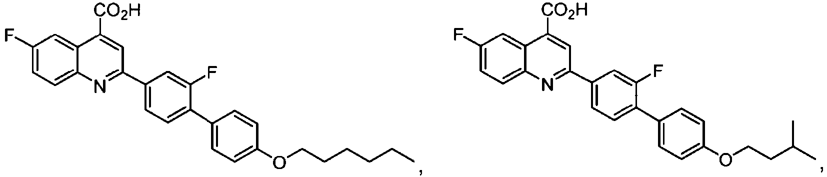 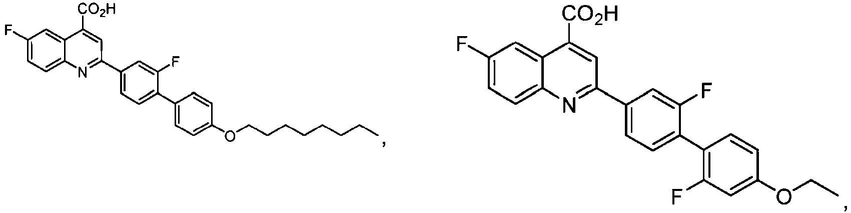 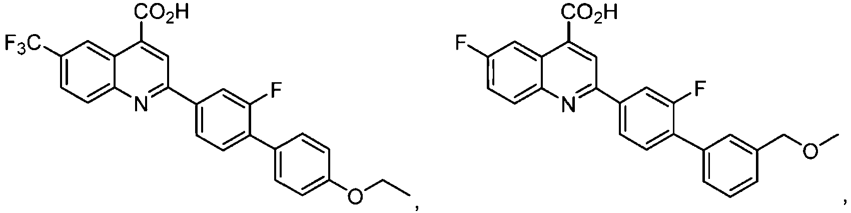 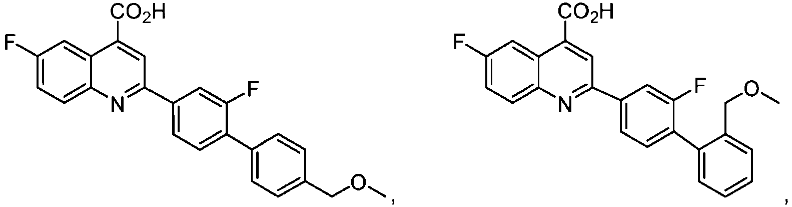 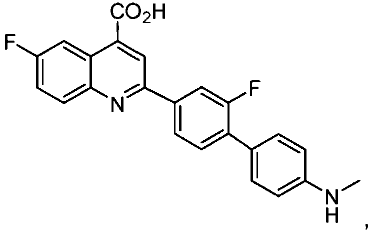 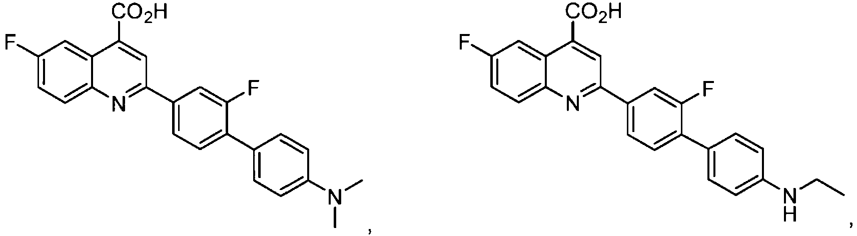 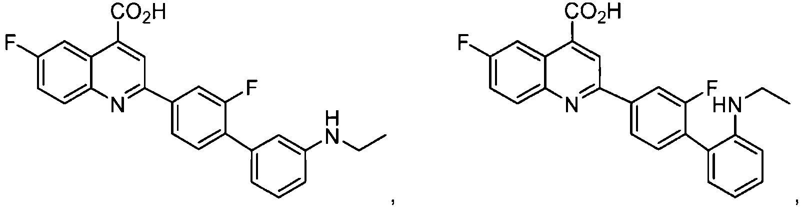 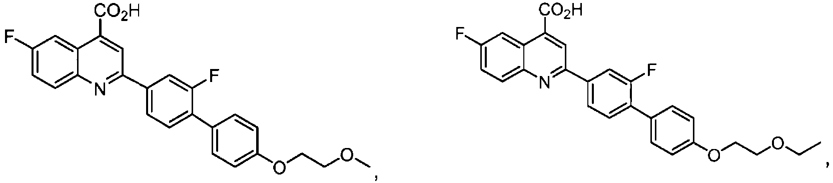 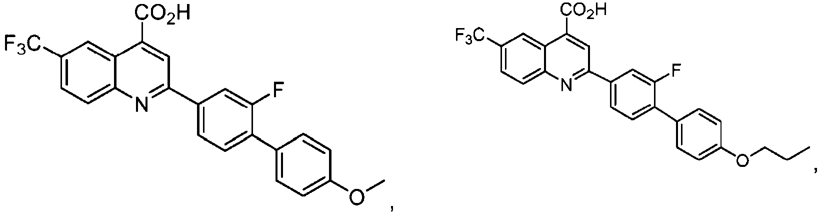 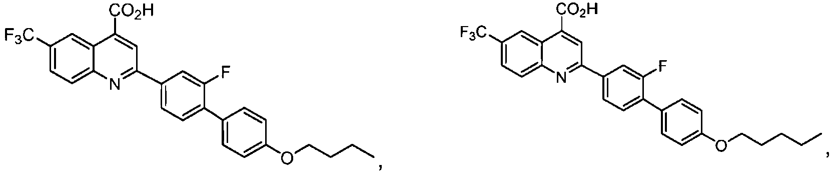 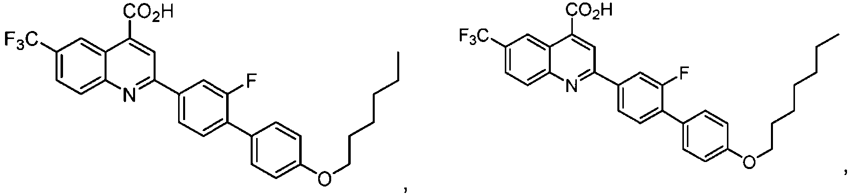 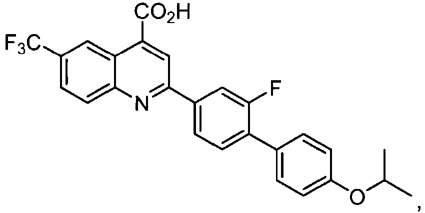 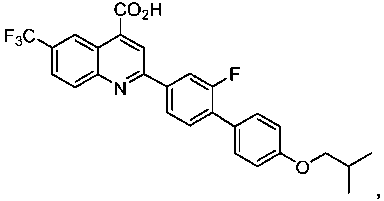 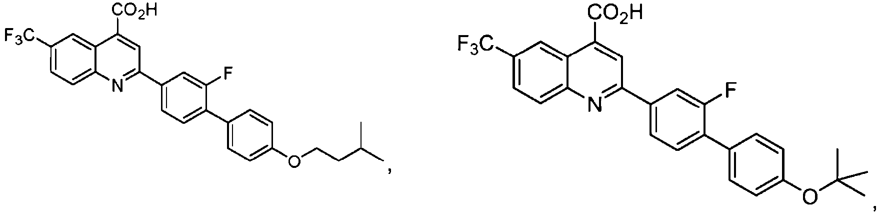 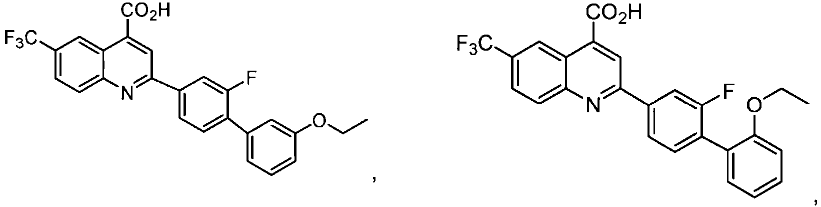 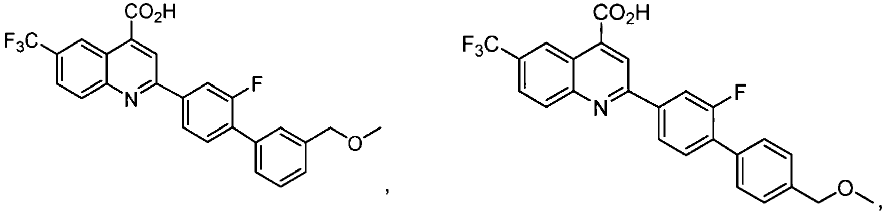 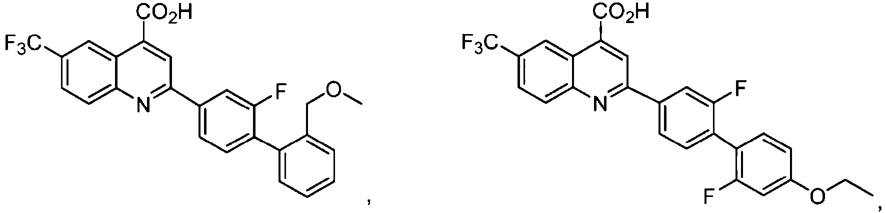 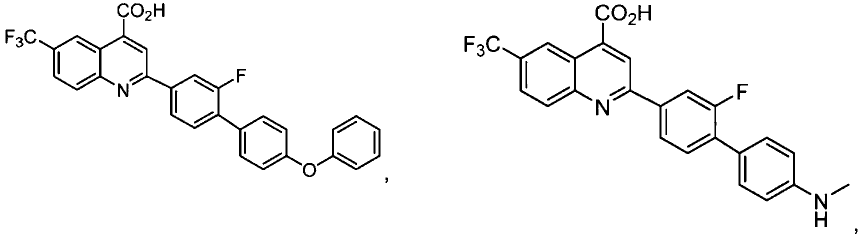 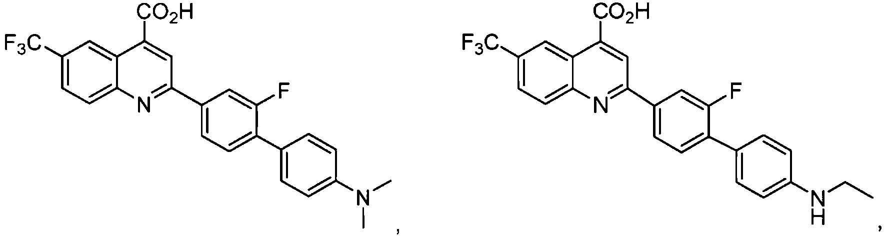 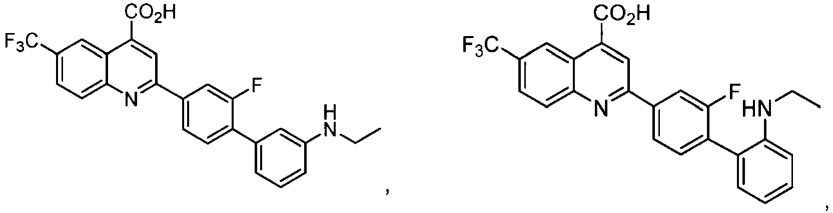 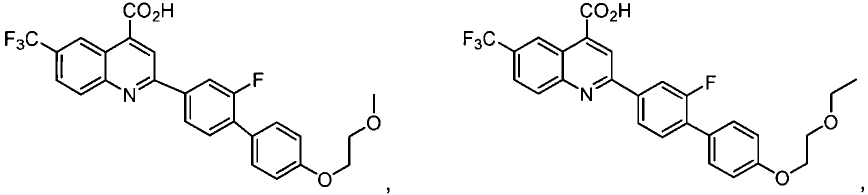 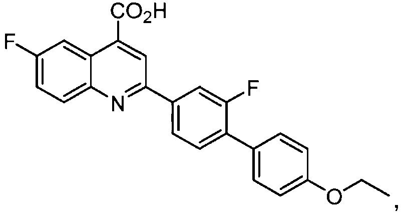 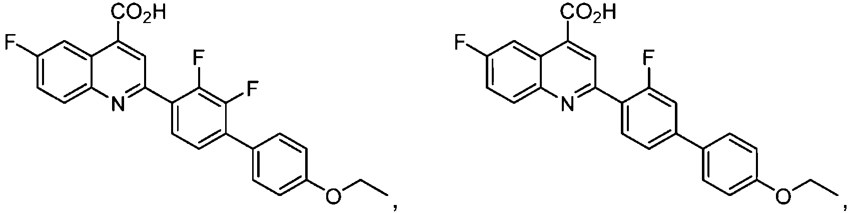 oder eine Kombination davon.

10. Eine pharmazeutische Zusammensetzung, die eine therapeutisch wirksame Menge einer Verbindung nach einem der Ansprüche 1 bis 9 oder eines pharmazeutisch annehmbaren Salzes davon und einen pharmazeutisch annehmbaren Träger umfasst.

11. Eine Verbindung nach einem der Ansprüche 1 bis 9 oder eine Zusammensetzung nach Anspruch 10 zur Verwendung bei der Behandlung von Krebs.

12. Die Verbindung oder Zusammensetzung nach Anspruch 11 zur Verwendung bei der Behandlung von hämatologischem Krebs.

13. Die Verbindung oder Zusammensetzung nach Anspruch 12, wobei der hämatologische Krebs ausgewählt ist aus chronischer myeloischer Leukämie (CML), akuter myeloischer Leukämie (AML), chronischer lymphatischer Leukämie (CLL), akuter lymphatischer Leukämie (ALL), Haarzell-Leukämie, chronischer myelomonozytärer Leukämie (CMML), juveniler myelomonozytärer Leukämie (JMML), großzelliger granulärer Lymphozyten-Leukämie (LGL), akuter lymphozytischer Leukämie, akuter lymphoblastischer Leukämie, B-Zell-Lymphom, T-Zell-Lymphom, Hodgkin-Lymphom, Non-Hodgkin-Lymphom, Haarzell-Lymphom, Burkitt-Lymphom, Hodgkin-Lymphom und Non-Hodgkin-Lymphom.

14. Die Verbindung oder Zusammensetzung nach Anspruch 12, wobei der hämatologische Krebs chronische myeloische Leukämie (CML) oder akute myeloische Leukämie (AML) ist.

## Revendications

1. Composé ayant une formule représentée par une structure : 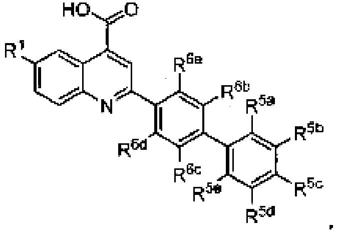
dans lequel R¹ est sélectionné parmi un halogène, - SF₅, -CF₃, et -CF₂CF₃ ;
dans lequel R^{5a} est -R²⁰, et -R²⁰ est sélectionné parmi un halogène, un -C2-C7 alkylamino et un -C2-C7 alcoxy ; et dans lequel chacun parmi R^{5b}, R^{5c}, R^{5d}, et R^{5e} est sélectionné indépendamment parmi un hydrogène, un halogène, -SF₅, -CN, -N₃, -OH, -NH₂, -CF₃, et -CF₂CF₃ ; ou
dans lequel R^{5b} est -R²⁰, et -R²⁰ est sélectionné parmi un halogène, un -C2-C7 alkylamino et un -C2-C7 alcoxy ; et dans lequel chacun parmi R^{5a}, R^{5c}, R^{5d}, et R^{5e} est sélectionné indépendamment parmi l'hydrogène, l'halogène, -SF₅, -CN, -N₃, -OH, -NH₂, -CF₃, et -CF₂CF₃ ; ou
dans lequel R^{5c} est -R²⁰, et -R²⁰ est sélectionné parmi un halogène, un -C2-C7 alkylamino et un -C2-C7 alcoxy ; et dans lequel chacun parmi R^{5a}, R^{5b}, R^{5d}, et R^{5e} est sélectionné indépendamment parmi un hydrogène, un halogène, -SF₅, -CN, -N₃, -OH, -NH₂, -CF₃, et -CF₂CF₃ ; ou
dans lequel chacun parmi R^{6a}, R^{6b}, R^{6c} et R^{6d} est sélectionné indépendamment parmi un hydrogène, un halogène, -SF₅, -CN, -N₃, -OH, -NH₂, un C1-C3 alkyle, un C1-C3 alcoxy, un C1-C3 haloalkyle, un C1-C3 aminoalkyle et un C1-C3 hydroxyalkyle, à condition qu'au moins l'un parmi R^{6a}, R^{6b}, R^{6c}, et R^{6d} ne soit pas un hydrogène ;
ou un sel pharmaceutiquement acceptable de celui-ci.

2. Composé selon la revendication 1, dans lequel R¹ est sélectionné parmi un halogène, -SF₅, -CF₃, et -CF₂CF₃.

3. Composé selon la revendication 2, dans lequel R¹ est -F.

4. Composé selon la revendication 1, dans lequel R¹ est sélectionné parmi -SF₅, -CF₃, et -CF₂CF₃.

5. Composé selon la revendication 1, dans lequel R^{5a} est -R²⁰ ; dans lequel R²⁰ est un halogène ; et dans lequel chacun parmi R^{5b}, R^{5c}, R^{5d}, et R^{5e} est sélectionné indépendamment parmi un hydrogène, un halogène, -SF₅, - CN, -N₃, -OH, -NH₂, -CF₃, et -CF₂CF₃ ; ou
dans lequel R^{5a} est -R²⁰ ; dans lequel -R²⁰ est sélectionné parmi un -C2-C7 alkylamino et un -C2-C7 alcoxy ; et dans lequel chacun parmi R^{5b}, R^{5c}, R^{5d}, et R^{5e} est sélectionné indépendamment parmi un hydrogène, un halogène, -SF₅, -CN, -N₃, -OH, -NH₂, -CF₃, et -CF₂CF₃.

6. Composé selon la revendication 1, dans lequel R^{5b} est -R²⁰ ; dans lequel R²⁰ est un halogène ; et dans lequel chacun parmi R^{5a}, R^{5c}, R^{5d}, et R^{5e} est sélectionné indépendamment parmi un hydrogène, un halogène, -SF₅, - CN, -N₃, -OH, -NH₂, -CF₃, et -CF₂CF₃ ; ou
dans lequel R^{5b} est -R²⁰ ; dans lequel -R²⁰ est sélectionné parmi un -C2-C7 alkylamino et un -C2-C7 alcoxy ; et dans lequel chacun parmi R^{5a}, R^{5c}, R^{5d}, et R^{5e} est sélectionné indépendamment parmi un hydrogène, un halogène, -SF₅, -CN, -N₃, -OH, -NH₂, -CF₃, et -CF₂CF₃.

7. Composé selon la revendication 1, dans lequel R^{5c} est -R²⁰ ; dans lequel R²⁰ est un halogène ; et dans lequel chacun parmi R^{5a}, R^{5b}, R^{5d}, et R^{5e} est sélectionné indépendamment parmi un hydrogène, un halogène, -SF₅, - CN, -N₃, -OH, -NH₂, -CF₃, et -CF₂CF₃ ; ou
dans lequel R^{5c} est -R²⁰ ; dans lequel -R²⁰ est sélectionné parmi un -C2-C7 alkylamino et un -C2-C7 alcoxy ; et dans lequel chacun parmi R^{5a}, R^{5b}, R^{5d}, et R^{5e} est sélectionné indépendamment parmi un hydrogène, un halogène, -SF₅, -CN, -N₃, -OH, -NH₂, -CF₃, et -CF₂CF₃.

8. Composé selon la revendication 1, ayant une structure représentée par une formule : 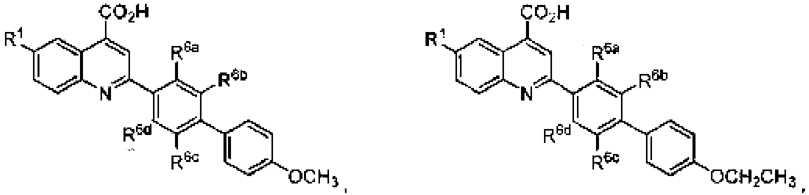 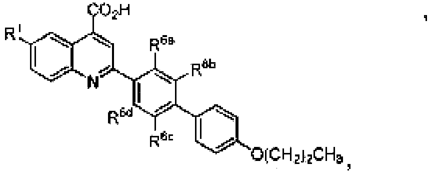 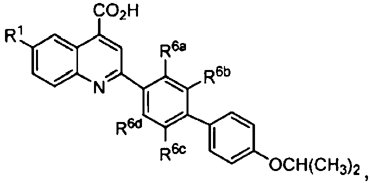 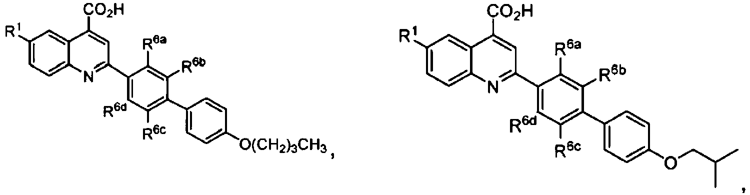 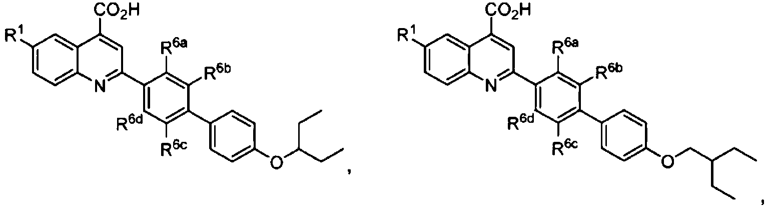 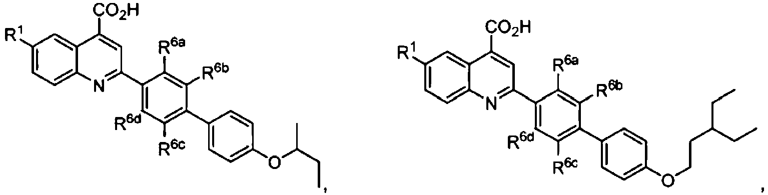 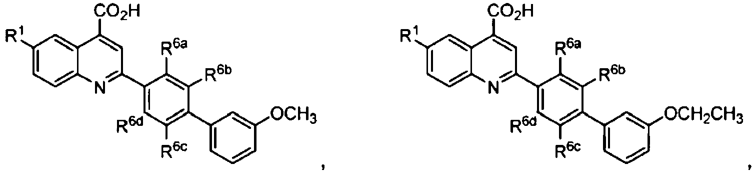 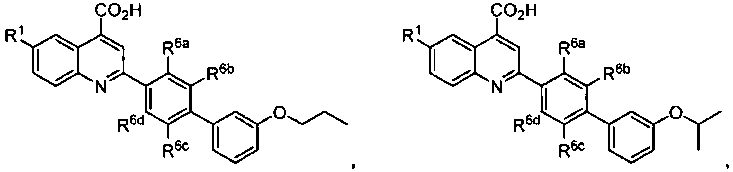 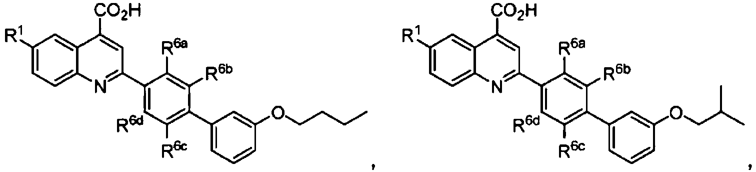 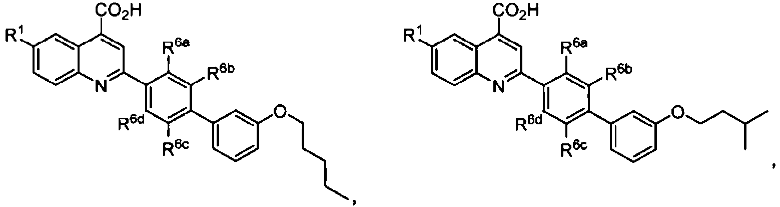 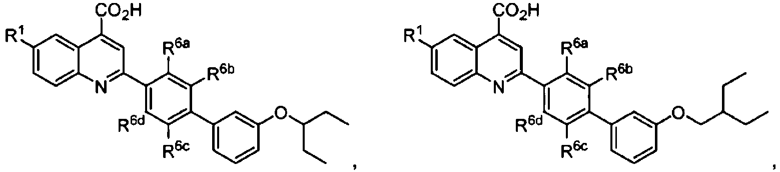 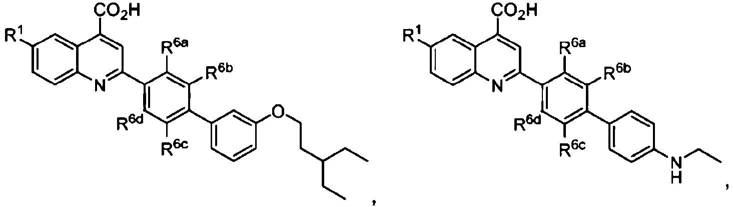 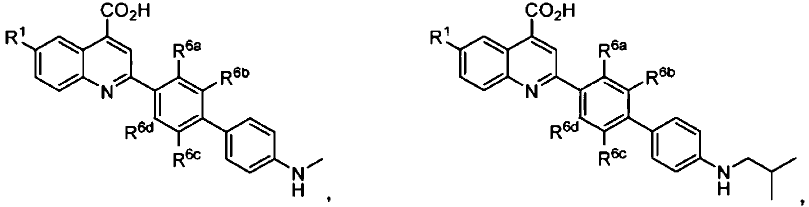 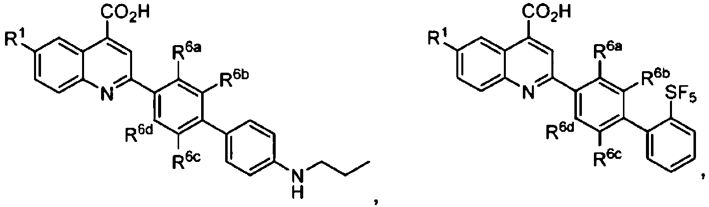 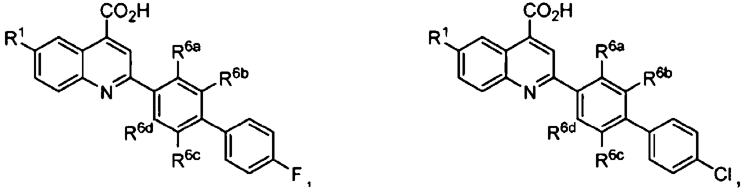 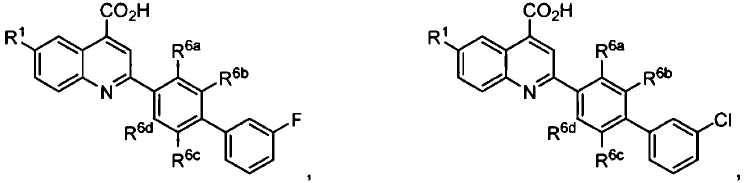 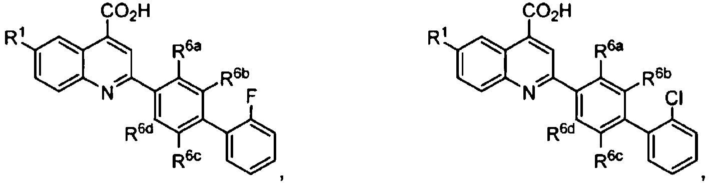 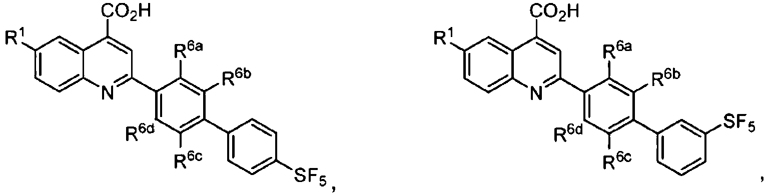 ou des combinaisons de celles-ci.

9. Composé selon la revendication 1, ayant une structure représentée par une formule : 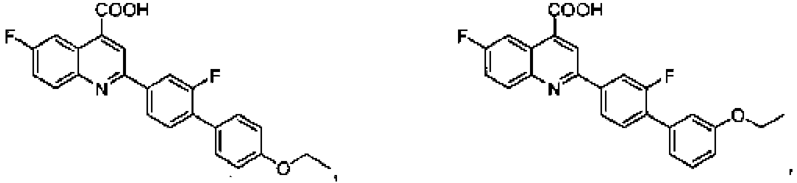 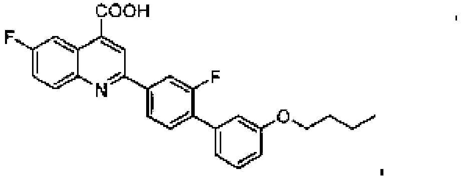 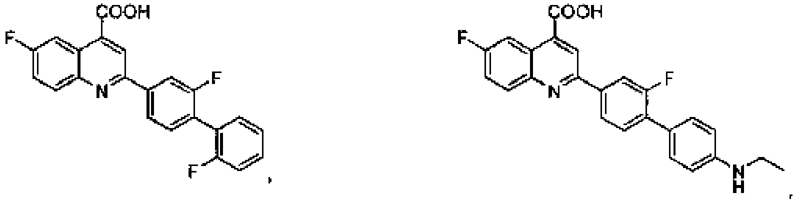 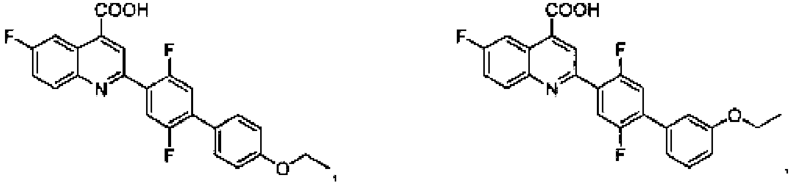 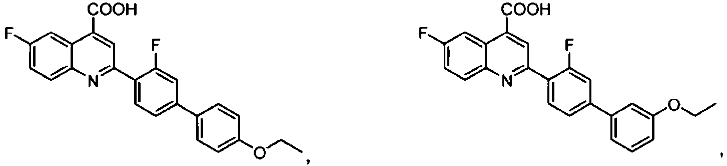 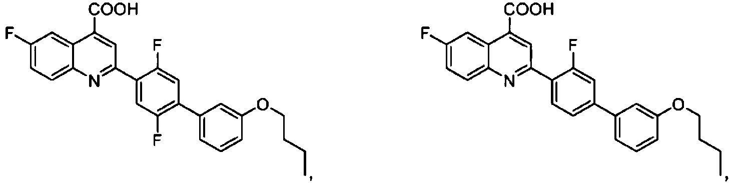 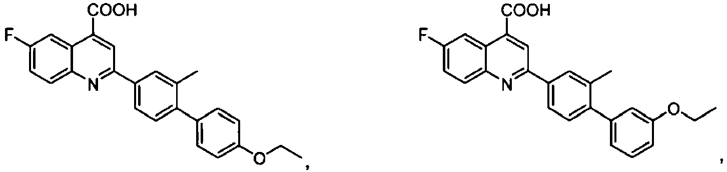 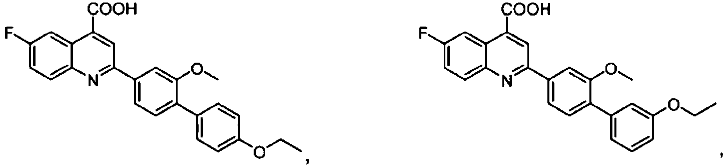 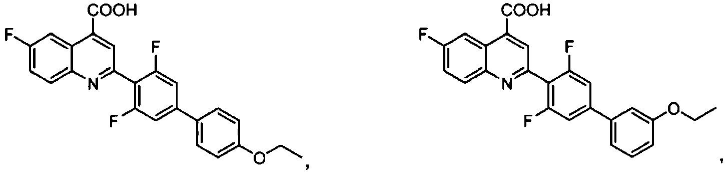 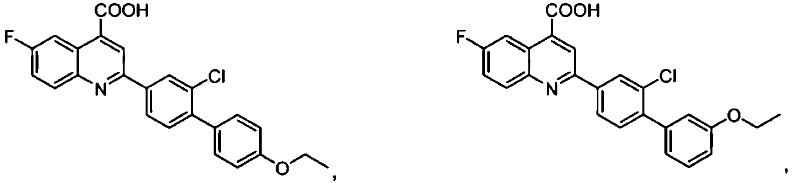 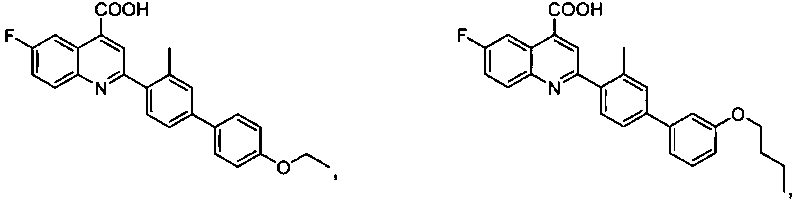 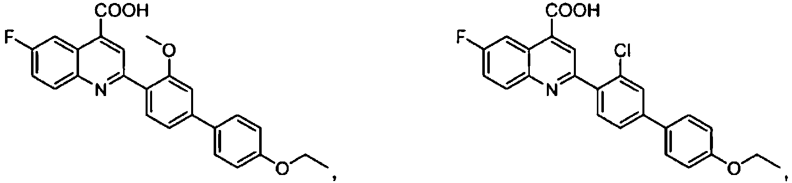 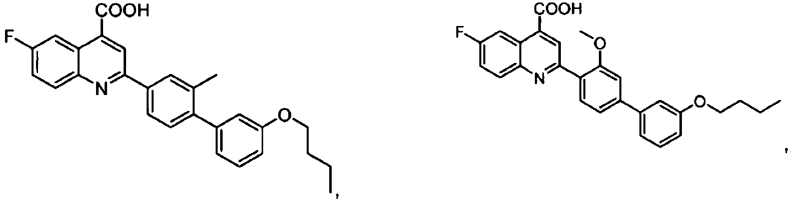 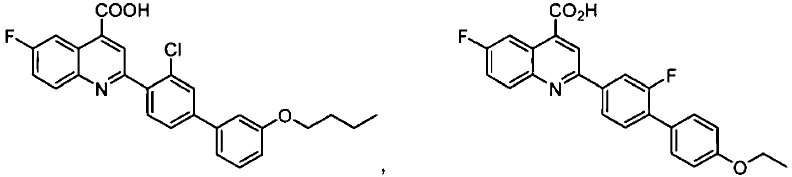 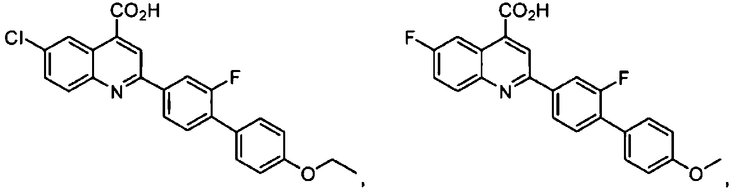 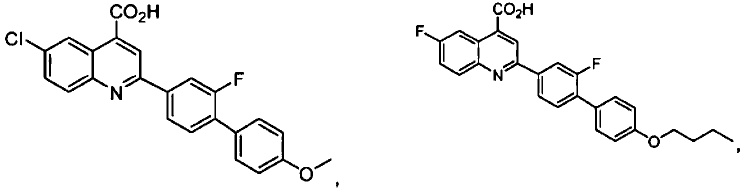 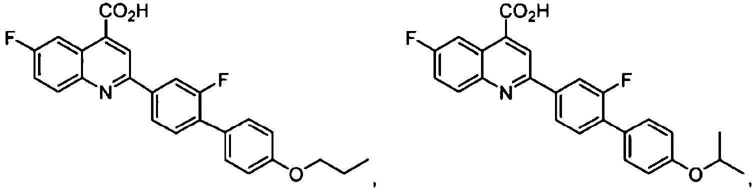 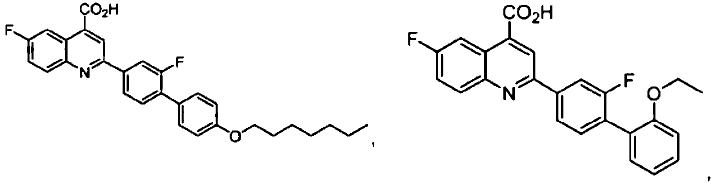 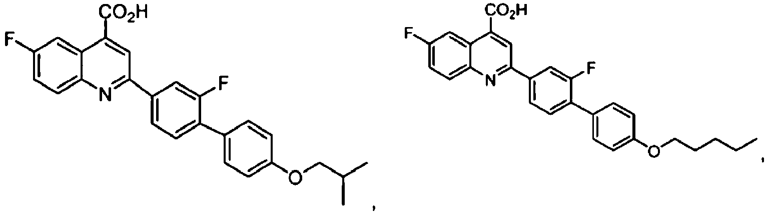 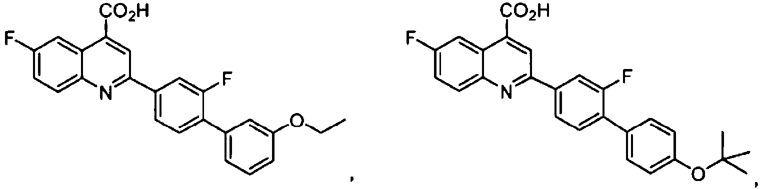 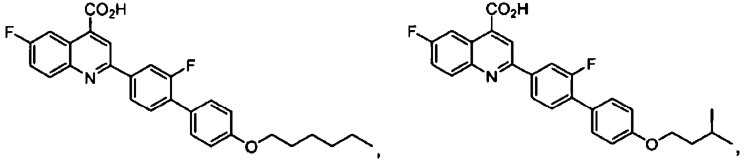 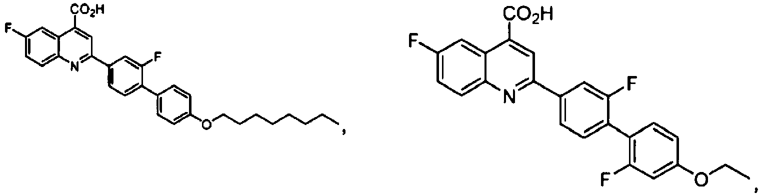 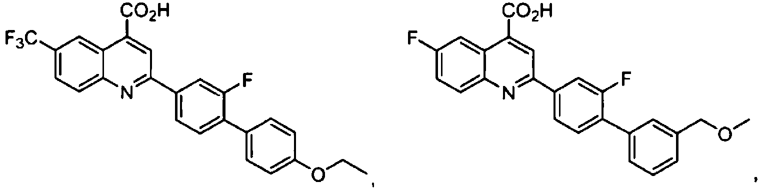 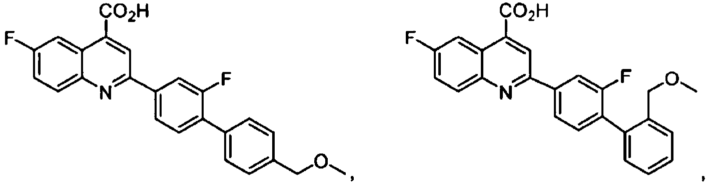 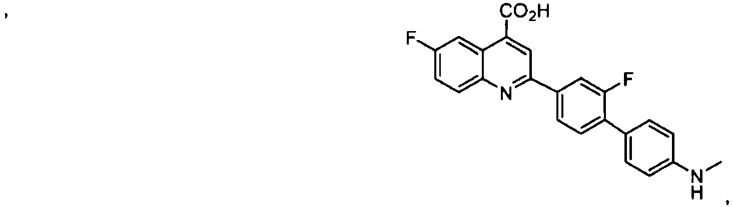 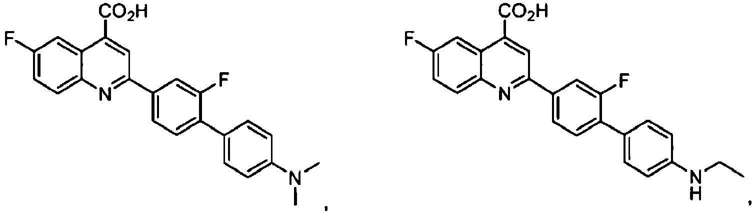 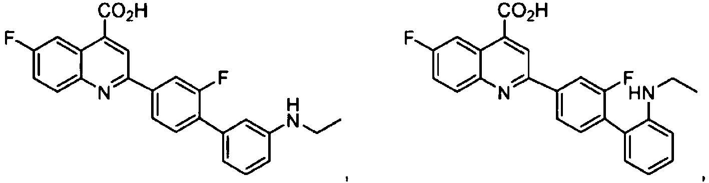 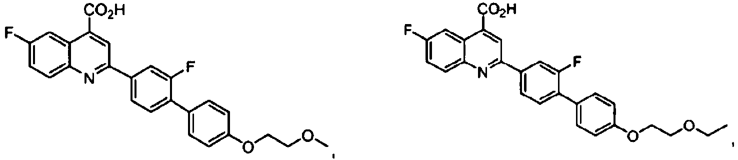 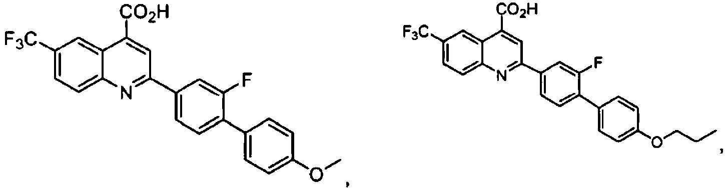 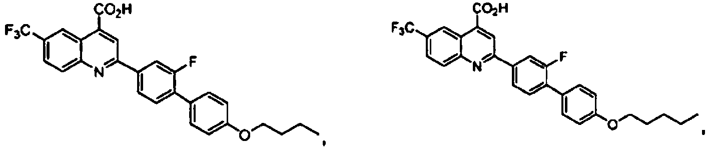 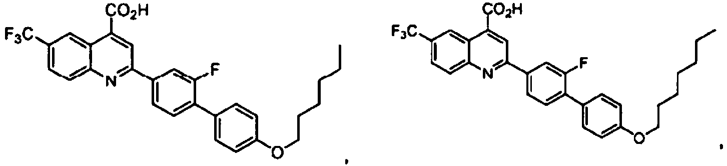 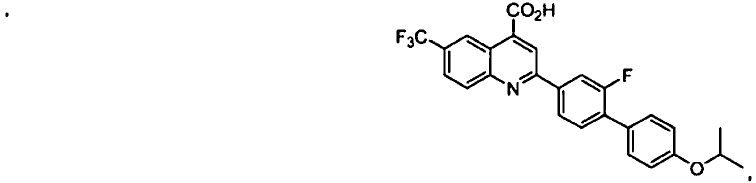 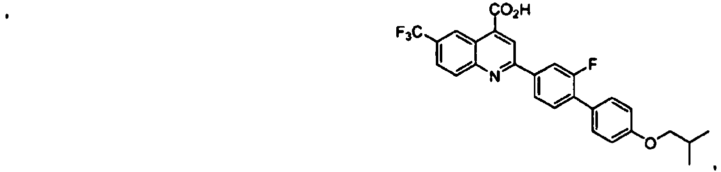 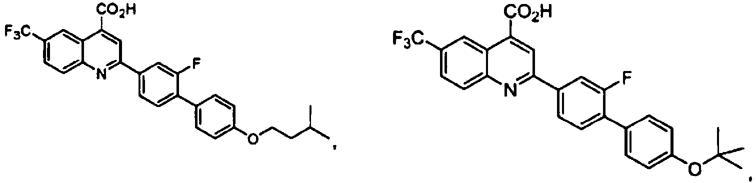 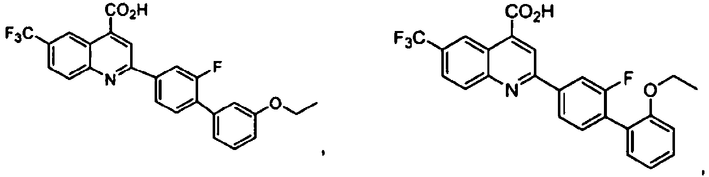 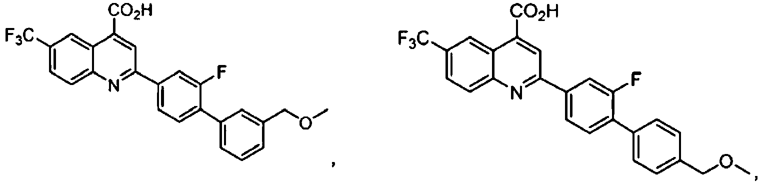 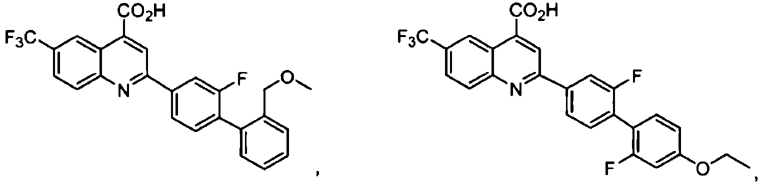 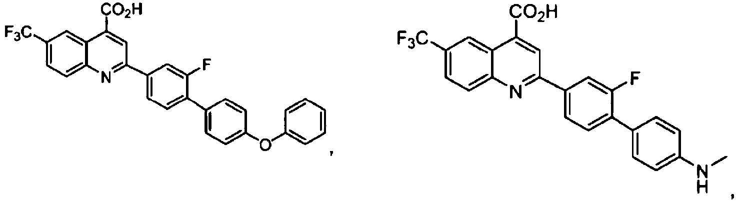 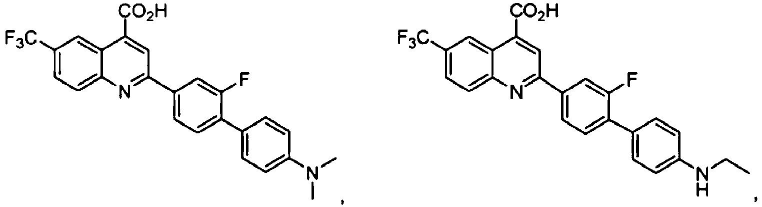 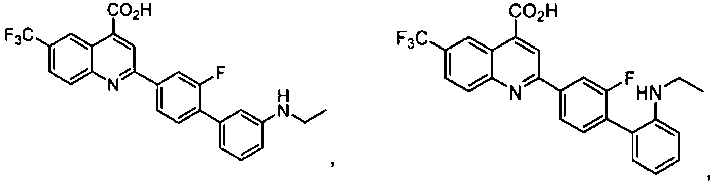 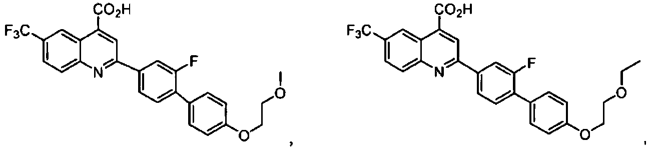 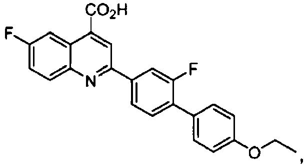 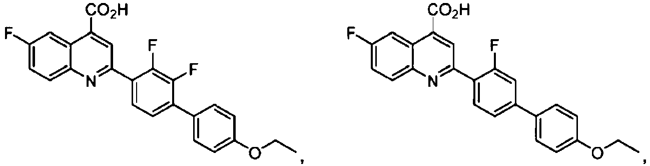 ou une combinaison de celles-ci.

10. Composition pharmaceutique comprenant une quantité thérapeutiquement efficace d'un composé selon l'une quelconque des revendications 1 à 9, ou un sel pharmaceutiquement acceptable de celui-ci, et un support pharmaceutiquement acceptable.

11. Composé selon l'une quelconque des revendications 1 à 9, ou composition selon la revendication 10, pour une utilisation dans le traitement d'un cancer.

12. Composé ou composition selon la revendication 11 pour une utilisation dans le traitement d'un cancer hématologique.

13. Composé ou composition selon la revendication 12, dans lequel ou laquelle le cancer hématologique est sélectionné parmi la leucémie myéloïde chronique (LMC), la leucémie myéloïde aiguë (LMA), la leucémie lymphoïde chronique (LLC), la leucémie lymphoïde aiguë (LLA), la leucémie à tricholeucocytes, la leucémie myélomonocytaire chronique (LMMC), la leucémie myélomonocytaire juvénile (LMMJ), la leucémie lymphoïde à grands granules (LGL), la leucémie lymphoïde aiguë, la leucémie lymphoblastique aiguë, le lymphome à cellules B, le lymphome à cellules T, le lymphome de Hodgkin, un lymphome non hodgkinien, le lymphome à tricholeucocytes, le lymphome de Burkett, le lymphome de Hodgkin et un lymphome non hodgkinien.

14. Composé ou composition selon la revendication 12, dans lequel ou laquelle le cancer hématologique est une leucémie myéloïde chronique (LMC) ou une leucémie myéloïde aiguë (LMA).
